(19) Europäisches Patentamt
European Patent Office
Office européen des brevets

(11) EP 1 502 603 A1

(12) **EUROPEAN PATENT APPLICATION**
published in accordance with Art. 158(3) EPC

(43) Date of publication:
**02.02.2005 Bulletin 2005/05**

(21) Application number: **03723099.2**

(22) Date of filing: **09.04.2003**

(51) Int Cl.[7]: **A61K 39/395**, A61P 9/00, A61P 29/00, A61P 31/04, A61P 31/12, A61P 31/14, A61P 35/00, A61P 37/02, A61P 37/04, A61P 37/08, A61P 43/00, G01N 33/15, G01N 33/50
// (C12P21/08, C12N15:09, 5:10, C07K16:18, C12P21:08), C12R1:91, C12N5:10, C12R1:91

(86) International application number:
**PCT/JP2003/004505**

(87) International publication number:
**WO 2003/084570 (16.10.2003 Gazette 2003/42)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.04.2002 JP 2002106951**

(71) Applicant: **KYOWA HAKKO KOGYO CO., LTD.**
**Chiyoda-ku, Tokyo 100-8185 (JP)**

(72) Inventors:
- **NAKAMURA, Kazuyasu**
**North Bethesda, MD 20852 (US)**
- **SHITARA, Kenya,**
**c/o Kyowa Hakko Kogyo Co.,Ltd.**
**Machida-shi, Tokyo 194-8533 (JP)**
- **HATANAKA, Shigeki,**
**c/o Kyowa Hakko Kogyo Co.,Ltd.**
**Tokyo 100-8185 (JP)**
- **NIWA, Rinpei, c/o Kyowa Hakko Kogyo Cp.,Ltd.**
**Machida-shi, Tokyo 194-8533 (JP)**
- **OKAZAKI, Akira,**
**c/o Kyowa Hakko Kogyo Co.,Ltd.**
**Machida-shi, Tokyo 194-8533 (JP)**

(74) Representative: **VOSSIUS & PARTNER**
**Siebertstrasse 4**
**81675 München (DE)**

(54) **DRUG CONTAINING ANTIBODY COMPOSITION APPROPRIATE FOR PATIENT SUFFERING FROM Fc gamma RIIIa POLYMORPHISM**

(57) A medicament for treating FcγRIIIa polymorphism patients who cannot be treated by a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain, which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain.

EP 1 502 603 A1

## Description

TECHNICAL FIELD

The present invention relates to a medicament for treating FcγRIIIa polymorphism patients, which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain.

BACKGROUND ART

Since antibodies have high binding activity, high binding specificity and high stability in blood, their applications to diagnosis, prevention and treatment of various human diseases have been attempted [*Monoclonal Antibodies: Principles and Applications*, Wiley-Liss, Inc., Chapter 2.1 (1995)]. Also, humanized antibodies such as human chimeric antibodies and human complementarity determining region (hereinafter referred to as "CDR")-grafted antibodies have been prepared from non-human animal antibodies by using genetic recombination techniques [*Nature*, 312, 643 (1984); *Proc. Natl. Acad Sci. USA*, 81, 6851 (1984); *Nature*, 321, 522 (1986); *Nature*, 332, 323 (1988)]. The human chimeric antibody is an antibody in which its antibody variable region (hereinafter referred to as "V region") is derived from a non-human animal antibody and its constant region (hereinafter referred to as "C region") is derived from a human antibody. The human CDR-grafted antibody is an antibody in which the CDR of a human antibody is replaced by CDR of a non-human animal antibody.

According to the development of humanized antibodies, problems such as high immunogenicity, low effector function and short blood half-life of non-human animal antibodies such as mouse antibodies were solved so that monoclonal antibodies could be applied as medicaments [*Immunol. Today*, 21, 364 (2000); *Immunol. Today*, 21, 403 (2000); *Ann. Allergy Asthma Immunol.*, 81, 105 (1998); *Nature Biotechnol.*, 16, 1015 (1998)]. In the United States, for example, five humanized antibodies have already been approved and there are on the market as antibodies for cancer treatment [*Nature Reviews Cancer*, 1, 119 (2001)].

These humanized antibodies actually show their effects to a certain degree in the clinical field, but therapeutic antibodies having higher efficacy are also in demand. For example, it has been reported that single use of an anti-CD20 human chimeric antibody, Rituxan (manufactured by IDEC) showed its efficacy of merely 48% (complete remission 6%, partial remission 42%) in its phase III clinical test on recurrent low malignancy non-Hodgkin lymphoma patients, and its average effect-keeping period was 12 months [*J. Clin. Oncol.*, 16, 2825 (1998)]. Although it has been reported that combination therapy of Rituxan and chemotherapy (CHOP: Cyclophosphamide, Doxorubicin, Vincristine) showed an efficacy of 95% (complete remission 55%, partial remission 45%) in the phase II clinical test on recurrent low malignancy and follicular non-Hodgkin lymphoma patients, side effects caused by CHOP were observed [*J. Clin. Oncol.*, 17, 268 (1999)]. It has been reported that single use of an anti-HER2 human CDR-grafted antibody, Herceptin (manufactured by Genentech) showed its efficacy of merely 15% in its phase III clinical test on metastatic breast cancer patients, and its average effect-keeping period was 9.1 months [*J. Clin. Oncol.*, 17, 2639 (1999)].

Various methods to reinforce therapeutic effects of therapeutic antibodies using such antigen-expressing cells as the direct target have been examined.

One of them is a method in which a radioisotope or a toxin is linked to an antibody and a target cell is directly injured [*Blood,* 96, 2934 (2000); *J. Clin. Oncol.,* 17, 3793 (1999)]. An anti-CD33 antibody, Mylotarg which is linked to calicheamicin (manufactured by Wyeth Labs) has already been approved and it is on the market in the United States. Also, anti-CD20 antibodies Zevalin (manufactured by IDEC), Bexxar (manufactured by Corixa) and the like which are linked to a radioisotope have been developed.

Also, a method for indirectly injuring target cells using a bi-specific antibody which is an antibody having two kinds of antigen binding specificity has been examined. For example, an antibody having one specificity for a target cell and the other for an effector cell, a radioisotope or a toxin has been produced [*Cyrr. Opin. Immunol.*, 11, 558 (1999); *J. Immunother.*, 22, 514 (1999); *Immunol. Today,* 21, 391 (2000)].

In addition, a method in which an antibody and an enzyme are linked, the antibody is specifically accumulated on the target cell, and then the target cell is specifically injured by administering an agent which is activated by the enzyme (ADEPT: antibody-dependent enzyme-mediated prodrug therapy) has also been examined [*Anticancer Res.,* 19, 605 (1999), *Cancer Res.,* 54, 2151 (1994)].

Although effects of these methods are currently inspected by various clinical tests, they have problems such as side effects by a radioisotope and a toxin [*Clin. Cancer Res.*, 2, 457 (1996), *J. Clin. Oncol.*, 17, 478 (1999)], producing method and cost in the bispecific antibody, and antigenicity of the enzyme to be used in ADEPT [*Cell Biophys.,* 21, 109 (1992)] and the like.

Antibodies of human antibody IgG1 and IgG3 subclasses have effector functions such as antibody-dependent

cell-mediated cytotoxic activity (hereinafter referred to as "ADCC activity") and complement-dependent cytotoxic activity (hereinafter referred to as "CDC activity") [*Chemical Immunology*, 65, 88 (1997), *Immunol. Today*, 20, 576 (1999)]. The above Rituxan is a human chimeric antibody of IgG1 subclass, and as the activity mechanism of its antitumor effect, importance of the induction of apoptosis by crosslinking of CD20 by the antibody has been suggested in addition to effector functions such as ADCC activity and CDC activity [*Cyrr. Opin. Immunol.*, 11, 541 (1999)]. Herceptin is also a human CDR-grafted antibody of IgG1 subclass, and importance of its ADCC activity as a cytotoxic activity has been reported *by in vitro* tests [*Cancer Immunol. Immunother.*, 37, 255 (1993)]. These facts suggest a possibility that therapeutic effects of antibodies can be improved by reinforcing effector functions, particularly ADCC activity.

ADCC activity is exerted via mutual functions of the Fc region of an IgG class antibody linked to an antigen on a target cell and the Fc receptor present on effector cells such as neutrophil, macrophage and NK cell (hereinafter referred to as "FcγR") [*Annu. Rev. Immunol.*, 18, 709 (2000); *Annu. Rev. Immunol.*, 19, 275 (2001)].

It has been found that FcγR is classified into three different classes called FcγRI (CD64), FcγRII (CD32) and FcγRIII (CD16). In human, FcγRII is further classified into FcγRIIa and FcγRIIb, and FcγRIII is further classified into FcγRIIIa and FcγRIIIb. FcγR is a membrane protein belonging to the immunoglobulin super family. FcγRII and FcγRIII comprise an α chain having an extracellular region of two immunoglobulin-like domains, and FcγRI comprises an α chain having extracellular region of three immunoglobulin-like domains, as a constituting component, and the α chain relates to the IgG binding activity. Furthermore, FcγRI and FcγRIII comprise a γ chain or ζ chain as a constituting component which has a signal transduction function by associating with the α chain [*Annu. Rev. Immunol.*, 18, 709 (2000); *Annu. Rev. Immunol.*, 19, 275 (2001)].

FcγR is classified into an activation receptor and an inhibitory receptor based on its functions [*Annu. Rev. Immunol.*, 19, 275 (2001)].

In the activating receptor, a sequence consisting of 19 amino acid residues, called immunoreceptor tyrosine-based activation motif (hereinafter referred to as "ITAM"), is present in the intracellular region of the α chain or associating γ chain or ζ chain. Tyrosine kinases such as Src and Syk, which mutually react with ITAM are activated by binding of an IgG immune complex to thereby induce various activation reactions.

In the inhibitory receptor, a sequence consisting of 13 amino acid residues, called immunoreceptor tyrosine-based inhibitory motif (hereinafter referred to as "ITIM"), is present in the intracellular region of the α chain. ITIM is phosphorylated via its association with the activating receptor, and various reactions including activation of a phosphatase called SHIP are induced to inhibit activation signal from the activation receptor.

In human, FcγRI, FcγRIIa and FcγRIIIa have a function as activating receptors. In FcγRI, an ITAM sequence is present in the intracellular region of the associated γ chain. FcγRI is expressed on macrophages, monocytes, dendritic cells, neutrophils, eosinophils and the like. FcγRIIa comprises a single α chain, and an ITAM-like sequence is present in the intracellular region. FcγRIIa is expressed on macrophages, mast cells, monocytes, dendritic cells, Langerhans cells, neutrophils, eosinophils, platelets and a part of B cells. In FcγRIIIa, an ITAM sequence is present in the intracellular region of the associated γ chain or ζ chain. FcγRIIIa is expressed on NK cells, macrophages, monocytes, mast cells, dendritic cells, Langerhans cells, eosinophil and the like, but is not expressed on neutrophils, B cells and T cells.

On the other hand, FcγRIIb comprises a single α chain, and the amino acid sequence of the extracellular region has homology of about 90% with the FcγRIIa, but since an ITMI sequence is present in the intracellular region, it functions as an inhibitory receptor. FcγRIIb is expressed on B cells, macrophages, mast cells, monocytes, dendritic cells, Langerhans cells, basophils, neutrophils and eosinophils, but is not expressed in NK cells and T cells. FcγRIIIb comprises a single α chain, and the amino acid sequence of the extracellular region has homology of about 95% with the FcγRIIIa, but is expressed specifically in neutrophils as a glycosylphosphatidylinositol (hereinafter to be referred to as "GPI") binding type membrane protein. The FcγRIIIb binds with an IgG immune complex but cannot activate cells by itself, and it is considered to function via its association with a receptor having an ITAM sequence such as FcγRIIa.

Based on tests using mice, it has been found that FcγR plays an important role in the antitumor activity of antibodies such as Rituxan, Herceptin and the like. That is, the antitumor effect of the antibodies increased in an inhibitory receptor FcγRIIb deficient mouse, whereas the antitumor effect of the antibodies decreased in an activating receptor FcγRI and RcγRIII deficient mouse [*Nature Medicine,* 6, 443 (2000)]. In addition, *in vitro* ADCC activity was hardly detected by an antibody whose binding activity to FcγR was reduced by mutating an amino acid mutation in the Fc region, and its antitumor effect in mice was significantly reduced [*Nature Medicine,* 6, 443 (2000)]. The above results shows a possibility to improve an antitumor effect of an antibody mainly via its ADCC activity, by increasing the activity of the antibody to bind to an activating receptor or by decreasing the activity of the antibody to bind to an inhibitory receptor.

Actually, Shields, R.L. *et al* have reported that the binding activity to an activating receptor FcγRIIIa was increased by mutating an amino acid in the Fc region of an antibody of human IgG1 subclass, and as the result, *in vitro* ADCC activity was increased about 2 times [*J. Biol. Chem.,* 276, 6591 (2001)]. However, increase in its *in vivo* antitumor effect has not been reported.

Furthermore, the ADCC activity of antibodies is also reinforced by artificially modifying a sugar chain binding

to the Fc region. It has been reported that the ADCC activity was increased when a bisecting sugar chain binding to the Fc region of the antibody was increased by introducing a β1,4-*N*-acetylglucosamine transferase III gene into CHO cell [*Nature Biotechnol*., 17, 176 (1999)]. In this case, however, detailed mechanism on the increase of the ADCC activities including the activity to bind to the FcγR has not been clarified.

Recently, it has been reported that the therapeutic effect of Rituxan in clinical tests is influenced by the polymorphism of FcγRIIIa in patients [*Blood*, 1, 754 (2002)]. Human FcγRIIIa has a polymorphism in which an amino acid residue at position 158 is Phe (hereinafter referred to as "FcγRIIIa(F)") and Val (hereinafter referred to as "FcγRIIIa (V)"). It is known that the antibody of human IgG1 subclass shows higher binding activity by a Val/Val homo type NK cell and induces much higher ADCC activity than Phe/Phe homo type FcγRIIIa and Phe/Val hetero type FcγRIIIa which are expressed on NK cell (hereinafter human having the Phe/Phe homo type or Phe/Val hetero type is referred to as "Phe carrier") [*Blood,* 90, 1109 (1997), *J. Clin. Invest*., 100, 1059 (1997), *J. Biol. Chem.,* 276, 6591 (2001)]. It has been shown that the efficacy of Rituxan one year after treatment of follicular non-Hodgkin lymphoma patients is 90% in the Val homo type, which is significantly higher than 51% of Phe carrier [*Blood,* 1, 754 (2002)].

It has been reported that the ratio of Phe carrier and Val homo type in FcγRIIIa is almost constant among various races, the Phe carrier is 80 to 90% and the Val homo type is 10 to 20% [*Blood,* 90, 1109 (1997), *J. Immunol. Methods*, 242, 127 (2000), *Blood*, 94, 4220 (1999)]. Accordingly, there are many reports relating to the binding activity of the antibody of human IgG1 subclass and FcγRIII, and although there are differences by the measuring methods, the binding constant (hereinafter referred to as "KA") has been reported to be from $10^5$ to $10^7$ $M^{-1}$ [*Biochem*., 34, 13320 (1995), *Adv. Immunol*., 57, 1 (1994), *Eur. J. Immunol*., 27, 1928 (1997), *J. Exp. Med*., 183, 2227 (1996), *Ann. Hematol*., 76, 231 (1998), *Ann. Rev. Immunol*., 9, 457 (1991)]. The antibody prepared by mutating the amino acid of the Fc region of human IgG1 subclass antibody as described above shows 1.1-fold and 2.17-fold higher binding activities for FcγRIIIa (V) and FcγRIIIa(F), respectively, at the maximum by ELISA compared to natural type human IgG1 (all produced by a human embryonic kidney cell strain 293 cell) [*J. Biol. Chem*., 276, 6591 (2001)].

DISCLOSURE OF THE INVENTION

The present invention relates to the following (1) to (28):

(1) A medicament for treating a patient who exerts such an affinity of a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain with a human Fcγ receptor IIIa that it is not enough for the antibody composition to exert sufficient therapeutic effect, which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain.
(2) The medicament according to (1), wherein the affinity that is not enough to exert sufficient therapeutic effect is an affinity that is not enough for the antibody composition to exert a sufficient antibody-dependent cell-mediated cytotoxic activity.
(3) The medicament according to (1) or (2), wherein the affinity that it is not enough to exert sufficient therapeutic effect is lower than at least one affinity selected from the group consisting of (a) and (b):

(a) a binding constant to the human Fcγ receptor IIIa at 25°C being $1\times10^7$ $M^{-1}$ when measured by a biosensor method according to BIAcore;
(b) a binding constant to the human Fcγ receptor IIIa at 25°C being $2\times10^6$ $M^{-1}$ when measured with an isothermal titration-type calorimeter.

(4) The medicament according to any one of (1) to (3), wherein the human Fcγ receptor IIIa is a human Fcγ receptor IIIa in which an amino acid residue at position 176 from the N-terminal methionine in the signal sequence is phenylalanine.
(5) The medicament according to any one of (1) to (4), wherein the patient is a patient having a human Fcγ receptor IIIa in which an amino acid residue at position 176 from the N-terminal methionine in the signal sequence is phenylalanine.
(6) The medicament according to any one of (1) to (5), wherein the patient is a patient having only human Fcγ receptor IIIa in which an amino acid residue at position 176 from the N-terminal methionine in the signal sequence is phenylalanine.
(7) The medicament according to any one of (1) to (6), wherein the cell resistant to the lectin is a cell, in which the activity of a protein is decreased or deleted, selected from the group consisting of the following (a), (b) and (c):

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose to the Golgi body.

(8) The medicament according to any one of (1) to (7), wherein the lection is selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

(9) The medicament according to any one of (1) to (8), wherein the cell is selected from the group consisting of a yeast, an animal cell, an insect cell and a plant cell.
(10) The medicament according to any one of (1) to (9), wherein the cell is selected from the group consisting of the following (a) to (j):

(a) a CHO cell derived from a Chinese hamster ovary tissue;
(b) a rat myeloma cell line YB2/3HL.P2.G11.16Ag.20 line;
(c) a mouse myeloma cell line NS0 cell;
(d) a mouse myeloma cell line SP2/0-Ag14 cell;
(e) a BHK cell derived from a Syrian hamster kidney tissue;
(f) a hybridoma cell producing an antibody;
(g) a human leukemic cell line Namalwa cell;
(h) an embryonic stem cell;
(i) a fertilized egg cell;
(j) a plant cell.

(11) The medicament according to any one of (1) to (10), wherein the antibody composition which comprises as an active ingredient an antibody molecule selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

(12) The medicament according to (11), wherein the antibody molecule belongs to an IgG class.
(13) The medicament according to any one of (1) to (12), wherein the antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain is an antibody composition having a higher antibody-dependent cell-mediated cytotoxic activity than the antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain.
(14) The medicament according to (13), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than the antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain.
(15) The medicament according to (14), wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.
(16) The medicament according to any one of (13) to (15), wherein the antibody composition having a higher antibody-dependent cell-mediated cytotoxic activity is an antibody composition having a ratio of a sugar chain in which fucose is not bound to *N*-acetylglucosamine in the reducing end in the sugar chain of 20% or more of total complex *N*-glycoside-linked sugar chains bound to the Fc region in the antibody composition.

(17) The medicament according to (16), wherein the antibody composition is an antibody composition produced by a CHO cell.
(18) The medicament according to any one of (1) to (17), which is a diagnostic agent, an preventing agent or a therapeutic agent for tumor-accompanied diseases, allergy-accompanied diseases, inflammatory-accompanied diseases, autoimmune diseases, cardiovascular diseases, viral infection-accompanied diseases or bacterial infection-accompanied diseases.
(19) Use of an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain for the manufacture of the medicament according to any one of (1) to (18).
(20) A method for screening a patient to which the medicament according to any one of (1) to (18) is effective, which comprises:

(i) contacting a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain or the medicament according to any one of (1) to (18), with an effector cell obtained from a patient;
(ii) measuring the amount of each of the medicament bound to the effector cell;
(iii) comparing the measured amounts;
(iv) selecting a patient in which the amount of the medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain which has been added to the effector cell is lower.

(21) The method according to (20), wherein the method for measuring the amount of the medicament bound to the effector cell is an immunological measuring method.
(22) A method for screening a patient to which the medicament according to any one of (1) to (18) is effective, which comprises

(i) contacting a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain or the medicament according to any one of (1) to (18), with an effector cell obtained from a patient;
(ii) measuring the activity caused by the contact of each of the medicaments with the effector cell;
(iii) comparing the measured activities;
(iv) selecting a patient in which the activity of the medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain is lower.

(23) The method according to (22), wherein the method for measuring the activity caused by the contact of the medicament reacted with the effector cell is a method selected from the group consisting of (a) to (e):

(a) a method for measuring an antibody-dependent cell-mediated cytotoxic activity;
(b) a method for measuring a complement-dependent cytotoxic activity;
(c) a method for measuring expression of a cytotoxic molecule;
(d) a method for measuring an intracellular signal transduction of a human Fcγ receptor IIIa;
(e) a method for measuring a molecule of which expression is varied by stimulating a human Fcγ receptor IIIa.

(24) The method according to any one of (20) to (23), wherein the effector cell is a cell which expresses a human Fcγ receptor IIIa.
(25) The method according to any one of (20) to (24), wherein the screening method is a method for screening a patient having a human Fcγ receptor IIIa in which an amino acid residue at position 176 from the N-terminal methionine in the signal sequence is phenylalanine.
(26) A medicament which comprises as an active ingredient an antibody composition produced by a cell resistant to a lectin which recognizes a sugar chain in which 1-position of fucose is bound to 6-position of N-acetylglucosamine in the reducing end through α-bond in a complex N-glycoside-linked sugar chain and is administered to a patient having a human Fcγ receptor IIIa in which an amino acid residue at position 176 from the N-terminal methionine in the signal sequence is phenylalanine who is screened by the method according to any one of (20)

to (25).

(27) The medicament according to any one of (1) to (18), which is administered to a patient having a human Fcγ receptor IIIa in which an amino acid residue at position 176 from the N-terminal methionine in the signal sequence is phenylalanine who is screened by the method according to any one of (20) to (26).

(28) Use of an antibody composition produced by a cell resistant to a lectin which recognizes a sugar chain in which 1-position of fucose is bound to 6-position of N-acetylglucosamine in the reducing end through α-bond in a complex N-glycoside-linked sugar chain for producing the medicament according to (26) or (27).

As cell resistant to a lectin which recognizes a sugar chain in which 1-position of fucose is bound to 6-position of *N*-acetylglucosamine in the reducing end through α-bond in a complex *N*-glycoside-linked sugar chain. (hereinafter referred to as "α1,6-fucose/lectin-resistant cell") used in the medicament of the present invention, any cell may be used, so long as it is a cell such as yeast, an animal cell, an insect cell or a plant cell which can be used for producing an antibody composition and is a cell resistant to a lectin which recognizes a sugar chain in which 1-position of fucose is bound to 6-position of *N*-acetylglucosamine in the reducing end through α-bond in a complex *N*-glycoside-linked sugar chain.

Examples include a hybridoma cell, a host cell for producing a human antibody or a humanized antibody, an embryonic stem cell and fertilized egg cell for producing a transgenic non-human animal which produces a human antibody, a myeloma cell, a cell derived from a transgenic non-human animal and the like which are resistant to lectin which recognizes a sugar chain in which 1-position of fucose is bound to 6-position of *N*-acetylglucosamine in the reducing end through α-bond in a complex *N*-glycoside-linked sugar chain. The myeloma cell can be used as a fusion cell for producing a hybridoma cell. Also, a hybridoma cell can be produced by immunizing a transgenic non-human animal with an antigen and removing spleen cells of the animal.

The lectin- resistant cell is a cell of which growth is not inhibited even when a lectin is applied at an effective concentration.

In the present invention, the effective concentration of a lectin which does not inhibit the growth can be decided depending on the cell line, and which is generally 10 μg/ml to 10.0 mg/ml, preferably 0.5 to 2.0 mg/ml. The effective concentration in the case where mutation is introduced into a parent cell is a concentration in which the parent cell cannot normally grow or higher than the concentration, and is a concentration which is preferably similar to, more preferably 2 to 5 times, still more preferably 10 times, and most preferably 20 times or more, higher concentration than the parent cell which cannot normally grow.

The parent cell is a cell before a certain treatment is applied, namely a cell before the step for selecting the α1,6-fucose/lectin-resistant cell used in the present invention is carried out or a cell before genetic engineering techniques for decreasing or deleting the above enzyme activity is carried out.

Although the parent cell is not particularly limited, the following cells are exemplified.

The parent cell of NS0 cell includes NS0 cells described in literatures such as *BIO/TECHNOLOGY*, 10, 169 (1992) and *Biotechnol. Bioeng.,* 73, 261 (2001). Furthermore, it includes NS0 cell line (RCB 0213) registered at RIKEN Cell Bank, The Institute of Physical and Chemical Research, sub-cell lines obtained by naturalizing these cell lines to media in which they can grow, and the like.

The parent cell of SP2/0-Ag14 cell includes SP2/0-Ag14 cells described in literatures such as *J. Immunol.*, 126, 317 (1981), *Nature*, 276, 269 (1978) and *Human Antibodies and Hybridomas*, 3, 129 (1992). Furthermore, it includes SP2/0-Ag14 cell (ATCC CRL-1581) registered at ATCC, sub-cell lines obtained by acclimating these cell lines to media in which they can grow (ATCC CRL-1581.1), and the like

The parent cell of CHO cell derived from Chinese hamster ovary tissue includes CHO cells described in literatures such as *Journal of Experimental Medicine,* 108, 945 (1958), *Proc. Natl. Acad Sci. USA*, 60, 1275 (1968), *Genetics*, 55, 513 (1968), *Chromosoma*, 41, 129 (1973), *Methods in Cell Science,* 18, 115 (1996), *Radiation Research*, 148, 260 (1997), *Proc. Natl. Acad Sci. USA*, 77, 4216 (1980), *Proc. Natl. Acad Sci. USA*, 60, 1275 (1968), *Cell*, 6, 121 (1975) and *Molecular Cell Genetics*, Appendix I, II (p. 883-900). Furthermore, it includes cell line CHO-K1 (ATCC CCL-61), cell line DUXB11 (ATCC CRL-9060) and cell line Pro-5 (ATCC CRL-1781) registered at ATCC, commercially available cell line CHO-S (Cat# 11619 of Life Technologies), sub-cell lines obtained by acclimating these cell lines to media in which they can grow, and the like.

The parent cell of a rat myeloma cell line YB2/3HL.P2.G11.16Ag.20 cell includes cell lines established from Y3/Ag1.2.3 cell (ATCC CRL-1631) such as YB2/3HL.P2.G11.16Ag.20 cell described in literatures such as *J. Cell. Biol.*, 93, 576 (1982) and *Methods Enzymol.*, 73B, 1 (1981). Furthermore, it includes YB2/3HL.P2.G11.16Ag.20 cell (ATCC CRL-1662) registered at ATCC, sub-lines obtained by acclimating these cell lines to media in which they can grow, and the like.

As the lectin which recognizes a sugar chain structure in which 1-position of fucose is bound to 6-position of *N*-acetylglucosamine in the reducing end through α-bond in the *N*-glycoside-linked sugar chain, any lectin can be used, so long as it can recognize the sugar chain structure. Examples include a *Lens culinaris* lectin LCA (lentil agglutinin

derived from *Lens culinaris*), a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

In the present invention, the α1,6-fucose/lectin-resistant cell may be any cell, so long as growth of the cell is not inhibited in the presence of a lectin at a definite effective concentration. Examples include cells in which the activity of at least one protein shown below is decreased or deleted, and the like.

(a) an enzyme protein relating to the synthesis of an intracellular sugar nucleotide, GDP-fucose, (hereinafter referred to as "GDP-fucose synthase");
(b) an enzyme protein relating to the sugar chain modification in which 1-position of fucose is bound to 6-position of *N*-acetylglucosamine in the reducing end through α-bond in a complex *N*-glycoside-linked sugar chain (hereinafter referred to as "α1,6-fucose modifying enzyme"); and
(c) a protein relating to the transportation of the intracellular sugar nucleotide, GDP-fucose, to the Golgi body (hereinafter referred to as "GDP-fucose transport protein").

The GDP-fucose synthase may be any enzyme, so long as it is an enzyme relating to the synthesis of the intracellular sugar nucleotide, GDP-fucose, as a supply source of fucose to a sugar chain, and includes an enzyme which has influence on the synthesis of the intracellular sugar nucleotide, GDP-fucose, and the like.

The intracellular sugar nucleotide, GDP-fucose, is supplied by a *de novo* synthesis pathway or a salvage synthesis pathway. Thus, all enzymes relating to the synthesis pathways are included in the GDP-fucose synthase.

The GDP-fucose synthase relating to the *de novo* synthesis pathway includes GDP-mannose 4-dehydratase (hereinafter referred to as "GMD"), GDP-keto-6-deoxymannose 3,5-epimerase, 4-reductase (hereinafter referred to as "Fx") and the like.

The GDP-fucose synthase relating to the salvage synthesis pathway includes GDP-beta-L-fucose pyrophosphorylase (hereinafter referred to as "GFPP"), fucokinase and the like.

The GDP-fucose synthase also includes an enzyme which has influence on the activity of the enzyme relating to the synthesis of the intracellular sugar nucleotide, GDP-fucose, and an enzyme which has influence on the structure of substances as the substrate of the enzyme.

The α1,6-fucose modifying enzyme includes any enzyme, so long as it is an enzyme relating to the reaction of binding of 1-position of fucose to 6-position of *N*-acetylglucosamine in the reducing end through α-bond in the complex *N*-glycoside-linked sugar chain. The enzyme relating to the reaction of binding of 1-position of fucose to 6-position of *N*-acetylglucosamine in the reducing end through α-bond in the complex *N*-glycoside-linked sugar chain includes an enzyme which has influence on the reaction of binding of 1-position of fucose to 6-position of *N*-acetylglucosamine in the reducing end through α-bond in the complex *N*-glycoside-linked sugar chain. Examples include α1,6-fucosyltransferase, α-L-fucosidase and the like.

Also, the enzyme relating to the reaction of binding of 1-position of fucose to 6-position of *N*-acetylglucosamine in the reducing end through α-bond in the complex *N*-glycoside-linked sugar chain includes an enzyme which has influence on the activity the enzyme relating to the reaction of binding of 1-position of fucose to 6-position of *N*-acetylglucosamine in the reducing end through α-bond in the complex *N*-glycoside-linked sugar chain and an enzyme which has influence on the structure of substances as the substrate of the enzyme.

The GDP-fucose transport protein may be any protein, so long as it is a protein relating to the transportation of the intracellular sugar nucleotide, GDP-fucose, to the Golgi body, and includes a GDP-fucose transporter and the like.

Furthermore, the GDP-fucose transport protein includes a protein which has an influence on the reaction to transport the intracellular sugar nucleotide, GDP-fucose, to the Golgi body, and specifically includes a protein which has an influence on the above protein relating to the transportation of the intracellular sugar nucleotide, GDP-fucose, to the Golgi body or has an influence on the expression thereof.

As a method for obtaining a cell used in the production of the medicament of the present invention, any technique can be used, so long as it is a technique which can select the α1,6-fucose/lectin-resistant cell. Specifically, the method includes a technique for decreasing or deleting the activity of the above protein. The technique for decreasing or deleting the above protein includes:

(a) a gene disruption technique which comprises targeting a gene encoding the protein,
(b) a technique for introducing a dominant negative mutant of a gene encoding the protein,
(c) a technique for introducing mutation into the protein,
(d) a technique for suppressing transcription and/or translation of a gene encoding the protein, and the like.

The present invention relates to a medicament for treating a patient who exerts such an affinity to a medicament comprising as an active ingredient an antibody composition produced by a cell unresistant to a lectin which recognizes a sugar chain in which 1-position of fucose is bound to 6-position of *N*-acetylglucosamine in the reducing

end through α-bond in a complex *N*-glycoside-linked sugar chain with a human Fcγ receptor IIIa that it is not enough for the antibody composition to exert sufficient therapeutic effect (hereinafter referred to as "conventional antibody medicament"), which comprises as an active ingredient an antibody composition produced by α1,6-fucose/lectin-resistant cell.

Such an affinity of the conventional antibody medicament with a human Fcγ receptor IIIa that it is not enough for the antibody composition to exert sufficient therapeutic effect means an affinity which is not sufficient for the antibody medicament to exert its ADCC activity.

Specifically, the affinity is considered to be not sufficient for an antibody medicament to exert its therapeutic effect in the case where a binding constant to the human Fcγ receptor IIIa at 25°C is lower than $1 \times 10^7$ $M^{-1}$ when measured by a biosensor method according to BIAcore or a binding constant to the human Fcγ receptor IIIa at 25°C is lower than $2 \times 10^6$ $M^{-1}$ when measured with an isothermal titration-type calorimeter.

The method for measuring affinity of an antibody composition and human Fcγ receptor IIIa includes a biosensor method using surface plasmon resonance, a measuring method by an isothermal titration-type calorimeter, and the like. The biosensor method using surface plasmon resonance is a method which monitors interaction between bio-molecules in real time by using the surface plasmon resonance phenomenon. When this method is used, it is unnecessary to label the bio-molecules. The measuring apparatus includes BIAcore series manufactured by Biacore and the like.

The measuring method using BIAcore includes measurement under optimum measuring conditions in accordance with the attached manufacture's instructions.

As the optimum measuring conditions, it is preferable that the amount of a substance to be immobilized on the sensor tip is within the range of equation 1, and the maximum binding amount is equal to or less than equation 2. In equation 1 and equation 2, the ligand represents a molecule to be immobilized on the sensor tip, the analyte represents a molecule to be added via the flow system, and "S" represents the number of binding sites of the ligand.

Equation 1

Minimum immobilizing amount

= 200 × l/s × (molecular weight of ligand (Dal)/molecular weight of analyte (Dal))

Maximum immobilizing amount

= 1000 × l/s × (molecular weight of ligand (Dal)/molecular weight of analyte (Dal))

Equation 2

Maximum binding amount

= molecular weight of analyte (Dal)

× immobilized amount of ligand (RU)/molecular weight of ligand (Dal) × s

Analysis according to the binding mode of protein can be carried out by setting the flow rate and washing conditions to such levels that a predetermined maximum binding amount can be maintained at the time of the measurement.

The isothermal titration-type calorimeter is an apparatus which can measure stoichiometry (quantitative ratio, hereinafter referred to as "N"), binding constant (KA) and enthalpy changing amount (ΔH) of the binding of a protein to a ligand. Any ligand can be used, so long as it is a molecule which binds to a protein, such as a protein, DNA or a low molecular compound.

The isothermal titration-type calorimeter measures the calorie generated or absorbed accompanied with the binding by carrying out titration of a protein and a ligand. By analyzing the titration curve, N, KA and ΔH are simultaneously obtained. The N, KA and ΔH obtained by the isothermal titration-type calorimeter are useful as parameters for quantitatively and thermodynamically describing the binding.

ADCC activity is the activity of an antibody bound to a cell surface antigen of a tumor cell or the like *in vivo* to activate an effector cell and thereby injure a tumor cell or the like via the binding of Fc region of the antibody and Fc receptor existing on the effector cell surface [*Monoclonal Antibodies: Principles and Applications,* Wiley-Liss, Inc.,

Chapter 2.1 (1995)]. The effector cell includes immunocytes such as a natural killer cell (hereinafter referred to as "NK cell"), a macrophage, a monocyte, a dendritic cell and a granulocyte.

The Fc receptor is classified into kinds such as Fcα receptor I, Fcε receptor I, Fcε receptor II, Fcγ receptor I, Fcγ receptor IIa, Fcγ receptor IIb, Fcγ receptor IIc, Fcγ receptor IIIa, Fcγ receptor IIIb and Fc receptor n.

The Fcγ receptor IIIa (hereinafter referred to as "FcγRIIIa") is one of the Fc receptors important for ADCC activity, which is expressed on cells such as NK cells, macrophages, monocytes, mast cells, dendritic cells, Langerhans cells and eosinophils [*Monoclonal Antibodies: Principles and Applications,* Wiley-Liss, Inc., Chapter 2.1 (1995)].

Also, in addition to the ADCC activity, the cytotoxic activity possessed by the antibody composition includes CDC activity [*Monoclonal Antibodies: Principles and Applications,* Wiley-Liss, Inc., Chapter 2.1 (1995)] and a growth inhibitory activity upon antigen-expressing cells by binding to the antigen.

Furthermore, it includes the growth inhibitory activity are those which accelerate apoptosis induction and differentiation induction of target cells [*Cancer Research,* 60, 7170 (2000); *Nature Medicine,* 1, 644 (1995); *Cell Growth Differ.,* 3, 401 (1992)].

The term "the antibody medicament is not enough in exerting ADCC activity" means that the antibody medicament cannot injure the targeting cell in a patient.

FcγRIIIa activates an immunocyte as an effector cell by the binding of an antibody and mediates ADCC activity to injure antigen-positive target cell by producing a cytotoxic molecule [*Monoclonal Antibodies: Principles and Applications,* Wiley-Liss, Inc., Chapter 2.1 (1995)].

The cytotoxic molecule is a molecule which directly or indirectly injures a target cell through the increase of its expression by a signal of FcγRIIIa on an effector cell. Examples include perforin, granzyme, active oxygen, nitrogen monoxide, granulysine, FasL and the like.

The immunocyte cell is a cell which exists *in vivo* and relates to various immune responses. The immunocompetent cell includes an NK cell, a macrophage, a monocyte, a mast cell, a dendritic cell, a Langerhans cell, a neutrophil, an eosinophil, a basophil, a B cell, a T cell and the like.

Genetic polymorphism (hereinafter simply referred to as "polymorphism") is present in the human FcγRIIIa. Specifically, the amino acid residue at position 176 from the N-terminal methionine of the human FcγRIIIa signal sequence is phenylalanine or valine.

The polymorphism is a mutation on a gene nucleotide sequence found in the same gene between normal individuals in the same species, which sometimes accompanies mutation of an amino acid as a result.

It is known that three expression systems, Phe/Phe or Val/Val homo type and Phe/Val hetero type, are present at position 176 from the N-terminal methionine of the human FcγRIIIa signal sequence, based on the combination of allele polymorphisms.

According to the present invention, the human FcγRIIIa includes all of these polymorphisms. A human FcγRIIIa having phenylalanine at the amino acid residue of position 176 from the N-terminal methionine of the signal sequence has lower affinity for the conventional antibody medicament in comparison with a human FcγRIIIa having valine at position 176 from the N-terminal methionine of the signal sequence. Accordingly, the medicament of the present invention exerts its effect particularly upon a patient having the human FcγRIIIa having phenylalanine at the amino acid residue of position 176 from the N-terminal methionine of the signal sequence.

In the present invention, the antibody composition may be any composition, so long as it comprises an antibody molecule having a complex *N*-glycoside-linked sugar chain in the Fc region.

The antibody molecule is a tetramer in which two molecules of each of two polypeptide chains, a heavy chain and a light chain (hereinafter referred to as "H chain" and "L chain", respectively), are respectively associated. Each of about a quarter of the N-terminal side of the H chain and about a half of the N-terminal side of the L chain (more than 100 amino acids for each) is called V region which is rich in diversity and directly relates to the binding with an antigen. The greater part of the moiety other than the V region is called C region. Based on homology with the C region, antibody molecules are classified into classes IgG, IgM, IgA, IgD and IgE.

Also, the IgG class is further classified into subclasses IgG1 to IgG4 based on homology with the C region.

The H chain is divided into four immunoglobulin domains VH, CH1, CH2 and CH3 from its N-terminal side, and a highly flexible peptide region called hinge region is present between CH1 and CH2 to divide CH1 and CH2. A structural unit comprising CH2 and CH3 after the hinge region is called Fc region to which a complex *N*-glycoside-linked sugar chain is bound and is also a region to which an Fc receptor, a complement and the like are bound (*Immunology Illustrated*, the Original, 5th edition, published on February 10, 2000, by Nankodo; *Handbook of Antibody Technology (Kotai Kogaku Nyumon)*, 1st edition on January 25, 1994, by Chijin Shokan).

Sugar chains of glycoproteins such as an antibody molecule are roughly divided into two types, namely a sugar chain which binds to asparagine (*N*-glycoside-linked sugar chain) and a sugar chain which binds to as serine or threonine (*O*-glycoside-linked sugar chain), based on the binding form to the protein moiety. The *N*-glycoside-linked sugar chains have a basic common core structure shown by the following structural formula (I):

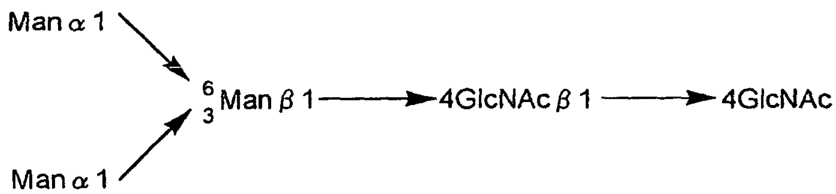

(I)

In formula (I), the sugar chain terminus which binds to asparagine is called a reducing end, and the opposite side is called a non-reducing end.

The *N*-glycoside-linked sugar chain may be any *N*-glycoside-linked sugar chain, so long as it comprises the core structure of formula (I). Examples include a high mannose type in which mannose alone binds to the non-reducing end of the core structure; a complex type in which the non-reducing end side of the core structure has one or more parallel branches of galactose-*N*-acetylglucosamine (hereinafter referred to as "Gal-GlcNAc") and the non-reducing end side of Gal-GlcNAc has a structure of sialic acid, bisecting *N*-acetylglucosamine or the like; a hybrid type in which the non-reducing end side of the core structure has branches of both of the high mannose type and complex type; and the like.

Since the Fc region in the antibody molecule has positions to which *N*-glycoside-linked sugar chains are separately bound, two sugar chains are bound per one antibody molecule. Since the *N*-glycoside-linked sugar chain which binds to an antibody molecule includes any sugar chain having the core structure represented by formula (I), a number of combinations of sugar chains may possible for the two *N*-glycoside-linked sugar chains which bind to the antibody.

Accordingly, in the present invention, the antibody composition which is produced by the α1,6-fucose/lectin-resistant cell may comprise an antibody molecule which is bound to the same sugar chain structure or an antibody molecule having different sugar chain structures, so long as the effect of the present invention is obtained from the composition.

The antibody molecule may be any antibody molecule, so long as it is a molecule comprising the Fc region of an antibody. Examples include an antibody, an antibody fragment, a fusion protein comprising an Fc region, and the like.

The antibody includes an antibody secreted by a hybridoma cell prepared from a spleen cell of an animal immunized with an antigen; an antibody prepared by genetic engineering technique, i.e., an antibody obtained by introducing an antibody expression vector to which gene encoding an antibody is inserted, into a host cell; and the like. Examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from mouse or the like, and can produce a monoclonal antibody having the desired antigen specificity.

The humanized antibody includes a human chimeric antibody, a human CDR-grafted antibody and the like.

A human chimeric antibody is an antibody which comprises an antibody H chain V region (hereinafter referred to as "HV" or "VH") and an antibody L chain V region (hereinafter referred to as "LV" or "VL"), both of a non-human animal, a human antibody H chain C region (hereinafter also referred to as "CH") and a human antibody L chain C region (hereinafter also referred to as "CL"). The non-human animal may be any animal such as mouse, rat, hamster or rabbit, so long as a hybridoma can be prepared therefrom.

The human chimeric antibody can be produced by obtaining cDNAs encoding VH and VL from a monoclonal antibody-producing hybridoma, inserting them into an expression vector for host cell having genes encoding human antibody CH and human antibody CL to thereby construct a human chimeric antibody expression vector, and then introducing the vector into a host cell to express the antibody.

The CH of a human chimeric antibody may be any CH, so long as it belongs to human immunoglobulin (hereinafter referred to as "hIg") can be used. Those belonging to the hIgG class are preferred and any one of the subclasses belonging to the hIgG class, such as hIgG1, hIgG2, hIgG3 and hIgG4, can be used. Also, as the CL of human chimeric antibody, any CL can be used, so long as it belongs to the hIg class, and those belonging to the κ class or λ class can also be used.

A human CDR-grafted antibody is an antibody in which amino acid sequences of any CDRs of VH and VL of a non-human animal antibody are grafted into appropriate positions of VH and VL of a human antibody.

The human CDR-grafted antibody can be produced by constructing cDNAs encoding V regions in which CDRs of VH and VL of a non-human animal antibody are grafted into CDRs of VH and VL of a human antibody, inserting them into an expression vector for host cell having genes encoding human antibody CH and human antibody CL to thereby construct a human CDR-grafted antibody expression vector, and then introducing the expression vector into

a host cell to express the human CDR-grafted antibody.

The CH of a human CDR-grafted antibody may be any CH, so long as it belongs to the hIg. Those of the hIgG class are preferred and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ class can also be used.

A human antibody is originally an antibody naturally existing in the human body, but it also includes antibodies obtained from a human antibody phage library, a human antibody-producing transgenic animal and a human antibody-producing transgenic plant, which are prepared based on the recent advance in genetic engineering, cell engineering and developmental engineering techniques.

Regarding the antibody existing in the human body, a lymphocyte capable of producing the antibody can be cultured by isolating a human peripheral blood lymphocyte, immortalizing it by its infection with EB virus or the like and then cloning it, and the antibody can be purified from the culture.

The human antibody phage library is a library in which antibody fragments such as Fab and single chain antibody are expressed on the phage surface by inserting a gene encoding an antibody prepared from a human B cell into a phage gene. A phage expressing an antibody fragment having binding activity for the desired antigen can be collected from the library based on the activity to bind to an antigen-immobilized substrate. The antibody fragment can be converted further into a human antibody molecule comprising two full H chains and two full L chains by genetic engineering techniques.

A human antibody-producing transgenic non-human animal is an animal in which a gene encoding a human antibody is introduced into cells. Specifically, a human antibody-producing transgenic non-human animal can be prepared by introducing a gene encoding a human antibody into ES cell derived from a mouse, transplanting the ES cell into an early stage embryo derived from other mouse and then developing it. By introducing a gene encoding a human antibody gene into a fertilized egg and developing it, the transgenic non-human animal can be also prepared. Regarding the preparation method of a human antibody from the human antibody-producing transgenic non-human animal, the human antibody can be produced and accumulated in a culture by obtaining a human antibody-producing hybridoma by a hybridoma preparation method usually carried out in non-human mammals and then culturing it.

The transgenic non-human animal includes cattle, sheep, goat, pig, horse, mouse, rat, fowl, monkey, rabbit and the like.

An antibody fragment is a fragment which comprises at least a part of the Fc region of the above-described antibody. The Fc region is a region at the C-terminal of H chain of an antibody, consists CH2 region and CH3 region, and includes a natural type and a mutant type. At least the part of the Fc region is preferably a fragment comprising CH2 region, more preferably a region comprising aspartic acid at position 1 present in the CH2 region. The Fc region of the IgG class is from Cys at position 226 to the C-terminal or from Pro at position 230 to the C-terminal according to the numbering of EU Index of Kabat *et al*. [*Sequences of Proteins of Immunological Interest,* 5$^{th}$ Ed., Public Health Service, National Institutes of Health, Bethesda, MD. (1991)]. The antibody fragment includes an H chain monomer, an H chain dimer and the like.

A fusion protein comprising a part of the Fc region is a composition in which an antibody comprising a part of the Fc region of an antibody or the antibody fragment is fused with a protein such as an enzyme or a cytokine (hereinafter referred to as "Fc fusion protein").

The ratio of sugar chains in which fucose is not bound to *N*-acetylglucosamine in the reducing end among the total complex *N*-glycoside-linked sugar chains bound to the Fc region contained in the antibody composition is a ratio of the number of a sugar chain in which fucose is not bound to *N*-acetylglucosamine in the reducing end in the sugar chain to the total number of the complex *N*-glycoside-linked sugar chains bound to the Fc region contained in the composition.

The sugar chain in which fucose is not bound to *N*-acetylglucosamine in the reducing end in the complex *N*-glycoside-linked sugar chain is a complex *N*-glycoside-linked sugar chain in which fucose is not bound to *N*-acetylglucosamine in the reducing end through α-bond. Specifically, it is a complex *N*-glycoside-linked sugar chain in which 1-position of fucose is not bound to 6-position of *N*-acetylglucosamine through α-bond.

Furthermore, the present invention relates to a medicament which comprises an antibody composition produced by the α1,6-fucose/lectin-resistant cell which has higher ADCC activity than a medicament comprising as an active ingredient an antibody composition produced by a cell unresistant to a lectin which recognizes a sugar chain structure in which 1-position of fucose is bound to 6-position of *N*-acetylglucosamine in the reducing end through α-bond in a complex *N*-glycoside-linked sugar chain.

The antibody composition having higher ADCC activity than the antibody composition produced by a cell unresistant to a lectin can be produced by the above α1,6-fucose/lectin-resistant cell.

ADCC activity is a cytotoxic activity in which an antibody bound to a cell surface antigen on a cell such as a tumor cell *in vivo* activates an effector cell through an Fc receptor existing on the antibody Fc region and effector cell surface and thereby injure the tumor cell and the like [*Monoclonal Antibodies: Principles and Applications,* Wiley-Liss,

Inc., Chapter 2.1 (1995)]. The effector cell includes a killer cell, a natural killer cell, an activated macrophages and the like.

When the ratio of sugar chains in which fucose is not bound to *N*-acetylglucosamine in the reducing end among the total complex *N*-glycoside-linked sugar chains binding to the Fc region in the antibody molecule is higher than that of the antibody composition produced by a cell unresistant to a lectin which recognizes a sugar chain structure in which 1-position of fucose is bound to 6-position of *N*-acetylglucosamine in the reducing end through α-bond in a complex *N*-glycoside-linked sugar chain, it has higher ADCC activity than the antibody composition produced by a cell unresistant to a lectin which recognizes a sugar chain structure in which 1-position of fucose is bound to 6-position of *N*-acetylglucosamine in the reducing end through α-bond in a complex *N*-glycoside-linked sugar chain.

As the ratio of sugar chains in which fucose is not bound to *N*-acetylglucosamine in the reducing end in the sugar chain among the total complex *N*-glycoside-linked sugar chains binding to the Fc region contained in the antibody composition is the higher, the ADCC activity of the antibody composition is the higher. The antibody composition having high ADCC activity includes an antibody composition in which the ratio of sugar chains in which fucose is not bound to *N*-acetylglucosamine in the reducing end among the total complex *N*-glycoside-linked sugar chains binding to the Fc region contained in the antibody composition is preferably 20% or more, more preferably 30% or more, still more preferably 40% or more, particularly preferably 50% or more and most preferably 100%.

Furthermore, the antibody composition having high ADCC activity produced by CHO cell includes an antibody composition in which the ratio of sugar chains in which fucose is not bound to *N*-acetylglucosamine in the reducing end among the total complex *N*-glycoside-linked sugar chains binding to the Fc region contained in the antibody composition is preferably 20% or more, more preferably 30% or more, still more preferably 40% or more, particularly preferably 50% or more and most preferably 100%.

The ratio of a sugar chain in which fucose is not bound to *N*-acetylglucosamine in the reducing end in the sugar chains contained in the composition which comprises an antibody molecule having complex *N*-glycoside-linked sugar chains in the Fc region can be determined by separating the sugar chain from the antibody molecule using a known method such as hydrazinolysis, enzyme digestion or the like [*Biochemical Experimentation Methods 23 - Method for Studying Glycoprotein Sugar Chain* (Japan Scientific Societies Press), edited by Reiko Takahashi (1989)], carrying out fluorescence labeling or radioisotope labeling of the released sugar chain, and then separating the labeled sugar chain by chromatography. Also, the separating sugar chain can be determined by analyzing it with the HPAED-PAD method [*J. Liq. Chromatogr.*, 6, 1577 (1983)].

Moreover, in the present invention, the antibody is preferably an antibody which recognizes a tumor-related antigen, an antibody which recognizes an allergy- or inflammation-related antigen, an antibody which recognizes cardiovascular disease-related antigen, an antibody which recognizes autoimmune disease-related antigen or an antibody which recognizes a viral or bacterial infection-related antigen. Also, the class of the antibody is preferably IgG.

The antibody which recognizes a tumor-related antigen includes anti-GD2 antibody [*Anticancer Res.,* 13, 331-336 (1993)], anti-GD3 antibody [*Cancer Immunol. Immunother.*, 36, 260-266 (1993)], anti-GM2 antibody [*Cancer Res.*, 54, 1511-1516 (1994)], anti-HER2 antibody [*Proc. Natl. Acad. Sci. USA*, 89, 4285-4289 (1992)], anti-CD52 antibody [*Proc. Natl. Acad. Sci. USA*, 89, 4285-4289 (1992)], anti-MAGE antibody [*British J. Cancer*, 83, 493-497 (2000)], anti-HM1.24 antibody [*Molecular Immunol.*, 36, 387-395 (1999)], anti-parathyroid hormone-related protein (PTHrP) antibody [*Cancer*, 88, 2909-2911 (2000)], anti-basic fibroblast growth factor antibody and anti-FGF8 antibody [*Proc. Natl. Acad. Sci. USA*, 86, 9911-9915 (1989)], anti-basic fibroblast growth factor receptor antibody and anti-FGF8 receptor antibody [*J. Biol. Chem.,* 265, 16455-16463 (1990)], anti-insulin-like growth factor antibody [*J. Neurosci. Res.*, 40, 647-659 (1995)], anti-insulin-like growth factor receptor antibody [*J. Neurosci. Res.,* 40, 647-659 (1995)], anti-PMSA antibody [*J. Urology*, 160, 2396-2401 (1998)], anti-vascular endothelial cell growth factor antibody [*Cancer Res.*, 57, 4593-4599 (1997)], anti-vascular endothelial cell growth factor receptor antibody [*Oncogene*, 19, 2138-2146 (2000)] and the like.

The antibody which recognizes an allergy- or inflammation-related antigen includes anti-interleukin 6 antibody [*Immunol. Rev.,* 127, 5-24 (1992)], anti-interleukin 6 receptor antibody [*Molecular Immunol.*, 31, 371-381 (1994)], anti-interleukin 5 antibody [*Immunol. Rev.,* 127, 5-24 (1992)], anti-interleukin 5 receptor antibody and anti-interleukin 4 antibody [*Cytokine*, 3, 562-567 (1991)], anti-interleukin 4 receptor antibody [*J. Immunol. Meth.,* 217, 41-50 (1998)], anti-tumor necrosis factor antibody [*Hybridoma*, 13, 183-190 (1994)], anti-tumor necrosis factor receptor antibody [*Molecular Pharmacol.,* 58, 237-245 (2000)], anti-CCR4 antibody [*Nature*, 400, 776-780 (1999)], anti-chemokine antibody [*J. Immuno. Meth.,* 174, 249-257 (1994)], anti-chemokine receptor antibody [*J. Exp. Med.*, 186, 1373-1381 (1997)] and the like. The antibody which recognizes a cardiovascular disease-related antigen includes anti-GpIIb/IIIa antibody [*J. Immunol.*, 152, 2968-2976 (1994)], anti-platelet-derived growth factor antibody [*Science*, 253, 1129-1132 (1991)], anti-platelet-derived growth factor receptor antibody [*J. Biol. Chem.,* 272, 17400-17404 (1997)] and anti-blood coagulation factor antibody [*Circulation*, 101, 1158-1164 (2000)] and the like.

The antibody which recognizes a viral or bacterial infection-related antigen includes anti-gp120 antibody [*Structure*, 8, 385-395 (2000)], anti-CD4 antibody [*J. Rheumatology*, 25, 2065-2076 (1998)], anti-CCR4 antibody and

anti-Vero toxin antibody [*J. Clin. Microbiol.*, 37, 396-399 (1999)] and the like.

Moreover, the present invention relates to a determination method for expecting effects before the administration of the medicament to a patient. Specifically, the method includes a method for screening a patient to which the medicament of the present invention is effective comprising the following steps (i) to (iii):

a method for selecting a patient to which the medicament of the present invention is effective, which comprises (i) contacting a conventional medicament or the medicament of the present invention with an effector cell obtained from a patient; (ii) measuring the amount of each of the medicaments bound to the effector cell;

(iii) comparing the measured amounts; and (iv) selecting a patient in which the amount of the medicament comprising an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain which is bound to the effector cell is low, or

a method for selecting a patient to which the medicament of the present invention is effective, which comprises (i) contacting a conventional antibody medicament or the medicament of the present invention with an effector cell obtained from a patient; (ii) measuring the activity caused by the contact of each of the medicaments with the effector cell; (iii) comparing the measured activities; and (iv) selecting a patient in which the activity of the medicament comprising an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain which is bound to the effector cell is low.

Hereinafter, the present invention is explained below in detail.

1. Preparation of host cell

The host cell for the production of an antibody composition used in the present invention can be prepared by the following techniques.

(1) Gene disruption technique which comprises targeting a gene encoding an enzyme

The host cell can be prepared by using a gene disruption technique by targeting a gene encoding a GDP-fucose synthase, an α1,6-fucose modifying enzyme or a GDP-fucose transport protein. The GDP-fucose synthase includes GMD, Fx, GFPP, fucokinase and the like. The α1,6-fucose modifying enzyme includes α-1,6-fucosyltransferase, α-L-fucosidase and the like. The GDP-fucose transport protein includes GDP-fucose transporter.

The gene disruption method may be any method, so long as it can disrupt the gene encoding the target enzyme. Examples include an antisense method, a ribozyme method, a homologous recombination method, an RNA-DNA oligonucleotide (RDO) method, an RNA interference (RNAi) method, a method using retrovirus, a method using transposon and the like. The methods are specifically described below.

(a) Preparation of host cell by the antisense method or the ribozyme method

The host cell can be prepared by targeting the GDP-fucose synthase, the α1,6-fucose modifying enzyme or the GDP-fucose transport protein according to the antisense or ribozyme method described in *Cell Technology*, 12, 239 (1993); *BIO/TECHNOLOGY*, 17, 1097 (1999); *Hum. Mol. Genet.*, 5, 1083 (1995); *Cell Technology,* 13, 255 (1994); *Proc. Natl. Acad. Sci. USA,* 96 , 1886 (1999); or the like, e.g., in the following manner.

A cDNA or a genomic DNA encoding the GDP-fucose synthase, the α1,6-fucose modifying enzyme or the GDP-fucose transport protein is prepared.

The nucleotide sequence of the prepared genomic DNA is determined.

Based on the determined DNA sequence, an antisense gene or ribozyme construct of an appropriate length comprising a part of a DNA which encodes the GDP-fucose synthase, the α1,6-fucose modifying enzyme or the GDP-fucose transport protein, its untranslated region or an intron is designed.

In order to express the antisense gene or ribozyme in a cell, a recombinant vector is prepared by inserting a fragment or total length of the prepared DNA into downstream of the promoter of an appropriate expression vector.

A transformant is obtained by introducing the recombinant vector into a host cell suitable for the expression vector.

The host cell can be obtained by selecting a transformant based on the activity of the GDP-fucose synthase, the α1,6-fucose modifying enzyme or the GDP-fucose transport protein. The host cell of the present invention can also be obtained by selecting a transformant based on the sugar chain structure of a glycoprotein on the cell membrane or the sugar chain structure of the produced antibody molecule.

As the host cell for preparing the host cell of the present invention, any cell such as yeast, an animal cell, an insect cell or a plant cell can be used, so long as it has a gene encoding the target GDP-fucose synthase, α1,6-fucose modifying enzyme or GDP-fucose transport protein. Examples include host cells described in the following item 3.

As the expression vector, a vector which is autonomously replicable in the host cell or can be integrated into the chromosome and comprises a promoter at such a position that the designed antisense gene or ribozyme can be transferred can be used. Examples include expression vectors described in the following item 3.

As the method for introducing a gene into various host cells, the methods for introducing recombinant vectors suitable for various host cells described in the following item 3 can be used.

The method for selecting a transformant based on the activity of the GDP-fucose synthase, the α1,6-fucose modifying enzyme or the GDP-fucose transport protein includes biochemical methods or genetic engineering techniques described in *New Biochemical Experimentation Series (Shin-Jikken Kagaku Koza)* 3 - *Saccharides (Toshitsu) I*, Glycoprotein (Totanpakushitu) (Tokyo Kagaku Dojin), edited by Japanese Biochemical Society (1988); *Cell Engineering (Saibo Kogaku)*, Supplement, Experimental Protocol Series, Glycobiology Experimental Protocol, Glycoprotein, Glycolipid and Proteoglycan (Shujun-sha), edited by Naoyuki Taniguchi, Akemi Suzuki, Kiyoshi Furukawa and Kazuyuki Sugawara (1996); *Molecular Cloning,* Second Edition; *Current Protocols in Molecular Biology;* and the like. The biochemical method includes a method in which the enzyme activity is evaluated using an enzyme-specific substrate and the like. The genetic engineering technique include the Northern analysis, RT-PCR and the like wherein the amount of mRNA of a gene encoding the enzyme is measured.

The method for selecting a transformant based on the sugar chain structure of a glycoprotein on the cell membrane includes the methods described later in the following item 1(5). The method for selecting a transformant based on the sugar chain structure of a produced antibody molecule includes the methods described in the following items 5 and 6.

As the method for preparing cDNA encoding the GDP-fucose synthase, the α1,6-fucose modifying enzyme or the GDP-fucose transport protein, the following method is exemplified.

<u>Preparation method of DNA:</u>

A total RNA or mRNA is prepared from human or non-human animal tissues or cells.

A cDNA library is prepared from the prepared total RNA or mRNA.

Degenerative primers are produced based on the amino acid sequence of the GDP-fucose synthase, the α1,6-fucose modifying enzyme or the GDP-fucose transport protein, and a gene fragment encoding the GDP-fucose synthase, the α1,6-fucose modifying enzyme or the GDP-fucose transport protein is obtained by PCR using the prepared cDNA library as the template.

A DNA encoding the GDP-fucose synthase, the α1,6-fucose modifying enzyme or the GDP-fucose transport protein can be obtained by screening the cDNA library using the obtained gene fragment as a probe.

As the mRNA of human or non-human tissues or cells, a commercially available product (e.g., manufactured by Clontech) may be used. Also, the mRNA can be prepared as poly(A)$^+$ RNA from a total RNA by the oligo(dT)-immobilized cellulose column method (*Molecular Cloning,* Second Edition) and the like, the total RNA being prepared from human or non-human animal tissues or cells by the guanidine thiocyanate-cesium trifluoroacetate method [*Methods in Enzymology,* <u>154</u>, 3 (1987)], the acidic guanidine thiocyanate phenol chloroform (AGPC) method [*Analytical Biochemistry*, <u>162</u>, 156 (1987); *Experimental Medicine*, <u>9</u>, 1937 (1991)] and the like.

In addition, mRNA can be prepared using a kit such as Fast Track mRNA Isolation Kit (manufactured by Invitrogen) or Quick Prep mRNA Purification Kit (manufactured by Pharmacia).

A method for preparing a cDNA library from the prepared mRNA of human or non-human animal tissues or cells includes the methods described in *Molecular Cloning,* Second Edition; *Current Protocols in Molecular Biology, A Laboratory Manual,* 2nd Ed. (1989); and the like, or methods using a commercially available kit such as SuperScript Plasmid System for cDNA Synthesis and Plasmid Cloning (manufactured by Life Technologies) or ZAP-cDNA Synthesis Kit (manufactured by STRATAGENE), and the like.

As the cloning vector for preparing the cDNA library, any vector such as a phage vector or a plasmid vector or the like can be used, so long as it is autonomously replicable in *Escherichia coli* K12. Examples include ZAP Express [manufactured by STRATAGENE, *Strategies*, <u>5</u>, 58 (1992)], pBluescript II SK(+) [*Nucleic Acids Research*, <u>17</u>, 9494 (1989)], Lambda ZAP II (manufactured by STRATAGENE), λgt10 and λgt11 [DNA Cloning, A Practical Approach, <u>1</u>, 49 (1985)], λTriplEx (manufactured by Clontech), λExCell (manufactured by Pharmacia), pT7T318U (manufactured by Pharmacia), pcD2 [*Mol. Cell. Biol.*, <u>3</u>, 280 (1983)], pUC18 [*Gene,* <u>33,</u> 103 (1985)] and the like.

Any microorganism can be used as the host microorganism for the preparation of the cDNA library, and *Escherichia coli* is preferably used. Examples include *Escherichia coli* XL1-Blue MRF' [manufactured by STRATAGENE, *Strategies*, <u>5</u>, 81 (1992)], *Escherichia coli* C600 [*Genetics*, <u>39</u>, 440 (1954)], *Escherichia coli* Y1088 [*Science,* <u>222</u>, 778 (1983)], *Escherichia coli* Y1090 [*Science,* <u>222</u>, 778 (1983)], *Escherichia coli* NM522 [*J. Mol. Biol.*, <u>166</u>, 1 (1983)],

*Escherichia coli* K802 [*J. Mol. Biol.*, 16, 118 (1966)], *Escherichia coli* JM105 [*Gene,* 38, 275 (1985)] and the like.

The cDNA library can be used as such in the subsequent analysis, and in order to obtain a full length cDNA as efficient as possible by decreasing the ratio of an infull length cDNA, a cDNA library prepared by using the oligo cap method developed by Sugano *et al*. [*Gene*, 138, 171 (1994); *Gene,* 200, 149 (1997); *Protein, Nucleic Acid and Protein*, 41, 603 (1996); *Experimental Medicine*, 11, 2491 (1993); *cDNA Cloning* (Yodo-sha) (1996); *Methods for Preparing Gene Libraries* (Yodo-sha) (1994)] can be used in the following analysis.

Based on the amino acid sequence of the GDP-fucose synthase, the α1,6-fucose modifying enzyme or the GDP-fucose transport protein, degenerative primers specific for the 5'-terminal and 3'-terminal nucleotide sequences of a nucleotide sequence presumed to encode the amino acid sequence are prepared , and DNA is amplified by PCR [*PCR Protocols,* Academic Press (1990)] using the prepared cDNA library as the template to obtain a gene fragment encoding the GDP-fucose synthase, the α1,6-fucose modifying enzyme or the GDP-fucose transport protein.

It can be confirmed that the obtained gene fragment is a DNA encoding the GDP-fucose synthase, the α1,6-fucose modifying enzyme or the GDP-fucose transport protein, by a method generally used for analyzing a nucleotide such as the dideoxy method of Sanger *et al*. [*Proc. Natl. Acad Sci. USA*, 74, 5463 (1977)] or by using a nucleotide sequence analyzer such as ABIPRISM 377 DNA Sequencer (manufactured by PE Biosystems) or the like.

A DNA encoding the GDP-fucose synthase, the α1,6-fucose modifying enzyme or the GDP-fucose transport protein can be obtained by carrying out colony hybridization or plaque hybridization (*Molecular Cloning,* Second Edition) for the cDNA or cDNA library synthesized from the mRNA contained in the human or non-human animal tissue or cell, using the gene fragment as a DNA probe.

Also, using the primers used for obtaining the gene fragment encoding the GDP-fucose synthase, the α1,6-fucose modifying enzyme or the GDP-fucose transport protein, a DNA encoding the GDP-fucose synthase, the α1,6-fucose modifying enzyme or the GDP-fucose transport protein can also be obtained by carrying out screening by PCR using the cDNA or cDNA library synthesized from the mRNA contained in human or non-human animal tissues or cells as the template.

The nucleotide sequence of the obtained DNA encoding the GDP-fucose synthase, the α1,6-fucose modifying enzyme or the GDP-fucose transport protein is analyzed from its terminus and determined by a method generally used for analyzing a nucleotide such as the dideoxy method of Sanger *et al*. [*Proc. Natl. Acad. Sci. USA*, 74, 5463 (1977)] or by using a nucleotide sequence analyzer such as ABIPRISM 377 DNA Sequencer (manufactured by PE Biosystems).

A gene encoding the GDP-fucose synthase, the α1,6-fucose modifying enzyme or the GDP-fucose transport protein can also be determined from genes in data bases by searching nucleotide sequence data bases such as GenBank, EMBL and DDBJ using a homology searching program such as BLAST based on the determined cDNA nucleotide sequence.

The cDNA encoding the GDP-fucose synthase, the α1,6-fucose modifying enzyme or the GDP-fucose transport protein can also be obtained by chemically synthesizing it with a DNA synthesizer such as DNA Synthesizer model 392 manufactured by Perkin Elmer using the phosphoamidite method, based on the determined DNA nucleotide sequence.

The method for preparing a genomic DNA encoding the GDP-fucose synthase, the α1,6-fucose modifying enzyme or the GDP-fucose transport protein includes known methods described in *Molecular Cloning,* Second Edition; *Current Protocols in Molecular Biology;* and the like. Furthermore, the genomic DNA can be prepared by using a kit such as Genome DNA Library Screening System (manufactured by Genome Systems) or Universal GenomeWalker™ Kits (manufactured by CLONTECH).

In addition, the host cell can also be obtained without using an expression vector, by directly introducing an antisense oligonucleotide or ribozyme into a host cell, which is designed based on the nucleotide sequence encoding the GDP-fucose synthase, the α1,6-fucose modifying enzyme or the GDP-fucose transport protein.

The antisense oligonucleotide or ribozyme can be prepared in the usual method or by using a DNA synthesizer. Specifically, it can be prepared based on the sequence information of an oligonucleotide having a corresponding sequence of continued 5 to 150 bases, preferably 5 to 60 bases, and more preferably 10 to 40 bases, among nucleotide sequences of a cDNA and a genomic DNA encoding the GDP-fucose synthase, the α1,6-fucose modifying enzyme or the GDP-fucose transport protein by synthesizing an oligonucleotide which corresponds to a sequence complementary to the oligonucleotide (antisense oligonucleotide) or a ribozyme comprising the oligonucleotide sequence.

The oligonucleotide includes oligo RNA and derivatives of the oligonucleotide (hereinafter referred to as "oligonucleotide derivatives").

The oligonucleotide derivatives includes oligonucleotide derivatives in which a phosphodiester bond in the oligonucleotide is converted into a phosphorothioate bond, an oligonucleotide derivative in which a phosphodiester bond in the oligonucleotide is converted into an N3'-P5' phosphoamidate bond, an oligonucleotide derivative in which ribose and a phosphodiester bond in the oligonucleotide are converted into a peptide-nucleic acid bond, an oligonucleotide derivative in which uracil in the oligonucleotide is substituted with C-5 propynyluracil, an oligonucleotide derivative in which uracil in the oligonucleotide is substituted with C-5 thiazoleuracil, an oligonucleotide derivative in which

cytosine in the oligonucleotide is substituted with C-5 propynylcytosine, an oligonucleotide derivative in which cytosine in the oligonucleotide is substituted with phenoxazine-modified cytosine, an oligonucleotide derivative in which ribose in the oligonucleotide is substituted with 2'-*O*-propylribose and an oligonucleotide derivative in which ribose in the oligonucleotide is substituted with 2'-methoxyethoxyribose [*Cell Technology (Saibo Kogaku)*, 16, 1463 (1997)].

(b) Preparation of host cell by homologous recombination

The host cell can be prepared by targeting a gene encoding the GDP-fucose synthase, the α1,6-fucose modifying enzyme or the GDP-fucose transport protein and modifying the target gene on chromosome through a homologous recombination technique.

The target gene on the chromosome can be modified by using a method described in *Manipulating the Mouse Embryo, A Laboratory Manual,* Second Edition, Cold Spring Harbor Laboratory Press (1994) (hereinafter referred to as "*Manipulating the Mouse Embryo, A Laboratory Manual*"); *Gene Targeting*, *A Practical Approach,* IRL Press at Oxford University Press (1993); *Biomanual Series 8, Gene Targeting*, *Preparation of Mutant Mice using ES cell,* Yodo-sha (1995) (hereinafter referred to as "*Preparation of Mutant Mice using ES Cells*"); or the like, for example, as follows.

A genomic DNA encoding the GDP-fucose synthase, the α1,6-fucose modifying enzyme or the GDP-fucose transport protein is prepared.

Based on the nucleotide sequence of the genomic DNA, a target vector is prepared for homologous recombination of a target gene to be modified (e.g., structural gene of the GDP-fucose synthase, the α1,6-fucose modifying enzyme or the GDP-fucose transport protein or a promoter gene).

The host cell can be produced by introducing the prepared target vector into a host cell and selecting a cell in which homologous recombination occurred between the target gene and target vector.

As the host cell, any cell such as yeast, an animal cell, an insect cell or a plant cell can be used, so long as it has a gene encoding the GDP-fucose synthase, the α1,6-fucose modifying enzyme or the GDP-fucose transport protein. Examples include the host cells described in the following item 3.

The method for preparing a genomic DNA encoding the GDP-fucose synthase, the α1,6-fucose modifying enzyme or the GDP-fucose transport protein includes the methods described in "Preparation method of genomic DNA" in the item 1(1)(a).

The target vector for the homologous recombination of the target gene can be prepared in accordance with a method described in *Gene Targeting*, *A Practical Approach*, IRL Press at Oxford University Press (1993); Biomanual Series 8, *Gene Targeting, Preparation of Mutant Mice using ES Cells,* Yodo-sha (1995); or the like. The target vector can be used as either a replacement type or an insertion type.

For introducing the target vector into various host cells, the methods for introducing recombinant vectors suitable for various host cells described in the following item 3, can be used.

The method for efficiently selecting a homologous recombinant includes a method such as the positive selection, promoter selection, negative selection or polyA selection described in *Gene Targeting, A Practical Approach,* IRL Press at Oxford University Press (1993); Biomanual Series 8, *Gene Targeting, Preparation of Mutant Mice using ES Cells,* Yodo-sha (1995); or the like. The method for selecting the homologous recombinant of interest from the selected cell lines includes the Southern hybridization method for genomic DNA (*Molecular Cloning,* Second Edition), PCR [*PCR Protocols*, Academic Press (1990)], and the like.

(c) Preparation of host cell by RDO method

The host cell of the present invention can be prepared by targeting a gene encoding the GDP-fucose synthase, the α1,6-fucose modifying enzyme or the GDP-fucose transport protein according to an RDO (RNA-DNA oligonucleotide) method, for example, as follows.

A cDNA or a genomic DNA encoding the GDP-fucose synthase, the α1,6-fucose modifying enzyme or the GDP-fucose transport protein is prepared.

The nucleotide sequence of the prepared cDNA or genomic DNA is determined.

Based on the determined DNA sequence, an RDO construct of an appropriate length comprising a part encoding the GDP-fucose synthase, the α1,6-fucose modifying enzyme or the GDP-fucose transport protein, a part of its untranslated region or a part of its intron, is designed and synthesized.

The host cell of the present invention can be obtained by introducing the synthesized RDO into a host cell and then selecting a transformant in which a mutation occurred in the target enzyme, i.e., the GDP-fucose synthase, the α1,6-fucose modifying enzyme or the GDP-fucose transport protein.

As the host cell, any cell such as yeast, an animal cell, an insect cell or a plant cell can be used, so long as it has a gene encoding the target GDP-fucose synthase, α1,6-fucose modifying enzyme or GDP-fucose transport protein. Examples include the host cells which will be described in the following item 3.

The method for introducing RDO into various host cells includes the methods for introducing recombinant vectors suitable for various host cells described in the following item 3.

The method for preparing cDNA encoding the GDP-fucose synthase, the α1,6-fucose modifying enzyme or the GDP-fucose transport protein includes the methods described in "Preparation method of DNA" in the item 1(1)(a).

The method for preparing a genomic DNA encoding the GDP-fucose synthase, the α1,6-fucose modifying enzyme or the GDP-fucose transport protein includes the methods in "Preparation method of genomic DNA" described in the item 1(1)(a).

The nucleotide sequence of the DNA can be determined by digesting it with appropriate restriction enzymes, cloning the fragments into a plasmid such as pBluescript SK(-) (manufactured by Stratagene), subjecting the clones to the reaction generally used as a method for analyzing a nucleotide sequence such as the dideoxy method of Sanger *et al*. [*Proc. Natl. Acad Sci. USA*, 74, 5463 (1977)] or the like, and then analyzing the clones using an automatic nucleotide sequence analyzer such as A.L.F. DNA Sequencer (manufactured by Pharmacia) or the like.

The RDO can be prepared in the usual method or by using a DNA synthesizer.

The method for selecting a transformant in which a mutation occurred, by introducing the RDO into the host cell, in the gene encoding the targeting enzyme, the GDP-fucose synthase, the α1,6-fucose modifying enzyme or the GDP-fucose transport protein includes the methods for directly detecting mutations in chromosomal genes described in *Molecular Cloning,* Second Edition, *Current Protocols in Molecular Biology* and the like.

Furthermore, the method described in the item 1(1)(a) for selecting a transformant based on the activity of the GDP-fucose synthase, the α1,6-fucose modifying enzyme or the GDP-fucose transport protein and the method for selecting a transformant based on the sugar chain structure of a glycoprotein on the cell membrane described in the following item 1(5) can also be used.

The construct of the RDO can be designed in accordance with the methods described in *Science,* 273, 1386 (1996); *Nature Medicine,* 4, 285 (1998); *Hepatology*, 25, 1462 (1997); *Gene Therapy*, 5, 1960 (1999); *J. Mol. Med*., 75, 829 (1997); *Proc. Natl. Acad Sci. USA*, 96, 8774 (1999); *Proc. Natl. Acad. Sci. USA*, 96, 8768 (1999); *Nuc. Acids. Res.,* 27, 1323 (1999); *Invest. Dematol*., 111, 1172 (1998); *Nature Biotech*., 16, 1343 (1998); *Nature Biotech.,* 18, 43 (2000); *Nature Biotech.,* 18, 555 (2000); and the like.

(d) Preparation of host cell by RNAi method

The host cell of the present invention can be prepared by targeting a gene encoding the GDP-fucose synthase, the α1,6-fucose modifying enzyme or the GDP-fucose transport protein according to the RNAi (RNA interference) method, for example, as follows.

A cDNA encoding the GDP-fucose synthase, the α1,6-fucose modifying enzyme or the GDP-fucose transport protein is prepared.

The nucleotide sequence of the prepared cDNA is determined.

Based on the determined DNA sequence, an RNAi gene construct of an appropriate length comprising a part encoding the GDP-fucose synthase, the α1,6-fucose modifying enzyme or the GDP-fucose transport protein or a part of its untranslated region, is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The host cell can be obtained by selecting a transformant based on the activity of the GDP-fucose synthase, the α1,6-fucose modifying enzyme or the GDP-fucose transport protein, or the sugar chain structure of the produced antibody molecule or of a glycoprotein on the cell membrane.

As the host cell, any cell such as yeast, an animal cell, an insect cell or a plant cell can be used, so long as it has a gene encoding the target GDP-fucose synthase, α1,6-fucose modifying enzyme or GDP-fucose transport protein. Examples include host cells described in the following item 3.

As the expression vector, a vector which is autonomously replicable in the host cell or can be integrated into the chromosome and comprises a promoter at such a position that the designed RNAi gene can be transferred is used. Examples include the expression vectors described in the following item 3.

As the method for introducing a gene into various host cells, the methods for introducing recombinant vectors suitable for various host cells, which will be described in the following item 3, can be used.

The method for selecting a transformant based on the activity having the GDP-fucose synthase, the α1,6-fucose modifying enzyme or the GDP-fucose transport protein includes the methods described in the item 1(1)(a).

The method for selecting a transformant based on the sugar chain structure of a glycoprotein on the cell membrane includes the methods which will be described in the following item 1(5). The method for selecting a transformant based on the sugar chain structure of a produced antibody molecule includes the methods described in the

following item 5 or 6.

The method for preparing cDNA encoding the GDP-fucose synthase, the α1,6-fucose modifying enzyme or the GDP-fucose transport protein includes the methods described in "Preparation method of DNA" in the item 1(1)(a) and the like.

In addition, the host cell of the present invention can also be obtained without using an expression vector, by directly introducing an RNAi gene designed based on the nucleotide sequence encoding the GDP-fucose synthase, the α1,6-fucose modifying enzyme or the GDP-fucose transport protein.

The RNAi gene can be prepared in the usual method or by using a DNA synthesizer.

The RNAi gene construct can be designed in accordance with the methods described in *Nature*, 391, 806 (1998); *Proc. Natl. Acad. Sci. USA,* 95, 15502 (1998); *Nature*, 395, 854 (1998); *Proc. Natl. Acad Sci. USA,* 96, 5049 (1999); *Cell,* 95, 1017 (1998); *Proc. Natl. Acad. Sci. USA*, 96, 1451 (1999); *Proc. Natl. Acad. Sci. USA*, 95, 13959 (1998); *Nature Cell Biol.*, 2, 70 (2000); and the like.

(e) Preparation of host cell by method using transposon

The host cell can be prepared by selecting a mutant based on the activity of the GDP-fucose synthase, the α1,6-fucose modifying enzyme or the GDP-fucose transport protein or the sugar chain structure of a produced antibody molecule or a glycoprotein on the cell membrane by using a transposon system described in *Nature Genet.*, 25, 35 (2000) or the like.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a host cell.

As the host cell, any cell such as yeast, an animal cell, an insect cell or a plant cell can be used, so long as it has a gene encoding the targeting GDP-fucose synthase, α1,6-fucose modifying enzyme or GDP-fucose transport protein. Examples include the host cells described in the following item 3. For introducing the gene into various host cells, the method for introducing recombinant vectors suitable for various host cells, which will be described in the following item 3, can be used.

The method for selecting a mutant based on the activity of the GDP-fucose synthase, the α1,6-fucose modifying enzyme or the GDP-fucose transport protein includes the methods described above in the item 1(1)(a).

The method for selecting a mutant based on the sugar chain structure of a glycoprotein on the cell membrane includes the methods be described in the following item 1(5). The method for selecting a mutant based on the sugar chain structure of a produced antibody molecule includes the methods described in the following item 5 or 6.

(2) Method for introducing dominant negative mutant of enzyme

The host cell can be prepared by targeting a gene encoding the GDP-fucose synthase, the α1,6-fucose modifying enzyme or the GDP-fucose transport protein according to a technique for introducing a dominant negative mutant of the enzyme. The GDP-fucose synthase includes GMD, Fx, GFPP, fucokinase and the like. The α1,6-fucose modifying enzyme includes α1,6-fucosyltransferase, α-L-focosidase and the like. The GDP-fucose transport protein includes GDP-fucose transporter and the like.

The enzymes catalyze specific reactions having substrate specificity, and dominant negative mutants of a gene encoding the enzymes can be prepared by disrupting the active center of the enzymes which catalyze the catalytic activity having substrate specificity. The preparation of a dominant negative mutant is specifically described as follows with reference to GMD among the target enzymes.

As a result of the analysis of the three-dimensional structure of GMD derived from *E. coli*, it has been found that 4 amino acids (threonine at position 133, glutamic acid at position 135, tyrosine at position 157 and lysine at position 161) have an important function on the enzyme activity [*Structure*, 8, 2 (2000)]. That is, when mutants were prepared by substituting the 4 amino acids with other different amino acids based on the three-dimensional structure information, the enzyme activity of all of the mutants was significantly decreased. On the other hand, changes in the ability of mutant GMD to bind to GMD coenzyme, NADP or its substrate, GDP-mannose were hardly observed in the mutants. Accordingly, a dominant negative mutant can be prepared by substituting the 4 amino acids which control the enzyme activity of GMD. A dominant negative mutant can be prepared by comparing the homology and predicting the three-dimensional structure using the amino acid sequence information based on the results of the GMD derived from *E. col.* Such a gene encoding substituted amino acid can be prepared by the site-directed mutagenesis described in *Molecular Cloning,* Second Edition, *Current Protocols in Molecular Biology* or the like.

The host cell can be prepared by using the prepared dominant negative mutant gene of the target enzyme according to the method described in *Molecular Cloning*, Second Edition, *Current Protocols in Molecular Biology, Manipulating the Mouse Embryo*, Second Edition or the like, for example, as follows.

A gene encoding the dominant negative mutant (hereinafter referred to as "dominant negative mutant gene") of the GDP-fucose synthase, the α1,6-fucose modifying enzyme or the GDP-fucose transport protein is prepared.

Based on the full length DNA of the prepared dominant negative mutant gene, a DNA fragment of an appropriate length containing a region encoding the protein is prepared, if necessary.

A recombinant vector is prepared by inserting the DNA fragment or full length DNA into downstream of the promoter of an appropriate expression vector.

A transformant is obtained by introducing the recombinant vector into a host cell suitable for the expression vector.

The host cell can be prepared by selecting a transformant based on the activity of the GDP-fucose synthase, the α1,6-fucose transport protein or the GDP-fucose transport protein, or the sugar chain structure of a produced antibody molecule or of a glycoprotein on the cell membrane.

As the host cell, any cell such as yeast, an animal cell, an insect cell or a plant cell can be used, so long as it has a gene encoding the GDP-fucose synthase, the α1,6-fucose transport protein or the GDP-fucose transport protein. Examples include the host cells described in the following item 3.

As the expression vector, a vector which is autonomously replicable in the host cell or can be integrated into the chromosome and comprises a promoter at a position where transcription of the DNA encoding the dominant negative mutant of interest can be effected is used. Examples include the expression vectors which will be described in the following item 3.

For introducing the gene into various host cells, the methods for introducing recombinant vectors suitable for various host cells, which will be described in the following item 3, can be used.

The method for selecting a mutant based on the activity of the GDP-fucose synthase, the α1,6-fucose transport protein or the GDP-fucose transport protein includes the methods described in above item 1(1)(a).

The method for selecting a mutant based on the sugar chain structure of a glycoprotein on he cell membrane includes the methods described in the following item 1(5). The method for selecting a transformant based on the sugar chain structure of a produced antibody molecule includes the methods described in the following item 5 or 6.

(3) Method for introducing mutation into enzyme

The host cell of the present invention can be prepared by introducing a mutation into a gene encoding the GDP-fucose synthase or the α1,6-fucose transport protein, and then by selecting a clone of interest in which the mutation occurred in the enzyme.

The GDP-fucose synthase includes GMD, Fx, GFPP, fucokinase and the like. The α1,6-fucose modifying enzyme includes α1,6-fucosyltransferase, α-L-focosidase and the like. The GDP-fucose transport protein includes GDP-fucose transporter and the like.

The method for introducing mutation into an enzyme includes 1) a method in which a desired clone is selected from mutants obtained by inducing a parent cell line into a mutagen or spontaneously generated mutants, based on the activity of the GDP-fucose synthase, the α1,6-fucose transport protein or the GDP-fucose transport protein, 2) a method in which a desired clone is selected from mutants obtained by a mutation-inducing treatment of a parent cell line with a mutagen or spontaneously generated mutants, based on the sugar chain structure of a produced antibody molecule, 3) a method in which a desired clone is selected from mutants obtained by a mutation-inducing treatment of a parent cell line with a mutagen or spontaneously generated mutants, based on the sugar chain structure of a glycoprotein on the cell membrane, and the like.

As the mutation-inducing treatment, any treatment can be used, so long as it can induce a point mutation or a deletion or frame shift mutation in the DNA of cells of the parent cell line.

Examples include treatment with ethyl nitrosourea, nitrosoguanidine, benzopyrene or an acridine pigment and treatment with radiation. Also, various alkylating agents and carcinogens can be used as mutagens. The method for allowing a mutagen to act upon cells includes the methods described in *Tissue Culture Techniques*, 3rd edition (Asakura Shoten), edited by Japanese Tissue Culture Association (1996), *Nature Genet*., 24, 314 (2000) and the like.

The spontaneously generated mutant includes mutants which are spontaneously formed by continuing subculture under general cell culture conditions without applying special mutation-inducing treatment.

The method for measuring the activity of the GDP-fucose synthase, the α1,6-fucose transport protein or the GDP-fucose transport protein includes the methods described above in the item 1(1)(a). The method for identifying the sugar chain structure of a glycoprotein on the cell membrane includes the methods described in the following item 1(5).

(4) Method for inhibiting transcription and/or translation of enzyme

The host cell of the present invention can be prepared by targeting a gene encoding the GDP-fucose synthase, the α1,6-fucose modifying enzyme or the GDP-fucose transport protein and inhibiting transcription and/or translation of the target gene according to the antisense RNA/DNA technique [*Bioscience and Industry*, 50, 322 (1992); *Chemistry*, 46, 681 (1991); *Biotechnology*, 9, 358 (1992); *Trends in Biotechnology*, 10, 87 (1992); *Trends in Biotechnology*, 10, 152 (1992); *Cell Engineering,* 16, 1463 (1997)], the triple helix technique [*Trends in Biotechnology*, 10, 132 (1992)] or the like

The GDP-fucose synthase includes GMD, Fx, GFPP, fucokinase and the like. The α1,6-fucose modifying enzyme includes α1,6-fucosyltransferase, α-L-focosidase and the like.

(5) Method for selecting clone resistant to lectin which recognizes sugar chain structure in which 1-position of fucose is bound to 6-position of *N*-acetylglucosamine in the reducing end through α-bond in the *N*-glycoside-linked sugar chain

The host cell can be prepared by using a method for selecting a clone resistant to a lectin which recognizes a sugar chain structure in which 1-position of fucose is bound to 6-position of *N*-acetylglucosamine in the reducing end through α-bond in the *N*-glycoside-linked sugar chain.

The method for selecting a clone resistant to a lectin which recognizes a sugar chain structure in which 1-position of fucose is bound to 6-position of *N*-acetylglucosamine in the reducing end through α-bond in the *N*-glycoside-linked sugar chain includes the methods using lectin described in *Somatic Cell Mol. Genet*., 12, 51 (1986) and the like.

As the lectin, any lectin can be used, so long as it is a lectin which recognizes a sugar chain structure in which 1-position of fucose is bound to 6-position of *N*-acetylglucosamine in the reducing end through α-bond in the *N*-glycoside-linked sugar chain. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

Specifically, the clone of the present invention resistant to a lectin which recognizes a sugar chain structure in which 1-position of fucose is bound to 6-position of *N*-acetylglucosamine in the reducing end through α-bond in the *N*-glycoside-linked sugar chain can be selected by culturing cells for 1 day to 2 weeks, preferably from 1 day to 1 week, using a medium comprising the lectin at a concentration of 1 μg/ml to 1 mg/ml, subculturing surviving cells or picking up a colony and transferring it into a culture vessel, and subsequently continuing the culturing using the lectin-containing medium.

The method for confirming that the cell is a lectin-resistant cell includes a method for confirming expression of the GDP-fucose synthase, α1,6-fucose modifying enzyme or the GDP-fucose transport protein, a method for culturing the cell in a medium to which lectin is directly added and the like. Specifically, when the expression amount of the mRNA of α1,6-fucosyltransferase which is one of α1,6-fucose modifying enzymes is measured, the decrease of the expression of mRNA demonstrates that the cell is a lectin-resistant cell.

2. Preparation of transgenic non-human animal or plant or the progenies

The antibody composition of the present invention can be prepared by using a transgenic non-human animal or plant or the progenies thereof in which a genomic gene is modified in such a manner that at least one activity of the protein selected from the group of the intracellular sugar nucleotide, GDP-fucose synthase, the α1,6-fucose modifying enzyme or the GDP-fucose transport protein is decreased or deleted. The transgenic non-human animal or plant or the progenies thereof can be prepared by targeting a gene encoding the above protein according to the method similar to that in the item 1.

In a transgenic non-human animal, the embryonic stem cell in which the activity of the GDP-fucose synthase, the α1,6-fucose modifying enzyme or the GDP-fucose transport protein is decreased or deleted can be prepared by applying the method similar to that in the item 1 to an embryonic stem cell of the intended non-human animal such as cattle, sheep, goat, pig, horse, mouse, rat, fowl, monkey or rabbit.

Specifically, a mutant clone is prepared in which a gene encoding the GDP-fucose synthase, the α1,6-fucose modifying enzyme or the GDP-fucose transport protein on the chromosome is inactivated or substituted with any sequence, by a known homologous recombination technique [e.g., *Nature*, 326, 6110, 295 (1987); *Cell*, 51, 3, 503 (1987); *etc*.]. Using the prepared mutant clone, a chimeric individual comprising an embryonic stem cell clone and a normal cell can be prepared by an injection chimera method into blastocyst of fertilized egg of an animal or by an aggregation chimera method. The chimeric individual is crossed with a normal individual, so that a transgenic non-human animal in which the activity of the GDP-fucose synthase, the α1,6-fucose modifying enzyme or the GDP-fucose transport protein is decreased or deleted in the whole body cells can be obtained.

The target vector for the homologous recombination of the target gene can be prepared in accordance with

a method described in *Gene Targeting, A Practical Approach,* IRL Press at Oxford University Press (1993); *Preparation of Mutant Mice using ES Cells;* or the like. The target vector can be used as any of a replacement type, an insertion type, a gene trap type and the like.

As the method for introducing the target vector into the embryonic stem cell, any method can be used, so long as it can introduce DNA into an animal cell. Examples include electroporation [*Cytotechnology*, 3, 133 (1990)], the calcium phosphate method (Japanese Published Unexamined Patent Application No. 227075/90), the lipofection method [*Proc. Natl. Acad Sci. USA*, 84, 7413 (1987)], the injection method [*Manipulating the Mouse Embryo*, Second Edition], a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method (*Manipulating Mouse Embryo,* Second Edition) and the like.

The method for efficiently selecting a homologous recombinant includes a method such as the positive selection, promoter selection, negative selection or polyA selection described in *Gene Targeting, A Practical Approach,* IRL Press at Oxford University Press (1993); *Preparation of Mutant Mice using ES Cells;* or the like. Specifically, in the case of the target vector containing hprt gene, it is introduced into the hprt gene-defected embryonic stem cell, the embryonic stem cell is cultured in a medium containing aminopterin, hypoxanthine and thymidine, and positive selection which selects the homologous recombinant of the hprt gene can be carried out by selecting a homogenous recombinant containing an aminopterin-resistant clone. In the case of the target vector containing a neomycin-resistant gene, the vector-introduced embryonic stem cell is cultured in a medium containing G418, and positive selection can be carried out by selecting a homogenous recombinant containing a neomycin-resistant gene. In the case of the target vector containing DT gene, the vector-introduced embryonic stem cell is cultured, and negative selection being capable of selecting a DT gene-free homogenous recombinant can be carried out by selecting the grown clone. Since the recombinants integrated into a chromosome randomly other than the homogenous recombination expresses the DT gene, they cannot grow due to the toxicity of DT. The method for selecting the homogenous recombinant of interest among the selected clones include the Southern hybridization for genomic DNA (*Molecular Cloning,* Second Edition), PCR [*PCR Protocols,* Academic Press (1990)] and the like.

When the embryonic stem cell is introduced into a fertilized egg by using an aggregation chimera method, in general, a fertilized egg at the development stage before 8-cell stage is preferably used. When the embryonic stem cell is introduced into a fertilized egg by using an injection chimera method, in general, it is preferred that a fertilized egg at the development stage from 8-cell stage to blastocyst stage is used.

When the fertilized egg is transplanted into a female mouse, it is preferred that a fertilized egg obtained from a pseudopregnant female mouse in which fertility is induced by mating with a male non-human mammal which is subjected to vasoligation is artificially transplanted or implanted. Although the psuedopregnant female mouse can be obtained by natural mating, the pseudopregnant female mouse in which fertility is induced can be obtained by mating with a male mouse after administration of a luteinizing hormone-releasing hormone (hereinafter referred to as "LHRH") or its analogue thereof. The analogue of LHRH includes [3,5-Dil-Tyr5]-LHRH, [Gln8]-LHRH, [D-Ala6]-LHRH, des-Gly10-[D-His(Bzl)6]-LHRH ethylamide and the like. Also, a fertilized egg cell in which the activity of the GDP-fucose synthase, the α1,6-fucose modifying enzyme or the GDP-fucose transport protein is decreased or deleted can be prepared by applying the method similar to that in the item 1 to fertilized egg of a non-human animal of interest such as cattle, sheep, goat, pig, horse, mouse, rat, fowl, monkey, rabbit or the like.

A transgenic non-human animal in which the activity of the GDP-fucose synthase, the α1,6-fucose modifying enzyme or the GDP-fucose transport protein is decreased or deleted can be prepared by transplanting the prepared fertilized egg cell into the oviduct or uterus of a pseudopregnant female using the embryo transplantation method described in *Manipulating Mouse Embryo*, Second Edition or the like, followed by childbirth by the animal.

In a transgenic plant, the callus in which the activity of the GDP-fucose synthase or the enzyme relating to the sugar chain modification in which 1-position of fucose is bound to 3-position or 6-position of *N*-acetylglucosamine in the reducing end through α-bond in a complex *N*-glycoside-linked sugar chain is decreased or deleted can be prepared by applying the method similar to that in the item 1 to a callus or cell of the plant of interest.

A transgenic plant in which the activity of the GDP-fucose synthase or the enzyme relating to the sugar chain modification in which 1-position of fucose is bound to 3-position or 6-position of *N*-acetylglucosamine in the reducing end through α-bond in a complex *N*-glycoside-linked sugar chain is decreased or deleted can be prepared by culturing the prepared callus in a medium comprising auxin and cytokinin to redifferentiate it in accordance with a known method [*Tissue Culture (Soshiki Baiyo),* 20 (1994); *Tissue Culture (Soshiki Baiyo),* 21 (1995); *Trends in Biotechnology,* 15, 45 (1997)].

3. Method for producing antibody composition

The antibody composition can be obtained by expressing it in a host cell using the methods described in *Molecular Cloning,* Second Edition; *Current Protocols in Molecular Biology; Antibodies, A Laboratory Manual*, Cold

Spring Harbor Laboratory, 1988 (hereinafter referred to as *"Antibodies*"); *Monoclonal Antibodies: Principles and Practice,* Third Edition, Acad. Press, 1996 (hereinafter referred to as *"Monoclonal Antibodies*"); and *Antibody Engineering, A Practical Approach,* IRL Press at Oxford University Press, 1996 (hereinafter referred to as "*Antibody Engineering*"), for example, as follows.

A full length cDNA encoding an antibody molecule is prepared, and a DNA fragment of an appropriate length comprising a DNA encoding the antibody molecule is prepared.

A recombinant vector is prepared by inserting the DNA fragment or the full length cDNA into downstream of the promoter of an appropriate expression vector.

A transformant which produces the antibody molecule can be obtained by introducing the recombinant vector into a host cell suitable for the expression vector.

As the host cell, the host cell of yeast, an animal cell, an insect cell, a plant cell or the like which can express the gene of interest described in the item 1 is used.

As the expression vector, a vector which is autonomously replicable in the host cell or can be integrated into the chromosome and comprises a promoter at such a position that the DNA encoding the antibody molecule of interest can be transferred is used.

The cDNA can be prepared from a human or non-human tissue or cell using, e.g., a probe or a primer specific for the DNA encoding the antibody molecule of interest according to the methods described in "Preparation method of DNA" in the item 1(1)(a).

When yeast is used as the host cell, the expression vector includes YEP13 (ATCC 37115), YEp24 (ATCC 37051), YCp50 (ATCC 37419) and the like.

Any promoter can be used, so long as it can function in yeast. Examples include a promoter of a gene of the glycolytic pathway such as a hexose kinase gene, PHO5 promoter, PGK promoter, GAP promoter, ADH promoter, gal 1 promoter, gal 10 promoter, heat shock protein promoter, MF$\alpha$1 promoter, CUP 1 promoter and the like.

The host cell includes yeast belonging to the genus *Saccharomyces*, the genus *Schizosaccharomyces,* the genus *Kluyveromyces*, the genus *Trichosporon*, the genus *Schwanniomyces* and the like, such as *Saccharomyces cerevisiae, Schizosaccharomyces pombe, Kluyveromyces lactis, Trichosporon pullulans* and *Schwanniomyces alluvius*.

As the method for introducing the recombinant vector, any method can be used, so long as it can introduce DNA into yeast. Examples include electroporation [*Methods in Enzymology*, 194, 182 (1990)], spheroplast method [*Proc. Natl. Acad Sci. USA*, 84, 1929 (1978)], lithium acetate method [*J. Bacteriol*., 153, 163 (1983)], a method described in *Proc. Natl. Acad Sci. USA*, 75, 1929 (1978) and the like.

When an animal cell is used as the host cell, the expression vector includes pcDNAI, pcDM8 (available from Funakoshi), pAGE107 [Japanese Published Unexamined Patent Application No. 22979/91; *Cytotechnology*, 3, 133 (1990)], pAS3-3 (Japanese Published Unexamined Patent Application No. 227075/90), pCDM8 [*Nature,* 329, 840 (1987)], pcDNAI/Amp (manufactured by Invitrogen), pREP4 (manufactured by Invitrogen), PAGE103 [*J. Biochemistry,* 101, 1307 (1987)], pAGE210 and the like.

Any promoter can be used, so long as it can function in an animal cell. Examples include a promoter of IE (immediate early) gene derived from cytomegalovirus (CMV), an early promoter derived from SV40, a promoter derived from retrovirus, a promoter derived from metallothionein, a heat shock promoter, an SR$\alpha$ promoter and the like. Also, an enhancer of the IE gene derived from human CMV may be used together with the promoter.

The host cell includes a human cell such as Namalwa cell, a monkey cell such as COS cell, a Chinese hamster cell such as CHO cell or HBT5637 (Japanese Published Unexamined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology*, 3, 133 (1990)], the calcium phosphate method (Japanese Published Unexamined Patent Application No. 227075/90), the lipofection method [*Proc. Natl. Acad. Sci. USA*, 84, 7413 (1987)], the injection method [*Manipulating the Mouse Embryo*, *A Laboratory Manual*], a method by using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method [*Manipulating Mouse Embryo*, Second Edition] and the like.

When an insect cell is used as the host, the protein can be expressed by the method described in *Current Protocols in Molecular Biology, Baculovirus Expression Vectors, A Laboratory Manual*, W.H. Freeman and Company, New York (1992), *Bio/Technology,* 6, 47 (1988) or the like.

That is, the protein can be expressed by co-introducing a recombinant gene-introducing vector and a baculovirus into an insect cell to obtain a recombinant virus in an insect cell culture supernatant and then infecting the insect cell with the recombinant virus.

The gene-introducing vector used in the method includes pVL1392, pVL1393, pBlueBacIII (all manufactured

by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [*Current Protocols in Molecular Biology*, *Baculovirus Expression Vectors*, *A Laboratory Manual,* W.H. Freeman and Company, New York (1992)], a *Trichoplusia* ni oocyte High 5 (manufactured by Invitrogen) and the like.

The method for the co-introducing the recombinant gene-introducing vector and the baculovirus for preparing the recombinant virus includes the calcium phosphate method (Japanese Published Unexamined Patent Application No. 227075/90), the lipofection method [*Proc. Natl. Acad. Sci. USA,* 84, 7413 (1987)] and the like.

When a plant cell is used as the host cell, the expression vector includes Ti plasmid, tobacco mosaic virus and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley and the like.

As the method for introducing the recombinant vector, any method can be used, so long as it can introduce DNA into a plant cell. Examples include a method using *Agrobacterium* (Japanese Published Unexamined Patent Application No. 140885/84, Japanese Published Unexamined Patent Application No. 70080/85, WO94/00977), electroporation (Japanese Published Unexamined Patent Application No. 251887/85), a method in which a particle gun (gene gun) is used (Japanese Patent No. 2606856, Japanese Patent No. 2517813) and the like.

As the method for expressing an antibody gene, secretion production, expression of a fusion protein of the Fc region with other protein and the like can be carried out in accordance with the method described in *Molecular Cloning,* Second Edition or the like, in addition to the direct expression.

When a gene is expressed by yeast, an animal cell, an insect cell or a plant cell into which a gene relating to the synthesis of a sugar chain is introduced, an antibody molecule to which a sugar or a sugar chain is added can be obtained depending on the introduced gene.

An antibody composition can be produced by culturing the obtained transformant in a medium to produce and accumulate the antibody molecule in the culture and then recovering it from the resulting culture. The method for culturing the transformant in a medium can be carried out in accordance with a general method which is used for the culturing of host cells.

As the medium for culturing a transformant obtained by using a yeast cell, as the host, the medium may be either a natural medium or a synthetic medium, so long as it comprises materials such as a carbon source, a nitrogen source and an inorganic salt which can be assimilated by the organism and culturing of the transformant can be efficiently carried out.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic salt includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out generally under aerobic conditions such as a shaking culture or submerged-aeration stirring culture. The culturing temperature is preferably at 15 to 40°C, and the culturing time is generally 16 hours to 7 days. During the culturing, the pH is maintained at 3.0 to 9.0. The pH is adjusted using an inorganic or organic acid, an alkali solution, urea, calcium carbonate, ammonia or the like.

Furthermore, if necessary, an antibiotic such as ampicillin or tetracycline can be added to the medium during the culturing.

When yeast transformed with a recombinant vector obtained by using an inducible promoter as the promoter is cultured, an inducer can be added to the medium, if necessary. For example, when yeast transformed with a recombinant vector obtained by using *lac* promoter is cultured, isopropyl-β-D-thiogalactopyranoside can be added to the medium, and when yeast transformed with a recombinant vector obtained by using *trp* promoter is cultured, indoleacrylic acid can be added to the medium.

When a transformant obtained by using an animal cell as the host cell is cultured, the medium includes generally used RPMI 1640 medium [*The Journal of the American Medical Association,* 199, 519 (1967)], Eagle's MEM medium [*Science*, 122, 501 (1952)], Dulbecco's modified MEM medium [*Virology*, 8, 396 (1959)], 199 medium [*Pro-*

*ceeding of the Society for the Biological Medicine*, 73, 1 (1950)] and Whitten's medium [*Developmental Engineering Experimentation Manual-Preparation of Transgenic Mice* (Kodan-sha), edited by M. Katsuki (1987)], the media to which fetal calf serum, *etc.* are added, and the like.

The culturing is carried out generally at a pH of 6 to 8 and 30 to 40°C for 1 to 7 days in the presence of 5% $CO_2$.

Furthermore, if necessary, an antibiotic such as kanamycin or penicillin can be added to the medium during the culturing.

The medium for culturing a transformant obtained by using an insect cell as the host cell includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature*, 195, 788 (1962)] and the like.

The culturing is carried out generally at a pH of 6 to 7 and 25 to 30°C for 1 to 5 days.

Furthermore, if necessary, an antibiotic such as gentamicin can be added to the medium during the culturing.

A transformant obtained by using a plant cell as the host cell can be cultured as a cell or by differentiating it into a plant cell or organ. The medium for culturing the transformant includes generally used Murashige and Skoog (MS) medium and White medium, wherein the media are added to a plant hormone such as auxin, cytokinin, and the like.

The culturing is carried out generally at a pH of 5 to 9 and 20 to 40°C for 3 to 60 days.

Furthermore, if necessary, an antibiotic such as kanamycin or hygromycin can be added to the medium during the culturing.

As discussed above, an antibody composition can be produced by culturing a transformant derived from a yeast cell, an animal cell, an insect cell or a plant cell, which comprises a recombinant vector into which a DNA encoding an antibody molecule is inserted, in accordance with a general culturing method, to thereby produce and accumulate the antibody composition, and then recovering the antibody composition from the culture.

As the method for expressing the gene encoding an antibody, secretion production, expression of a fusion protein and the like can be carried out in accordance with the method described in *Molecular Cloning,* Second Edition in addition to the direct expression.

The method for producing an antibody composition includes a method of intracellular expression in a host cell, a method of extracellular secretion from a host cell, and a method of production on a host cell membrane outer envelope. The method can be selected by changing the host cell used or the structure of the antibody composition produced.

When the antibody composition is produced in a host cell or on a host cell membrane outer envelope, it can be positively secreted extracellularly in accordance with the method of Paulson *et al*. [*J. Biol. Chem*., 264, 17619 (1989)], the method of Lowe *et al*. [*Proc. Natl. Acad Sci. USA*, 86, 8227 (1989), *Genes Develop*., 4, 1288 (1990)], the methods described in Japanese Published Unexamined Patent Application No. 336963/93 and Japanese Published Unexamined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector according to a gene recombination technique, introducing the expression vector into the host cell and then expressing the antibody molecule.

Also, its production amount can be increased in accordance with the method described in Japanese Published Unexamined Patent Application No. 227075/90 according to a gene amplification system using a dihydrofolate reductase gene.

In addition, the antibody composition can also be produced by using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby produce and accumulate the antibody composition and then recovering the antibody composition from the animal or plant individual.

The method for producing an antibody composition using an animal individual includes a method in which the antibody composition of interest is produced in an animal constructed by introducing a gene in accordance with a known method [*American Journal of Clinical Nutrition,* 63, 639S (1996); *American Journal of Clinical Nutrition,* 63, 627S (1996); *Bio/Technology*, 9, 830 (1991)].

In the case of an animal individual, an antibody composition can be produced by rearing a transgenic non-human animal into which a DNA encoding an antibody molecule is introduced to thereby produce and accumulate the antibody composition in the animal, and then recovering the antibody composition from the animal.

The place of the animal where the composition is produced and accumulated includes milk (Japanese Published Unexamined Patent Application No. 309192/88) and eggs of the animal. As the promoter used in these cases, any promoter can be used, so long as it can function in an animal. Preferred examples include mammary gland cell-specific promoters such as α casein promoter, β casein promoter, β lactoglobulin promoter, whey acidic protein promoter

and the like.

The method for producing an antibody composition using a plant individual includes a method in which an antibody composition is produced by cultivating a transgenic plant into which a DNA encoding an antibody molecule is introduced by a known method [*Tissue Culture (Soshiki Baiyo)*, 20 (1994); *Tissue Culture (Soshiki Baiyo),* 21 (1995); *Trends in Biotechnology,* 15, 45 (1997)] to produce and accumulate the antibody composition in the plant, and then recovering the antibody composition from the plant.

Regarding an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted by using ultrasonic oscillator, French press, Manton Gaulin homogenizer, dynomill or the like to obtain a cell-free extract. A purified product of the antibody composition can be obtained from a supernatant obtained by centrifuging the cell-free extract according to a general enzyme isolation purification techniques such as solvent extraction; salting out or desalting with ammonium sulfate; precipitation with an organic solvent; anion exchange chromatography using a resin such as diethylaminoethyl (DEAE)-Sepharose or DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose or phenyl-Sepharose, gel filtration using a molecular sieve; affinity chromatography; chromatofocusing; electrophoresis such as isoelectric focusing; and the like which may be used alone or in combination.

Also, when the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized by using a protein denaturing agent. The antibody composition is made into a normal three-dimensional structure by diluting or dialyzing the solubilized solution, and then a purified product of the antibody composition is obtained by the same isolation purification method.

When the antibody composition is secreted extracellularly, the antibody composition or derivatives thereof can be recovered from the culture supernatant. That is, the culture is treated according to a technique such as centrifugation to obtain a soluble fraction, and a purified preparation of the antibody composition can be obtained from the soluble fraction by the same isolation purification method.

The thus obtained antibody composition includes an antibody, the fragment of the antibody, a fusion protein comprising the Fc region of the antibody, and the like.

As an example for obtaining antibody compositions, methods for producing a humanized antibody composition and Fc fusion protein are described below in detail, but other antibody compositions can also be obtained in a manner similar to the method.

A. Preparation of humanized antibody composition

(1) Construction of humanized antibody expression vector

A humanized antibody expression vector is an expression vector for animal cell into which genes encoding H chain and L chain C regions of a human antibody are inserted, and which can be constructed by cloning each of genes encoding CH and CL of a human antibody into an expression vector for animal cell.

The C regions of a human antibody may be CH and CL of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding CH and CL of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [*Cytotechnology*, 3, 133 (1990)], pAGE103 [*J. Biochem*., 101, 1307 (1987)], pHSG274 [*Gene,* 27, 223 (1984)], pKCR [*Proc. Natl. Acad Sci. USA*, 78, 1527 (1981), pSG1 β d2-4 [*Cytotechnology*, 4, 173 (1990)] and the like. The promoter and enhancer used in the expression vector for animal cell includes SV40 early promoter and enhancer [*J. Biochem*., 101, 1307 (1987)], Moloney mouse leukemia virus LTR promoter [*Biochem. Biophys. Res. Commun*., 149, 960 (1987)], immunoglobulin H chain promoter [*Cell*, 41, 479 (1985)] and enhancer [*Cell,* 33, 717 (1983)], and the like.

The humanized antibody expression vector may be either of a type in which genes encoding the H chain and L chain of an antibody exist on separate vectors or of a type in which both genes exist on the same vector (hereinafter referred to "tandem type"). In respect of easiness of construction of a humanized antibody expression vector, easiness of introduction into animal cells, and balance between the expression amounts of the H and L chains of an antibody in animal cells, a tandem type of the humanized antibody expression vector is more preferred [*J. Immunol. Methods*,

167, 271 (1994)].

The constructed humanized antibody expression vector can be used for expression of a human chimeric antibody and a human CDR-grafted antibody in animal cells.

(2) Preparation method of cDNA encoding V region of non-human animal antibody

cDNAs encoding VH and VL of a non-human animal antibody such as a mouse antibody can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding VH and a recombinant phage or recombinant plasmid comprising a cDNA encoding VL is isolated from the library by using a C region part or a V region part of an existing mouse antibody as the probe. Full nucleotide sequences of VH and VL of the mouse antibody of interest on the recombinant phage or recombinant plasmid are determined, and full length amino acid sequences of VH and VL are deduced from the nucleotide sequences.

As the non-human animal, any animal such as mouse, rat, hamster or rabbit can be used, so long as a hybridoma cell can be produced therefrom.

The method for preparing a total RNA from a hybridoma cell includes the guanidine thiocyanate-cesium trifluoroacetate method [*Methods in Enzymology*, 154, 3 (1987)] and the like, and the method for preparing mRNA from total RNA includes an oligo(dT)-immobilized cellulose column method [*Molecular Cloning: A Laboratory Manual*, Cold Spring Harbor Lab. Press New York (1989)] and the like. In addition, a kit for preparing mRNA from a hybridoma cell includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods [*Molecular Cloning: A Laboratory Manual,* Cold Spring Harbor Lab. Press New York (1989), *Current Protocols in Molecular Biology,* Supplement 1-34], methods using a commercially available kit such as SuperScript™, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized by using mRNA extracted from a hybridoma cell as the template is inserted may be any vector, so long as the cDNA can be inserted. Examples include ZAP Express [*Strategies*, 5, 58 (1992)], pBluescript II SK(+) [*Nucleic Acids Research*, 17, 9494 (1989)], λzapII (manufactured by Stratagene), λgt10 and λgt11 [*DNA Cloning, A Practical Approach*, I, 49 (1985)], Lambda BlueMid (manufactured by Clontech), λExCell, pT7T3 18U (manufactured by Pharmacia), pcD2 [*Mol. Cell. Biol.*, 3, 280 (1983)], pUC18 [*Gene*, 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies*, 5, 81 (1992)], C600 [*Genetics*, 39, 440 (1954)], Y1088 and Y1090 [*Science,* 222, 778 (1983)], NM522 [*J. Mol. Biol.*, 166, 1 (1983)], K802 [*J. Mol. Biol.*, 16, 118 (1966)], JM105 [*Gene*, 38, 275 (1985)] and the like.

As the method for selecting a cDNA clone encoding VH and VL of a non-human animal antibody from the cDNA library, a colony hybridization or a plaque hybridization using an isotope- or fluorescence-labeled probe can be used [*Molecular Cloning: A Laboratory Manual*, Cold Spring Harbor Lab. Press New York (1989)]. The cDNA encoding VH and VL can also be prepared by preparing primers and carrying out polymerase chain reaction [hereinafter referred to as "PCR"; *Molecular Cloning: A Laboratory Manual*, Cold Spring Harbor Lab. Press New York (1989); *Current Protocols in Molecular Biology,* Supplement 1-34] using a cDNA synthesized from mRNA or a cDNA library as the template.

The nucleotide sequences of the cDNAs can be determined by digesting the selected cDNAs with appropriate restriction enzymes, cloning the fragments into a plasmid such as pBluescript SK(-) (manufactured by Stratagene), carrying out the reaction of a generally used nucleotide sequence analyzing method such as the dideoxy method of Sanger *et al*. [*Proc. Natl. Acad. Sci., USA*, 74, 5463 (1977)], and then analyzing the clones using an automatic nucleotide sequence analyzer such as A.L.F. DNA Sequencer (manufactured by Pharmacia).

Whether or not the obtained cDNAs encode the full length amino acid sequences of VH and VL of the antibody comprising a secretory signal sequence can be confirmed by deducing the full length amino acid sequences of VH and VL from the determined nucleotide sequence and comparing them with the full length amino acid sequences of VH and VL of known antibodies [*Sequences of Proteins of Immunological Interest,* US Dep. Health and Human Services (1991)].

(3) Analysis of amino acid sequence of V region of non-human animal antibody

Regarding the full length amino acid sequences of VH and VL of the antibody comprising a secretory signal sequence, the length of the secretory signal sequence and the N-terminal amino acid sequences can be deduced and subgroups to which they belong can also be found, by comparing them with the full length amino acid sequences of VH and VL of known antibodies [*Sequences of Proteins of Immunological Interest*, US Dep. Health and Human Services (1991)]. In addition, the amino acid sequences of each CDR of VH and VL can also be found by comparing them with the amino acid sequences of VH and VL of known antibodies [*Sequences of Proteins of Immunological Interest*, US Dep. Health and Human Services (1991)].

(4) Construction of human chimeric antibody expression vector

A human chimeric antibody expression vector can be constructed by cloning cDNAs encoding VH and VL of a non-human animal antibody into upstream of genes encoding CH and CL of a human antibody in the humanized antibody expression vector described in the item 3(1). For example, a human chimeric antibody expression vector can be constructed by linking each of cDNAs encoding VH and VL of a non-human animal antibody to a synthetic DNA comprising nucleotide sequences at the 3'-terminals of VH and VL of a non-human animal antibody and nucleotide sequences at the 5'-terminals of CH and CL of a human antibody and also having a recognizing sequence of an appropriate restriction enzyme at both terminals, and by cloning them into upstream of genes encoding CH and CL of a human antibody contained in the humanized antibody expression vector constructed described in the item 3(1) in such a manner that they can be expressed in a suitable form.

(5) Construction of cDNA encoding V region of human CDR-grafted antibody

cDNAs encoding VH and VL of a human CDR-grafted antibody can be obtained as follows. First, amino acid sequences of the frameworks (hereinafter referred to as "FR") of VH and VL of a human antibody for grafting CDR of VH and VL of a non-human animal antibody is selected. As the amino acid sequences of FRs of VH and VL of a human antibody, any amino acid sequences can be used so long as they are derived from a human antibody. Examples include amino acid sequences of FRs of VH and VL of human antibodies registered at databases such as Protein Data Bank, amino acid sequences common in each subgroup of FRs of VH and VL of human antibodies [*Sequences of Proteins of Immunological Interest,* US Dep. Health and Human Services (1991)] and the like. In order to produce a human CDR-grafted antibody having enough activities, it is preferred to select an amino acid sequence having homology as high as possible (at least 60% or more) with amino acid sequences of VH and VL of a non-human animal antibody of interest.

Next, the amino acid sequences of CDRs of VH and VL of the non-human animal antibody of interest are grafted to the selected amino acid sequences of FRs of VH and VL of a human antibody to design amino acid sequences of VH and VL of the human CDR-grafted antibody. The designed amino acid sequences are converted into DNA sequences by considering the frequency of codon usage found in nucleotide sequences of antibody genes [*Sequences of Proteins of Immunological Interest*, US Dep. Health and Human Services (1991)], and the DNA sequences encoding the amino acid sequences of VH and VL of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the humanized antibody expression vector described in the item 3(1) by introducing recognizing sequences of an appropriate restriction enzyme into the 5'-terminals of the synthetic DNA on both terminals. After the PCR, the amplified product is cloned into a plasmid such as pBluescript SK(-) (manufactured by Stratagene) and the nucleotide sequences are determined by the method in the item 3(2) to thereby obtain a plasmid having DNA sequences encoding the amino acid sequences of VH and VL of the desired human CDR-grafted antibody.

(6) Construction of human CDR-grafted antibody expression vector

A human CDR-grafted antibody expression vector can be constructed by cloning the cDNAs encoding VH and VL of the human CDR-grafted antibody constructed in the item 3(5) into upstream of the gene encoding CH and CL of a human antibody in the humanized antibody expression vector described in the item 3(1). For example, recognizing sequences of an appropriate restriction enzyme are introduced into the 5'-terminals of both terminals of a synthetic DNA fragment, among the synthetic DNA fragments which are used in the item 3(5) for constructing the VH and VL of the human CDR-grafted antibody, so that they are cloned into upstream of the genes encoding CH and CL of a human antibody in the humanized antibody expression vector described in the item 3(1) in such a manner that they

can be expressed in a suitable form, to thereby construct the human CDR-grafted antibody expression vector.

(7) Stable production of humanized antibody

A transformant capable of stably producing a human chimeric antibody and a human CDR-grafted antibody (both hereinafter referred to as "humanized antibody") can be obtained by introducing the humanized antibody expression vector described in the items 3(4) and (6) into an appropriate animal cell.
The method for introducing a humanized antibody expression vector into an animal cell includes electroporation [Japanese Published Unexamined Patent Application No. 257891/90, *Cytotechnology*, 3, 133 (1990)] and the like.
As the animal cell into which a humanized antibody expression vector is introduced, the animal cell capable of producing the humanized antibody prepared in the above item 1 can be used.
Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a syrian hamster kidney, a human myeloma cell such as Namalwa cell, and the like, a Chinese hamster ovary cell CHO/DG44 cell, a rat myeloma YB2/0 cell and the host cells of the present invention described in the item 5 are preferred.
A transformant introduced with the humanized antibody expression vector capable of stably producing the humanized antibody can be selected by using a medium for animal cell culture comprising an agent such as G418 sulfate (hereinafter referred to as "G418"; manufactured by SIGMA) and the like in accordance with the method described in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The humanized antibody can be produced and accumulated in the culture supernatant by culturing the obtained transformant in a medium. The amount of the humanized antibody produced and the antigen binding activity of the humanized antibody in the culture supernatant can be measured by a method such as enzyme-linked immunosorbent assay [hereinafter referred to as "ELISA"; *Antibodies, Monoclonal Antibodies,* Cold Spring Harbor Laboratory, Chapter 14 (1998); *Monoclonal Antibodies: Principles and Practice,* Academic Press Limited (1996)] or the like. Also, the amount of the humanized antibody produced by the transformant can be increased by using a DHFR gene amplification system in accordance with the method described in Japanese Published Unexamined Patent Application No. 257891/90.
The humanized antibody can be purified from a medium culturing the transformant by using a protein A column [*Antibodies, A Laboratory Manual*, Cold Spring Harbor Laboratory, Chapter 8 (1998); *Monoclonal Antibodies: Principles and Practice,* Academic Press Limited (1996)]. In addition, purification methods generally used for the purification of proteins can also be used. For example, the purification can be carried out through the combination of gel filtration, ion exchange chromatography and ultrafiltration. The molecular weight of the H chain, L chain or antibody molecule as a whole of the purified humanized antibody can be measured, e.g., by polyacrylamide gel electrophoresis [hereinafter referred to as "SDS-PAGE"; *Nature,* 227, 680 (1970)], Western blotting [*Antibodies, A Laboratory Manual,* Cold Spring Harbor Laboratory, Chapter 12 (1998), *Monoclonal Antibodies: Principles and Practice,* Academic Press Limited (1996)] or the like.

B. Preparation of Fc fusion protein

(1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.
The Fc region of a human antibody includes regions containing CH2 and CH3, a part of a hinge region and/or CH1 in addition to regions containing CH2 and CH3. Also, it can be any Fc region so long as at least one amino acid of CH2 or CH3 may be deleted, substituted, added or inserted, and substantially has the binding activity to the Fcγ receptor.
As the genes encoding each of the Fc region of a human antibody and the protein to be fused, a chromosomal DNA comprising an exon and an intron can be used, and a cDNA can also be used. The method for linking the genes and the Fc region includes PCR using each of the gene sequences as the template (*Molecular Cloning,* Second Edition; *Current Protocols in Molecular Biology,* Supplement 1-34).
As the expression vector for animal cell, any vector can be used, so long as a gene encoding the C region of a human antibody can be inserted thereinto and expressed therein. Examples include pAGE107 [*Cytotechnology*, 3,

133 (1990)], pAGE103 [*J. Biochem.,* 101, 1307 (1987)], pHSG274 [*Gene,* 27, 223 (1984)], pKCR [*Proc. Natl. Acad Sci. USA*, 78, 1527 (1981), pSG1 β d2-4 [*Cytotechnology*, 4, 173 (1990)] and the like. The promoter and enhancer in the expression vector for animal cell include SV40 early promoter and enhancer [*J. Biochem.,* 101, 1307 (1987)], Moloney mouse leukemia virus LTR [*Biochem. Biophys. Res. Commun.*, 149, 960 (1987)], immunoglobulin H chain promoter [*Cell,* 41, 479 (1985)] and enhancer [*Cell,* 33, 717 (1983)], and the like.

(2) Obtaining of DNA encoding Fc region of human antibody and protein to be fused

A DNA encoding the Fc region of a human antibody and the protein to be fused can be obtained in the following manner.

A cDNA is synthesized by extracting mRNA from a cell or tissue which expresses the protein of interest to be fused with Fc. The synthesized cDNA is cloned into a vector such as a phage or a plasmid to obtain a cDNA library. A recombinant phage or recombinant plasmid comprising cDNA encoding the protein of interest is isolated from the library by using the gene sequence part of the protein of interest as the probe. A full nucleotide sequence of the protein of interest on the recombinant phage or recombinant plasmid is determined, and a full length amino acid sequence is deduced from the nucleotide sequence.

As the non-human animal, any animal such as mouse, rat, hamster or rabbit can be used, so long as a cell or tissue can be extirpated therefrom.

The method for preparing a total RNA from a cell or tissue includes the guanidine thiocyanate-cesium trifluoroacetate method [*Methods in Enzymology*, 154, 3 (1987)] and the like, and the method for preparing mRNA from total RNA includes an oligo (dT)-immobilized cellulose column method (*Molecular Cloning,* Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods (*Molecular Cloning,* Second Edition; *Current Protocols in Molecular Biology*, Supplement 1-34); methods using a commercially available kit such as SuperScript™, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express [*Strategies,* 5, 58 (1992)], pBluescript II SK(+) [*Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.*, 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies*, 5, 81 (1992)], C600 [*Genetics*, 39, 440 (1954)], Y1088 and Y1090 [*Science*, 222, 778 (1983)], NM522 [*J. Mol. Biol.*, 166, 1 (1983)], K802 [*J. Mol. Biol.*, 16, 118 (1966)], JM105 [*Gene*, 38, 275 (1985)] and the like.

As the method for selecting a cDNA clone encoding the protein of interest from the cDNA library, a colony hybridization or a plaque hybridization using an isotope- or fluorescence-labeled probe can be used (*Molecular Cloning*, Second Edition). The cDNA encoding the protein of interest can also be prepared by preparing primers and using a cDNA synthesized from mRNA or a cDNA library as the template according to PCR.

The method for fusing the protein of interest with the Fc region of a human antibody includes PCR. For example, synthesized oligo DNAs (primers) are designed at the 5'-terminal and 3'-terminal of the gene sequence encoding the protein of interest, and PCR is carried out to prepare a PCR product. In the same manner, any primers are designed for the gene sequence encoding the Fc region of a human antibody to be fused and a PCR product is obtained. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequence of the DNA can be determined by digesting the gene sequence linked by the above method with appropriate restriction enzymes, cloning the fragments into a plasmid such as pBluescript SK(-) (manufactured by Stratagene), carrying out analysis by using a generally used nucleotide sequence analyzing method such as the dideoxy method of Sanger *et al*. [*Proc. Natl. Acad Sci. USA,* 74, 5463 (1977)] or an automatic nucleotide sequence analyzer such as ABI PRISM 377DNA Sequencer (manufactured by PE Biosystems).

Whether or not the obtained cDNA encodes the full length amino acid sequences of the Fc fusion protein

containing a secretory signal sequence can be confirmed by deducing the full length amino acid sequence of the Fc fusion protein from the determined nucleotide sequence and comparing it with the amino acid sequence of interest.

(3) Stable production of Fc fusion protein

A transformant capable of stably producing an Fc fusion protein can be obtained by introducing the Fc fusion protein expression vector described in the item (1) into an appropriate animal cell.
The method for introducing the Fc fusion protein expression vector into an animal cell include electroporation [Japanese Published Unexamined Patent Application No. 257891/90, *Cytotechnology*, 3, 133 (1990)] and the like.
As the animal cell into which the Fc fusion protein expression vector is introduced, any cell can be used, so long as it is an animal cell which can produce the Fc fusion protein.
Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a syrian hamster kidney, a human myeloma cell such as Namalwa cell, and the like. A Chinese hamster ovary cell CHO/DG44 cell, a rat myeloma YB2/0 cell and the host cells used in the method of the present invention described in the item 1 are preferred.
A transformant introduced with the Fc fusion protein expression vector and capable of stably producing the Fc fusion protein expression vector can be selected by using a medium for animal cell culture comprising an agent such as G418 and the like in accordance with the method described in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum to these media, and the like. The Fc fusion protein can be produced and accumulated in the culture supernatant by culturing the obtained transformant in a medium. The amount of the Fc fusion protein produced and the antigen binding activity of the Fc fusion protein in the culture supernatant can be measured by a method such as ELISA. Also, the amount of the Fc fusion protein produced by the transformant can be increased by using a *dhfr* gene amplification system in accordance with the method described in Japanese Published Unexamined Patent Application No. 257891/90.
The Fc fusion protein can be purified from a culture supernatant culturing the transformant by using a protein A column or a protein G column (*Antibodies,* Chapter 8; *Monoclonal Antibodies*). In addition, purification methods generally used for purifying proteins can also be used. For example, the purification can be carried out through the combination of a gel filtration, an ion exchange chromatography and an ultrafiltration. The molecular weight as a whole of the purified Fc fusion protein molecule can be measured by SDS-PAGE [*Nature,* 227, 680 (1970)], Western blotting (*Antibodies,* Chapter 12; *Monoclonal Antibodies*) or the like.
Thus, methods for producing an antibody composition using an animal cell as the host cell have been described, but, as described above, it can also be produced by yeast, an insect cell, a plant cell, an animal individual or a plant individual by similar methods of the animal cell.
When the host cell is capable of expressing the antibody molecule, the antibody composition of the present invention can be produced by preparing the cell capable of expressing an antibody molecule according to the method described in the above item 1, culturing the cell, and recovering the antibody composition of interest.

4. Activity evaluation of antibody composition

As the method for measuring the amount of the protein in purified antibody composition, its binding activity to an antigen and its effector function, the known method described in *Monoclonal Antibodies, Antibody Engineering* or the like can be used.
For example, in the case where the antibody composition is a humanized antibody, the binding activity to an antigen and the binding activity to an antigen-positive cultured clone can be measured by methods such as ELISA, an immunofluorescent method [*Cancer Immunol. Immunother*., 36, 373 (1993)] and the like. The cytotoxic activity against an antigen-positive cultured clone can be evaluated by measuring CDC activity, ADCC activity [*Cancer Immunol. Immunother*., 36, 373 (1993)] and the like.
Therapeutic effects of different agents can be compared by an *in vivo* test using a disease model which uses an experimental animal such as mouse, rat, hamster, guinea pig, rabbit, dog, pig or monkey. In addition, the effects can also be compared by an *in vitro* cytotoxic activity measurement using a cell relating to diseases or an established cell thereof as the target.
The *in vivo* test can be carried out by transplanting a target cell such as a cell relating to diseases or an established cell line thereof, into the body of an experimental animal, administering each agent, for example, intraperitoneally, intravenously or subcutaneously, and observing the morbid state of the experimental animal. For example,

therapeutic effect of an agent can be examined by measuring growth of a tumor, survived days of an experimental animal, a blood component concentration of the agent, weight of an organ and the like.

The *in vitro* cytotoxic activity can be obtained by measuring ADCC activity, CDC activity and the like.

5. Analysis of sugar chains of antibody molecule expressed in various cells

The sugar chain structure binding to an antibody molecule expressed in various cells can be analyzed in accordance with the general analysis of the sugar chain structure of a glycoprotein. For example, the sugar chain which is bound to IgG molecule comprises a neutral sugar such as galactose, mannose, fucose, an amino sugar such as *N*-acetylglucosamine and an acidic sugar such as sialic acid, and can be analyzed by a method such as a sugar chain structure analysis by using sugar composition analysis, two dimensional sugar chain mapping or the like.

(1) Analysis of neutral sugar and amino sugar compositions

The sugar chain composition binding to an antibody molecule can be analyzed by carrying out acid hydrolysis of sugar chains with trifluoroacetic acid or the like to release a neutral sugar or an amino sugar and measuring the composition ratio.

Examples include a method by using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr.*, 6, 1577 (1983)].

The composition ratio can also be analyzed by a fluorescence labeling method by using 2-aminopyridine. Specifically, the composition ratio can be calculated in accordance with a known method [*Agric. Biol. Chem.*, 55(1), 283-284 (1991)] by labeling an acid-hydrolyzed sample with a fluorescence by 2-aminopyridylation and then analyzing the composition by HPLC.

(2) Analysis of sugar chain structure

The sugar chain structure binding to an antibody molecule can be analyzed by the two dimensional sugar chain mapping method [*Anal. Biochem.,* 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with such results of known sugar chains.

Specifically, sugar chains are released from an antibody by subjecting the antibody to hydrazinolysis, and the released sugar chain are subjected to fluorescence labeling with 2-aminopyridine (hereinafter referred to as "PA") [*J. Biochem.*, 95, 197 (1984)], and then the sugar chains are separated from an excess PA-treating reagent by gel filtration, and subjected to reverse phase chromatography. Thereafter, each peak of the separated sugar chains are subjected to normal phase chromatography. From these results, the sugar chain structure can be deduced by plotting the results on a two dimensional sugar chain map and comparing them with the spots of a sugar chain standard (manufactured by Takara Shuzo) or a literature [*Anal. Biochem.,* 171, 73 (1988)].

The structure deduced by the two dimensional sugar chain mapping method can be confirmed by further carrying out mass spectrometry such as MALDI-TOF-MS of each sugar chain.

6. Immunological determination method for identifying the sugar chain structure binding to antibody molecule

An antibody composition comprises an antibody molecule in which different sugar chains are bound to the Fc region of the antibody are different in structure. The antibody composition included as an active ingredient in the therapeutic agent of the present invention, in which the ratio of sugar chains in which 1-position of fucose is not bound to 6-position of *N*-acetylglucosamine in the reducing end through $\alpha$ bond to the total complex *N*-glycoside-linked sugar chains is 20% or more, has high ADCC activity. The antibody composition can be identified by using the method for analyzing the sugar chain structure binding to an antibody molecule described in the item 5. Also, it can be identified by an immunological determination method using a lectin.

The sugar chain structure binding to an antibody molecule can be identified by the immunological determination method using a lectin in accordance with the known immunological determination method such as Western staining, IRA (radioimmunoassay), VIA (viroimmunoassay), EIA (enzymoimmunoassay), FIA (fluoroimmunoassay) or MIA (metalloimmunoassay) described in literatures [*Monoclonal Antibodies: Principles and Applications,* Wiley-Liss, Inc. (1995); *Immunoassay (Koso Meneki Sokuteiho)*, 3rd Ed., Igakushoin (1987); *Enzyme Antibody Method (Koso*

*Kotaiho),* Revised Edition, Gakusai Kikaku (1985)] and the like.

A lectin which recognizes the sugar chain structure binding to an antibody molecule comprised in an antibody composition is labeled, and the labeled lectin is allowed to react with a sample, antibody composition. Then, the amount of the complex of the labeled lectin with the antibody molecule is measured.

The lectin used for identifying the sugar chain structure binding to an antibody molecule includes WGA (wheat-germ agglutinin derived from *T. vulgaris),* ConA (cocanavalin A derived from *C. ensiformis*), RIC (toxin derived from *R. communis*), L-PHA (leucoagglutinin derived from *P. vulgaris*), LCA (lentil agglutinin derived from *L. culinaris*), PSA (pea lectin derived from *P. sativum*), AAL (*Aleuria aurantia* lectin), ACL *(Amaranthus caudatus* lectin), BPL (*Bauhinia purpurea* lectin), DSL (*Datura stramonium* lectin), DBA (*Dolichos biflorus* agglutinin), EBL (elderberry balk lectin), ECL (*Erythrina cristagalli* lectin), EEL (*Euonymus eoropaeus* lectin), GNL (*Galanthus nivalis* lectin), GSL (*Griffonia simplicifolia* lectin), HPA (*Helix pomatia* agglutinin), HHL (*Hippeastrum* hybrid lectin), Jacalin, LTL (*Lotus tetragonolobus* lectin), LEL (*Lycopersicon esculentum* lectin), MAL (*Maackia amurensis* lectin), MPL (*Maclura pomifera* lectin), NPL (*Narcissus pseudonarcissus* lectin), PNA (peanut agglutinin), E-PHA (*Phaseolus vulgaris* erythroagglutinin), PTL (*Psophocarpus tetragonolobus* lectin), RCA (*Ricinus communis* agglutinin), STL (*Solanum tuberosum* lectin), SJA (*Sophora japonica* agglutinin), SBA (soybean agglutinin), UEA (*Ulex europaeus* agglutinin), VVL (*Vicia villosa* lectin) and WFA (*Wisteria floribunda* agglutinin).

In order to identify the antibody composition of the present invention, the sugar chain structure can be analyzed in detail by using a lectin which specifically recognizes a sugar chain structure wherein fucose is bound to the *N*-acetylglucosamine in the reducing end in the complex *N*-glycoside-linked sugar chain. Examples include *Lens culinaris* lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

7. Method for screening a patient to which an antibody medicament produced by a lectin-resistant cell is effective

An example of the method for screening a patient to which the medicament of the present invention is effective is a method wherein an effector cell is collected from the body of a patient and allowed to contact with the medicament of the present invention or a conventional antibody medicament, the amount or activity of the medicament bound to the effector cell of the medicament of the present invention or the conventional antibody medicament reacted with the effector cell is measured, and the bound amount or activity shown by the conventional antibody medicament is compared with the bound amount or activity shown by the medicament of the present invention, thereby selecting a patient having a lower amount or activity of the effector cell-bound medicament which comprises an antibody composition produced by a cell unresistant to a lectin which recognizes a sugar chain in which 1-position of fucose is bound to 6-position of N-acetylglucosamine in the reducing end through $\alpha$-bond in a complex N-glycoside-linked sugar chain.

The method for collecting an effector cell from a patient includes a surgical technique, collection of body fluids and the like. If necessary, the effector cell may be concentrated or purified from the collected sample by an immunological technique, or a specific gravity separation, adsorption or the like method.

As the method for measuring the amount of a medicament bound to the effector cell, it may be any method, so long as it can detect antibody molecules. Examples include immunological assay methods such as tissue immunostaining, enzyme immunoassay, radioimmunoassay, flow cytometry, Scatchard plot method, immunoblotting, aggregation reaction, complement fixation reaction, hemolysis reaction, precipitation reaction, colloidal gold method and chromatography.

As the method for measuring the activity induced by a medicament bound to an effector cell, it may be any method, so long as it can detect the activity of antibody molecules. Examples include an ADCC activity measuring method, a CDC activity measuring method, a method for measuring expression of a cytotoxic molecule, a method for measuring intracellular signal transduction of the human Fc$\gamma$ receptor IIIa, and a method for measuring a molecule whose expression changes in a human Fc$\gamma$ receptor IIIa-expressing effector cell.

The method for measuring ADCC activity is a method in which an effector cell to which the antibody medicament of the present invention is bound is allowed to contact with an antigen-expressing target cell, and injury of the target cell is detected.

The target cell includes an established cell line, a red blood cell to which an antigen is adhered and a target cell collected from a patient.

The method for detecting injury of a target cell include immunological assay methods such as a method in which a target cell is labeled with a radioisotope, a pigment, a fluorescent material or the like, and a method in which a biological activity of an enzyme or amount of a pigment possessed by an unlabeled target cell is measured.

The method for measuring CDC activity is a method in which a complement to which the antibody medicament of the present invention is bound is allowed to contact with an antigen-expressing target cell, and injury of the target cell is detected.

The target cell includes an established cell line, a red blood cell to which an antigen is adhered and a target cell collected from a patient.

The method for measuring expression of a cytotoxic molecule is a method in which a substance produced from an effector cell to which the antibody medicament of the present invention is bound is measured.

The substance produced from an effector cell includes perforin, granzyme, active oxygen, nitrogen monoxide, granulysine, FasL and the like.

The method for measuring a substance includes an immunological assay which uses an antibody capably of specifically reacting with the substance and a bioassay which measures cytotoxic activity of the substance released into the extracellular moiety.

The method for measuring signal transduction of the human Fcγ receptor IIIa in an effector cell is a method in which phosphorylation of a signal transduction molecule in an effector cell to which the antibody medicament of the present invention is bound is detected.

The signal transduction molecule in effector cells includes γ chain, ζ chain, ZAP-70, PLC-γ and the like.

The method for measuring phosphorylation of a signal transduction molecule downstream of the human Fcγ receptor IIIa includes Western blotting, immunoprecipitation and the like.

As the method for measuring molecule whose expression changes in a human Fcγ receptor IIIa-expressing effector cell, a method for measuring the expression of a molecule on an effector cell to which the antibody medicament of the present invention is bound can be used.

The molecule whose expression changes in an effector cell includes CD 69, CD 25, CD 71 and the like expressed on the activated NK cell.

The method for measuring expression of a molecule on the effector cell include flow cytometry and immune staining methods such as tissue immunostaining.

The screening method of the present invention is particularly useful in screening a patient in which the amino acid residue at position 176 from the N-terminal methionine of the human FcγRIIIa signal sequence is phenylalanine, for which the medicament of the present invention is most effective.

In addition, by applying the medicament of the present invention to a patient selected by the screening method of the present invention, the patient can be effectively treated. It is useful to patients who cannot be treated by conventional medicaments.

The screening method of a patient for applying the medicament of the present invention can be carried out by the following method (a) or (b), in addition to the above-described method:

(a) a method for selecting a patient based on the nucleotide sequence of a patient's gene encoding the amino acid at position 176 from the N-terminal methionine of FcγRIIIa signal sequence;
(b) a method for selecting a patient based on an immunological technique, by collecting an effector cell of a patient and using an antibody capable of specifically recognizing a polymorphism of the amino acid at position 176 from the N-terminal methionine of the human FcγRIIIa signal sequence.

The method (a) includes a method in which genome is prepared by collecting cells from a patient and using a commercially available genomic DNA extraction kit or the like, and the nucleotide sequence of a gene in the genome encoding the amino acid at position 176 from the N-terminal methionine of the human FcγRIIIa signal sequence is analyzed, and a method in which only a partial region of the genome containing said polymorphism is amplified by using PCR, and then the nucleotide sequence of the amplified DNA fragment is analyzed. Specifically, it can be determined by the latter method that a patient has a phenylalanine homo type allele when, in analyzing the nucleotide sequence, the first nucleotide of the codon encoding the amino acid at position 176 from the N-terminal methionine of the human FcγRIIIa signal sequence is T, or a valine homo type when it is G or a hetero type when it is a mixed signal of T and G.

In addition, instead of analyzing the nucleotide sequence, the polymorphism can also be determined by treating the amplified fragment obtained by PCR with a restriction enzyme which recognizes only the gene sequence coding for one of the polymorphisms, and observing electrophoresis pattern of the amplified fragment after the treatment. Specifically, since the amplified fragment prepared from a patient having FcγRIIIa in which the amino acid at position 176 from the N-terminal sequence of the human FcγRIIIa is phenylalanine is not digested with a restriction enzyme *Nla*III, while that of a patient wherein it is valine is digested with *Nla*III, it can be distinguished whether the patient is a phenylalanine homo type, valine homo type or a hetero type of both, by determining whether the amplified fragment is digested or not digested or shows a mixed pattern of both by *Nla*III.

The method (b) includes a method in which an effector cell of a patient is stained by using an antibody capable of specifically recognizing polymorphism of the amino acid at position 176 from the N-terminal methionine of the human FcγRIIIa signal sequence, and the result is determined by using a flow cytometry or a immune staining method such as tissue immunostaining. The method for collecting an effector cell from a patient includes a surgical technique, collection from a body fluid and the like.

8. Method for treating patient using antibody medicament produced by lectin-resistant cell

As the method for treating a patient by using the medicament of the present invention, the medicament can be administered as a therapeutic agent alone, but generally, it is preferred to provide it as a pharmaceutical formulation produced by an appropriate method well known in the technical field of pharmaceutical, by mixing it with one or more pharmaceutically acceptable carriers.

It is preferred to select a route of administration which is most effective in treatment. Examples include oral administration and parenteral administration, such as buccal, tracheal, rectal, subcutaneous, intramuscular and intravenous adminstrations. In the case of an antibody preparation, intravenous administration is preferred.

The dosage form includes sprays, capsules, tablets, granules, syrups, emulsions, suppositories, injections, ointments, tapes and the like.

The pharmaceutical preparation suitable for oral administration include emulsions, syrups, capsules, tablets, powders, granules and the like.

Liquid preparations such as emulsions and syrups can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be prepared by using, as additives, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerine; and the like.

The pharmaceutical preparation suitable for parenteral administration includes injections, suppositories, sprays and the like.

Injections may be prepared by using a carrier such as a salt solution, a glucose solution or a mixture thereof. Also, powdered injections can be prepared by freeze-drying the antibody composition in the usual way and adding sodium chloride thereto.

Suppositories may be prepared by using a carrier such as cacao butter, hydrogenated fat or carboxylic acid.

Also, sprays may be prepared by using the antibody composition as such or using a carrier which does not stimulate the buccal or airway mucous membrane of the patient and can facilitate absorption of the antibody composition by dispersing it as fine particles.

The carrier includes lactose, glycerine and the like. Depending on the properties of the antibody composition and the carrier, it is possible to produce pharmaceutical preparations such as aerosols and dry powders. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

As the method for treating a patient by using the medicament of the present invention, it is preferred to select a patient to which the medicament of the present invention is effective in advance according to the method described in the item 6, followed by administering the medicament shown below to the selected patient.

Particularly, high therapeutic effects can be obtained by selecting a patient having a human Fcγ receptor IIIa in which an amino acid residue at position 176 from the N-terminal methionine in the signal sequence is phenylalanine and administering the medicament of the present invention to the patient.

The present invention will be described below in detail based on Examples; however, Examples are only simple illustrations, and the scope of the present invention is not limited thereto.

BRIEF DESCRIPTION OF THE DRAWINGS

Fig. 1 shows binding activities of two types of purified anti-GD3 chimeric antibodies to GD3, measured by changing the antibody concentration. The ordinate and the abscissa show the binding activity to GD3 and the antibody concentration, respectively. ○ and ● show the activities of YB2/0-GD3 chimeric antibody and CHO-GD3 chimeric antibody, respectively.

Fig. 2 shows ADCC activities of two types of purified anti-GD3 chimeric antibodies to a human melanoma cell line G-361. The ordinate and the abscissa show the cytotoxic activity and the antibody concentration, respectively. ○ and ● show the activities of YB2/0-GD3 chimeric antibody and CHO-GD3 chimeric antibody, respectively.

Fig. 3 shows binding activities of two types of purified anti-CCR4 chimeric antibodies to a human CCR4 peptide, measured by changing the antibody concentration. The ordinate and the abscissa show the binding activity to the human CCR4 peptide and the antibody concentration, respectively. ○ and ● show the activities of KM2760-1 and KM3060, respectively.

Fig. 4 shows ADCC activities of two types of purified anti-CCR4 chimeric antibodies to a human CCR4-expressing cell CCR4/EL-4. The ordinate and the abscissa show the cytotoxic activity and the antibody concentration, respectively. o and ● show the activities of KM2760-1 and KM3060, respectively.

Fig. 5 shows the results of binding activities of purified anti-CD20 chimeric antibody KM3065 and Rituxan™ to a human CD20-expressing cell Raji cell, measured by changing the antibody concentration by using an immunofluorescent method. The ordinate and the abscissa show the relative fluorescence intensity at each concentration and the antibody concentration, respectively. ■ and ○ show the activities of Rituxan™ and KM3065, respectively.

Fig. 6 shows ADCC activities of purified anti-CD20 chimeric antibody KM3065 and Rituxan™ to a human CD20-expressing cell. In A, B and C, Raji cell, Ramos cell and WIL2-S cell, respectively, are used as target cells. The ordinate and the abscissa show the cytotoxic activity and the antibody concentration, respectively. ■ and ○ show the activities of Rituxan™ and KM3065, respectively.

Fig. 7 shows binding activities of various anti-GD3 chimeric antibodies to shFcγRIIIa(F) and shFcγRIIIa(V). The ordinate and the abscissa show the binding activity and the antibody concentration, respectively. ○, □, ● and ■ show the activities of YB2/0-GD3 chimeric antibody to shFcγRIIIa(F), YB2/0-GD3 chimeric antibody to shFcγRIIIa (V), CHO-GD3 chimeric antibody to shFcγRIIIa(F), and CHO-GD3 chimeric antibody to shFcγRIIIa(V), respectively.

Fig. 8 shows binding activities of various anti-CCR4 chimeric antibodies to shFcγRIIIa(F) and shFcγRIIIa(V). The ordinate and the abscissa show the binding activity and the antibody concentration, respectively. ○, □, ● and ■ show the activities of KM2760-1 to shFcγRIIIa(F), KM2760-1 to shFcγRIIIa(V), KM3060 to shFcγRIIIa(F), and KM3060 to shFcγRIIIa(V), respectively.

Fig. 9 shows binding activities of various anti-FGF-8 chimeric antibodies to shFcγRIIIa(F) and shFcγRIIIa(V). The ordinate and the abscissa show the binding activity and the antibody concentration, respectively. o, □, ● and ■ show the activities of YB2/0-FGF8 chimeric antibody to shFcγRIIIa(F), YB2/0-FGF8 chimeric antibody to shFcγRIIIa (V), CHO-FGF8 chimeric antibody to shFcγRIIIa(F), and CHO-FGF8 chimeric antibody to shFcγRIIIa(V), respectively.

Fig. 10 shows binding activities of various anti-CD20 chimeric antibodies to shFcγRIIIa(F) and shFcγRIIIa(V). The ordinate and the abscissa show the binding activity and the antibody concentration, respectively. Fig. 10A shows the binding activity to shFcγRIIIa(F) and Fig. 10B shows the binding activity to shFcγRIIIa(V). o and ■ show the binding activities of KM3065 and Rituxan™, respectively.

Fig. 11 shows binding activities of various anti-CCR4 chimeric antibodies to shFcγRIIIa. The ordinate and the abscissa show the binding activity and the antibody concentration, respectively. Fig. 11A shows the binding activity of LCArCHO-CCR4 antibody (48%) and Fig. 11B shows the binding activity of KM3060. ● and ○ show the binding activities to shFcγRIIIa(F) and shFcγRIIIa(V), respectively.

Fig. 12 shows binding activities of various anti-GD3 chimeric antibodies to shFcγRIIIa. The ordinate and the abscissa show the binding activity and the antibody concentration, respectively. Fig. 12A shows the binding activity of LCArCHO-GD3 antibody (42%), Fig. 12B shows the binding activity of LCArCHO-GD3 chimeric antibody (80%) and Fig. 12C shows the binding activity of CHO-GD3 chimeric antibody. ● and ○ show the binding activities to shFcγRIIIa(F) and shFcγRIIIa(V), respectively.

Fig. 13 shows the results of binding activity of a chimeric antibody to shFcγRIIIa, measured by using BIAcore 2000. As a representative example, results using an anti-CCR4 chimeric antibody KM2760-1 and 10 μg/ml shFcγRIIIa (F) solution were shown.

Fig. 14 shows the results of binding activities of various anti-CCR4 chimeric antibodies to shFcγRIIIa, measured by using BIAcore 2000. Binding and dissociation reaction parts of each of shFcγRIIIa(V) and shFcγRIIIa(F) were shown. Fig. 14A, Fig. 14B, Fig. 14C, and Fig. 14D show results of KM2760-1 to shFcγRIIIa(F), KM2760-1 to shFcγRIIIa(V), KM3060 to shFcγRIIIa(F), and KM3060 to shFcγRIIIa(V), respectively.

Fig. 15 shows the results of binding activities of various anti-FGF8 chimeric antibodies to shFcγRIIIa, measured by using BIAcore 2000. Binding and dissociation reaction parts of each of shFcγRIIIa(V) and shFcγRIIIa(F) were shown. Fig. 15A, Fig. 15B, Fig. 15C and Fig. 15D show results of YB2/0-FGFB chimeric antibody to shFcγRIIIa (F), YB2/0-FGF8 chimeric antibody to shFcγRIIIa(V), CHO-FGF8 chimeric antibody to shFcγRIIIa(F), and CHO-FGF8 chimeric antibody to shFcγRIIIa(V), respectively.

Fig. 16 shows the results of binding activities of various anti-CD20 chimeric antibodies to shFcγRIIIa, measured by using BIAcore 2000. Binding and dissociation reaction parts of each of shFcγRIIIa(V) and shFcγRIIIa(F) were shown. Fig. 16A, Fig. 16B, Fig. 16C and Fig. 16D show results of KM3065 to shFcγRIIIa(F), KM3065 to shFcγRIIIa (V), Rituxan™ to shFcγRIIIa(F), and Rituxan™ to shFcγRIIIa(V), respectively.

Fig. 17 shows an analysis example of DNA sequencer of the polymorphism of the amino acid at position 176 of

FcγRIIIa of healthy donors. From the upper row drawing, signals of Phe/Phe type, Phe/Val type and Val/Val type are respectively shown. The arrow shows the position of the first nucleotide of the codon encoding the amino acid at position 176 having genetic polymorphism.

Fig. 18 shows ADCC activities per $10^4$ of NK cells when peripheral blood mononuclear cells of 20 donors were used as effecter cells. ● and ○ show the activities in which the chimeric antibody produced by CHO cell and the antibody produced by YB2/0 cell, respectively, were added at 10 ng/ml. Dotted lines show the reaction of the same donor.

Fig. 19 shows binding activities of an antibody to human peripheral blood-derived NK cells by using an immunofluorescent method. The abscissa and the ordinate show the fluorescence intensity and the cell number, respectively. Fig. 19A and Fig. 19B show results when an anti-CCR4 chimeric antibody and an anti-CD20 chimeric antibody, respectively, are allowed to react, and each antibody is shown in the drawings.

Fig. 20 shows expression intensity of CD56-positive cell, i.e., CD69 on the surface of NK cell, when human peripheral blood-derived NK cells are allowed to react with an antibody and antigen-expressing cells by using an immunofluorescent method. The abscissa and the ordinate show the fluorescence intensity and the cell number, respectively. Fig.20A, Fig.20B and Fig. 20C show results when an anti-CCR4 chimeric antibody was reacted at 10 μg/ml for 4 hours, an anti-CCR4 chimeric antibody was reacted at 10 μg/ml for 24 hours, and an anti-CD20 chimeric antibody was reacted at 0.1 μg/ml for 21 hours, respectively, and each antibody and reaction time are shown in the drawings.

Fig. 21 shows construction steps of plasmid pKANTEX1334H and plasmid pKANTEX1334.

Fig. 22 shows binding activities of two types of purified anti-FGF8 chimeric antibodies to a human FGF-8 peptide, measured by changing the antibody concentration. The ordinate and the abscissa show the binding activity with a human FGF-8 peptide and the antibody concentration, respectively. ○ and ● show the activities of YB2/0-FGF8 chimeric antibody and CHO-FGF8 chimeric antibody, respectively.

Fig. 23 shows a construction step of plasmid pBS-2B8L.

Fig. 24 shows a construction step of plasmid pBS-2B8Hm.

Fig. 25 shows a construction step of plasmid pKANTEX2B8P.

Fig. 26 shows results of ADCC activities of anti-CCR4 human chimeric antibodies produced by lectin-resistant clones. The ordinate and the abscissa show the cytotoxic activity and the antibody concentration, respectively. □, ■, ♦ and ▲ show the activities of antibodies produced by the clone 5-03, the clone CHO/CCR4-LCA, the clone CHO/CCR4-AAL and the clone CHO/CCR4-PHA, respectively.

Fig. 27 shows the results of evaluation of ADCC activities of anti-CCR4 human chimeric antibodies produced by lectin-resistant clones. The ordinate and the abscissa show the cytotoxic activity and the antibody concentration, respectively. □, Δ and ● show activities of antibodies produced by the clone YB2/0 (KM2760#58-35-16), the clone 5-03 and the clone CHO/CCR4-LCA, respectively.

Fig. 28 shows the results of evaluation of ADCC activities of anti-GD3 chimeric antibodies. The ordinate and the abscissa show the degree of the cytotoxic activity of the target cell calculated by the equation in the item 2 of Example 2 and the concentration of the anti-GD3 chimeric antibody in the reaction solution, respectively.

Fig. 29 are photographs showing electrophoresis patterns of SDS-PAGE of purified shFcγRIIIa(F) and shFcγRIIIa (V) under reducing conditions (using gradient gel from 4 to 15%). Lanes 1, 2 and M show electrophoresis patterns of shFcγRIIIa(F), shFcγRIIIa(V) and high molecular weight markers, respectively.

## BEST MODE FOE CARRYING OUT THE INVENTION

### Example 1

Activity evaluation of anti-GD3 chimeric antibody:

1. Binding activity of anti-GD3 chimeric antibody to GD3 (ELISA)

Binding activities of the two types of the purified anti-GD3 chimeric antibodies produced by various animal cells obtained in the item 3 of Reference Example 1 to GD3 were measured by the ELISA described in the item 2 of Reference Example 1.

Fig. 1 shows results of the examination of the binding activity measured by changing the concentration of the anti-GD3 chimeric antibody to be added. As shown in Fig. 1, the two types of the anti-GD3 chimeric antibodies showed almost the identical binding activity to GD3. The result shows that antigen binding activities of these antibodies are constant independently of the types of the antibody-producing animal cells.

2. *In vitro* antibody-dependent cell-mediated cytotoxic activity (ADCC activity) of anti-GD3 chimeric antibody

In order to evaluate *in vitro* antibody-dependent cell-mediated cytotoxic activity of the two types of the purified anti-GD3 chimeric antibodies produced by various animal cells obtained in the item 3 of Reference Example 1, ADCC activities were measured in accordance with the following method.

(1) Preparation of target cell solution

A human melanoma cell line G-361 (ATCC CRL 1424) was cultured in the RPMI1640-FBS(10) medium to prepare $1\times10^6$ cells, and the cells were radioisotope-labeled by reacting them with 3.7 MBq equivalents of a radioactive substance $Na_2{}^{51}CrO_4$ at 37°C for 1 hour. After the reaction, the cells were washed three times through their suspension in the RPMI1640-FBS(10) medium and centrifugation, resuspended in the medium and then allowed to react at 4°C for 30 minutes on ice for spontaneous dissolution of the radioactive substance. After centrifugation, the precipitate was adjusted to $2\times10^5$ cells/ml by adding 5 ml of the RPMI1640-FBS(10) medium and used as the target cell solution.

(2) Preparation of human effector cell solution

From a healthy donor, 50 ml of venous blood was collected, and gently mixed with 0.5 ml of heparin sodium (manufactured by Shimizu Pharmaceutical). The mixture was centrifuged to isolate a mononuclear cell layer using Lymphoprep (manufactured by Nycomed Pharma AS) in accordance with the manufacture's instructions. After washing with the RPMI1640-FBS(10) medium by centrifugation three times, the resulting precipitate was re-suspended to give a density of $2\times10^6$ cells/ml by using the medium and used as the effector cell solution.

(3) Measurement of ADCC activity

Into each well of a 96 well U-shaped bottom plate (manufactured by Falcon), 50 µl of the target cell solution prepared in the item 2(1) of Example 1 ($1\times10^4$ cells/well) was dispensed. Next, 100 µl of the effector cell solution prepared in the item 2(2) of Example 1 was added thereto ($2\times10^5$ cells/well, the ratio of effector cells to target cells becomes 20:1). Subsequently, each of the anti-GD3 chimeric antibodies was added at various concentrations, followed by reaction at 37°C for 4 hours. After the reaction, the plate was centrifuged, and the amount of $^{51}Cr$ in the supernatant was measured with a γ-counter. The amount of spontaneously released $^{51}Cr$ was calculated by the same operation using only the medium instead of the effector cell solution and the antibody solution, and measuring the amount of $^{51}Cr$ in the supernatant. The amount of total released $^{51}Cr$ was calculated by the same operation as above using only the medium instead of the antibody solution and adding 1 M hydrochloric acid instead of the effector cell solution, and measuring the amount of $^{51}Cr$ in the supernatant. The ADCC activity was calculated from the following equation (1):

$$\text{ADCC activity (\%)} = \frac{\text{amount of } {}^{51}\text{Cr in sample supernatant - spontaneously released amount of } {}^{51}\text{Cr}}{\text{total released amount of } {}^{51}\text{Cr - spontaneously released amount of } {}^{51}\text{Cr}} \times 100$$

(1)

The results are shown in Fig. 2. As shown in Fig. 2, the YB2/0-GD3 chimeric antibody had 100 times or more higher ADCC activity than the CHO-GD3 chimeric antibody. The results show that the antibody produced by the α1,6-fucose/lectin resistant cell has remarkably higher ADCC activity than the antibody produced by the α1,6-fucose/lectin unresistant cell.

3. Analysis of sugar chain bound to antibody molecule

Next, sugar chains bound to the Fc region of an antibody composition were analyzed according to the method of Example 5 in WO 00/61739. The result shows that the YB2/0-GD3 chimeric antibody and the CHO-GD3 chimeric antibody had contents of sugar chains bound to the Fc region of each antibody in which 1-position of fucose is not bound to 6-position of *N*-acetylglucosamine in the reducing end at 53% and 7%, respectively. The results show that the antibody produced by the α1,6-fucose/lectin resistant cell has high ADCC activity because of the high content of sugar chains bound to the Fc region of the antibody in which 1-position of fucose is not bound to 6-position of *N*-acetylglucosamine in the reducing end.

Example 2

Activity evaluation of anti-CCR4 chimeric antibody:

1. Binding activity of anti-CCR4 chimeric antibody to CCR4 partial peptide (ELISA)

Binding activities of the two types of the purified anti-CCR4 chimeric antibodies produced by various animal cells obtained in the item 3 of Reference Example 2 to a CCR4 partial peptide were measured by the ELISA shown in the item 2 of Reference Example 2.

Fig. 3 shows results of the examination of the binding activity measured by changing the concentration of the anti-CCR4 chimeric antibody to be added. As shown in Fig. 3, the two types of the anti-CCR4 chimeric antibodies showed the similar binding activity to the CCR4 partial peptide. The result shows that antigen binding activities of these antibodies are constant independently of the types of the antibody-producing animal cells in the same manner as the case of the anti-GD3 chimeric antibody.

2. *In vitro* antibody-dependent cell-medicated cytotoxic activity (ADC activity) of anti-CCR4 chimeric antibody

In order to evaluate *in vitro* ADCC activity of the two types of the purified anti-CCR4 chimeric antibodies produced by various animal cells obtained in the item 3 of Reference Example 2, ADCC activities were measured in accordance with the following method.

(1) Preparation of target cell suspension

Cells ($1.5\times10^6$) of a human CCR4-highly expressing cell, CCR4/EL-4 cell, described in WO01/64754 were prepared and a 5.55 MBq equivalent of a radioactive substance $Na_2{}^{51}CrO_4$ was added thereto, followed by reaction at 37°C for 1.5 hours to thereby label the cells with a radioisotope. After the reaction, the cells were washed three times by suspension in a medium and subsequent centrifugation, resuspended in the medium and then incubated at 4°C for 30 minutes on ice for spontaneous dissociation of the radioactive substance. After centrifugation, the cells were adjusted to give a density of $2\times10^5$ cells/ml by adding 7.5 ml of the medium and used as a target cell suspension.

(2) Preparation of human effector cell suspension

From a healthy donor, 60 ml of peripheral blood was collected, 0.6 ml of heparin sodium (manufactured by Shimizu Pharmaceutical) was added thereto, followed by gently mixing. The mixture was centrifuged (800 g, 20 minutes) to isolate a mononuclear cell layer using Lymphoprep (manufactured by AXIS SHIELD) in accordance with the manufacture's instructions. After washing with the RPMI1640-FBS(10) medium by centrifugation three times, the resulting precipitate was resuspended to give a density of $5\times10^6$ cells/ml by using the medium and used as the effector cell solution.

(3) Measurement of ADCC activity

Into each well of a 96 well U-shaped bottom plate (manufactured by Falcon), 50 μl of the target cell solution prepared in the item 2(1) of Example 2 ($1\times10^4$ cells/well) was dispensed. Next, 100 μl of the effector cell solution prepared in the item 2(2) of Example 2 was added thereto ($5\times10^5$ cells/well, the ratio of effector cells to target cells becomes 50:1). Subsequently, each of the anti-CCR4 chimeric antibodies was added at various concentrations, followed by reaction at 37°C for 4 hours. After the reaction, the plate was centrifuged, and the amount of $^{51}Cr$ in the supernatant was measured with a γ-counter. The amount of spontaneously released $^{51}Cr$ was calculated by the same operation as above using only the medium instead of the effector cell solution and the antibody solution, and measuring the amount of $^{51}Cr$ in the supernatant. The amount of total released $^{51}Cr$ was calculated by the same operation as above by adding 1 mol/l hydrochloric acid instead of the antibody solution and the effector cell solution, and measuring the amount of $^{51}Cr$ in the supernatant. The ADCC activity was calculated from the above-described equation (1).

The results are shown in Fig. 4. As shown in Fig. 4, only about 30% of the cytotoxic activity was recognized in KM3060 even at the highest antibody concentration of 10 μg/ml. On the other hand, KM2760-1 showed an almost constant high value of about 80% at an antibody concentration of 0.01 μg/ml or more. Furthermore, the antibody concentration which had cytotoxic activity of about 30% similar to that of KM3060 was about 0.0003 μg/ml. That is, the difference of the concentrations was $3\times10^4$-fold or more. The above results show that the YB2/0 cell-derived antibody has high ADCC activity in the same manner as the result in the anti-GD3 chimeric antibody. The above results show that the antibody produced by the α1,6-fucose/lectin resistant cell has remarkably higher ADCC activity than the antibody produced by the α1,6-fucose/lectin unresistant cell.

3. Analysis of sugar chain bound to antibody molecule

Sugar chains bound to the Fc region of the antibody composition were analyzed according to the method of Example 5 in WO 00/61739. The result shows that the YB2/0-derived antibody and the CHO-derived antibody had contents of sugar chains bound to the Fc region of each antibody in which 1-position of fucose is not bound to 6-position of *N*-acetylglucosamine in the reducing end at 87% and 8%, respectively. The results show that the antibody produced by the α1,6-fucose/lectin resistant cell has high ADCC activity because of the high content of sugar chains bound to the Fc region of the antibody in which 1-position of fucose is not bound to 6-position of *N*-acetylglucosamine in the reducing end.

Example 3

Evaluation of activity of an anti-CD20 chimeric antibody:

(1) Binding activity of anti-CD20 chimeric antibody on CD20-expressing cell (immunofluorescence technique)

Binding activity of the purified anti-CD20 chimeric antibody obtained in the item 3 of Reference Example 4 was evaluated by a immunofluorescence technique using a flow cytometer. A human lymphoma cell line Raji cell (JCRB 9012), which was a CD20-positive cell was dispensed at $2\times10^5$ cells into a 96 well U-shape plate (manufactured by Falcon). An antibody solution (a concentration of 0.039 to 40 μg/ml) prepared by diluting the anti-CD20 chimeric antibody with an FACS buffer (1% BSA-PBS, 0.02% EDTA, 0.05% $NaN_3$) was added thereto at 50 μl/well and allowed to react on ice under a shade for 30 minutes. After washing twice with the FACS buffer at 200 μl/well, a solution prepared by diluting a PE-labeled anti-human IgG antibody (manufactured by Coulter) 100-folds with FACS buffer was added thereto at 50 μl/well. After the reaction on ice under a shade for 30 minutes, the well were washed three times at 200 μl/well, the cells were finally suspended in 500 μl to measure the fluorescence intensity by a flow cytometer. The results are shown in Fig. 5. Increase in the fluorescence intensity depending on the antibody concentration was found in both KM3065 and Rituxan™, and it was confirmed that they show almost the identical binding activity.

(2) *In vitro* cytotoxic activity (ADCC activity) of anti-CD20 chimeric antibody

In order to evaluate *in vitro* cytotoxic activity of the purified anti-CD20 chimeric antibodies obtained in the item 3 of Reference Example 4, the ADCC activity was measured in accordance with the following method.

(a) Preparation of target cell solution

A human B lymphocyte cultured cell line WIL2-S cell (ATCC CRL8885), Ramos cell (ATCC CRL1596) or Raji cell (JCRB9012) cultured in RPMI1640-FCS(10) medium [RPMI1640 medium containing 10% FCS (manufactured by GIBCO BRL)] was washed with RPMI1640-FCS(5) medium [RPIM1640 medium containing 5% FCS (manufactured by GIBCO BRL)] by centrifugation and suspension, and prepared to give a density of $2\times10^5$ cells/ml with RPMI1640-FCS(5) medium as the target cell solution.

(b) Preparation of effector cell solution

From a healthy donor, 50 ml of venous blood was collected, and gently mixed with 0.5 ml of heparin sodium (manufactured by Shimizu Pharmaceutical). The mixture was centrifuged to isolate a mononuclear cell layer using Lymphoprep (manufactured by AXIS SHIELD) in accordance with the manufacture's instructions. After washing with the RPMI1640-FBS(10) medium by centrifugation three times, the resulting precipitate was re-suspended to give a density of $2\times10^6$ cells/ml using the medium and used as the effector cell solution.

(c) Measurement of ADCC activity

Into each well of a 96 well U-shaped bottom plate (manufactured by Falcon), 50 μl of the target cell solution prepared in the item (a) ($1 \times 10^4$ cells/well) was dispensed. Next, 50 μl of the effector cell solution prepared in the item (b) was added thereto ($2 \times 10^5$ cells/well, the ratio of effector cells to target cells becomes 20:1). Subsequently, each of the anti-CD20 chimeric antibodies was added to give a final concentration from 0.3 to 3000 ng/ml and a total amount of 150 μl, followed by reaction at 37°C for 4 hours. After the reaction, the plate was centrifuged, and the lactic acid

dehydrogenase (LDH) activity in the supernatant was measured by obtaining absorbance data using CytoTox96 Non-Radioactive Cytotoxicity Assay (manufactured by Promega) according to the attached manufacture's instructions. Absorbance data at spontaneously release from target cells were obtained by using the medium alone without using the effector cell solution and the antibody solution, and absorbance data at spontaneously release from effector cells were obtained by using the medium alone without using the target cell solution and the antibody solution, in the same manner as above. Regarding absorbance data of the total released target cells, the same procedures as above were carried out by using the medium alone without using the antibody solution and the effector cell solution, adding 15 μL of 9% Triton X-100 solution 45 minutes before completion of the reaction, and measuring the LDH activity of the supernatant. The ADCC activity was carried out by the following equation:

$$\text{Cytotoxic activity (\%)} = \frac{\left[\text{Absorbance of the sample}\right] - \left[\text{Absorbance at spontanously release from effector cells}\right] - \left[\text{Absorbance at spontanously release from target cells}\right]}{\left[\text{Absorbance at total release from target cells}\right] - \left[\text{Absorbance at spontanously release from target cells}\right]} \times 100$$

Fig. 6 shows results in which the three clones were used as the target. Fig. 6A, Fig. 6B and Fig. 6C show the results using Raji cell (JCRB9012), Ramos cell (ATCC CRL1596) and WIL2-S cell (ATCC CRL8885), respectively. As shown in Fig. 6, KM3065 has higher ADCC activity than Rituxan™ at all antibody concentrations, and has the highest maximum cytotoxic activity value. The above results show that the antibody produced by the α1,6-fucose/lectin resistant cell has remarkably higher ADCC activity than the antibody produced by the α1,6-fucose/lectin unresistant cell.

5. Analysis of sugar chain bound to antibody molecule

Sugar chains bound to the Fc region of the antibody composition were analyzed according to the method of Example 5 in WO 00/61739. The result shows that KM3065 and the CHO-GD3 antibody had contents of sugar chains bound to the Fc region of each antibody in which 1-position of fucose is not bound to 6-position of *N*-acetylglucosamine in the reducing end at 96% and 6%, respectively. The results show that the antibody produced by the α1,6-fucose/lectin resistant cell has high ADCC activity because the content of sugar chains bound to the Fc region of the antibody in which 1-position of fucose is not bound to 6-position of *N*-acetylglucosamine in the reducing end.

Example 4

Evaluation of binding activity of various chimeric antibodies to shFcγRIIIa(F) and shFcγRIIIa(V) (ELISA)

Experiments were carried out with ELISA by examining the influence of the polymorphism of the amino acids at position 176 from the N terminal methionine in human FcγRIIIa on the binding activity of the antibody produced by the α1,6-fucose/lectin-resistant cell to human FcγRIIIa.

1. Evaluation of binding activity of anti-GD3 chimeric antibodies

The binding activity of the two types of the anti-GD3 chimeric antibodies, YB2/0-GD3 chimeric antibody and CHO-GD3 chimeric antibody described in the item 3 of Reference Example 1, to shFcγRIIIa(F) and shFcγRIIIa(V) described in the item 4 of Reference Example 6 was measured by ELISA as follows.

According to the method described in the item 2 of Reference Example 1, GD3 was immobilized at 200 pmol/well on a 96 well plate for ELISA (manufactured by Greiner). 1% BSA-PBS was added at 100 μl/well and allowed to react at room temperature for 1 hour to block the remaining active groups. After washing each well with Tween-PBS, a solution of each anti-GD3 chimeric antibody diluted with 1% BSA-PBS was added at 50 μl/well and allowed to react at room temperature for 1 hour. After the reaction and subsequent washing of each well with Tween-PBS, a 2.3 μg/ml solution of shFcγRIIIa(F) or shFcγRIIIa(V) diluted with 1% BSA-PBS was added at 50 μl/well and allowed to react at room temperature for 1 hour. After the reaction and subsequent washing with Tween-PBS, a solution of a mouse antibody against His-tag, Tetra·His Antibody (manufactured by QIAGEN), adjusted to 1 μg/ml with 1% BSA-PBS was

added at 50 μl/well and allowed to react at room temperature for 1 hour. After the reaction and subsequent washing with Tween-PBS, a peroxidase-labeled goat anti-mouse IgG1 antibody solution (manufactured by ZYMED) diluted 200-fold with 1% BSA-PBS was added at 50 μl/well and allowed to react at room temperature for 1 hour. After the reaction and subsequent washing with Tween-PBS, the ABTS substrate solution was added at 50 μl/well to develop color, and 10 minutes thereafter, the reaction was stopped by adding 5% SDS solution at 50 μl/well. Thereafter, OD415 was measured. It was confirmed that, by adding each of the anti-GD3 chimeric antibodies to another plate prepared in the same manner and carrying out the ELISA described in item 2 of Reference Example 1, there is no difference in the each amount of the anti-GD3 chimeric antibody bound to the plate.

The results of the measurement of the binding activity of the various anti-GD3 chimeric antibodies to shFcγRIIIa(F) and shFcγRIIIa(V) are shown in Fig. 7. As shown in Fig. 7, shFcγRIIIa(V) showed higher binding activity to the chimeric antibodies than shFcγRIIIa(F). Also, the YB2/0-GD3 chimeric antibody showed 20 to 30 times or more higher binding activity to both types of shFcγRIIIa than the CHO-GD3 chimeric antibody. Furthermore, the binding activity of the YB2/0-GD3 chimeric antibody to shFcγRIIIa(F) was 5 times or more higher than that of the CHO-GD3 chimeric antibody to shFcγRIIIa(V). Moreover, the CHO-GD3 chimeric antibody showed little binding activity to shFcγRIIIa(F). The above results show that the antibody produced by the α1,6-fucose/lectin-unresistant cell binds to only FcγRIIIa having the polymorphism in which the amino acid at position 176 from the N-terminal is valine, whereas the antibody produced by the α1,6-fucose/lectin-resistant cell has high binding activity to FcγRIIIa having any polymorphism. That is, these results show that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

2. Evaluation of binding activity of anti-CCR4 chimeric antibodies

The binding activity of the two types of the anti-CCR4 chimeric antibodies, KM2760-1 and KM3060 described in the item 3 of Reference Example 2, to shFcγRIIIa(F) and shFcγRIIIa(V) was measured by ELISA as follows.

According to the method described in the item 2 of Reference Example 2, a human CCR4 extracellular peptide conjugate was immobilized at 1.0 μl/well on a 96 well plate for ELISA (manufactured by Greiner). After washing with PBS, 1% BSA-PBS was added at 100 μl/well and allowed to react at room temperature for 1 hour to block the remaining active groups. After washing each well with Tween-PBS, a solution of each anti-CCR4 chimeric antibody diluted with 1% BSA-PBS was added at 50 μl/well and allowed to react at room temperature for 1 hour. After the reaction and subsequent washing of each well with Tween-PBS, a 2.3 μg/ml solution of shFcγRIIIa(F) or shFcγRIIIa(V) diluted with 1% BSA-PBS was added at 50 μl/well and allowed to react at room temperature for 1 hour. After the reaction and subsequent washing with Tween-PBS, a solution of a mouse antibody against His-tag, Tetra·His Antibody (manufactured by QIAGEN), adjusted to 1 μg/ml with 1% BSA-PBS was added at 50 μl/well and allowed to react at room temperature for 1 hour. After the reaction and subsequent washing with Tween-PBS, a peroxidase-labeled goat anti-mouse IgG1 antibody solution (manufactured by ZYMED) diluted 200-fold with 1% BSA-PBS was added at 50 μl/well and allowed to react at room temperature for 1 hour. After the reaction and subsequent washing with Tween-PBS, the ABTS substrate solution was added at 50 μl/well to develop color, and 10 minutes thereafter, the reaction was stopped by adding 5% SDS solution at 50 μl/well. Thereafter, OD415 was measured. In addition, it was confirmed that, by adding each of the anti-CCR4 chimeric antibodies to another plate prepared in the same manner and carrying out the ELISA described in item 2 of Reference Example 2, there is no difference in the amount of the anti-CCR4 chimeric antibodies bound to the plate.

The results of the measurement of the binding activity of the various anti-CCR4 chimeric antibodies to shFcγRIIIa(F) and shFcγRIIIa(V) are shown in Fig. 8. As shown in Fig. 8, shFcγRIIIa(V) showed higher binding activity to the chimeric antibodies than shFcγRIIIa(F). Also, KM2760-1 showed 30 to 50 times or more higher binding activity to both types of shFcγRIIIa than KM3060. Furthermore, the binding activity of KM2760-1 to shFcγRIIIa(F) was 10 times or more higher than that of KM3060 to shFcγRIIIa(V). Moreover, KM3060 showed little binding activity to shFcγRIIIa (F). The above results show that the antibody produced by the α1,6-fucose/lectin-unresistant cell binds to only FcγRIIIa having the polymorphism in which the amino acid at position 176 from the N-terminal is valine, whereas the antibody produced by the α1,6-fucose/lectin-resistant cell has high binding activity to FcγRIIIa having any polymorphism. That is, these results show that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

3. Evaluation of binding activity of anti-FGF-8 chimeric antibodies

The binding activity of the two types of the anti-FGF-8 chimeric antibodies, YB2/0-FGF8 chimeric antibody and CHO-FGF8 chimeric antibody described in the item 3 of Reference Example 3, to shFcγRIIIa(F) and shFcγRIIIa (V) was measured by ELISA as follows.

According to the method described in the item 2 of Reference Example 3, a human FGF-8 peptide conjugate was immobilized at 1.0 µl/well on a 96 well plate for ELISA (manufactured by Greiner). After washing with PBS, 1% BSA-PBS was added at 100 µl/well and allowed to react at room temperature for 1 hour to block the remaining active groups. After washing each well with Tween-PBS, a solution of each anti-FGF-8 chimeric antibody diluted with 1% BSA-PBS was added at 50 µl/well and allowed to react at room temperature for 1 hour. After the reaction and subsequent washing of each well with Tween-PBS, a solution of shFcγRIIIa(F) or shFcγRIIIa(V) prepared by diluting it to 2.3 µg/ml with 1% BSA-PBS was added at 50 µl/well and allowed to react at room temperature for 1 hour. After the reaction and subsequent washing with Tween-PBS, a solution of a mouse antibody against His-tag, Tetra·His Antibody (manufactured by QIAGEN), adjusted to 1 µg/ml with 1% BSA-PBS was added at 50 µl/well and allowed to react at room temperature for 1 hour. After the reaction and subsequent washing with Tween-PBS, a peroxidase-labeled goat anti-mouse IgG1 antibody solution (manufactured by ZYMED) diluted 200-fold with 1% BSA-PBS was added at 50 µl/well and allowed to react at room temperature for 1 hour. After the reaction and subsequent washing with Tween-PBS, the ABTS substrate solution was added at 50 µl/well to develop color, and 10 minutes thereafter, the reaction was stopped by adding 5% SDS solution at 50 µl/well. Thereafter, OD415 was measured. In addition, it was confirmed that, by adding each of the anti-FGF-8 chimeric antibodies to another plate prepared in the same manner and carrying out the ELISA described in item 2 of Reference Example 3, there is no difference in the amount of the anti-FGF-8 chimeric antibodies bound to the plate.

The results of the measurement of the binding activity of the various anti-FGF-8 chimeric antibodies to shFcγRIIIa(F) and shFcγRIIIa(V) are shown in Fig. 9. As shown in Fig. 9, shFcγRIIIa(V) showed higher binding activity to the chimeric antibodies than shFcγRIIIa(F). Also, the YB2/0-FGF8 chimeric antibody showed 25 to 30 times or more higher binding activity to both types of shFcγRIIIa than the CHO-FGF8 chimeric antibody. Furthermore, the binding activity of the YB2/0-FGF8 chimeric antibody to shFcγRIIIa(F) was 10 times or more higher than that of the CHO-FGF8 chimeric antibody to shFcγRIIIa(V). Moreover, the CHO-FGF8 chimeric antibody showed little binding activity to shFcγRIIIa(F). The above results show that the antibody produced by the α1,6-fucose/lectin-unresistant cell binds to only FcγRIIIa having the polymorphism in which the amino acid at position 176 from the N-terminal is valine, whereas the antibody produced by the α1,6-fucose/lectin-resistant cell has high binding activity to FcγRIIIa having any polymorphism. That is, these results show that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

4. Evaluation of binding activity of anti-CD20 chimeric antibodies

The binding activity of the two types of the anti-CD20 chimeric antibodies, KM3065 and Rituxan™ described in the item 3 of Reference Example 4, to shFcγRIIIa was measured by ELISA as follows.

A solution of a mouse antibody against His-tag, Tetra.His Antibody (manufactured by QIAGEN), adjusted to 5 µg/ml was added at 50 µl/well on a 96 well plate for ELISA (manufactured by Greiner), and allowed to react at 4°C overnight for adsorption. After washing with PBS, 1% BSA-PBS was added at 100 µl/well and allowed to react at room temperature for 1 hour to block the remaining active groups. After washing each well with Tween-PBS, a 1 µg/ml solution of shFcγRIIIa(F) or shFcγRIIIa(V) described in the item 4 of Example 7 diluted with 1% BSA-PBS was added at 50 µl/well and allowed to react at room temperature for 2 hours. After the reaction and subsequent washing of each well with Tween-PBS, a solution of each of various anti-CD20 chimeric antibodies diluted with 1% BSA-PBS was added at 50 µl/well and allowed to react at room temperature for 2 hours. After the reaction and subsequent washing of each well with Tween-PBS, a peroxidase-labeled goat anti-human IgG(γ) antibody solution (manufactured by American Qualex) diluted 6,000-fold with 1% BSA-PBS was added at 50 µl/well and allowed to react at room temperature for 1 hour. After the reaction and subsequent washing with Tween-PBS, an ABTS substrate solution [solution prepared by dissolving 0.55 g of 2,2'-azino-bis(3-ethylbenzothiazoline-6-sulfonic acid) ammonium salt in 1 liter of 0.1 M citrate buffer (pH 4.2) and adding 1 µl/ml of hydrogen peroxide to the solution just before use] was added at 50 µl/well to develop color, and 10 minutes thereafter, the reaction was stopped by adding 5% SDS solution at 50 µl/well. Thereafter, the absorbance at 415 nm was measured. In addition, it was confirmed that, by adding each of the diluted antibody solutions to a plate for ELISA coated with a goat antibody against human IgG (manufactured by American Qualex) instead of the mouse antibody against His-tag and detecting the peroxidase-labeled goat anti-human IgG(γ) antibody solution (manufactured by American Qualex) in the same manner, there is no difference in the amount of the anti-CD20 chimeric

antibodies used in the diluted antibody solutions.

The results of the measurement of the binding activity of the various anti-CD20 chimeric antibodies to shFcγRIIIa(F) and shFcγRIIIa(V) are shown in Fig. 10. As shown in Fig. 10, shFcγRIIIa(V) showed higher binding activity to the chimeric antibodies than shFcγRIIIa(F). Also, KM3065 showed about 50 to 73 times higher binding activity to both types of shFcγRIIIa than Rituxan™. Furthermore, the binding activity of KM3065 to shFcγRIIIa(F) was several times higher than that of Rituxan™ to shFcγRIIIa(V). Moreover, Rituxan™ showed little binding activity to shFcγRIIIa(F). The above results show that the antibody produced by the α1,6-fucose/lectin-unresistant cell binds to only FcγRIIIa having the polymorphism in which the amino acid at position 176 from the N-terminal is valine, whereas the antibody produced by the α1,6-fucose/lectin-resistant cell has high binding activity to FcγRIIIa having any polymorphism. That is, these results show that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

5. Evaluation of binding activity of various anti-CCR4 chimeric antibodies produced by lectin-resistant CHO cells

The binding activities of the anti-CCR4 chimeric antibody KM3060 produced by CHO/DG44 cell described in the item 3(2) of Reference Example 2 and the antibody produced by the clone CHO/CCR4-LCA described in the item 2 of Reference Example 5 to shFcγRIIIa(F) and shFcγRIIIa(V) described in the item 4 of Reference Example 6 were measured by using the ELISA described in the above item 2.

Fig. 11 shows the results of measurement of binding activities of the various anti-CCR4 chimeric antibodies to shFcγRIIIa(F) and shFcγRIIIa(V), respectively. As shown in Fig. 17, the antibody produced by the clone CHO/CCR4-LCA showed high binding activities to shFcγRIIIa(V) and shFcγRIIIa(F), respectively, whereas KM3060 showed high binding activity to only shFcγRIIIa(V) and little binding activity to shFcγRIIIa(V). The above results show that the chimeric antibody produced by LCA lectin-resistant CHO cell of the present invention has higher binding activities to shFcγRIIIa(F) as well as shFcγRIIIa(V) than the chimeric antibody produced by the CHO/DG44 cell without depending on the polymorphism of shFcγRIIIa. That is, these results show that the antibody produced by the LCA lectin-resistant CHO cell showed higher therapeutic effects on patients having any polymorphism of FcγRIIIa than the antibody produced by the LCA lectin-unresistant CHO cell, and particularly has superior therapeutic effects on patients having polymorphism of FcγRIIIa in which the amino acid at position 176 from the N-terminal is phenlyalanine.

6. Evaluation of binding activity of various anti-GD3 chimeric antibodies produced by lectin-resistant CHO cells

The binding activities of the CHO-GD3 chimeric antibody produced by CHO/DG44 cell described in the item 1(2) of Reference Example 1 and the antibody produced by the clone CHO/GD3-LCA-1 and the antibody produced by the clone CHO/GD3-LCA-2 described in the item 3 of Reference Example 5 to shFcγRIIIa(F) and shFcγRIIIa(V) described in the item 4 of Reference Example 6 were measured by using the ELISA described in the above item 1 of Example 4.

Fig. 12 shows binding activities of the various anti-GD3 chimeric antibodies to shFcγRIIIa(F) and shFcγRIIIa(V), respectively. As shown in Fig. 12, both the antibody produced by the clone CHO/GD3-LCA-1 and the antibody produced by the clone CHO/GD3-LCA-2 showed high binding activities to shFcγRIIIa(V) and shFcγRIIIa(F), respectively, whereas CHO-GD3 chimeric antibody showed high binding activity to only shFcγRIIIa(V) and little binding activity to shFcγRIIIa(V). The above results show that the chimeric antibody produced by LCA lectin-resistant CHO cell of the present invention has higher binding activities to shFcγRIIIa(F) as well as shFcγRIIIa(V) than the chimeric antibody produced by the CHO/DG44 cell without depending on the polymorphism of shFcγRIIIa. That is, these results show that the antibody produced by the LCA lectin-resistant CHO cell showed higher therapeutic effects on patients having any polymorphism of FcγRIIIa than the antibody produced by the LCA lectin-unresistant CHO cell, and particularly has superior therapeutic effects on patients having polymorphism of FcγRIIIa in which the amino acid at position 176 from the N-terminal is phenlyalanine.

Example 5

Evaluation of binding activities of various chimeric antibodies to shFcγRIIIa(F) and shFcγRIIIa(V) (biosensor method)

Experiments were carried out according to a biosensor method by examining the influence of the polymorphism of the amino acids at position 176 from the N terminal methionine in human FcγRIIIa on the binding activity of the antibody produced by the α1,6-fucose/lectin-resistant cell to human FcγRIIIa.

1. Evaluation of binding activities of anti-CCR4 chimeric antibody and anti-FGF-8 chimeric antibody

The binding activities of two types of anti-CCR4 chimeric antibodies, KM2760-1 and KM3060, described in the item 3 of Reference Example 2 and the two types of anti-FGF-8 chimeric antibodies, YB2/0-FGF8 chimeric antibody and CHO-FGF8 chimeric antibody described in the item 3 of Reference Example 3 to shFcγRIIIa(F) and shFcγRIIIa(V) described in the item 4 of Reference Example 6 were measured by using BIAcore 2000 (manufactured by Pharmacia) as follows and the results were compared.

Herein, HBS-EP (manufactured by Pharmacia) was used as the buffer for the dilution of samples and during the measurement. First, a sensor tip SA (manufactured by Pharmacia) was set, and 10 µl of a biotinylated antigen peptide adjusted to 0.5 µg/ml was added at a flow rate of 10 µl/min. Thereafter, the tip surface was washed by adding 5 µl of 10 mmol/l glycine-hydrochloric acid solution (pH 2.0). In this manner, 421.9 RU of the biotinylated compound 1 (human CCR4 extracellular region peptide) was immobilized to the flow cell (hereinafter referred to as "FC") 1, and 349.9 RU of the biotinylated compound 2 (human FGF-8 peptide) to FC 2.

At a flow rate of 5 µl/min, 20 µl of 10 µg/ml solution of each of various chimeric antibodies was added to FC 1 and FC 2 to bind the antibody. After 90 seconds, 15 µl of a diluted solution of shFcγRIIIa(F) or shFcγRIIIa(V) was added thereto and then the dissociation reaction was monitored for 3 minutes. After the dissociation reaction, the tip surface was recycled by adding 5 µl of 10 mmol/l glycine-hydrochloric acid solution (pH 2.0). This cycle was carried out at various concentrations (from 22.3 to 714.3 nM) of shFcγRIIIa(F) and shFcγRIIIa(V) solutions to obtain a sensorgram at each concentration. Typical sensorgrams are shown in Fig. 13. The sensorgram of each chimeric antibody was prepared as a sensorgram of specific reaction by subtracting the sensorgram obtained for the nonspecific antigen peptide-immobilized FC.

Sensorgrams of the binding of anti-CCR4 chimeric antibody to shFcγRIIIa(F) or shFcγRIIIa(V) are shown in Fig. 14, and sensorgrams of the binding of anti-FGF-8 chimeric antibody to shFcγRIIIa(F) or shFcγRIIIa(V) in Fig. 15. Using the thus obtained sensorgrams at various concentrations, the binding rate constant (hereinafter referred to as "Ka"), the dissociation rate constant (hereinafter referred to as "Kd") and the binding constant (hereinafter referred to as "KA") of anti-CCR4 chimeric antibody to shFcγRIIIa(F) or shFcγRIIIa(V) shown in Table 1 and Ka, Kd and KA of anti-FGF-8 chimeric antibody to shFcγRIIIa(F) or shFcγRIIIa(V) shown in Table 2 were calculated by a nonlinear analysis [*J. Immunol. Methods,* 200, 121 (1997)] using analysis software BIAevaluation 3.0 attached to BIAcore 2000. However, regarding the binding of CHO/DG44 cell-derived chimeric antibodies (KM3060 and CHO-FGF8 chimeric antibody) to shFcγRIIIa(F), it was difficult to carry out the above analysis because of the extremely quick dissociation, so that KA was calculated from the equilibrium value of the binding and the concentration of shFcγRIIIa(F) [*Protein-Protein Interactions*, Cold Spring Harbor Laboratory Press (2002)].

Table 1

| | Ka ($\times 10^5 M^{-1}s^{-1}$) | Kd ($\times 10^{-2} s^{-1}$) | KA ($\times 10^7 M^{-1}$) |
|---|---|---|---|
| KM2760-1 | | | |
| shFcγRIIIa(F) | 2.36 | 1.66 | 1.42 |
| shFcγRIIIa(V) | 1.60 | 0.508 | 3.14 |
| KM3060 | | | |
| shFcγRIIIa(F) | | | 0.052 |
| shFcγRIIIa(V) | 1.04 | 2.99 | 0.349 |

Table 2

| | Ka ($\times 10^5 M^{-1}s^{-1}$) | Kd ($\times 10^{-2} s^{-1}$) | KA ($\times 10^7 M^{-1}$) |
|---|---|---|---|
| YB2/0-FGF chimeric antibody | | | |
| shFcγRIIIa(F) | 2.39 | 1.81 | 1.32 |
| shFcγRIIIa(V) | 1.53 | 0.554 | 2.76 |
| CHO-FGF8 chimeric antibody | | | |
| shFcγRIIIa(F) | | | 0.115 |
| shFcγRIIIa(V) | 0.963 | 3.25 | 0.297 |

As a result, the results of anti-CCR4 chimeric antibody and anti-FGF-8 chimeric antibody almost coincided,

and similar to the case of the analysis by ELISA, shFcγRIIIa(V) showed higher binding activity to the chimeric antibodies than shFcγRIIIa(F), which was 2 to 7 times higher in KA. In addition, the chimeric antibody produced by YB2/0 cell showed 9 to 27 times or more higher binding activity to both shFcγRIIIa than the chimeric antibody produced by the CHO/DG44 cell, and its binding activity to shFcγRIIIa(F) was 4 times or more higher than the binding activity of the chimeric antibody produced by the CHO/DG44 cell to shFcγRIIIa(V). These results show that the chimeric antibody produced by the YB2/0 cell has higher binding activity to shFcγRIIIa than that of the chimeric antibody produced by the CHO/DG44 cell without depending on the polymorphism of shFcγRIIIa.

That is, it is shown that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

2. Evaluation of binding activity of anti-CD20 chimeric antibody

The binding activities of two types of anti-CCR4 chimeric antibodies, KM3065 and Rituxan™, described in the item 3 of Reference Example 4 to shFcγRIIIa(F) and shFcγRIIIa(V) described in the item 4 of Reference Example 6 were compared by measuring them using BIAcore 2000 (manufactured by Pharmacia) as follows.

First, a sensor tip CM5 (manufactured by BIACORE) was set, and 4596.6RU of a mouse antibody against His-tag, Tetra·His Antibody (manufactured by QIAGEN), diluted to 10 μg/ml with 10 mM sodium acetate solution (pH 4.0) was immobilized. Herein, HBS-EP (manufactured by Pharmacia) was used as the buffer for the dilution of samples and during the measurement. At a flow rate of 5 μl/min, 20 μl of shFcγRIIIa(F) or shFcγRIIIa(V) diluted to 5 μg/ml was added thereto to bind shFcγRIIIa. After 60 seconds, 15 μl of a diluted solution of the anti-CD20 chimeric antibody or Rituxan™ was added thereto and then the dissociation reaction was monitored for 4 minutes. After the dissociation reaction, the tip surface was regenerated by adding 5 μl of 7.5 mM hydrochloric acid solution.. This cycle was carried out for the anti-CD20 chimeric antibody at various antibody concentrations (from 20 to 0.625 μg/ml) to obtain a sensorgram of binding to shFcγRIIIa(F) and shFcγRIIIa(V). The sensorgram of each anti-CD20 chimeric antibody was prepared by subtracting the sensorgram obtained by adding the buffer instead of the antibody. Sensorgrams of the binding of anti-CD20 chimeric antibody to shFcγRIIIa(F) and shFcγRIIIa(V) are shown in Fig. 16. Using the thus obtained sensorgrams at various concentrations, Ka, Kd and KA of the binding of anti-CD20 chimeric antibody to shFcγRIIIa(F) or shFcγRIIIa(V) shown in Table 3 were calculated by the nonlinear analysis using analysis software BIAevaluation 3.0 attached to BIAcore 2000. However, determining the binding of Rituxan™ to shFcγRIIIa(F) was difficult because of the extremely quick dissociation, so that KA was calculated from the equilibrium value of the binding and the concentration of shFcγRIIIa(F).

Table 3

| | Ka ($\times 10^5 M^{-1} s^{-1}$) | Kd ($\times 10^{-2} s^{-1}$) | KA ($\times 10^7 M^{-1}$) |
|---|---|---|---|
| KM3065 | | | |
| shFcγRIIIa(F) | 2.86 | 1.84 | 1.56 |
| shFcγRIIIa(V) | 2.88 | 0.56 | 5.17 |
| Rituxan | | | |
| shFcγRIIIa(F) | - | - | 0.28 |
| shFcγRIIIa(V) | 0.34 | 0.71 | 0.48 |

As a result, similar to the case of the analysis by ELISA, shFcγRIIIa(V) showed higher binding activity than shFcγRIIIa(F) to the chimeric antibodies, which was about 2 to 3 times higher in KA. In addition, KM3065 produced by YB2/0 cell showed higher binding activity to both shFcγRIIIa than Rituxan™, and its binding activity to shFcγRIIIa(F) was 3 times or more higher than the binding activity of Rituxan™ to shFcγRIIIa(V). These results show that the chimeric antibody produced by the YB2/0 cell has higher binding activity to shFcγRIIIa than that of the chimeric antibody produced by the CHO cell without depending on the polymorphism of shFcγRIIIa. That is, it is shown that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

Example 6

Analysis of correlation between polymorphism of FcγRIIIa and ADCC activity

Analysis was carried out on the correlation between polymorphism of the amino acid at position 176 from the N-terminal methionine of SEQ ID NO:11 or 13 in the human FcγRIIIa (hereinafter referred to as "polymorphism of the amino acid at position 176") and ADCC activity of antibodies produced by α1,6 fucose/lectin resistant cells.

1. Analysis of polymorphism of gene encoding FcγRIIIa contained in human peripheral blood

(1) Extraction of genomic DNA from human blood

From each of randomly selected 20 healthy donors, 30 ml of peripheral blood was collected and gently mixed with 0.3 ml of heparin sodium (manufactured by Shimizu Pharmaceutical). Genomic DNA of each volunteer was extracted from 2 ml of each sample using QIAamp DNA Blood Midi Kit (manufactured by Quiagen). The remaining 28 ml was used in the measurement of ADCC activity carried out in the item 3 of Example 6.

(2) Analysis of polymorphism of FcγRIIIa gene

The analysis was carried out in accordance with a known method [*Blood,* 90, 1109 (1997)]. The method carried out using the genomic DNA obtained from each donor in the item 1(1) of Example 6 is shown below.

a. Specific amplification of FcγRIIIa gene by allele-specific PCR

Using 5 ng of genomic DNA, PCR was carried out by a hot start method in 50 μl of a reaction solution comprising 500 nM of primers (SEQ ID NOs:28 and 29, consigned to Genset), 200 μM each of dNTP (manufactured by Takara Shuzo), 2.5 U of Taq Polymerase (manufactured by Promega) and 1 × TaqBuffer (manufactured by Promega). By using GeneAmp PCR System 9700 (manufactured by Applied Biosystems), the reaction solution was denatured at 95°C for 10 minutes and then the reaction was carried out by 35 cycles of heating at 95°C for 1 minute, 56°C for 1.5 minutes and 72°C for 1.5 minutes as one cycle, followed by incubation at 72°C for 8 minutes. It was confirmed by 1.5% agarose gel electrophoresis that the amplified fragment had the intended size (about 1.7 kbp).

b. Sequence determination

Analysis of the polymorphism of the amino acid at position 176 of FcγRIIIa was carried out by a nucleotide sequence analysis using the amplified fragment obtained in the above item a. as the template. The sequence-determining PCR reaction solution (20 μl) comprises 7 μl of the PCR product purified by QIAquick PCR Purification Kit (manufactured by Quiagen), 1.5 μM of primers (manufactured by Genset, their sequences are shown below) and 5 fold-diluted reaction mixture (manufactured by Applied Biosystems, Big Dye Terminator Kit). GeneAmp PCR System 9700 (manufactured by Applied Biosystems) was used. The reaction solution was denatured at 94°C for 5 minutes and then the reaction was carried out by 25 cycles of heating at 96°C for 10 seconds, 50°C for 5 seconds and 60°C for 4 minutes as one cycle. After the reaction, the PCR product was purified by using Dye Ex Spin Kit (manufactured by Quiagen). The analysis was carried out by using ABI 377 Sequencer (manufactured by Applied Biosystems), and the polymorphism of the amino acid at position 176 of FcγRIIIa was determined by the waveform of the sequencer. An example of the analysis is shown in Fig. 17. A sample of a donor whose genotype encoding the amino acid at position 176 is a phenylalanine homo type (hereinafter referred to as "Phe/Phe type") shows a signal in which the first nucleotide of the codon encoding the amino acid at position 176 is T, a sample of a donor of a hetero type of phenylalanine and valine (hereinafter referred to as "Phe/Val type") shows a mixed signal in which the first nucleotide of the codon encoding the amino acid at position 176 is T and G, and a sample of a donor of a valine homo type (hereinafter referred to as "Val/Val type") shows a signal in which the first nucleotide of the codon encoding the amino acid at position 176 is G. As a result of the polymorphism analysis on all of the 20 donors, 15 of which were the Phe/Phe type, 4 were the Phe/Val type, and 1 was the Val/Val type.

2. Measurement of the ratio of NK cells in peripheral blood mononuclear cells (immunofluorescent method)

The existing ratio of NK cells included in the peripheral blood mononuclear cells derived from the 20 donors was measured by an immunofluorescent method. Using an FITC-labeled anti-CD3 antibody/PE-labeled anti-CD56 antibody mixed solution (manufactured by Coulter), or an FITC-labeled mouse IgG1/PE-labeled mouse IgG1 mixed

solution (manufactured by Coulter) as a negative control, 4×$10^5$ cells of the peripheral blood mononuclear cells obtained in the item 1(1) of Example 6 were stained in accordance with the manufacture's instructions and then analyzed by using a flow cytometer EPICS XL-MCL (manufactured by Coulter). In the histogram of staining with the FITC-labeled anti-CD3 antibody/PE-labeled anti-CD56 antibody, the ratio of cells contained in the CD3-negative CD56-positive cell fractions among the total cells was regarded as the NK cell ratio. As a result, dispersion in the NK cell ratio was observed among the donors, but clear correlation was not found between the NK cell ratio and the polymorphism of the amino acid at position 176 of FcγRIIIa.

Table 4

| Donor No. | #1 | #2 | #3 | #4 | #5 |
|---|---|---|---|---|---|
| Genotype | Phe/Phe | Phe/Phe | Phe/Phe | Phe/Val | Phe/Val |
| NK cell ratio (%) | 12.5 | 26.2 | 26.4 | 13.2 | 9.83 |

| Donor No. | #6 | #7 | #8 | #9 | #10 |
|---|---|---|---|---|---|
| Genotype | Phe/Phe | Phe/Phe | Phe/Phe | Phe/Phe | Phe/Phe |
| NK cell ratio (%) | 10.5 | 19.1 | 12.8 | 15.9 | 21.3 |

| Donor No. | #11 | #12 | #13 | #14 | #15 |
|---|---|---|---|---|---|
| Genotype | Phe/Phe | Phe/Phe | Phe/Phe | Val/Val | Phe/Phe |
| NK cell ratio (%) | 36.6 | 24.6 | 11.4 | 9.12 | 22.8 |

| Donor No. | #16 | #17 | #18 | #19 | #20 |
|---|---|---|---|---|---|
| Genotype | Phe/Val | Phe/Phe | Phe/Val | Phe/Phe | Phe/Phe |
| NK cell ratio (%) | 20.6 | 13 | 28.8 | 28.4 | 18.9 |

3. *In vitro* cytotoxic activity (ADCC activity) of anti-CD20 antibody and anti-GD3 antibody

A correlation between polymorphism and ADCC activity was analyzed by measuring the ADCC activity using, as the effector cells, peripheral blood mononuclear cells in which the polymorphism of the amino acid at position 176 of FcγRIIIa had been determined. The method is shown below. The anti-CD20 chimeric antibody KM 3065 (the content of sugar chains to which α1,6-fucose is not bound is 96%) described in the item 3 of Reference Example 4, the anti-CD20 chimeric antibody Rituxan™ (the content of sugar chains to which α1,6-fucose is not bound is 6%), the anti-GD3 chimeric antibody YB2/0-GD3 chimeric antibody (the content of sugar chains to which α1,6-fucose is not bound is 53%) described in the item 3(1) of Reference Example 1, and the anti-GD3 chimeric antibody CHO-GD3 chimeric antibody (the content of sugar chain to which α1,6-fucose is not bound is 7%) described in the item 3(2) of Reference Example 1 were used.

(1) Preparation of target cell suspension

A human B lymphocyte cultured cell line Raji cell (JCRB 9012), WIL2-S cell (ATCC CRL 8885) or G-361 cell (ATCC CRL 1424) cultured using a RPMI 1640-FCS(10) medium (RPMI 1640 medium (manufactured by GIBCO BRL) containing 10% FCS) was mixed with a radioactive substance $Na_2{}^{51}CrO_4$ in a 3.7 MBq equivalent amount per 1×$10^5$ cells, and allowed to react at 37°C for 1 hour to label the cells with the radioactive substance. After the reaction, the cells were washed three times by repeating a step of suspending in the RPMI 1640-FCS(10) medium and separating by centrifugation, re-suspended in the medium and then allowed to stand at 4°C for 30 minutes for spontaneous dissociation of the radioactive substance. After centrifugation, the cells were suspended in the RPMI 1640-FCS(10) medium to give a density of 1×$10^5$ cells/ml and used as the target cell suspension.

(2) Preparation of effector cell suspension

From each of the 20 donors in the item 1(1) of Example 6, 28 ml of the peripheral blood sample was collected and centrifuged (800 g, 20 minutes) by using Lymphoprep (manufactured by AXIS SHIELD) in accordance with the manufacture's instructions to separate a mononuclear cell layer. The layer was washed by centrifugation three times by using RPMI 1640-FCS(10) medium and then re-suspended in the same medium to give a density of $2\times10^6$ cells/ml to be used as the effector cell suspension.

(3) Measurement of ADCC activity

In each well of a 96 well U-bottomed plate (manufactured by Falcon), 50 µl ($1\times10^4$ cells/well) of the target cell suspension prepared in the above item (1) was dispensed. Next, 100 µl of the effector cell suspension prepared in the above item (2) was added thereto ($2\times10^5$ cells/well, the ratio of the effector cell to the target cell is 20:1). Also, the anti-CD20 chimeric antibody KM 3065 or Rituxan™ was added to respective wells into which the Raji cell and WIL2-S cell had been dispensed, and the anti-GD3 chimeric antibody YB2/0-GD3 chimeric antibody or CHO-GD3 chimeric antibody to respective wells into which the G-361 cell had been dispensed, respectively, to give a final concentration of 10 ng/ml to adjust the total volume to 200 µl, and then the reaction was carried out at 37°C for 4 hours. After the reaction, the plate was centrifuged and the amount of $^{51}$Cr in each supernatant was measured by using a γ-counter. The amount of spontaneously released $^{51}$Cr was obtained from a well in which the reaction was carried out by adding the medium instead of the antibody solution and effector cell suspension, and the amount of total released $^{51}$Cr by adding 1 N hydrochloric acid instead of the antibody solution and effector cell suspension, and the antibody-independent cytotoxicity data by adding the medium instead of the antibody solution. The cytotoxic activity was calculated by the following equation:

$$\text{ADCC activity (\%)} = \frac{\text{amount of } ^{51}\text{Cr in sample supernatant - spontaneously released amount of } ^{51}\text{Cr}}{\text{total released amount of } ^{51}\text{Cr - spontaneously released amount of } ^{51}\text{Cr}} \times 100$$

4. Analysis of correlation between polymorphism of the amino acid at position 176 of FcγRIIIa and ADCC activity per $10^4$ NK cells

In order to reduce the dispersion of the ADCC activity due to individual difference in the NK cell ratio, values of the ADCC activity per $10^4$ NK cells were calculated based on the following equations by using the values of ADCC activity obtained in the item 3 of Example 6 and the NK cell ratio obtained in the item 2 of Example 6.

PBMC (effector) per well: $2\times10^5$ cells

The number of NK cells per well: $2\times10^5$ cells × NK cell ratio (%)/100

∴ ADCC activity per one NK cell:

$$\text{ADCC (\%)} \div \text{(the number of NK cells per well)}$$

$$= \text{ADCC (\%)} \div (2\times10^5 \text{ cells} \times \text{NK cell ratio (\%)}/100)$$

∴ ADCC activity per $10^4$ NK cells:

$$\text{(ADCC activity per one NK cell)} \times 10^4 \text{ (cells)}$$

$$= \text{ADCC (\%)} \div (2\times10^5 \text{ cells} \times \text{NK cell ratio (\%)}/100) \times 10^4$$

$$= \text{ADCC (\%)} \div \text{NK cell ratio (\%)} \times 5$$

The polymorphism of the amino acid at position 176 of FcγRIIIa from each donor was divided based on the presence or absence of the Val allele, with the ADCC activity per $10^4$ NK cells in each case was shown in Fig. 18 and Table 5.

Table 5

| Genotype of effector cell: Phe/Phe type | | | |
|---|---|---|---|
| Experiment system | (i) ADCC activity of the antibody produced by CHO/DG44 cell per $10^4$ of NK cells (%) | (ii) ADCC activity of the antibody produced by YB2/0 cell per $10^4$ NK cells (%) | Increasing ratio [(ii) ÷ (i)] |
| Anti-CD20 chimeric antibody × Raji cell | 1.2 ± 0.40 | 13 ± 2.8 | 11 times |
| Anti-CD20 chimeric antibody × WIL2-S cell | 4.8 ± 2.3 | 20 ± 5.9 | 4.2 times |
| Anti-GD3 chimeric antibody × G-361 cell | -1.5 ± 1.2 | 7.2 ± 2.8 | (∞) |
| Genotype of effector cell: Phe/Val type + Val/Val type | | | |
| Experiment system | (i) ADCC activity of the antibody produced by CHO/DG44 cell per $10^4$ of NK cells (%) | (ii) ADCC activity of the antibody produced by YB2/0 cell per $10^4$ NK cells (%) | Increasing ratio [(ii) ÷ (i)] |
| Anti-CD20 chimeric antibody × Raji cell | 5.6 ± 1.7 | 19 ± 4.9 | 3.4 times |
| Anti-CD20 chimeric antibody × WIL2-S cell | 12 ± 3.0 | 27 ± 8.3 | 2.3 times |
| Anti-GD3 chimeric antibody × G-361 cell | 2.4 ± 2.5 | 14 ± 1.9 | 5.8 times |
| The numerals of the columns (i) and (ii) represents a mean value of each group ± standard deviation. | | | |

Fig. 18 and Table 5 show the results using antigens, target cells and effector cells in which the chimeric antibody produced by the YB2/0 cell showed high ADCC activity than the chimeric antibody produced by the CHO/DG44 cell in any types of the polymorphism of the amino acid at position 176 of FcγRIIIa. Also, when the effector cell was Phe/Phe type donors, the chimeric antibody produced by the CHO/DG44 cell showed almost no ADCC activity. On the other hand, the chimeric antibody produced by the YB2/0 cell showed high ADCC activity to the Phe/Phe type, too, and the increasing ratio of ADCC activity was particularly high when effector cells of the Phe/Phe type donors were used.

That is, it is shown that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine. In addition, the results of this example also show that patients in which the antibody of the invention produced by the α1,6-fucose/lectin-resistant cells is particularly effective can be selected by measuring the ADCC activity using effector cells of the patients.

Example 7

Evaluation of binding activity of various anti-CCR4 chimeric antibodies to shFcγRIIIa(F) and shFcγRIIIa(V) by using isothermal titration-type calorimeter:

Influences of the polymorphism of the amino acid at position 176 from the N-terminal methionine of SEQ ID NO:11 in the human FcγRIIIa on the binding activity of the antibody produced by the α1,6-fucose/lectin-resistant cell to human FcγRIIIa were analyzed using by an isothermal titration-type calorimeter.

Using the following equation, the molar absorption coefficient (280 nm) of each protein was calculated from the amino acid sequence information of anti-CCR4 chimeric antibody described in WO 01/64754 and the information on the shFcγRIIIa(F) described in SEQ ID NO:11.

$$E \text{ (absorbance coefficient at 280 nm: L mol}^{-1}\text{ cm}^{-1}) = A \times n1 + B \times n2 + C \times n3$$

A: molar absorption coefficient of Trp at 280 nm = 5550 (L $mol^{-1}$ $cm^{-1}$)
B: molar absorption coefficient ofTyr at 280 nm = 1340 (L $mol^{-1}$ $cm^{-1}$)
C: molar absorption coefficient of cystine at 280 nm = 200 (L $mol^{-1}$ $cm^{-1}$)
n1: the number of tryptophan per 1 antibody molecule
n2: the number of tyrosine per 1 antibody molecule
n3: the number of cystine per 1 antibody molecule

As a result, the molar absorption coefficient of the anti-CCR4 chimeric antibody (KM 2760-1 described in the item 3(1) of Reference Example 2 or KM3060 described in the item 3(2) of Reference Example 2) was calculated to be 203,000 $M^{-1}cm^{-1}$. Also, the molar absorption coefficient of the shFcγRIIIa(F) and shFcγRIIIa(V) described in the item 4 of Reference Example 6-4 was calculated to be 38,900 $M^{-1}cm^{-1}$.

The following describes on the evaluation of binding activity of various anti-CCR4 chimeric antibodies for the shFcγRIIIa(F) and shFcγRIIIa(V) using an isothermal titration-type calorimeter VP-ITC (manufactured by MicroCal). Each of the anti-CCD4 chimeric antibody and shFcγRIIIa was dialyzed against a buffer (50 mM $NaH_2PO_4$, 150 mM NaCl, pH 7.4). The dialyzed antibody was filled in a cell (capacity 1.44 ml), and the dialyzed shFcγRIIIa(F) or shFcγRIIIa (V) in an injector syringe (capacity: about 0.3 ml), and a titration profile was obtained by injecting the injector syringe solution at 10 μl into the cell solution at 25°C. Immediately after the measurement, titration was carried out by using the injector syringe solution as such but changing the cell solution to the buffer (50 mM $NaH_2PO_4$, 150 mM NaCl, pH 7.4), thereby obtaining data on the heat of dilution. The same samples as those filled in the cell and injector syringe were collected for use in the measurement of absorbance. Using a spectrophotometer for ultraviolet and visible region, the absorbance of the collected samples at 280 nm was measured by using a cell of 1 cm in cell length. Using the aforementioned molar absorbance coefficient E (L $mol^{-1}cm^{-1}$), molar concentration of each of the samples filled in the cell and injector syringe was calculated by the following equation.

$$\text{Molar concentration} = \text{absorbance of sample at 280 nm} \div \text{molar absorbance coefficient}$$

The titration data was corrected by using the data on the heat of dilution and then a titration profile in which the abscissa is the molar ratio of the shFcγRIIIa to the antibody by using the molar concentration calculated by the above equation. The values of N (stoichiometry of binding: the number of shFc·RIIIa binding to one antibody molecule), KA ( binding constant) and ΔH (enthalpy changing amount of the binding) were obtained by the least square method such that a theoretical curve having the three parameters N, KA and ΔH best-fitted to the titration data. A series of the above data analyses were carried out using a software Origin (manufactured by MicroCal). In addition, regarding the binding of KM2760-1 with shFcγRIIIa(F) or shFcγRIIIa(V) and the binding of KM3060 with shFcγRIIIa(V), two independent measurements were carried out. All of the analytical results are shown in Table 6.

Table 6

| Antibody | shFcγRIIIa | Antibody concentration ($\times 10^{-6}$ mol•$L^{-1}$) | shFcγRIIIa concentration ($\times 10^{-6}$ mol•$L^{-1}$) | N | KA ($\times 10^{-6}$ L•$mol^{-1}$) |
|---|---|---|---|---|---|
| KM2760-1 | shFcγRIIIa(V) | 3.1 | 60.1 | 1.11 | 17.1 |
| KM2760-1 | shFcγRIIIa(V) | 3.1 | 67.8 | 1.02 | 16.5 |
| KM2760-1 | shFcγRIIIa(F) | 2.67 | 58.8 | 1.19 | 3.65 |
| KM2760-1 | shFcγRIIIa(F) | 2.97 | 65.1 | 1.17 | 3.94 |
| KM3060 | shFcγRIIIa(V) | 2.55 | 60.1 | 1.11 | 0.96 |
| KM3060 | shFcγRIIIa(V) | 5.75 | 117.2 | 1.19 | 0.52 |
| KM3060 | shFcγRIIIa(F) | 14 | 199.4 | 0.99 | 0.14 |

As shown in Table 6, the binding constant KA between KM2760-1 and shFcγRIIIa(F) was $3.8\times10^{6}$ L·$mol^{-1}$

(mean value of two measurements), the binding constant KA between KM2760-1 and shFcγRIIIa(V) was $16.8\times10^6$ $L\cdot mol^{-1}$ (mean value of two measurements), the binding constant KA between KM3060 and shFcγRIIIa(F) was $0.14\times10^6$ $L\cdot mol^{-1}$, and the binding constant KA between KM3060 and shFcγRIIIa(V) was $0.74\times10^6$ $L\cdot mol^{-1}$ (mean value of two measurements). The above results show that the chimeric antibody produced by CHO/DG44 cell only showed low binding activity to every polymorphism of FcγRIIIa, particularly only markedly low binding activity to the phenylalanine type, while the chimeric antibody produced by YB2/0 cell showed higher FcγRIIIa-binding activity than that of the chimeric antibody produced by CHO/DG44 cell, independent of the polymorphism of the amino acid at position 176 of FcγRIIIa. That is, it is shown that the antibody produced by the α1,6-fucose/lectin-resistant cell showed higher therapeutic effects on patients having any polymorphism of FcγRIIIa than the antibody produced by the α1,6-fucose/lectin-unresistant cell, and particularly has superior therapeutic effects on patients having polymorphism of FcγRIIIa in which the amino acid at position 176 from the N-terminal is phenlyalanine.

Example 8

Measurement of effector cell-binding activity of antibody produced by lectin-resistant cells

As shown below, NK cells were isolated as CD3-negative, CD14-negative, CD19-negative, CD36-negative and an IgE-negative cell from human peripheral blood mononuclear cells using a magnetic cell separation method (MACS), and the binding activity of antibodies to the cells was evaluated by an immunofluorescent method using a flow cytometry.

1. Preparation of human peripheral blood-derived NK cells

From a healthy person, 50 ml of vein blood was collected and gently mixed with 0.5 ml of heparin sodium (manufactured by Shimizu Pharmaceutical). A mononuclear cell layer was separated by centrifugation (800 g, 20 minutes) using Lymphoprep (manufactured by AXIS SHIELD) in accordance with the manufacture's instructions. After washing with a buffer for MACS (PBS containing 0.5% BSA and 2 mM EDTA), CD3-negative, CD14-negative, CD19-negative, CD36-negative and an IgE-negative cell were obtained by using NK Cell Isolation Kit (manufactured by Miltenyi Biotech) in accordance with the manufacture's instructions. Most of the thus obtained cell groups were CD3-negative and CD56-positive showing that they were NK cells. Accordingly, these NK cell groups were used in the analysis.

2. Binding activity of antibodies for NK cells (immunofluorescent method)

An anti-CCR4 chimeric antibody or an anti-CD20 chimeric antibody was diluted with a buffer for FACS (PBS containing 1% BSA, 0.02% EDTA and 0.05% $NaN_3$) to give a concentration of 10 μg/ml, added to $1.3\times10^5$ cells of the human peripheral blood-derived NK cell obtained in the above and then allowed to react on ice for 30 minutes. After washing with the buffer for FACS, a solution prepared by 100 fold-diluting a PE-labeled anti-human IgG antibody (manufactured by Coulter) using the buffer for FACS was added at 50 μl. After 30 minutes of reaction on ice under shade, the cells were washed and finally suspended in 500 μl, and then the fluorescence intensity was measured by using a flow cytometer. Results of the anti-CCR4 chimeric antibodies KM 2760 and KM 3060 are shown in Fig. 19A, and results of the anti-CD20 chimeric antibodies KM 3065 and Rituxan™ in Fig. 19B. KM 2760 in the case of the anti-CCR4 chimeric antibodies and KM 3065 in the case of the anti-CD20 chimeric antibodies respectively showed high binding activity. The above results show that the high ADCC activity of the antibody composition of the invention produced by the α1,6-fucose/lectin-resistant cells is due to high binding activity to the FcγRIIIa on the effector cells.

In addition, it was shown based on the results of this example that, in selecting a patient to which the antibody composition produced by the α1,6-fucose/lectin-resistant cells according to the present invention is effective, a patient to which the medicament comprising the antibody composition produced by the α1,6-fucose/lectin-resistant cells according to the present invention is effective can be selected by comparing the binding activity of the antibody composition for effector cells of patients with the binding activity of an antibody composition produced by α1,6-fucose/lectin-sensitive cells, and by selecting a patient in which the binding activity for the medicament comprising the antibody composition produced by the α1,6-fucose/lectin-sensitive cells is low.

Example 9

Measurement of increased expression of CD69 molecule which is induced in effector cells by antibodies produced by lectin-resistant cell

Using human peripheral blood-derived NK cells obtained by the similar method of the item 1 of Example 8, analysis was carried out on the expression of an activated marker CD69 on the surface of NK cells when an anti-CCR4 chimeric antibody or anti-CD20 chimeric antibody was allowed to react with corresponding antigen-expressing cell (target cell) by the following method.

1. Co-culturing of NK cell and target cell in the presence of anti-CCR4 chimeric antibody

NALM-6 cell [*Proc. Natl. Acad. Sci. USA,* 79, 4386 (1982)] which was CCR4-expressing cell was used as the target cell. The NALM-6 cell was cultured in RPMI 1640-FCS(10) medium [RPMI 1640 medium (manufactured by Invitrogen) containing 10% FCS], centrifuged, adjusted to give a density of $1\times10^6$ cells/ml in the same medium and then dispensed at 50 μl/well ($5\times10^4$ cells/well) into a 96 well U-bottom culture plate (manufactured by Falcon). Also, the human peripheral blood-derived NK cell obtained by the similar method of the item 1 of Example 8 was adjusted to give a density of $1\times10^6$ cells/ml in the RPMI 1640-FCS(10) medium and dispensed at 50 μl/well ($5\times10^4$ cells/well, ratio of the NK cell to the target cell is 1:1). Thereafter, the anti-CCR4 chimeric antibody was added to give a final concentration of 10 μg/ml to adjust the total volume to 200 μl, followed by culturing at 37°C in the presence of 5% $CO_2$.

2. Co-culturing of NK cell and target cell in the presence of anti-CD20 chimeric antibody

Raji cell (JCRB CCL 86) which was CD20-expressing cell was used as the target cell. The Raji cell was cultured in RPMI 1640-FCS(10) medium, centrifuged, adjusted to give a density of $2\times10^6$ cells/ml in the same medium and then dispensed at 50 μl/well ($1\times10^5$ cells/well) into the 96 well U-bottom culture plate. Also, the human peripheral blood-derived NK cell obtained by the similar method of item 1 of Example 8 was adjusted to give a density of $4\times10^6$ cells/ml in the RPMI 1640-FCS(10) medium and dispensed at 50 μl ($2\times10^5$ cells/well, ratio of the NK cell to the target cell is 2:1). Thereafter, the anti-CD20 chimeric antibody was added to give a final concentration of 0.1 μg/ml to adjust the total volume to 200 μl, followed by culturing at 37°C in the presence of 5% $CO_2$.

3. Analysis of the expression of CD69 on the NK cell surface (immunofluorescent method)

The cells cultured as in the above item 1 or 2 were recovered and washed with the buffer for FACS, and an FITC-labeled anti-CD69 antibody (manufactured by Pharmingen) and a PE-labeled anti-CD56 antibody (manufactured by Coulter) were added thereto in accordance with the respective manufacture's instructions and allowed to react on ice for 30 minutes. The cells were washed with the buffer for FACS and finally suspended at 500 μl, and then the fluorescence intensity of CD69 in the CD56-positive cell group was measured by using a flow cytometer.

Expression intensity of CD69 in the CD56-positive cell fractions after reacting 10 μg/ml in concentration of each of the anti-CCR4 chimeric antibodies KM 2760 and KM 3060 for 4 hours is shown in Fig. 20A, and that after 72 hours of the reaction in Fig. 20. Also, expression intensity of CD69 in the CD56-positive cell fractions after reacting 0.1 μg/ml in concentration of each of the anti-CD20 chimeric antibodies KM 3065 and Rituxan™ for 21 hours is shown in Fig. 20C. KM 2760 in the case of the anti-CCR4 chimeric antibodies and KM 3065 in the case of the anti-CD20 chimeric antibodies respectively showed tendency to increase of the expression of CD69 on the CD56-positive cells, namely NK cells, under any conditions. The above results show that expression of the CD69 molecule on the effector cells is strongly induced when the antibody composition which is resistance to LCA lectin according to the present invention showed high ADCC activity.

It was shown based on the results of this example that, in selecting a patient to which the antibody composition produced by the α1,6-fucose/lectin-resistant cells according to the present invention is effective, a patient to which the medicament comprising the antibody composition produced by the α1,6-fucose/lectin-resistant cells according to the present invention is effective can be selected by comparing a difference between the activated marker molecule of effector cells when the antibody composition is allowed to contact with effector cells of patients and the molecule when allowed to contact with the antibody composition produced by the α1,6-fucose/lectin-unresistant cells, and by selecting a patient in which the expression of the activated marker molecule of effector cells is low and the binding activity to the medicament comprising the antibody composition produced by α1,6-fucose/lectin-unresistant cells is low.

Reference Example 1

Preparation of anti-ganglioside GD3 human chimeric antibody:

1. Preparation of cell stably producing anti-ganglioside GD3 human chimeric antibody

By using the expression vector pChi641LHGM4 for anti-ganglioside GD3 (hereinafter referred to as "GD3") human chimeric antibody described in WO00/61739, cells capable of stably producing an anti-GD3 human chimeric antibody (hereinafter referred to as "anti-GD3 chimeric antibody") were prepared as described below.

(1) Preparation of producing cell using rat myeloma YB2/0 cell

After introducing 5 μg of the anti-GD3 chimeric antibody expression vector pChi641LHGM4 into $4\times10^6$ cells of rat myeloma YB2/0 cell [ATCC CRL-1662, *J. Cell. Biol.*, 93, 576 (1982)] by electroporation [*Cytotechnology*, 3, 133 (1990)], the cells were suspended in 40 ml of RPMI1640-FBS(10) [RPMI1640 medium (manufactured by LIFE TECHNOLOGIES) comprising 10% fetal bovine serum (hereinafter referred to as "FBS", manufactured by LIFE TECHNOLOGIES)] and dispensed at 200 μl/well into a 96 well culture plate (manufactured by Sumitomo Bakelite). After culturing at 37°C for 24 hours in a 5% $CO_2$ incubator, G418 was added to give a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-GD3 chimeric antibody in the supernatant was measured by the ELISA shown in the item 2 of Reference Example 1.

Regarding the transformants in wells in which production of the anti-GD3 chimeric antibody was observed in culture supernatants, in order to increase the amount of the antibody production using a DHFR gene amplification system, each of them was suspended in the RPMI1640-FBS(10) medium comprising 0.5 mg/ml G418 and 50 nmol/L DHFR inhibitor, methotrexate (hereinafter referred to as "MTX"; manufactured by SIGMA) to give a density of 1 to $2\times10^5$ cells/ml, and the suspension was dispensed at 2 ml into each well of a 24 well plate (manufactured by Greiner). Transformants showing 50 nmol/L MTX resistance were induced by culturing at 37°C for 1 to 2 weeks in a 5% $CO_2$ incubator. The antigen binding activity of the anti-GD3 chimeric antibody in culture supernatants in wells in which growth of transformants was observed was measured by the ELISA shown in the item 2 of Reference Example 1.

Regarding the transformants in wells in which production of the anti-GD3 chimeric antibody was observed in culture supernatants, the MTX concentration was increased to 100 nmol/L and then to 200 nmol/L, and a transformant capable of growing in the RPMI1640-FBS(10) medium comprising 0.5 mg/ml G418 and 200 nmol/L MTX and also capable of producing the anti-GD3 chimeric antibody in a large amount was finally obtained by the similar method as described above. The obtained transformant was made into a single cell (hereinafter referred to as "cloning") by limiting dilution twice. Also, using the determination method of transcription product of a1,6-fucoslytransferase gene described in Example 8 of WO00/61739, a cell line producing a relatively low level of the transcription product was selected as a suitable clone.

The obtained anti-GD3 chimeric antibody-producing transformed cell clone 7-9-51 has been deposited on April 5, 1999, as FERM BP-6691 in National Institute of Bioscience and Human Technology, Agency of Industrial Science and Technology (Higashi 1-1-3, Tsukuba, Ibaraki, Japan) (present name: International Patent Organism Depositary, National Institute of Advanced Industrial Science and Technology (Tsukuba Central 6, 1, Higashi 1-Chome Tsukuba-shi, Ibaraki-ken, Japan)).

(2) Preparation of producing cell using CHO/DG44 cell

After introducing 4 μg of the anti-GD3 chimeric antibody expression vector pChi641LHGM4 into $1.6\times10^6$ cells of CHO/DG44 cell [*Proc. Natl. Acad Sci. USA*, 77, 4216 (1980)] by electroporation [*Cytotechnology*, 3, 133 (1990)], the cells were suspended in 10 ml of IMDM-FBS(10)-HT(1) [IMDM medium (manufactured by LIFE TECHNOLOGIES) comprising 10% FBS (manufactured by LIFE TECHNOLOGIES) and 1× concentration of HT supplement (manufactured by LIFE TECHNOLOGIES)] and dispensed at 200 μl/well into a 96 well culture plate (manufactured by Iwaki Glass). After culturing at 37°C for 24 hours in a 5% $CO_2$ incubator, G418 was added to give a concentration of 0.5 mg/ml, followed by culturing for 1 to 2 weeks. The culture supernatant was recovered from wells in which colonies of transformants showing G418 resistance were formed and growth of colonies was observed, and the antigen binding activity of the anti-GD3 chimeric antibody in the supernatant was measured by the ELISA shown in the item 2 of Reference Example 1.

Regarding the transformants in wells in which production of the anti-GD3 chimeric antibody was observed in culture supernatants, in order to increase the amount of the antibody production using a DHFR gene amplification system, each of them was suspended in an IMDM-dFBS(10) medium [IMDM medium comprising 10% dialyzed fetal

bovine serum (hereinafter referred to as "dFBS"; manufactured by LIFE TECHNOLOGIES)] comprising 0.5 mg/ml G418 and 10 nmol/L MTX to give a density of 1 to $2\times10^5$ cells/ml, and the suspension was dispensed at 0.5 ml into each well of a 24 well plate (manufactured by Iwaki Glass). Transformants showing 10 nmol/L MTX resistance were induced by culturing at 37°C for 1 to 2 weeks in a 5% $CO_2$ incubator. Regarding the transformants in wells in which their growth was observed, the MTX concentration was increased to 100 nmol/L, and a transformant capable of growing in the IMDM-dFBS(10) medium comprising 0.5 mg/ml G418 and 100 nmol/L MTX and of producing the anti-GD3 chimeric antibody in a large amount was finally obtained by the similar method as described above. Cloning was carried out for the obtained transformant by limiting dilution twice, and the obtained transformant cell clone was named DCHI01-20.

2. Measurement of binding activity of antibody to GD3 (ELISA)

The binding activity of the antibody to GD3 was measured as described below.

Into 2 ml of an ethanol solution containing 10 µg of dipalmitoylphosphatidylcholine (manufactured by SIGMA) and 5 µg of cholesterol (manufactured by SIGMA), 4 nmol of GD3 (manufactured by Snow Brand Milk Products) was dissolved. Into each well of a 96 well plate for ELISA (manufactured by Greiner), 20 µl of the solution was dispensed (40 pmol/well in GD3 concentration), followed by air-drying, 1% bovine serum albumin (hereinafter referred to as "BSA"; manufactured by SIGMA)-containing PBS (hereinafter referred to as "1% BSA-PBS") was dispensed at 100 µl/well, and then the reaction was carried out at room temperature for 1 hour to block remaining active groups. After discarding 1% BSA-PBS, a culture supernatant of a transformant or a diluted solution of a human chimeric antibody was dispensed at 50 µl/well to carry out the reaction at room temperature for 1 hour. After the reaction, each well was washed with 0.05% Tween 20 (manufactured by Wako Pure Chemical Industries)-containing PBS (hereinafter referred to as "Tween-PBS"), a peroxidase-labeled goat anti-human IgG (H & L) antibody solution (manufactured by American Qualex) diluted 3,000 times with 1% BSA-PBS was dispensed at 50 µl/well as a secondary antibody solution, and then the reaction was carried out at room temperature for 1 hour. After the reaction and subsequent washing with Tween-PBS, ABTS substrate solution [solution prepared by dissolving 0.55 g of 2,2'-azino-bis(3-ethylbenzothiazoline-6-sulfonic acid) ammonium salt in 1 liter of 0.1 mol/L citrate buffer (pH 4.2) and adding 1 µl/ml of hydrogen peroxide to the solution just before use (hereinafter the same solution was used)] was dispensed at 50 µl/well for color development, and 5 minutes thereafter, the reaction was stopped by adding a 5% SDS solution at 50 µl/well. Then, absorbance at 415 nm (hereinafter referred to as "OD415") was measured.

3. Purification of anti-GD3 chimeric antibody

(1) Culturing of producing cell derived from YB2/0 cell and purification of antibody

The anti-GD3 chimeric antibody-producing transformed cell clone 7-9-51 obtained in the item 1(1) of Reference Example 1 was suspended in the Hybridoma-SFM medium (manufactured by LIFE TECHNOLOGIES) comprising 0.2% BSA, 200 nmol/L MTX and 100 nmol/L triiodothyronine (hereinafter referred to as "T3"; manufactured by SIGMA) to give a density of $3\times10^5$ cells/ml and cultured in a 2.0 liter spinner bottle (manufactured by Iwaki Glass) under stirring at a rate of 50 rpm. After culturing at 37°C for 10 days in a constant temperature chamber, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named YB2/0-GD3 chimeric antibody.

(2) Culturing of producing cell derived from CHO/DG44 cell and purification of antibody

The anti-GD3 chimeric antibody-producing transformed cell clone DCHI01-20 obtained in the item 1(2) of Reference Example 1 was suspended in the EX-CELL302 medium (manufactured by JRH Biosciences) comprising 3 mmol/L L-Gln, 0.5% fatty acid concentrated solution (hereinafter referred to as "CDLC"; manufactured by LIFE TECHNOLOGIES) and 0.3% Pluronic F68 (hereinafter referred to as "PF68"; manufactured by LIFE TECHNOLOGIES) to give a density of $1\times10^6$ cells/ml, and the suspension was dispensed at 50 ml into 175 $mm^2$ flasks (manufactured by Greiner). After culturing at 37°C for 4 days in a 5% $CO_2$ incubator, the culture supernatant was recovered. The anti-GD3 chimeric antibody was purified from the culture supernatant using a Prosep-A (manufactured by Bioprocessing) column in accordance with the manufacture's instructions. The purified anti-GD3 chimeric antibody was named CHO -GD3 chimeric antibody.

4. Analysis of purified anti-GD3 chimeric antibodies

In accordance with a known method [*Nature*, 227, 680 (1970)], 4 μg of each of two types of the purified anti-GD3 chimeric antibodies produced from respective animal cells obtained in the item 3 of Reference Example 1, was subjected to SDS-PAGE to analyze the molecular weight and purity. A single band of about 150 kilodaltons (hereinafter referred to as "Kd") in molecular weight was found under non-reducing conditions, and two bands of about 50 Kd and about 25 Kd under reducing conditions, in each of the purified anti-GD3 chimeric antibodies. The molecular weights almost coincided with the molecular weights deduced from the cDNA nucleotide sequences of H chain and L chain of the antibody (H chain: about 49 Kd, L chain: about 23 Kd, whole molecule: about 144 Kd), and also coincided with the reports stating that the IgG class antibody has a molecular weight of about 150 Kd under non-reducing conditions and is degraded into H chains having a molecular weight of about 50 Kd and L chains having a molecular weight of about 25 Kd under reducing conditions due to cutting of the disulfide bond (hereinafter referred to as "S-S bond") in the molecule [*Antibodies: Laboratory Manual*, Cold Spring Harbor Laboratory (1988); *Monoclonal Antibodies: Principles and Practice,* Academic Press Limited (1996)], so that it was confirmed that each anti-GD3 chimeric antibody was expressed and purified as an antibody molecule having the true structure.

Reference Example 2

1. Preparation of cells stably producing anti-chemokine receptor CCR4 human chimeric antibody

By using an expression vector pKANTEX2160 for an anti-chemokine receptor CCR4 (hereinafter referred to as "CCR4") human chimeric antibody described in WO01/64754, cells capable of stably producing an anti-CCR4 human chimeric antibody (hereinafter referred to as "anti-CCR4 chimeric antibody") were prepared as follows.

(1) Preparation of producing cell using rat myeloma YB2/0 cell

After introducing 10 μg of the anti-CCR4 chimeric antibody expression vector pKANTEX2160 into $4\times10^6$ cells of rat myeloma YB2/0 cell (ATCC CRL 1662) [*J. Cell. Biol.*, 93, 576 (1982)] by electroporation [*Cytotechnology*, 3, 133 (1990)], the cells were suspended in 40 ml of Hybridoma-SFM-FBS(5) [Hybridoma-SFM medium (manufactured by Invitrogen) comprising 5% FBS (manufactured by PAA Laboratories)] and dispensed at 200 μl/well into a 96 well culture plate (manufactured by Sumitomo Bakelite). After culturing at 37°C for 24 hours in a 5% $CO_2$ incubator, G418 was added to give a concentration of 1.0 mg/ml, followed by culturing for 1 to 2 weeks. Culture supernatant was recovered from wells in which growth of transformants showing G418 resistance was observed by the formation of colonies, and antigen binding activity of the anti-CCR4 chimeric antibody in the supernatant was measured by the ELISA described in the item 2 of Reference Example 2.

Regarding the transformants in wells in which production of the anti-CCR4 chimeric antibody was observed in culture supernatants, in order to increase an amount of the antibody production using a DHFR gene amplification system, each of them was suspended in the Hybridoma-SFM-FBS(5) medium comprising 1.0 mg/ml G418 and 50 nmol/L DHFR inhibitor MTX (manufactured by SIGMA) to give a density of 1 to $2\times10^5$ cells/ml, and the suspension was dispensed at 1 ml into each well of a 24 well plate (manufactured by Greiner). After culturing at 37°C for 1 to 2 weeks in a 5% $CO_2$ incubator, transformants showing 50 nmol/L MTX resistance were induced. Antigen binding activity of the anti-CCR4 chimeric antibody in culture supernatants in wells in which growth of transformants was observed was measured by the ELISA described in the item 2 of Reference Example 2.

Regarding the transformants in wells in which production of the anti-CCR4 chimeric antibody was observed in culture supernatants, the MTX concentration was increased to 100 nmol/l and then to 200 nmol/l, and a transformant capable of growing in the Hybridoma-SFM-FBS(5) medium comprising 200 nmol/L MTX and of producing the anti-CCR4 chimeric antibody in a large amount was finally obtained by the similar method as described above. Cloning was carried out for the obtained transformant by limiting dilution twice, and the obtained transformant cell clone was named KM2760#58-35-16. Also, using the determination method of transcription product of α1,6-fucosyltransferase gene described in Example 8 of WO00/61739, a clone producing a relatively low level of the transcription product was selected and used as a suitable clone.

(2) Preparation of producing cell using CHO/DG44 cell

After introducing 4 μg of the anti-CCR4 chimeric antibody expression vector pKANTEX2160 into $1.6\times10^6$ cells of CHO/DG44 cell [*Proc. Natl. Acad. Sci. USA*, 77, 4216 (1980)] by electroporation [*Cytotechnology*, 3, 133 (1990)], the cells were suspended in 10 ml of IMDM-dFBS(10)-HT(1) [IMDM medium (manufactured by Invitrogen) comprising 10% dFBS (manufactured by Invitrogen) and 1× concentration ofHT supplement (manufactured by Invitrogen)] and

dispensed at 100 µl/well into a 96 well culture plate (manufactured by Iwaki Glass). After culturing at 37°C for 24 hours in a 5% $CO_2$ incubator, the medium was changed to IMDM-dFBS(10) (IMDM medium comprising 10% of dialyzed FBS), followed by culturing for 1 to 2 weeks. Culture supernatant was recovered from wells in which the growth was observed due to formation of a transformant showing HT-independent growth, and an expression amount of the anti-CCR4 chimeric antibody in the supernatant was measured by the ELISA described in the item 2 of Reference Example 2.

Regarding the transformants in wells in which production of the anti-CCR4 chimeric antibody was observed in culture supernatants, in order to increase an amount of the antibody production using a DHFR gene amplification system, each of them was suspended in the IMDM-dFBS(10) medium comprising 50 nmol/L MTX to give a density of 1 to $2\times10^5$ cells/ml, and the suspension was dispensed at 0.5 ml into each well of a 24 well plate (manufactured by Iwaki Glass). After culturing at 37°C for 1 to 2 weeks in a 5% $CO_2$ incubator, transformants showing 50 nmol/L MTX resistance were induced. Regarding the transformants in wells in which the growth was observed, the MTX concentration was increased to 200 nmol/L by the similar method as above, and a transformant capable of growing in the IMDM-dFBS(10) medium comprising 200 nmol/L MTX and of producing the anti-CCR4 chimeric antibody in a large amount was finally obtained. The obtained transformant was named clone 5-03.

2. Binding activity of antibody to CCR4 partial peptide (ELISA)

Compound 1 (SEQ ID NO:1) was selected as a human CCR4 extracellular region peptide capable of reacting with the anti-CCR4 chimeric antibody. In order to use it in the activity measurement by ELISA, a conjugate with BSA (manufactured by Nacalai Tesque) was prepared by the following method and used as the antigen. That is, 100 ml of a DMSO solution comprising 25 mg/ml SMCC [4-(*N*-maleimidomethyl)-cyclohexane-1-carboxylic acid *N*-hydroxysuccinimide ester] (manufactured by Sigma) was added dropwise to 900 ml of a 10 mg BSA-containing PBS solution under stirring with a vortex, followed by gently stirring for 30 minutes. To a gel filtration column such as NAP-10 column equilibrated with 25 ml of PBS, 1 ml of the reaction solution was applied and then eluted with 1.5 ml of PBS and the resulting eluate was used as a BSA-SMCC solution (BSA concentration was calculated based on OD280 measurement). Next, 250 ml of PBS was added to 0.5 mg of Compound 1 and then completely dissolved by adding 250 ml of DMF, and the BSA-SMCC solution was added thereto under vortex, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-compound 1 solution.

The prepared conjugate was dispensed at 0.05 µg/ml and 50 µl/well into a 96 well ELISA plate (manufactured by Greiner) and incubated for adhesion at 4°C overnight. After washing each well with PBS, 1% BSA-PBS was added thereto in 100 µl/well and allowed to react at room temperature to block the remaining active groups. After discarding 1% BSA-PBS, culture supernatant of a transformant and variously diluted solutions of a purified human chimeric antibody were added thereto at 50 µl/well and allowed to react at room temperature for 1 hours. After the reaction, each well was washed with Tween-PBS, and then a peroxidase-labeled goat anti-human IgG(H&L) antibody solution (manufactured by American Qualex) diluted 3,000-fold with 1% BSA-PBS as the secondary antibody solution was added at 50 µl/well and allowed to react at room temperature for 1 hour. After the reaction and subsequent washing with Tween-PBS, the ABTS substrate solution was added at 50 µl/well for color development, and 5 minutes thereafter, the reaction was stopped by adding a 5% SDS solution at 50 µl/well. Thereafter, the absorbance at $OD_{415}$ was measured.

3. Purification of anti-CCR4 chimeric antibody

(1) Culturing of producing cell derived from YB2/0 cell and purification of antibody

The anti-CCR4 chimeric antibody-expressing transformant cell clone KM2760#58-35-16 obtained in the item 1(1) of Reference Example 2 was suspended in Hybridoma-SFM (manufactured by Invitrogen) medium comprising 200 nmol/L MTX and 5% of Daigo's GF21 (manufactured by Wako Pure Chemical Industries) to give a density of $2\times10^5$ cells/ml and subjected to fed-batch shaking culturing with a spinner bottle (manufactured by Iwaki Glass) in a constant temperature chamber of 37°C. After culturing for 8 to 10 days and recovering the culture supernatant, the anti-CCR4 chimeric antibody was purified using Prosep-A (manufactured by Millipore) column and gel filtration. The purified anti-CCR4 chimeric antibody was named KM2760-1.

(2) Culturing of producing cell derived from CHO-DG44 cell and purification of antibody

The anti-CCR4 chimeric antibody-producing transformant clone 5-03 obtained in the item 1(2) of Reference Example 2 was cultured at 37°C in a 5% $CO_2$ incubator using IMDM-dFBS(10) medium in a 182 $cm^2$ flask (manufactured by Greiner). When the cell density reached confluent after several days, the culture supernatant was discarded, and

the cells were washed with 25 ml of PBS buffer and then mixed with 35 ml of EXCELL 301 medium (manufactured by JRH Biosciences). After culturing at 37°C for 7 days in a 5% $CO_2$ incubator, the culture supernatant was recovered. The anti-CCR4 chimeric antibody was purified from the culture supernatant using Prosep-A (manufactured by Millipore) column in accordance with the manufacture's instructions. The purified anti-CCR4 chimeric antibody was named KM3060.

4. Analysis of purified anti-CCR4 chimeric antibodies

Each 4 μg of the two types of the anti-CCR4 chimeric antibodies produced by and purified from various animal cells, obtained in the item 3 of Reference Example 2 was subjected to SDS-PAGE in accordance with a known method [*Nature,* 227, 680 (1970)], and the molecular weight and purity were analyzed. In each of the purified anti-CCR4 chimeric antibodies, a single band corresponding to the molecular weight of about 150 Kd was found under non-reducing conditions, and two bands of about 50 Kd and about 25 Kd were found under reducing conditions. The molecular weights almost coincided with the molecular weights deduced from the cDNA nucleotide sequences of antibody H chain and L chain (H chain: about 49 Kd, L chain: about 24 Kd, whole molecule: about 146 Kd) and further coincided with reports stating that an IgG class type antibody has a molecular weight of about 150 Kd under non-reducing conditions and is resolved into H chain having a molecular weight of about 50 Kd and L chain having a molecular weight of about 25 Kd under reducing conditions caused by cutting an S-S bond in the molecule [*Antibodies: A Laboratory Manual*, Cold Spring Harbor Laboratory (1988), *Monoclonal Antibodies: Principles and Practice,* Academic Press Limited (1996)], thus confirming that the anti-CCR4 chimeric antibody was expressed and purified as an antibody molecule having a correct structure.

Reference Example 3

Preparation of anti-fibroblast growth factor-8 chimeric antibody

1. Isolation and analysis of cDNA encoding the V region of a mouse antibody against fibroblast growth factor-8 (hereinafter referred to as "FGF-8")

(1) Preparation of mRNA from hybridoma cells which produces a mouse antibody against FGF-8

About 8 μg of mRNA was prepared from $1 \times 10^7$ cells of a hybridoma KM1334 (FERM BP-5451) which produces a mouse antibody against FGF-8 (anti-FGF-8 mouse antibody), using a mRNA preparation kit Fast Track mRNA Isolation Kit (manufactured by Invitrogen) according to the attached manufacture's instructions.

(2) Production of cDNA libraries of anti-FGF-8 mouse antibody H chain and L chain

A cDNA having *Eco*RI-*Not*I adapters on both termini was synthesized from 5 μg of the KM1334 mRNA obtained in the item 1(1) of Reference Example 3 by using Time Saver cDNA Synthesis Kit (manufactured by Amersham Pharmacia Biotech) according to the attached manufacture's instructions. A full amount of the prepared cDNA was dissolved in 20 μl of sterile water and then fractionated by agarose gel electrophoresis, and about 1.5 kb of a cDNA fragment corresponding to the H chain of an IgG class antibody and about 1.0 kb of a cDNA fragment corresponding to the L chain of a κ class were recovered each at about 0.1 μg. Next, 0.1 μg of the cDNA fragment of about 1.5 kb and 0.1 μg of the cDNA fragment of about 1.0 kb were respectively digested with restriction enzyme *Eco*RI and then ligated with 1 μg of λZAPII vector whose termini had been dephosphorylated with calf intestine alkaline phosphatase, using λZAPII Cloning Kit (manufactured by Stratagene) according to the attached manufacture's instructions.

Using Gigapack II Packaging Extracts Gold (manufactured by Stratagene), 4 μl of each reaction solution after ligation was packaged in λ phage according to the attached manufacture's instructions, and *Escherichia coli* XL1-Blue [*Biotechniques,* 5, 376 (1987)] was infected with an adequate amount of the package to obtain about $8.1 \times 10^4$ and $5.5 \times 10^4$ phage clones as H chain cDNA library and L chain cDNA library, respectively, of KM1334. Next, respective phages were immobilized on a nylon membrane according to a known method [*Molecular Cloning: A Laboratory Manual,* Cold Spring Harbor Lab. Press New York (1989)].

(3) Cloning of cDNAs encoding H chain and L chain of anti-FGF-8 mouse antibody

Nylon membranes of the H chain cDNA library and L chain cDNA library of KM1334 prepared in the item 1 (2) in Reference Example 3 were detected using a cDNA of the C region of a mouse antibody [H chain is a DNA fragment containing mouse Cγ1 cDNA (*J. Immunol*., 146, 2010 (1991)), L chain is a DNA fragment containing mouse

Cκ cDNA (*Cell*, 22, 197 (1980))] as a probe, using ECL Direct Nucleic Acid Labeling and Detection Systems (manufactured by Amersham Pharmacia Biotech) according to the attached manufacture's instructions, and phage clones strongly linked to the probe, 10 clones for each of H chain and L chain, were obtained. Next, each phage clone was converted into a plasmid by the *in vivo* excision method according to the attached manufacture's instructions attached to λZAPII Cloning Kit (manufactured by Stratagene). A nucleotide sequence of a cDNA contained in each of the obtained plasmids was determined by the dideoxy method [*Molecular Cloning: A Laboratory Manual,* Cold Spring Harbor Lab. Press New York (1989)] by using Big Dye Terminator Kit ver. 2 (manufactured by Applied Biosystems). As a result, a plasmid pKM1334H7-1 containing a full length and functional H chain cDNA and a plasmid pKM1334L7-1 containing L chain cDNA, having an ATG sequence considered to be the initiation codon in the 5'-terminal of the cDNA were obtained.

(4) Analysis of amino acid sequence of V region of anti-FGF-8 mouse antibody

A full length nucleotide sequence of VH contained in the plasmid pKM1334H7-1 and a deduced complete length amino acid sequence are represented by SEQ ID NO:14 and SEQ ID NO:15, respectively, and a full length nucleotide sequence of VL contained in the plasmid pKM1334L7-1 and a deduced complete length amino acid sequence are represented by SEQ ID NO:16 and SEQ ID NO:17, respectively. As a result of comparing these sequences to both known sequence data of mouse antibodies [*Sequences of Proteins of Immunological Interest,* U.S. Dept. Health and Human Services (1991)] and the comparison with the results of analysis of N-terminal amino acid sequences of H chain and L chain of the purified anti-FGF-8 mouse antibody KM1334, carried out by their automatic Edman degradation using a protein sequencer PPSQ-10 (manufactured by Shimadzu), it was found that each of the isolated cDNA is a full length cDNA encoding the anti-FGF-8 mouse antibody KM1334 containing a secretory signal sequence, and positions 1 to 19 in the amino acid sequence represented by SEQ ID NO:15 and positions 1 to 19 in the amino acid sequence described in SEQ ID NO:17 are secretory signal sequences of H chain and L chain, respectively.

Next, novelty of the amino acid sequences (sequences excluding secretory signal sequence) of VH and VL of the anti-FGF-8 mouse antibody KM1334 was examined. Using GCG Package (version 9.1, manufactured by Genetics Computer Group) as a sequence analyzing system, an amino acid sequence data base of known proteins (PIR-Protein (Release 56.0)) was searched by the BLAST method [*J. Mol. Biol.*, 215, 403 (1990)]. As a result, completely coincided sequences were not found for both of the H chain and L chain, so that it was confirmed that the VH and VL of the anti-FGF-8 mouse antibody KM1334 are novel amino acid sequences.

Also, the CDR of VH and VL of the anti-FGF-8 mouse antibody KM1334 was identified by comparing with amino acid sequences of known antibodies. Amino acid sequences of CDR 1, 2 and 3 of VH of the anti-FGF-8 mouse antibody KM1334 are represented by SEQ ID NOs:18, 19 and 20, respectively, and amino acid sequences of CDR 1, 2 and 3 of VL in SEQ ID NOs:21, 22 and 23, respectively.

2. Stable expression of anti-FGF-8 chimeric antibody using animal cell

(1) Construction of anti-FGF-8 chimeric antibody expression vector pKANTEX1334

An anti-FGF-8 chimeric antibody expression vector pKANTEX1334 was constructed as follows using the vector pKANTEX93 for humanized antibody expression described in WO97/10354 and the plasmids pKM1334H7-1 and pKM1334L7-1 obtained in the item 1(3) of Reference Example 3.

Using 50 ng of the plasmid pKM1334H7-1 obtained in the item 1(3) of Reference Example 3 as the template and by adding synthetic DNAs having the nucleotide sequences described in SEQ ID NOs:24 and 25 (manufactured by GENSET) as primers to give a final concentration of 0.3 μM, PCR were carried out in a system of 50 μl by first heating at 94°C for 2 minutes and subsequent 30 cycles of heating at 94°C for 15 seconds, at 55°C for 30 seconds and at 68°C for 1 minute according to the attached manufacture's instructions attached to KOD plus polymerase (manufactured by TOYOBO). The reaction solution was precipitated with ethanol, dissolved in sterile water and then allowed to react at 37°C for 1 hour by using 10 units of a restriction enzyme *Apa*I (manufactured by Takara Shuzo) and 10 units of a restriction enzyme *Not*I (manufactured by New England Biolabs). About 0.3 μg of an *Apa*I-*Not*I fragment of about 0.47 kb was recovered By fractionating the reaction solution by agarose gel electrophoresis.

Next, 3 μg of the vector pKANTEX93 for humanized antibody expression was allowed to react at 37°C for 1 hour by using 10 units of restriction enzyme *Apa*I (manufactured by Takara Shuzo) and 10 units of restriction enzyme *Not*I (manufactured by New England Biolabs). About 2 μg of an *Apa*I-*Not*I fragment of about 12.75 kb was recovered, by fractionating the reaction solution by an agarose gel electrophoresis.

Next, 0.1 μg of the *Not*I-*Apa*I fragment derived from the PCR product and 0.1 μg of the *Not*I-*Apa*I fragment derived from the plasmid pKANTEX93, obtained in the above, were added to 10 μl of sterile water in total amount and ligated by using Ligation High (manufactured by TOYOBO). The plasmid pKANTEX1334H shown in Fig. 21 was obtained by transforming *Escherichia coli* JM109 by using the recombinant plasmid DNA solution obtained in this manner.

Next, using 50 ng of the plasmid pKM1334L7-1 obtained in the item 1(3) of Reference Example 3 as the template and by adding synthetic DNAs having the nucleotide sequences described in SEQ ID NOs:26 and 27 (manufactured by GENSET) as primers to give a final concentration of 0.3 μM, PCR was carried out in a system of 50 μl by first heating at 94°C for 2 minutes and subsequent 30 cycles of heating at 94°C for 15 seconds, at 55°C for 30 seconds and 68°C for 1 minute according to the attached manufacture's instructions attached to KOD plus polymerase (manufactured by TOYOBO). The reaction solution was precipitated with ethanol, dissolved in sterile water and then allowed to react at 37°C for 1 hour by using 10 units of a restriction enzyme *Eco*RI (manufactured by Takara Shuzo) and 10 units of a restriction enzyme *Bsi*WI (manufactured by New England Biolabs). About 0.3 μg of an *Eco*RI-*Bsi*WI fragment of about 0.44 kb was recovered by fractionating the reaction solution by agarose gel electrophoresis.

Next, 3 μg of the plasmid pKANTEX1134H obtained in the above was allowed to react at 37°C for 1 hour by using 10 units of a restriction enzyme *Eco*RI (manufactured by Takara Shuzo) and a restriction enzyme *Bsi*WI (manufactured by New England Biolabs). About 2 μg of an *Eco*RI-*Bsi*WI fragment of about 13.20 kb was recovered by fractionating said reaction solution by an agarose gel electrophoresis.

Next, 0.1 μg of the *Eco*RI-*Bsi*WI fragment derived from the PCR product and 0.1 μg of the *Eco*RI-*Bsi*WI fragment derived from the plasmid pKANTEX1334H, obtained in the above, were added to 10 μl of sterile water in total amount and ligated by using Ligation High (manufactured by TOYOBO). The plasmid pKANTEX1334 shown in Fig. 21 was obtained by transforming *Escherichia coli* JM109 using the recombinant plasmid DNA solution obtained in this manner.

As a result of carrying out analysis of a nucleotide sequence using 400 ng of the obtained plasmid by the dideoxy method (*Molecular Cloning,* Second Edition) using Big Dye Terminator Kit ver. 2 (manufactured by Applied Biosystems), it was confirmed that a plasmid comprising a cloned DNA of interest was obtained.

3. Preparation of anti-fibroblast growth factor-8 human chimeric antibody

1. Preparation of cells stably producing anti-fibroblast growth factor-8 human chimeric antibody

By using an expression vector pKANTEX134 of an anti-FGF-8 human chimeric antibody described in the item 2 of Reference Example 3, cells stably producing the anti-FGF-8 human chimeric antibody (hereinafter referred to as "anti-FGF-8 chimeric antibody") was prepared as follows.

(1) Preparation of producing cell using rat myeloma YB2/0 cell

After introducing 10 μg of the anti-FGF-8 chimeric antibody expression vector pKANTEX1334 into $4\times10^6$ cells of rat myeloma YB2/0 cell [ATCC CRL 1662; *J. Cell. Biol.*, 93, 576 (1982)] by electroporation [*Cytotechnology*, 3, 133 (1990)], the cells were suspended in 40 ml of Hybridoma-SFM-FBS(5) and dispensed at 200 μl/well into a 96 well culture plate (manufactured by Sumitomo Bakelite). After culturing at 37°C for 24 hours in a 5% $CO_2$ incubator, G418 was added to give a concentration of 0.5 mg/ml, followed by culturing for 1 to 2 weeks. Culture supernatants were recovered from wells in which colonies of transformants showing G418 resistance were formed and their growth was confirmed, and antigen-binding activity of the anti-FGF-8 chimeric antibody in the supernatants was measured by the ELISA described in the item 4 of Reference Example 3.

Regarding the transformants in wells in which production of the anti-FGF-8 chimeric antibody was found in the culture supernatants, in order to increase the antibody production amount by using a *dhfr* gene amplification system, each of them was suspended to give a density of 1 to $2\times10^5$ cells/ml in the Hybridoma-SFM-FBS(5) medium containing 0.5 mg/ml G418 and 50 nmol/l DHFR inhibitor MTX (manufactured by SIGMA) and dispensed at 1 ml into each well of a 24 well plate (manufactured by Greiner). After culturing at 37°C for 1 to 2 weeks in a 5% $CO_2$ incubator, transformants showing 50 nmol/l MTX resistance were induced. Antigen-binding activity of the anti-FGF-8 chimeric antibody in culture supernatants in wells where growth of transformants was observed was measured by the ELISA described in the item 4 of Reference Example 3.

Regarding the transformants in wells in which production of the anti-FGF-8 chimeric antibody was found in culture supernatants, the MTX concentration was increased to 100 nmol/l and then to 200 nmol/l by a method similar to the above to thereby finally obtain a transformant 5-D capable of growing in the Hybridoma-SFM-FBS(5) medium containing 0.5 mg/ml G418 and 200 nmol/l MTX and also highly producing the anti-FGF-8 chimeric antibody. The resulting transformant was subjected to cloning by limiting dilution, and the resulting transformant cell clone was named 5-D-10. Also, using the determination method of transcription product of α1,6-fucosyltransferase gene described in Example 8 of WO00/61739, a clone producing a relatively low level of the transcription product was selected and used as a suitable clone.

(2) Preparation of producing cell using CHO/DG44 cell

In accordance with the method described in the item 1(2) of Reference Example 2, the anti-FGF-8 chimeric antibody expression plasmid pKANTEX1334 was introduced into CHO/DG44 cell and gene amplification was carried out by using the drug MTX to obtain a transformant highly producing the anti-FGF-8 chimeric antibody. The antibody expression amount was measured using the ELISA described in the item 4 of Reference Example 3. The resulting transformant was cloned twice by limiting dilution, and the resulting transformant cell clone was named 7-D-1-5.

4. Binding activity of antibody to FGF-8 partial peptide (ELISA)

Compound 2 (SEQ ID NO:2) was selected as a human FGF-8 peptide with which the anti-FGF-8 chimeric antibody can react. For the activity measurement by the ELISA, a conjugate with BSA (manufactured by Nacalai Tesque) was prepared by the following method and used as the antigen. That is, 100 ml of a 25 mg/ml SMCC [4-(*N*-maleimidomethyl)cyclohexane-1-carboxylic acid *N*-hydroxysuccinimide ester] (manufactured by SIGMA)-DMSO solution was added dropwise to 900 ml of a PBS solution containing 10 mg of BSA under stirring, followed by slowly stirred for 30 minutes. To a gel filtration column such was NAP-10 column or the like which had been equilibrated with 25 ml of PBS, 1 ml of the reaction solution was applied, and the eluate eluted with 1.5 ml of PBS was used as a BSA-SMCC solution (BSA concentration was calculated from OD280 measurement). Next, 250 ml of PBS was added to 0.5 mg of Compound 2, 250 ml of DMF was added thereto and completely dissolved, and then the above BSA-SMCC solution (1.25 mg as BSA) was added thereto under stirring, followed by slow stirring for 3 hours. The reaction solution was dialyzed against PBS at 4°C overnight, sodium azide was added thereto to give a final concentration of 0.05% and then filtered through a 0.22 µm filter and used as a BSA-compound 2 solution.

The conjugate prepared in the above was dispensed at 1 µg/ml and 50 µl/well into a 96 well plate for ELISA (manufactured by Greiner) and adhered thereto by allowing it to stand at 4°C overnight. After washing with PBS, 1% BSA-PBS was added at 100 µl/well and allowed to react at room temperature for 1 hour to block the remaining active groups. After 1% BSA-PBS was discarded, culture supernatant of the transformant or each of various dilution solutions of purified chimeric antibody was added at 50 µl/well and allowed to react at room temperature for 1 hour. After washing each well with Tween-PBS, culture supernatant of a transformant or a purified antibody was added at 50 µl/well and allowed to react at room temperature for 1 hour. After the reaction and subsequent washing of each well with Tween-PBS, a peroxidase-labeled goat anti-human IgG (H&L) antibody solution (manufactured by American Qualex) diluted 3,000-fold with 1% BSA-PBS was added as a secondary antibody solution at 50 µl/well and allowed to react at room temperature for 1 hour. After the reaction and subsequent washing with Tween-PBS, the ABTS substrate solution was added at 50 µl/well to develop color, and the reaction was stopped 15 minutes thereafter by adding 5% SDS solution at 50 µl/well. Thereafter, OD415 was measured.

5. Purification of anti-FGF-8 chimeric antibody

(1) Culturing of YB2/0 cell-derived producing cell and purification of antibody

The anti-FGF-8 chimeric antibody-expressing transformant 5-D obtained in the item 3(1) of Reference Example 3 was cultured in Hybridoma-SFM (manufactured by Invitrogen) medium containing 200 nmol/l of MTX and 5% Daigo's GF21 (manufactured by Wako Pure Chemical Industries) in a 182 $cm^2$ flask (manufactured by Greiner) at 37°C in a 5% $CO_2$ incubator. After culturing for 8 to 10 days, the anti-FGF-8 chimeric antibody was purified from the culture supernatant recovered by using Prosep-A (manufactured by Millipore) column in accordance with the attached manufacture's instructions. The purified anti-FGF-8 chimeric antibody was named YB2/0-FGF8 chimeric antibody.

(2) Culturing of CHO-DG44 cell-derived antibody-producing cells and purification of antibody

The anti-FGF-8 chimeric antibody-producing transformant cell clone 7-D-1-5 obtained in the item 3(2) of Reference Example 3 was cultured in the IMDM-dFBS(10) medium in a 182 $cm^2$ flask (manufactured by Greiner) at 37°C in a 5% $CO_2$ incubator. At the stage where the cell density reached confluent several days thereafter, the culture supernatant was discarded, the cells were washed with 25 ml of PBS buffer and then 35 ml of EXCELL301 medium (manufactured by JRH Biosciences) was added thereto. After the culturing for 7 days at 37°C in a 5% $CO_2$ incubator, the culture supernatant was recovered. The anti-FGF-8 chimeric antibody was purified from the culture supernatant by using Prosep-A (manufactured by Millipore) column in accordance with the manufacture's instructions. The purified anti-FGF-8 chimeric antibody was named CHO-FGF8 chimeric antibody.

6. Analysis of purified anti-FGF-8 chimeric antibody

Each 4 µg of the two anti-FGF-8 chimeric antibodies produced by respective animal cells and purified in the item 5 of Reference Example 3 was subjected to SDS-PAGE according to a known method [*Nature,* 227, 680 (1970)] and the molecular weight and purity were analyzed. In each of the purified anti-FGF-8 chimeric antibodies, a single band of about 150 Kd in molecular weight was found under non-reducing conditions and two bands of about 50 Kd and about 25 Kd were found under reducing conditions. These molecular weights almost coincided with the molecular weights deduced from the cDNA nucleotide sequences of the antibody H chain and L chain (H chain: about 50 Kd, L chain: about 24 Kd, whole molecule: about 148 Kd), and also coincided with the reports showing that the IgG class antibody shows a molecular weight of about 150 Kd under non-reducing conditions and is degraded into H chain having a molecular weight of about 50 Kd and L chain having a molecular weight of about 25 Kd under reducing conditions due to cleavage of the intramolecular S-S bond [*Antibodies: A Laboratory Manual,* Cold Spring Harbor Laboratory (1988); *Monoclonal Antibodies: Principles and Practice,* Academic Press Limited (1996)]. Thus it was confirmed that the anti-FGF-8 chimeric antibodies were expressed and purified as antibody molecules having correct structures.

7. Binding activity of anti-FGF-8 chimeric antibody to FGF-8 partial peptide (ELISA)

Binding activities of the two types of the purified anti-FGF-8 chimeric antibodies produced by various animal cells obtained in the item 5 of Reference Example 3 to an FGF-8 partial peptide were measured by the ELISA shown in the item 4 of Reference Example 3.

Fig. 22 shows results of the examination of the binding activity measured by changing the concentration of the anti-FGF-8 chimeric antibody to be added. As shown in Fig. 22, the two types of the anti-FGF-8 chimeric antibodies showed the similar binding activity to the FGF-8 partial peptide. The result shows that antigen binding activities of these antibodies are constant independently of the types of the antibody-producing animal cells.

Reference Example 4

Preparation of an anti-CD20 human chimeric antibody:

1. Preparation of anti-CD20 human chimeric antibody expression vector

(1) Construction of cDNA encoding VL of anti-CD20 mouse monoclonal antibody

A cDNA (represented by SEQ ID NO:30) encoding the amino acid sequence of VL of an anti-CD20 mouse monoclonal antibody 2B8 described in WO94/11026 was constructed by PCR as follows.

First, nucleotide sequences of amplified DNA primers at the time of the PCR including restriction enzyme recognizing nucleotide sequences for cloning into a vector for humanized antibody expression were added to the 5'-terminal and 3'-terminal of the nucleotide sequence of the VL described in WO94/11026. A designed nucleotide sequence was divided from the 5'-terminal side into a total of 6 nucleotide sequences each having about 100 bases (adjacent nucleotide sequences are designed in such a manner that their termini have an overlapping sequence of about 20 nucleotides), and 6 synthetic DNA fragments, actually those represented by SEQ ID NOs:31, 32, 33, 34, 35 and 36, were prepared from them in alternate order of a sense chain and an antisense chain (consigned to GENSET).

Each oligonucleotide was added to 50 µl of a reaction mixture [KOD DNA polymerase-attached PCR Buffer #1 (manufactured by TOYOBO), 0.2 mM dNTPs, 1 mM magnesium chloride, 0.5 µM M13 primer M4 (manufactured by Takara Shuzo) and 0.5 µM M13 primer RV (manufactured by Takara Shuzo)] to give a final concentration of 0.1 µM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO) thereto, subsequent 25 cycles of heating at 94°C for 30 seconds, 55°C for 30 seconds and 74°C for 1 minute as one cycle and then further heating at 72°C for 10 minutes. After 25 µl of the reaction mixture was subjected to agarose gel electrophoresis, a VL PCR product of about 0.44 kb was recovered by using QIAquick Gel Extraction Kit (manufactured by QIAGEN).

Next, 0.1 µg of a DNA obtained by digesting a plasmid pBluescript II SK(-) (manufactured by Stratagene) with a restriction enzyme *Sma*I (manufactured by Takara Shuzo) and about 0.1 µg of the PCR product obtained in the above were added to sterile water to adjust the total volume to 7.5 µl, and then 7.5 µl of solution I of TAKARA ligation kit ver. 2 (manufactured by Takara Shuzo) and 0.3 µl of the restriction enzyme *Sma*I (manufactured by Takara Shuzo) were added thereto for the reaction at 22°C for 2 hours. Using the recombinant plasmid DNA solution obtained in this manner, *E. coli* DH5α strain (manufactured by TOYOBO) was transformed. Each plasmid DNA was prepared from the transformant clones and allowed to react using BigDye Terminator Cycle Sequencing Ready Reaction Kit v2.0 (manufactured

by Applied Biosystems) in accordance with the manufacture's instructions attached thereto, and then the nucleotide sequence was analyzed by a DNA sequencer ABI PRISM 377 manufactured by the same company. In this manner, plasmid pBS-2B8L shown in Fig. 23 having the nucleotide sequence of interest was obtained.

(2) Construction of cDNA encoding VH of anti-CD20 mouse monoclonal antibody

A cDNA (represented by SEQ ID NO:37) encoding the amino acid sequence of VH of the anti-CD20 mouse monoclonal antibody 2B8 described in WO94/11026 was constructed by PCR as follows.

First, nucleotide sequences of amplified DNA primers at the time of PCR including a restriction enzyme recognizing sequence for cloning into a vector for humanized antibody expression were added to the 5'-terminal and 3'-terminal of the nucleotide sequence of the VH described in WO94/11026. A designed nucleotide sequence was divided from the 5'-terminal side into a total of 6 nucleotide sequences each having about 100 bases (adjacent nucleotide sequences are designed in such a manner that their termini have an overlapping sequence of about 20 bases), and 6 synthetic DNA fragments, actually those represented by SEQ ID NOs:38, 39, 40, 41, 42 and 43, were prepared from them in alternate order of a sense chain and an antisense chain (consigned to GENSET).

Each oligonucleotide was added to 50 µl of a reaction mixture [KOD DNA polymerase-PCR Buffer #1 (manufactured by TOYOBO), 0.2 mM dNTPs, 1 mM magnesium chloride, 0.5 µM M13 primer M4 (manufactured by Takara Shuzo) and 0.5 µM M13 primer RV (manufactured by Takara Shuzo)] to give a final concentration of 0.1 µM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO), subsequent 25 cycles of heating at 94°C for 30 seconds, 55°C for 30 seconds and 74°C for 1 minute as one cycle and then heating at 72°C for 10 minutes. After 25 µl of the reaction mixture was subjected to agarose gel electrophoresis, a VH PCR product of about 0.49 kb was recovered by using QIAquick Gel Extraction Kit (manufactured by QIAGEN).

Next, 0.1 µg of a DNA obtained by digesting the plasmid pBluescript II SK(-) (manufactured by Stratagene) with the restriction enzyme *Sma*I (manufactured by Takara Shuzo) and about 0.1 µg of the PCR product obtained in the above were added to sterile water to adjust the total volume to 7.5 µl, and then 7.5 µl of solution I of TAKARA ligation kit ver. 2 (manufactured by Takara Shuzo) and 0.3 µl of the restriction enzyme *Sma*I (manufactured by Takara Shuzo) were added thereto to carry out the reaction at 22°C overnight.

Using the recombinant plasmid DNA solution obtained in this manner, *E. coli* DH5α strain (manufactured by TOYOBO) was transformed. Each plasmid DNA was prepared from the transformant clones and allowed to react using BigDye Terminator Cycle Sequencing Ready Reaction Kit v2.0 (manufactured by Applied Biosystems) in accordance with the manufacture's instructions attached thereto, and then the nucleotide sequence was analyzed by the DNA sequencer ABI PRISM 377 manufactured by the same company. In this manner, the plasmid pBS-2B8H shown in Fig. 24 comprising the nucleotide sequence of interest was obtained.

Next, in order to substitute the amino acid residue at position 14 from Ala to Pro, the synthetic DNA represented by SEQ ID NO:45 was designed, and base substitution was carried out by PCR using LA PCR *in vitro* Mutagenesis Primer Set for pBluescript II (manufactured by Takara Shuzo) as follows. After 50 µl of a reaction mixture [LA PCR Buffer II (manufactured by Takara Shuzo), 2.5 units of TaKaRa LA Taq, 0.4 mM dNTPs, 2.5 mM magnesium chloride, 50 nM T3 BcaBEST Sequencing primer (manufactured by Takara Shuzo) and 50 nM of the primer for mutagenesis (SEQ ID NO:44, manufactured by GENSET)] containing 1 ng of the plasmid pBS-2B8H was prepared, the PCR was carried out by using a DNA thermal cycler GeneAmp PCR System 9600 (manufactured by Perkin Elmer) by 25 cycles of heating at 94°C for 30 seconds, 55°C for 2 minutes and 72°C for 1.5 minutes as one cycle. After 30 µl of the reaction mixture was subjected to agarose gel electrophoresis, a PCR product of about 0.44 kb was recovered by using QIAquick Gel Extraction Kit (manufactured by QIAGEN) and made into 30 µl of an aqueous mixture. In the same manner, PCR was carried out by using 50 µl of a reaction mixture [LA PCR Buffer II (manufactured by Takara Shuzo), 2.5 units of TaKaRa LA Taq, 0.4 mM dNTPs, 2.5 mM magnesium chloride, 50 nM T7 BcaBEST Sequencing primer (manufactured by Takara Shuzo) and 50 nM MUT B1 primer (manufactured by Takara Shuzo)] containing 1 ng of the plasmid pBS-2B8H. After 30 µl of the reaction mixture was subjected to agarose gel electrophoresis, a PCR product of about 0.63 kb was recovered by using QIAquick Gel Extraction Kit (manufactured by QIAGEN) and made into 30 µl of aqueous solution. Next, 0.5 µl of each of 0.44 kb PCR product and 0.63 kb PCR product thus obtained were added to 47.5 µl of a reaction mixture [LA PCR Buffer II (manufactured by Takara Shuzo), 0.4 mM dNTPs, and 2.5 mM magnesium chloride], and using a DNA thermal cycler GeneAmp PCR System 9600 (manufactured by Perkin Elmer), annealing of the DNA was carried out by heating the reaction mixture at 90°C for 10 minutes, cooling it to 37°C over 60 minutes and then keeping it at 37°C for 15 minutes. After carrying out the reaction at 72°C for 3 minutes by adding 2.5 units of TaKaRa LA Taq (manufactured by Takara Shuzo), 10 pmol of each of T3 BcaBEST Sequencing primer (manufactured by Takara Shuzo) and T7 BcaBEST Sequencing primer (manufactured by Takara Shuzo) were added thereto to give the reaction mixture of 50 µl, which was subjected to 10 cycles of heating at 94°C for 30 seconds, 55°C for 2 minutes and 72°C for 1.5 minutes as one cycle. After 25 µl of the reaction mixture was purified using QIA quick PCR purification

kit (manufactured by QIAGEN), a half volume thereof was allowed to react at 37°C for 1 hour using 10 units of a restriction enzyme *Kpn*I (manufactured by Takara Shuzo) and 10 units of a restriction enzyme *Sac*I (manufactured by Takara Shuzo). The reaction mixture was fractionated by using agarose gel electrophoresis to recover a *Kpn*I-*Sac*I fragment of about 0.59 kb.

Next, 1 μ g of pBluescript II SK(-) (manufactured by Stratagene) was allowed to react at 37°C for 1 hour by using 10 units of the restriction enzyme *Kpn*I (manufactured by Takara Shuzo) and 10 units of the restriction enzyme *Sac*I (manufactured by Takara Shuzo), and then the reaction mixture was subjected to agarose gel electrophoresis to recover a *Kpn*I-*Sac*I fragment of about 2.9 kb.

The PCR product-derived *Kpn*I-*Sac*I fragment and plasmid pBluescript II SK(-)-derived *Kpn*I-*Sac*I fragment thus obtained were ligated by using Solution I of DNA Ligation Kit Ver. 2 (manufactured by Takara Shuzo) in accordance with the manufacture's instructions attached thereto. Using the recombinant plasmid DNA solution obtained in this manner, *E. coli* DH5α strain (manufactured by TOYOBO) was transformed. Each plasmid DNA was prepared from the transformant clones, and allowed to react by using BigDye Terminator Cycle Sequencing Ready Reaction Kit v2.0 (manufactured by Applied Biosystems) in accordance with the manufacture's instructions attached thereto, and then the nucleotide sequence was analyzed by the DNA sequencer ABI PRISM 377 manufactured by the same company.

In this manner, plasmid pBS-2B8Hm shown in Fig. 24 comprising the nucleotide sequence of interest was obtained.

(3) Construction of anti-CD20 human chimeric antibody expression vector

An anti-CD20 human chimeric antibody (hereinafter referred to as "anti-CD20 chimeric antibody") expression vector pKANTEX2B8P was constructed as follows by using pKANTEX93, a vector for expression of humanized antibody [*Mol. Immunol.*, 37, 1035 (2000)] and the plasmids pBS-2B8L and pBS-2B8Hm obtained in items 1(1) and (2) of this Reference Example 4(E1).

After 2 μg of the plasmid pBS-2B8L obtained in item 1(1) in Reference Example 4(E1) was allowed to react at 55°C for 1 hour by using 10 units of a restriction enzyme *Bsi*WI (manufactured by New England Biolabs), followed by reaction at 37°C for 1 hour using 10 units of a restriction enzyme *Eco*RI (manufactured by Takara Shuzo). The reaction mixture was fractionated by agarose gel electrophoresis to recover a *Bsi*WI-*Eco*RI fragment of about 0.41 kb.

Next, 2 μg of pKANTEX93, a vector for expression of humanized antibody, was allowed to react at 55°C for 1 hour by using 10 units of the restriction enzyme *Bsi*WI (manufactured by New England Biolabs), followed by reaction at 37°C for 1 hour using 10 units of the restriction enzyme *Eco*RI (manufactured by Takara Shuzo). The reaction mixture was fractionated by agarose gel electrophoresis to recover a *Bsi*WI-*Eco*RI fragment of about 12.75 kb.

Next, the plasmid pBS-2B8L-derived *Bsi*WI-*Eco*RI fragment and plasmid pKANTEX93-derived *Bsi*WI-*Eco*RI fragment thus obtained were ligated by using Solution I of DNA Ligation Kit Ver. 2 (manufactured by Takara Shuzo) in accordance with the manufacture's instructions attached thereto. By using the recombinant plasmid DNA solution obtained in this manner, *E. coli* DH5α strain (manufactured by TOYOBO) was transformed to obtain plasmid pKANTEX2B8-L shown in Fig. 25.

Next, 2 μg of the plasmid pBS-2B8Hm obtained in item 1(2) of Reference Example 4(E1) was allowed to react at 37°C for 1 hour by using 10 units of a restriction enzyme *Apa*I (manufactured by Takara Shuzo), followed by reaction at 37°C for 1 hour using 10 units of a restriction enzyme *Not*I (manufactured by Takara Shuzo). The reaction mixture was fractionated by agarose gel electrophoresis to recover an *Apa*I-*Not*I fragment of about 0.45 kb.

Next, 3 μg of the plasmid pKANTEX2B8-L was allowed to react at 37°C for 1 hour by using 10 units of the restriction enzyme *Apa*I (manufactured by Takara Shuzo), followed by reaction at 37°C for 1 hour using 10 units of the restriction enzyme *Not*I (manufactured by Takara Shuzo). The reaction mixture was fractionated by agarose gel electrophoresis to recover an *Apa*I-*Not*I fragment of about 13.16 kb.

Next, the plasmid pBS-2B8Hm-derived *Apa*I-*Not*I fragment and plasmid pKANTEX2B8-L-derived *Apa*I-*Not*I fragment thus obtained were ligated by using Solution I of DNA Ligation Kit Ver. 2 (manufactured by Takara Shuzo) in accordance with the manufacture's instructions attached thereto. *E. coli* DH5α strain (manufactured by TOYOBO) was transformed by using the recombinant plasmid DNA solution obtained in this manner, and each plasmid DNA was prepared from the transformant clones.

The nucleotide sequence of the thus obtained plasmid was analyzed by using BigDye Terminator Cycle Sequencing Ready Reaction Kit v 2.0 (manufactured by Applied Biosystems) and the DNA sequencer 377 of the same company, and it was confirmed that the plasmid pKANTEX2B8P shown in Fig. 25 into which the DNA of interest had been cloned was obtained.

2. Stable expression of anti-CD20 chimeric antibody by using animal cell

(1) Preparation of production cell by using rat myeloma YB2/0 cell

The anti-CD20 chimeric antibody was expressed in animal cells by using the anti-CD20 chimeric antibody expression vector, pKANTEX2B8P, obtained in item 1(3) of Reference Example 4(E1) as follows.

After 10 μg of the plasmid pKANTEX2B8P was introduced into 4×$10^6$ cells of a rat myeloma cell line, YB2/0 (ATCC CRL 1662) by electroporation [*Cytotechnology*, 3, 133 (1990)], the cells were suspended in 40 ml of H-SFM medium (manufactured by GIBCO-BRL supplemented with 5% fetal calf serum (FCS)) and dispensed at 200 μl/well into a 96 well microtiter plate (manufactured by Sumitomo Bakelite). After culturing at 37°C for 24 hours in a 5% $CO_2$ incubator, G418 was added thereto to give a concentration of 1 mg/ml, followed by culturing for 1 to 2 weeks. Culture supernatants were recovered from wells where colonies of transformants showing G418 resistance were formed and transformants became confluent, and the produced amount of the human IgG antibody in the culture supernatant was measured by ELISA described in item 2(2) of this Reference Example 4(E1).

Regarding a transformant in a well where expression of human IgG antibody was found in the culture supernatant, in order to increase the amount of antibody expression using a *dhfr* gene amplification system, it was suspended in H-SFM medium containing 1 mg/ml G418 and 50 nM methotrexate (hereinafter referred to as "MTX", manufactured by SIGMA) as an inhibitor of the *dhfr* gene product dihydrofolate reductase (hereinafter referred to as "DHFR") to give a density of 1 to 2×$10^5$ cells/ml, and the suspension was dispensed at 1 ml into each well of a 24 well plate (manufactured by Greiner). Culturing was carried out at 37°C for 1 to 2 weeks in a 5% $CO_2$ incubator to induce transformants showing 50 nM MTX resistance. When a transformant became confluent in a well, the produced amount of the human IgG antibody in the culture supernatant was measured by ELISA described in item 2(2) of this Reference Example 4 (E1). Regarding a transformant in well where expression of human IgG antibody was found in the culture supernatant, the MTX concentration was increased to 100 nM and then to 200 nM by the similar method to the above to finally obtain a transformant capable of growing in H-SFM medium containing 1 mg/ml G418 and 200 nM MTX and also performing high expression of the anti-CD20 chimeric antibody. The obtained transformant was made into a single clone (cloning) by limiting dilution to obtain a clone KM3065 which expresses an anti-CD20 chimeric antibody. Also, using the determination method of transcription product of α1,6-fucosyltransferase gene described in Example 8 of WO00/61739, a clone producing a relatively small amount of the transcription product was selected and used as a suitable clone.

The obtained transformant clone KM3065 which produces the anti-CD20 chimeric antibody has been deposited on December 21, 2001, as FERM 7834 in International Patent Organism Depositary, National Institute of Advanced Industrial Science and Technology (Tsukuba Central 6, 1, Higashi 1-Chome Tsukuba-shi, Ibarakiken, Japan).

(2) Measurement of human IgG antibody concentration in culture supernatant (ELISA)

A goat anti-human IgG (H & L) antibody (manufactured by American Qualex) was diluted with a phosphate buffered saline (hereinafter referred to as "PBS") to give a concentration of 1 μg/ml, dispensed at 50 μl/well into a 96 well plate for ELISA (manufactured by Greiner) and then allowed to stand at 4°C overnight for adhesion. After washing with PBS, 1% bovine serum albumin (hereinafter referred to as "BSA"; manufactured by AMPC)-containing PBS (hereinafter referred to as "1% BSA-PBS") was added thereto at 100 μl/well and allowed to react at room temperature for 1 hour to block the remaining active groups. After discarding 1% BSA-PBS, culture supernatant of a transformant and variously diluted solutions of a purified human chimeric antibody were added thereto at 50 μl/well and allowed to react at room temperature for 2 hours. After the reaction, each well was washed with 0.05% Tween 20-containing PBS (hereinafter referred to as "Tween-PBS"), and then, as a secondary antibody solution, a peroxidase-labeled goat anti-human IgG (H & L) antibody solution (manufactured by American Qualex) 3,000 fold-diluted with 1% BSA-PBS was added thereto at 50 μl/well and allowed to react at room temperature for 1 hour. After the reaction and subsequent washing with Tween-PBS, an ABTS substrate solution (a solution prepared by dissolving 0.55 g of 2,2'-azino-bis (3-ethylbenzothiazoline-6-sulfonic acid)ammonium in 1 liter of 0.1 M citrate buffer (pH 4.2), and adding 1 μl/ml hydrogen peroxide just before use) was dispensed at 50 μl/well for coloration, and the absorbance at 415 nm (hereinafter referred to as "$OD_{415}$") was measured.

3. Purification of anti-CD20 chimeric antibody from culture supernatant

The transformant cell clone KM3065 capable of expressing the anti-CD20 chimeric antibody, obtained in item 2(1) of Reference Example 4, was suspended in H-SFM (manufactured by GIBCO-BRL) containing 200 nM MTX and 5% of Daigo's GF21 (manufactured by Wako Pure Chemical Industries), to give a density of 1×$10^5$ cells/ml, and dispensed at 50 ml into a 182 $cm^2$ flask (manufactured by Greiner). The cells were cultured at 37°C for 7 days in a 5% $CO_2$ incubator, and the culture supernatant was recovered when they became confluent. The anti-CD20 chimeric antibody KM3065 was purified from the culture supernatant using a Prosep-A (manufactured by Millipore) column in accordance with the manufacture's instructions attached thereto. About 3 μg of the obtained anti-CD20 chimeric antibody KM3065 was subjected to electrophoresis in accordance with the known method [*Nature*, 227, 680 (1970)] to examine its molecular weight and purity. As a result, the purified anti-CD20 chimeric antibody KM3065 was about 150 kilodaltons (hereinafter referred to as "Kd") under non-reducing condition, and two bands of about 50 Kd and about 25 Kd were observed under reducing conditions. The sizes of the protein coincided with reports stating that an IgG type antibody has a molecular weight of about 150 Kd under non-reducing conditions and is degraded into H chain having a molecular weight of about 50 Kd and L chain having a molecular weight of about 25 Kd under reducing conditions due to cutting of the intramolecular disulfide bond (hereinafter referred to as "S-S bond") [*Antibodies*, *A Laboratory Manual*, Cold Spring Harbor Laboratory, Chapter 14 (1988); *Monoclonal Antibodies: Principles and Practice*, Academic Press Limited (1996)] and also almost coincided with the electrophoresis pattern of Rituxan™. Accordingly, it was confirmed that the anti-CD20 chimeric antibody KM3065 is expressed as the antibody molecule of a correct structure.

Reference Example 5

Preparation of lectin-resistant CHO/DG44 cell and production of antibody using the cell:

1. Preparation of lectin-resistant CHO/DG44 cell

CHO/DG44 cells were cultured in a 75 $cm^2$ flask for adhesion culture (manufactured by Greiner) in IMDM-FBS (10) medium [IMDM medium comprising 10% fetal bovine serum (FBS) and 1× concentration of HT supplement (manufactured by GIBCO BRL)] to grow until they reached a stage of just before confluent. After washing the cells with 5 ml of Dulbecco PBS (manufactured by Invitrogen), 1.5 ml of 0.05% trypsin (manufactured by Invitrogen) diluted with Dulbecco PBS was added thereto and cultured at 37°C for 5 minutes to remove the cells from the flask bottom. The removed cells were recovered by a centrifugation operation generally used in cell culture and suspended in IMDM-FBS (10) medium to give a density of 1($10^5$ cells/ml, and then 0.1 μg/ml of an alkylating agent MNNG (manufactured by Sigma) was added or not added thereto. After culturing at 37°C for 3 days in a $CO_2$ incubator (manufactured by TABAI), the culture supernatant was discarded, and the cells were again washed, removed and recovered by the same operations as described above, suspended in IMDM-FBS(10) medium and then inoculated into an adhesion culture 96 well plate (manufactured by IWAKI Glass) to give a density of 1,000 cells/well. To each well, as the final concentration in medium, 1 mg/ml *Lens culinaris* agglutinin (hereinafter referred to as "LCA", manufactured by Vector), 1 mg/ml *Aleuria aurantia* agglutinin (*Aleuria aurantia* lectin; hereinafter referred to as "AAL", manufactured by Vector) or 1 mg/ml kidney bean agglutinin (*Phaseolus vulgaris* leucoagglutinin; hereinafter referred to as "L-PHA", manufactured by Vector) was added. After culturing at 37°C for 2 weeks in a $CO_2$ incubator, the appeared colonies were obtained as lectin-resistant clone CHO/DG44. Regarding the obtained lectin-resistant clone CHO/DG44, an LCA-resistant clone was named clone CHO-LCA, an AAL-resistant clone was named clone CHO-AAL and an L-PHA-resistant clone was named clone CHO-PHA. When the resistance of these clones to various kinds of lectin was examined, it was found that the clone CHO-LCA was also resistant to AAL and the clone CHO-AAL was also resistant LCA. In addition, the clone CHO-LCA and the clone CHO-AAL also showed a resistance to a lectin which recognizes a sugar chain structure identical to the sugar chain structure recognized by LCA and AAL, namely a lectin which recognizes a sugar chain structure in which 6-position of fucose is bound to 1-position of *N*-acetylglucosamine residue in the reducing end through α-bond in the *N*-glycoside-linked sugar chain. Specifically, it was found that the clone CHO-LCA and the clone CHO-AAL can show resistance and survive even in a medium supplemented with 1 mg/ml at a final concentration of a pea agglutinin (*Pisum sativum* agglutinin; hereinafter referred to as "PSA", manufactured by Vector). In addition, even when the alkylating agent MNNG was not added, it was able to obtain lectin-resistant clones.

3. Production of anti-ganglioside GD3 human chimeric antibody by using lectin-resistant CHO/DG44 cell and evaluation of activity of the antibody

(1) Preparation of anti-CCR4 human chimeric antibody-producing cell

An anti-CCR4 human chimeric antibody expression plasmid pKANTEX2160 was introduced into the three kinds of the lectin-resistant clones obtained in the above item 1 by the method described in the item 1(2) of Reference Example 2, and gene amplification by MTX was carried out to prepare an anti-CCR4 human chimeric antibody-producing clone. By measuring an amount of antibody expression by the ELISA described in the item 2 of Reference Example 2, antibody-expressing transformants were obtained from each of the clone CHO-LCA, the clone CHO-AAL and the clone CHO-PHA. Regarding each of the obtained transformants, a transformant derived from the clone CHO-LCA was named clone CHO/CCR4-LCA, a transformant derived from the clone CHO-AAL was named clone

CHO/CCR4-AAL and a transformant derived from the clone CHO-PHA was named clone CHO/CCR4-PHA. Further, the clone CHO/CCR4-LCA, as a name of Nega-13, has been deposited on September 26, 2001, as PERM BP-7756 in International Patent Organism Depositary, National Institute of Advanced Industrial Science and Technology (Tsukuba Central 6, 1, Higashi 1-Chome Tsukuba-shi, Ibaraki-ken, Japan).

(2) Production of anti-CCR4 chimeric antibody by using lectin-resistant CHO cell and evaluation of activity of the antibody

Using the three kinds of the transformants obtained in the above item (1), purified antibodies were obtained by the method described in the item 3 of Reference Example 1. The antigen binding activity of the purified anti-CCR4 human chimeric antibodies was evaluated by the ELISA described in the item 2 of Reference Example 2. The antibodies produced by all transformants showed an antigen binding activity similar to that of the antibody produced by a recombinant clone (clone 5-03) prepared in Reference Example 2 using normal CHO/DG44 cell as the host. Using these purified antibodies, ADCC activity of each of the purified anti-CCR4 human chimeric antibodies was evaluated in accordance with the method described in the item 2 of Example 2. The results are shown in Fig. 26. In comparison with the antibody produced by the clone 5-03, about 100 fold-increased ADCC activity was observed in the antibodies produced by the clone CHO/CCR4-LCA and the clone CHO/CCR4-AAL. On the other hand, no significant increase in the ADCC activity was observed in the antibody produced by the clone CHO/CCR4-PHA. Also, when ADCC activities of the antibodies produced by the clone CHO/CCR4-LCA and the YB2/0 clone were compared in accordance with the method described in the item 7 of Reference Example 1, it was found that the antibody produced by the clone CHO/CCR4-LCA shows higher ADCC activity compared to the antibody produced by clone 5-03, similar to the case of the antibody KM2760-1 produced by the YB2/0 clone prepared in Reference Example 2 (Fig. 27).

(3) Sugar chain analysis of antibody produced by lectin-resistant CHO cell

Sugar chains of the anti-CCR4 chimeric antibodies purified in the item 2(2) of Reference Example 5 were analyzed according to the method described in Example 5 of WO00/61739. Table 7 shows the result of ratios of α1,6-fucose-free sugar chains in each of the antibodies.

Table 7

| Antibody producing cell | Ratio of α1,6-fucose-free complex biantennary sugar chains (%) |
|---|---|
| Clone 5-03 | 9 |
| Clone CHO/CCR4-LCA | 48 |
| Clone CHO/CCR4-AAL | 27 |
| Clone CHO/CCR4-PHA | 8 |

In comparison with the antibody produced by the clone 5-03, the ratio of α1,6-fucose-free sugar chains was increased from 9% to 48% in the antibody produced by the clone CHO/CCR4-LCA. The ratio of α1,6-fucose-free sugar chains was increased from 9% to 27% in the antibody produced by the clone CHO/CCR4-AAL. On the other hand, changes in the sugar chain pattern and the ratio of α1,6-fucose-free sugar chains were hardly found in the PHA-resistant clone when compared with the clone 5-03. From consideration together with the results in the above item (2), the antibody composition produced by the lectin-resistant cell in which the ratio of α1,6-fucose-free sugar chains is 20% or more showed remarkably high ADCC activity than the antibody composition produced by the lectin-unresistant cell.

3. Production of anti-ganglioside GD3 human chimeric antibody by using lectin-resistant CHO/DG44 cell and evaluation of activity of the antibody

(1) Preparation of cells stably producing anti-GD3 chimeric antibody

Into $1.6 \times 10^6$ cells of the clone CHO/DG44 and the clone CHO-LCA prepared in the item 1 of Reference Example 5, the anti-GD3 chimeric antibody expression vector pChi641LHGM4 described in WO00/61739 was introduced by electroporation [*Cytotechnology*, 3, 133 (1990)], and the cells were suspended in 10 ml of IMDM medium (manufactured by Invitrogen, to be referred to as IMDM-dFBS(10) medium) containing dialyzed fetal bovine serum (manufactured by Invitrogen) at 10% volume ratio and dispensed at 200 µl/well into a 96 well culture plate (manufactured by Iwaki Glass). The cells were cultured for 2 weeks in a 5% $CO_2$ incubator. Culture supernatants were recovered from wells where colonies of transformants showing medium nucleic acid component-independent growth were formed and their growth was confirmed, and then, antigen-binding activity of the anti-GD3 chimeric antibody in the culture supernatant was measured by the ELISA shown in the item 2 of Reference Example 1.

In order to increase antibody production using the DHFR gene amplification system, transformants in wells where production of an anti-GD3 chimeric antibody were detected in the culture supernatant were suspended to give a density of $1\times10^5$ cells/ml in the IMDM-dFBS(10) medium containing 50 nM methotrexate (manufactured by Sigma, hereinafter referred to as "MTX"), and the suspension was dispensed at 0.5 ml into a 24 well plate (manufactured by Iwaki Glass). After culturing at 37°C for 2 weeks in a 5% $CO_2$ incubator, transformants showing 50 nM MTX resistance were induced. The transformants in wells where their growth was observed were cultured at 37°C for 2 weeks by increasing the MTX concentration to 200 nM by a method similar to the above to induce transformants showing 200 nM MTX resistance. The transformants in wells where their growth was observed were cultured at 37°C for 2 weeks by increasing the MTX concentration to 500 nM by a method similar to the above to induce transformants showing 500 nM MTX resistance. Finally, stable transformants which can grow in the IMDM-dFBS(10) medium containing 500 nM MTX and also can highly produce the anti-GD3 chimeric antibody were obtained. Regarding the thus obtained transformants, cloned clones were obtained by carrying out single cell isolation (cloning) by a limiting dilution method. Cloned clones obtained using the clone CHO-LCA as the host cell for gene introduction were named clone CHO/GD3-LCA-1 and clone CHO/GD3-LCA-2. A clone obtained using the clone CHO-DG44 as the host cell was named clone CHO/GD3. The clone CHO/GD3-LCA-1 has been deposited on November 11, 2002, as FERM BP-8236 in International Patent Organism Depositary, National Institute of Advanced Industrial Science and Technology (Tsukuba Central 6, 1, Higashi 1-Chome Tsukuba-shi, Ibaraki-ken, Japan).

(2) Production of anti-GD3 chimeric antibody by using lectin-resistant CHO cell and evaluation of activity of the antibody

Each of the anti-GD3 chimeric antibody-producing transformant cell clone, the clone CHO/GD3-LCA-1 and the clone CHO/GD3-LCA-2 obtained in the above item (1) was suspended in a commercially available serum-free medium, EX-CELL 301 medium (manufactured by JRH) to give a density of $1 \times 10^6$ cells/ml and dispensed at 35 ml into 175 $cm^2$ flasks (manufactured by Greiner). After culturing at 37°C for 7 days in a 5% $CO_2$ incubator, culture supernatants were recovered. Each of the anti-GD3 chimeric antibodies was purified from the culture supernatants by using Prosep-A (manufactured by Bioprocessing) column according to the attached manufacture's instructions. As the purified anti-GD3 chimeric antibodies, the antibody produced by the clone CHO/GD3-LCA-1 was named CHO/GD3-LCA-1 antibody and the antibody produced by the clone CHO/GD3-LCA-2 was named CHO/GD3-LCA-2 antibody. Also, the usual antibody produced by the clone CHO/DG44 which was used as control was named CHO/GD3 antibody. Antibodies produced by any transformants showed similar antigen binding activity. By using these purified antibodies, the ADCC activity of each of the anti-GD3 human chimeric antibodies was evaluated according to the method described in the item 2 of Example 1. The results are shown in Table 28. As shown in Table 28, among the three types of the purified anti GD3 chimeric antibodies, the CHO/GD3-LCA-2 antibody showed the highest ADCC activity, and then the CHO/GD3-LCA-1 antibody and the CHO-GD3 antibody showed high ADCC activity in this order. The above results show that the ADCC activity of the produced antibody was increased in the LCA lectin-resistant CHO/DG44 clone.

(4) Sugar chain analysis of anti-GD3 chimeric antibody

Sugar chains of the anti-GD3 chimeric antibodies purified in the item 3(2) of Reference Example 5 were analyzed according to the method described in Example 5 of WO00/61739. Table 8 shows the result of ratios of α1,6-fucose-free sugar chains in each of the antibodies.

Table 8

| Sugar chain analysis of anti-GD3 chimeric antibody | |
|---|---|
| Kind of antibody | Ratio of α1,6-fucose-free complex biantennary sugar chains (%) |
| CHO/GD3 antibody | 9 |
| CHO/GD3-LCA-1 antibody | 42 |
| CHO/GD3-LCA-1 antibody | 80 |

As shown in Table 8, the ratio of α1,6-fucose-free complex biantennary sugar chain was increased from 9% to 42% in the CHO/GD3-LCA-1 antibody in comparison with that in the control CHO/GD3 antibody. Also, the ratio of α1,6-fucose-free complex biantennary sugar chains was increased from 9% to 80% in the CHO/GD3-LCA-2 antibody.

Reference Example 6

Preparation of soluble human FcγRIIIa protein

1. Construction of a soluble human FcγRIIIa protein expression vector

(1) Preparation of human peripheral blood monocyte cDNA

From a healthy donor, 30 ml of vein blood was collected, gently mixed with 0.5 ml of heparin sodium (manufactured by Shimizu Pharmaceutical) and then mixed with 30 ml of physiological saline (manufactured by Otsuka Pharmaceutical). After the mixing, 10 ml of each mixture was gently overlaid on 4 ml of Lymphoprep (manufactured by NYCOMED PHARMA AS) and centrifuged at 2,000 rpm for 30 minutes at room temperature. The separated monocyte fractions in respective centrifugation tubes were combined and suspended in 30 ml of RPMI1640-FBS(10). After centrifugation at room temperature and at 1,200 rpm for 15 minutes, the supernatant was discarded and the cell were suspended in 20 ml of RPMI1640-FBS(10). This washing operation was repeated twice and then $2\times10^6$ cells/ml of peripheral blood monocyte suspension was prepared using RPMI1640-FBS(10).
After 5 ml of the resulting peripheral blood monocyte suspension was centrifuged at room temperature and at 800 rpm for 5 minutes, the supernatant was discarded and the residue was suspended in 5 ml of PBS. After centrifugation at room temperature and at 800 rpm for 5 minutes, the supernatant was discarded and total RNA was extracted by QIAamp RNA Blood Mini Kit (manufactured by QIAGEN) in accordance with the manufacture's instructions.
A single-stranded cDNA was synthesized by reverse transcription reaction to 2 μg of the obtained total RNA, in a series of 40 μl containing oligo(dT) as primers using SUPERSCRITP™ Preamplification System for First Strand cDNA Synthesis (manufactured by Life Technologies) according to the attached manufacture's instructions.

(2) Obtaining of cDNA encoding human FcγRIIIa protein

A cDNA encoding a human FcγRIIIa protein (hereinafter referred to as "hFcγRIIIa") was prepared as follows.
First, a specific forward primer containing a translation initiation codon (represented by SEQ ID NO:3) and a specific reverse primer containing a translation termination codon (represented by SEQ ID NO:4) were designed from the nucleotide sequence of hFcγRIIIa cDNA [*J. Exp. Med.,* 170, 481 (1989)].
Next, using a DNA polymerase ExTaq (manufactured by Takara Shuzo), 50 μl of a reaction solution [1× concentration ExTaq buffer (manufactured by Takara Shuzo), 0.2 mmol/l dNTPs, 1 μmol/l of the above gene-specific primers (SEQ ID NOs:3 and 4)] containing 5 μl of 20-fold diluted solution of the human peripheral blood monocyte-derived cDNA solution prepared in the item 1(1) of Reference Example 6 was prepared, and PCR was carried out. The PCR was carried out by 35 cycles of a reaction at 94°C for 30 seconds, at 56°C for 30 seconds and at 72°C for 60 seconds as one cycle.
After the PCR, the reaction solution was purified by using QIAquick PCR Purification Kit (manufactured by QIAGEN) and dissolved in 20 μl of sterile water. The products were digested with restriction enzymes *Eco*RI (manufactured by Takara Shuzo) and *Bam*HI (manufactured by Takara Shuzo) and subjected to 0.8% agarose gel electrophoresis to recover about 800 bp of a specific amplification fragment.
On the other hand, 2.5 μg of a plasmid pBluescript II SK(-) (manufactured by Stratagene) was digested with restriction enzymes *Eco*RI (manufactured by Takara Shuzo) and *Bam*HI (manufactured by Takara Shuzo), and digested products were subjected to 0.8% agarose gel electrophoresis to recover a fragment of about 2.9 kbp.
The human peripheral blood monocyte cDNA- derived amplification fragment and plasmid pBluescript II SK (-)-derived fragment obtained in the above were ligated by using DNA Ligation Kit Ver. 2.0 (manufactured by Takara Shuzo). The strain *Escherichia coli* DH5α (manufactured by TOYOBO) was transformed by using the reaction solution, and a plasmid DNA was isolated from each of the resulting ampicillin-resistant colonies according to a known method.
A nucleotide sequence of the cDNA inserted into each plasmid was determined by using DNA Sequencer 377 (manufactured by Parkin Elmer) and BigDye Terminator Cycle Sequencing FS Ready Reaction Kit (manufactured by Parkin Elmer) according to the attached manufacture's instructions. It was confirmed that all of the inserted cDNAs whose sequences were determined by this method encodes a complete ORF sequence of the cDNA encoding hFcγRIIIa. As a result, cDNAs encoding two types of hFcγRIIIa were obtained. One is a sequence represented by SEQ ID NO:5, and pBSFcγRIIIa5-3 was obtained as a plasmid containing the sequence. The amino acid sequence corresponding to the nucleotide sequence represented by SEQ ID NO:5 is represented by SEQ ID NO:6. Another is a sequence represented by SEQ ID NO:7, and pBSFcγRIII a5-3 was obtained as a plasmid containing the sequence. The amino acid sequence corresponding to the nucleotide sequence represented by SEQ ID NO:7 is represented by SEQ ID NO: 8. SEQ ID NO:5 and SEQ ID NO:7 are different in nucleotide at position 538 showing T and G, respectively. As a result,

in the corresponding amino acid sequences, the position 176 in the sequence is Phe and Val, respectively. Herein, hFcγRIIIa of the amino acid sequence represented by SEQ ID NO:6 is named hFcγRIIIa(F), and hFcγRIIIa(V) of the amino acid sequence represented by SEQ ID NO:8 is named hFcγRIIIa(V).

(3) Obtaining of a cDNA encoding soluble hFcγRIIIa(F)

A cDNA encoding soluble hFcγRIIIa(F) (hereinafter referred to as "shFcγRIIIa(F)") having the extracellular region of hFcγRIIIa(F) (positions 1 to 193 in SEQ ID NO:6) and a His-tag sequence at the C-terminal was constructed as follows.

First, a primer FcgR3-1 (represented by SEQ ID NO:9) specific for the extracellular region was designed from the nucleotide sequence of cDNA encoding hFcγRIIIa(F) (represented by SEQ ID NO:5).

Next, using a DNA polymerase ExTaq (manufactured by Takara Shuzo), 50 μl of a reaction solution [1× concentration ExTaq buffer (manufactured by Takara Shuzo), 0.2 mmol/l dNTPs, 1 μmol/l of the primer FcgR3-1, 1 μmol/l of the primer M13M4 (manufactured by Takara Shuzo)] containing 5 ng of the plasmid pBSFcγRIIIa5-3 prepared in the item 1(2) of Reference Example 6 was prepared, and PCR was carried out. The PCR was carried out by 35 cycles of a reaction at 94°C for 30 seconds, at 56°C for 30 seconds and at 72°C for 60 seconds as one cycle.

After the PCR, the reaction solution was purified by using QIAquick PCR Purification Kit (manufactured by QIAGEN) and dissolved in 20 μl of sterile water. The products were digested with restriction enzymes *Pst*I (manufactured by Takara Shuzo) and *Bam*HI (manufactured by Takara Shuzo) and subjected to 0.8% agarose gel electrophoresis to recover about 110 bp of a specific amplification fragment.

On the other hand, 2.5 μg of the plasmid pBSFcγRIIIa5-3 was digested with restriction enzymes *Pst*I (manufactured by Takara Shuzo) and *Bam*HI (manufactured by Takara Shuzo), and the digested products were subjected to 0.8% agarose gel electrophoresis to recover a fragment of about 3.5 kbp.

The hFcγRIIIa(F) cDNA-derived amplification fragment and plasmid pBSFcγRIIIa5-3-derived fragment obtained in the above were ligated by using DNA Ligation Kit Ver. 2.0 (manufactured by Takara Shuzo). The strain *Escherichia coli* DH5α (manufactured by TOYOBO) was transformed by using the reaction solution, and a plasmid DNA was isolated from each of the resulting ampicillin-resistant colonies according to a known method.

A nucleotide sequence of the cDNA inserted into each plasmid was determined by using DNA Sequencer 377 (manufactured by Parkin Elmer) and BigDye Terminator Cycle Sequencing FS Ready Reaction Kit (manufactured by Parkin Elmer) according to the attached manufacture's instructions. It was confirmed that all of the inserted cDNAs whose sequences were determined by this method encodes a complete ORF sequence of the cDNA encoding shFcγRIIIa(F) of interest. A plasmid DNA containing absolutely no reading error of bases in the sequence accompanied by PCR was selected from them. Hereinafter, this plasmid is named pBSFcγRIIIa+His3.

The thus determined full length cDNA sequence for shFcγRIIIa(F) is represented by SEQ ID NO:10, and its corresponding amino acid sequence containing a signal sequence is represented by SEQ ID NO:11. In SEQ ID NO: 11, the amino acid residue at position 176 from the N-terminal methionine was phenylalanine.

(4) Obtaining of a cDNA encoding soluble hFcγRIIIa(V)

A cDNA encoding soluble hFcγRIIIa(V) (hereinafter referred to as "shFcγRIIIa(V)") having the extracellular region of hFcγRIIIa(V) (positions 1 to 193 in SEQ ID NO:8) and a His-tag sequence at the C-terminal was constructed as follows.

After digesting 3.0 μg of the plasmid pBSFcγRIIIa3 obtained in the item 1(2) of Reference Example 6 with a restriction enzyme *Alw*NI (manufactured by New England Biolabs), followed by 0.8% agarose gel electrophoresis to collect a fragment of about 2.7 kbp containing the 5'-terminal side of hFcγRIIIa(V).

After digesting 3.0 μg of the plasmid pBSFcγRIIIa+His3 obtained in the item 1(3) of Reference Example 6 with a restriction enzyme *Alw*NI (manufactured by New England Biolabs), the digested product was subjected to 0.8% agarose gel electrophoresis to recover a fragment of about 0.92 kbp containing the 3'-terminal side of hFcγRIIIa and His-tag sequence.

The DNA fragment containing the 5'-terminal side of hFcγRIIIa(V) and DNA fragment containing the 3'-terminal side of hFcγRIIIa and His-tag sequence obtained in the above were ligated by using DNA Ligation Kit Ver. 2.0 (manufactured by Takara Shuzo). The strain *Escherichia coli* DH5α (manufactured by TOYOBO) was transformed by using the reaction solution, and a plasmid DNA was isolated from each of the obtained ampicillin-resistant colonies according to a known method.

A nucleotide sequence of the cDNA inserted into each plasmid was determined by using DNA Sequencer 377 (manufactured by Parkin Elmer) and BigDye Terminator Cycle Sequencing FS Ready Reaction Kit (manufactured by Parkin Elmer) according to the attached manufacture's instructions. It was confirmed that all of the inserted cDNAs whose sequences were determined by this method encodes a complete ORF sequence of the cDNA encoding shFcγRIIIa(V) of interest. A plasmid DNA containing absolutely no reading error of bases in the sequence accompanied by PCR was selected from them. Hereinafter, this plasmid is named pBSFcγRIIIa+His2.

The thus determined full length cDNA sequence for shFcγRIIIa(F) is represented by SEQ ID NO:12, and its corresponding amino acid sequence containing a signal sequence is represented by SEQ ID NO:13. In SEQ ID NO: 13, the amino acid residue at position 176 from the N-terminal methionine was valine.

(5) Construction of shFcγRIIIa(F) and shFcγRIIIa(V) expression vector

shFcγRIIIa(F) or shFcγRIIIa(V) expression vector was constructed as follows.

After 3.0 μg of each of the plasmids pBSFcγRIIIa+His3 and pBSFcγRIIIa+His2 obtained in the items 1(3) and (4) of Reference Example 6 was digested with restriction enzymes *Eco*RI (manufactured by Takara Shuzo) and *Bam*HI (manufactured by Takara Shuzo), the digested products were subjected to 0.8% agarose gel electrophoresis to recover each of fragments of about 620 bp.

Separately, 2.0 μg of the plasmid pKANTEX93 described in WO97/10354 was digested with restriction enzymes *Eco*RI (manufactured by Takara Shuzo) and *Bam*HI (manufactured by Takara Shuzo), and the digested products were subjected to 0.8% agarose gel electrophoresis to recover a fragment of about 10.7 kbp.

Either of the DNA fragments containing shFcγRIIIa(F) cDNA and shFcγRIIIa(V) cDNA was ligated with the plasmid pKANTEX93-derived fragment by using DNA Ligation Kit Ver. 2.0 (manufactured by Takara Shuzo). The strain *Escherichia coli* DH5α (manufactured by TOYOBO) was transformed by using the reaction solution, and a plasmid DNA was isolated from each of the resulting ampicillin-resistant colonies according to a known method.

A nucleotide sequence of the cDNA inserted into each plasmid was determined by using DNA Sequencer 377 (manufactured by Parkin Elmer) and BigDye Terminator Cycle Sequencing FS Ready Reaction Kit (manufactured by Parkin Elmer) in accordance with the manual attached thereto. It was confirmed that the plasmids whose sequences were determined by this method encodes the shFcγRIII(F) cDNA or shFcγRIII(V) cDNA of interest. Hereinafter, the expression vector containing the shFcγRIII(F) cDNA and the expression vector containing the shFcγRIII(V) cDNA were named pKANTEXFcγRIIIa(F)-His and pKANTEXFcγRIIIa(V)-His, respectively.

2. Preparation of cell stably producing shFcγRIIIa(F) and shFcγRIIIa(V)

Cells stably producing shFcγRIIIa(F) or shFcγRIIIa(V) were prepared by introducing the shFcγRIIIa(F) expression vector pKANTEXFcγRIIIa(F)-His or shFcγRIIIa(V) expression vector pKANTEXFcγRIIIa(V)-His constructed in the item 1 of Reference Example 6 into rat myeloma YB2/0 cell [ATCC CRL-1662, *J. Cell. Biol.*, 93, 576 (1982)],

pKANTEXFcγRIIIa(F)-His or pKANTEXFcγRIIIa(V)-His was digested with a restriction enzyme *Aat*II to obtain a linear fragment, 10 μg of each thereof was introduced into $4\times10^6$ cells by electroporation [*Cytotechnology*, 3, 133 (1990)], and the resulting cells were suspended in 40 ml of Hybridoma-SFM-FBS(10) and dispensed at 200 μl/well into a 96 well culture plate (manufactured by Sumitomo Bakelite). After culturing at 37°C for 24 hours in a 5% $CO_2$ incubator, G418 was added to give a concentration of 1.0 mg/ml, followed by culturing for 1 to 2 weeks. Culture supernatants were recovered from wells in which colonies of transformants showing G418 resistance were formed and their growth was confirmed, and expression amount of shFcγRIIIa(F) or shFcγRIIIa(V) in the supernatants was measured by the ELISA described in the item 3 of Reference Example 6.

Regarding the transformants in wells in which expression of the shFcγRIIIa(F) or shFcγRIIIa(V) was confirmed in the culture supernatants, in order to increase the production amount by using a *dhfr* gene amplification system, each of them was suspended to give a density of 1 to $2\times10^5$ cells/ml in the Hybridoma-SFM-FBS(10) medium containing 1.0 mg/ml G418 and 50 nmol/l DHFR inhibitor MTX (manufactured by SIGMA) and dispensed at 2 ml into each well of a 24 well plate (manufactured by Greiner). After culturing at 37°C for 1 to 2 weeks in a 5% $CO_2$ incubator, transformants showing 50 nmol/l MTX resistance were induced. A production amount of shFcγRIIIa(F) or shFcRIIIa(V) in culture supernatants in wells where growth of transformants was observed was measured by the ELISA described in the item 3 of Reference Example 6.

Regarding the transformants in wells in which production of the shFcγRIIIa(F) or shFcγRIIIa(V) was found in culture supernatants, the MTX concentration was increased to 100 nmol/l and then to 200 nmol/l sequentially by a method similar to the above to thereby finally obtain a transformant capable of growing in the Hybridoma-SFM-FBS (10) medium containing 1.0 mg/ml G418 and 200 nmol/l MTX and also of highly producing shFcγRIIIa(F) or shFcγRIIIa (V). Regarding the obtained transformants, cloning was carried out twice by limiting dilution to obtain shFcγRIIIa(F)-producing transformant clone KC 1107 and shFcγRIIIa(V)-producing transformant clone KC1111.

3. Detection of shFcγRIIIa(F) and shFcγRIIIa(V) (ELISA)

shFcγRIIIa(F) and shFcγRIIIa(V) in culture supernatants or purified shFcγRIIIa(F) and shFcγRIIIa(V) were

detected or determined by the ELISA shown below.

A solution of a mouse antibody against His-tag, Tetra·His Antibody (manufactured by QIAGEN), adjusted to 5 μg/ml with PBS was dispensed at 50 μl/well into each well of a 96 well plate for ELISA (manufactured by Greiner) and allowed to react at 4°C for 12 hours or more. After the reaction, 1% BSA-PBS was added at 100 μl/well and allowed to react at room temperature for 1 hour to block the remaining active groups. After 1% BSA-PBS was discarded, culture supernatant of the transformant or each of various diluted solutions of purified shFcγRIIIa(F) or shFcγRIIIa(V) was added at 50 μl/well and allowed to react at room temperature for 1 hour. After the reaction and subsequent washing of each well with Tween-PBS, a biotin-labeled mouse anti-human CD16 antibody solution (manufactured by PharMingen) diluted 50-fold with 1% BSA-PBS was added at 50 μl/well and allowed to react at room temperature for 1 hour. After the reaction and subsequent washing with Tween-PBS, a peroxidase-labeled Avidin D solution (manufactured by Vector) diluted 4,000-fold with 1% BSA-PBS was added at 50 μl/well and allowed to react at room temperature for 1 hour. After the reaction and subsequent washing with Tween-PBS, the ABTS substrate solution was added at 50 μl/well to develop color, 5 minutes thereafter, the reaction was stopped by adding 5% SDS solution at 50 μl/well. Thereafter, OD415 was measured.

4. Purification of shFcγRIIIa

The shFcγRIIIa(F)-producing transformant cell clone KC1107 and shFcγRIIIa(F)-producing transformant cell clone KC1111 obtained in the item 2 of Reference Example 6 was suspended in Hybridoma-SFM-GF(5) [Hybridoma-SFM medium (manufactured by LIFE TECHNOLOGIES) containing 5% Daigo's GF21 (manufactured by Wako Pure Chemical Industries)] to give a density of $3\times10^5$ cells/ml and dispensed at 50 ml into 182 $cm^2$ flasks (manufactured by Greiner). After culturing at 37°C for 4 days in a 5% $CO_2$ incubator, the culture supernatants were recovered. shFcγRIIIa(F) and shFcγRIIIa(V) were purified from the culture supernatants by using Ni-NTA agarose (manufactured by QIAGEN) column according to the attached manufacture's instructions.

5. Analysis of purified shFcγRIIIa(F) and shFcγRIIIa(V)

Concentrations of purified shFcγRIIIa(F) and shFcγRIIIa(V) obtained in the item 4 of Reference Example 6 was calculated by amino acid composition analysis as follows. A part of purified shFcγRIIIa(F) or shFcγRIIIa(V) was suspended in 6 mol/l hydrochloric acid-1% phenol solution, and hydrolyzed in a gas phase at 110°C for 20 hours. Work Station manufactured by Waters was used for the hydrolysis. Amino acids after the hydrolysis were converted into PTC-amino acid derivatives in accordance with the method of Bidlingmeyer *et al*. [*J. Chromatogr*., 336, 93 (1984)] and analyzed by using PicoTag Amino Acid Analyzer (manufactured by Waters).

Next, about 0.5 μg of purified shFcγRIIIa(F) or shFcγRIIIa(V) was subjected to SDS-PAGE under reducing conditions according to a known method [*Nature*, 227, 680 (1970)] to analyze its molecular weight and purity. The results are shown in Fig. 28. As shown in Fig. 29, a broad band of 36 to 38 Kd in molecular weight was detected in purified shFcγRIIIa(F) or shFcγRIIIa(V). Since it is known that five sites to which *N*-glycoside-linked sugar chains can be bound are present in the extracellular region of hFcγRIIIa [*J. Exp. Med*., 170, 481 (1989)], it was considered that the broad molecular weight distribution of purified shFcγRIIIa(F) or shFcγRIIIa(V) is based on the irregularity of sugar chain addition. On the other hand, when the N-terminal amino acid sequence of purified shFcγRIIIa(F) or shFcγRIIIa (V) was analyzed by automatic Edman degradation using a protein sequencer PPSQ-10 (manufactured by Shimadzu), a sequence expected from the cDNA of shFcγRIIIa(F) or shFcγRIIIa(V) was obtained, so that it was confirmed that shFcγRIIIa(F) or shFcγRIIIa(V) of interest was purified.

## INDUSTRIAL APPLICABILITY

The present invention provides a medicament for treating FcγRIIIa polymorphism patients who cannot be treated with a medicament which comprises as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain., which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain.

Free Text in Sequence Listing

SEQ ID NO:3 - Explanation of synthetic sequence: synthetic DNA
SEQ ID NO:9 - Explanation of synthetic sequence: synthetic DNA
SEQ ID NO:24 - Explanation of synthetic sequence: synthetic DNA
SEQ ID NO:25 - Explanation of synthetic sequence: synthetic DNA
SEQ ID NO:26 - Explanation of synthetic sequence: synthetic DNA
SEQ ID NO:27 - Explanation of synthetic sequence: synthetic DNA
SEQ ID NO:28 - Explanation of synthetic sequence: synthetic DNA
SEQ ID NO:29 - Explanation of synthetic sequence: synthetic DNA
SEQ ID NO:31 - Explanation of synthetic sequence: synthetic DNA
SEQ ID NO:32 - Explanation of synthetic sequence: synthetic DNA
SEQ ID NO:33 - Explanation of synthetic sequence: synthetic DNA
SEQ ID NO:34 - Explanation of synthetic sequence: synthetic DNA
SEQ ID NO:35 - Explanation of synthetic sequence: synthetic DNA
SEQ ID NO:36 - Explanation of synthetic sequence: synthetic DNA
SEQ ID NO:38 - Explanation of synthetic sequence: synthetic DNA
SEQ ID NO:39 - Explanation of synthetic sequence: synthetic DNA
SEQ ID NO:40 - Explanation of synthetic sequence: synthetic DNA
SEQ ID NO:41 - Explanation of synthetic sequence: synthetic DNA
SEQ ID NO:42 - Explanation of synthetic sequence: synthetic DNA
SEQ ID NO:43 - Explanation of synthetic sequence: synthetic DNA
SEQ ID NO:44 - Explanation of synthetic sequence: synthetic DNA

## Claims

1. A medicament for treating a patient who exerts such an affinity of a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain with a human Fcγ receptor IIIa that it is not enough for the antibody composition to exert sufficient therapeutic effect, which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain.

2. The medicament according to claim 1, wherein the affinity that it is not enough to exert sufficient therapeutic effect is an affinity that is not enough for the antibody composition to exert a sufficient antibody-dependent cell-mediated cytotoxic activity.

3. The medicament according to claim 1 or 2, wherein the affinity that it is not enough to exert sufficient therapeutic effect is lower than at least one affinity selected from the group consisting of (a) and (b):

   (a) a binding constant to the human Fcγ receptor IIIa at 25°C being $1\times10^7$ $M^{-1}$ when measured by a biosensor method according to BIAcore;
   (b) a binding constant to the human Fcγ receptor IIIa at 25°C being $2\times10^6$ $M^{-1}$ when measured with an isothermal titration-type calorimeter.

4. The medicament according to any one of claims I to 3, wherein the human Fcγ receptor IIIa is a human Fcγ receptor IIIa in which an amino acid residue at position 176 from the N-terminal methionine in the signal sequence is phenylalanine.

5. The medicament according to any one of claims 1 to 4, wherein the patient is a patient having a human Fcγ receptor IIIa in which an amino acid residue at position 176 from the N-terminal methionine in the signal sequence is phenylalanine.

6. The medicament according to any one of claims 1 to 5, wherein the patient is a patient having only human Fcγ receptor IIIa in which an amino acid residue at position 176 from the N-terminal methionine in the signal sequence is phenylalanine.

7. The medicament according to any one of claims 1 to 6, wherein the cell resistant to the lectin is a cell, in which the activity of a protein is decreased or deleted, selected from the group consisting of the following (a), (b) and (c):

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose to the Golgi body.

8. The medicament according to any one of claims 1 to 7, wherein the lection is selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

9. The medicament according to any one of claims 1 to 8, wherein the cell is selected from the group consisting of a yeast, an animal cell, an insect cell and a plant cell.

10. The medicament according to any one of claims 1 to 9, wherein the cell is selected from the group consisting of the following (a) to (j):

(a) a CHO cell derived from a Chinese hamster ovary tissue;
(b) a rat myeloma cell line YB2/3HL.P2.G11.16Ag.20 line;
(d) a mouse myeloma cell line NS0 cell;
(d) a mouse myeloma cell line SP2/0-Ag14 cell;
(e) a BHK cell derived from a Syrian hamster kidney tissue;
(f) a hybridoma cell producing an antibody;
(g) a human leukemic cell line Namalwa cell;
(h) an embryonic stem cell;
(i) a fertilized egg cell;
(j) a plant cell.

11. The medicament according to any one of claims 1 to 10, wherein the antibody composition which comprises as an active ingredient the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

12. The medicament according to claim 11, wherein the antibody molecule belongs to an IgG class.

13. The medicament according to any one of claims 1 to 12, wherein the antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain is an antibody composition having a higher antibody-dependent cell-mediated cytotoxic activity than the antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain.

14. The medicament according to claim 13, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to

the Fc region in the antibody composition than the antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain.

**15.** The medicament according to claim 14, wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

**16.** The medicament according to any one of claims 13 to 15, wherein the antibody composition having a higher antibody-dependent cell-mediated cytotoxic activity is an antibody composition having a ratio of a sugar chain in which fucose is not bound to *N*-acetylglucosamine in the reducing end in the sugar chain of 20% or more of total complex *N*-glycoside-linked sugar chains bound to the Fc region in the antibody composition.

**17.** The medicament according to claim 16, wherein the antibody composition is an antibody composition produced by a CHO cell.

**18.** The medicament according to any one of claims 1 to 17, which is a diagnostic agent, an preventing agent or a therapeutic agent for tumor-accompanied diseases, allergy-accompanied diseases, inflammatory-accompanied diseases, autoimmune diseases, cardiovascular diseases, viral infection-accompanied diseases or bacterial infection-accompanied diseases.

**19.** Use of an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain for the manufacture of the medicament according to any one of claims 1 to 18.

**20.** A method for screening a patient to which the medicament according to any one of claims 1 to 18 is effective, which comprises:

(i) contacting a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain or the medicament according to any one of claims 1 to 18, with an effector cell obtained from a patient;
(ii) measuring the amount of each of the medicaments bound to the effector cell;
(iii) comparing the measured amounts;
(iv) selecting a patient in which the amount of the medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain which has been added to the effector cell is lower.

**21.** The method according to claim 20, wherein the method for measuring the amount the medicament bound to the target cell is an immunological measuring method.

**22.** A method for screening a patient to which the medicament according to any one of claims 1 to 18 is effective, which comprises

(i) contacting a medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain or the medicament according to any one of claims 1 to 18, with an effector cell obtained from a patient;
(ii) measuring the activity caused by the contact of each of the medicaments with the effector cell;
(iii) comparing the measured activities;
(iv) selecting a patient in which the activity of the medicament comprising as an active ingredient an antibody composition produced by a cell unresistant to a lectin which recognizes a sugar chain in which 1-position of fucose is bound to 6-position of *N*-acetylglucosamine in the reducing end through α-bond in a complex *N*-glycoside-linked sugar chain is lower.

**23.** The method according to claim 22, wherein the method for measuring the activity caused by the contact of the medicament reacted with the target cell is a method selected from the group consisting of (a) to (e):

(a) a method for measuring an antibody-dependent cell-mediated cytotoxic activity;
(b) a method for measuring a complement-dependent cytotoxic activity;
(c) a method for measuring expression of a cytotoxic molecule;
(d) a method for measuring an intracellular signal transduction of a human Fcγ receptor IIIa;
(e) a method for measuring a molecule of which expression is varied by stimulating a human Fcγ receptor IIIa.

**24.** The method according to any one of claims 20 to 23, wherein the effector cell is a cell which expresses a human Fcγ receptor IIIa.

**25.** The method according to any one of claims 20 to 24, wherein the screening method is a method for screening a patient having a human Fcγ receptor IIIa in which an amino acid residue at position 176 from the N-terminal methionine in the signal sequence is phenylalanine.

**26.** A medicament which comprises as an active ingredient an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain and is administered to a patient having a human Fcγ receptor IIIa in which an amino acid residue at position 176 from the N-terminal methionine in the signal sequence is phenylalanine who is screened by the method according to any one of claims 20 to 25.

**27.** The medicament according to any one of claims 1 to 18, which is administered to a patient having a human Fcγ receptor IIIa in which an amino acid residue at position 176 from the N-terminal methionine in the signal sequence is phenylalanine who is screened by the method according to any one of claims 20 to 25.

**28.** Use of an antibody composition produced by a cell resistant to a lectin which recognizes a sugar chain in which 1-position of fucose is bound to 6-position of *N*-acetylglucosamine in the reducing end through α-bond in a complex *N*-glycoside-linked sugar chain for the manufacture of the medicament according to claim 26 or 27.

FIG. 1

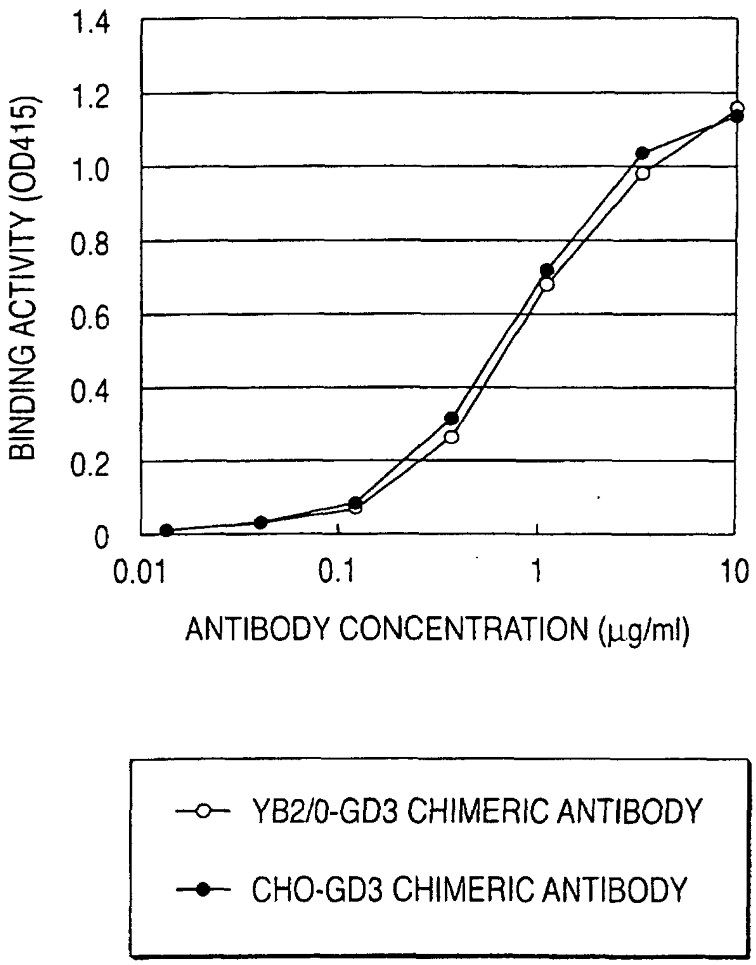
1.4
1.2
1.0
0.8
0.6
0.4
0.2
0
BINDING ACTIVITY (OD415)
0.01
0.1
1
10
ANTIBODY CONCENTRATION (μg/ml)
YB2/0-GD3 CHIMERIC ANTIBODY
CHO-GD3 CHIMERIC ANTIBODY

FIG. 2

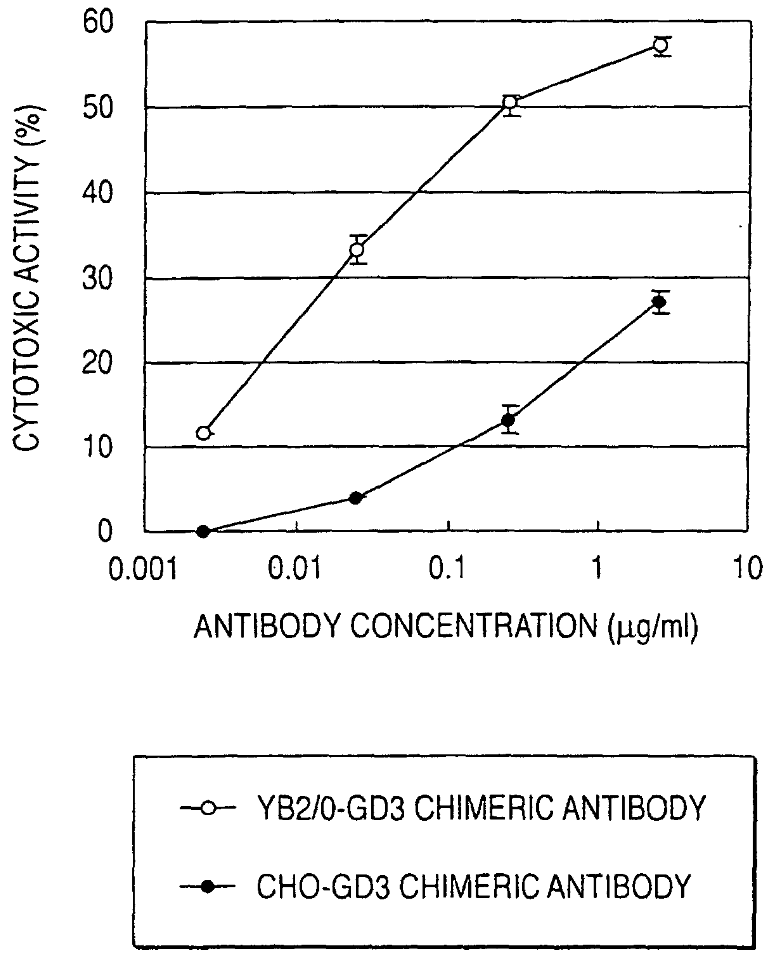
60
50
40
30
20
10
0
CYTOTOXIC ACTIVITY (%)
0.001
0.01
0.1
1
10
ANTIBODY CONCENTRATION (μg/ml)
YB2/0-GD3 CHIMERIC ANTIBODY
CHO-GD3 CHIMERIC ANTIBODY

FIG. 3

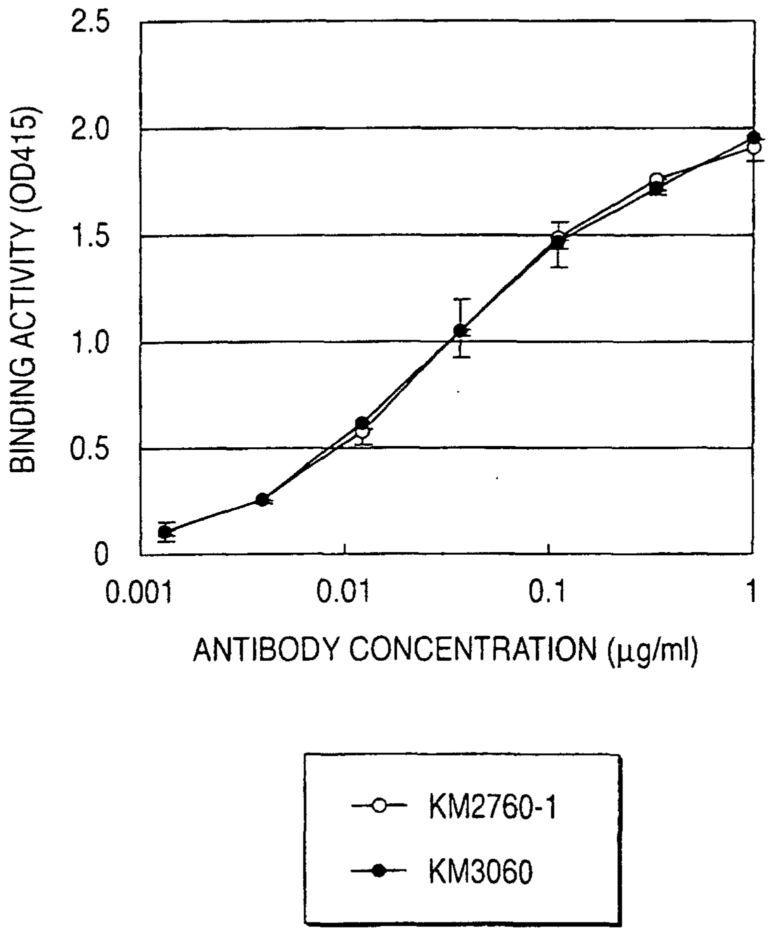
2.5
2.0
1.5
1.0
0.5
0
BINDING ACTIVITY (OD415)
0.001
0.01
0.1
1
ANTIBODY CONCENTRATION (μg/ml)
KM2760-1
KM3060

FIG. 4

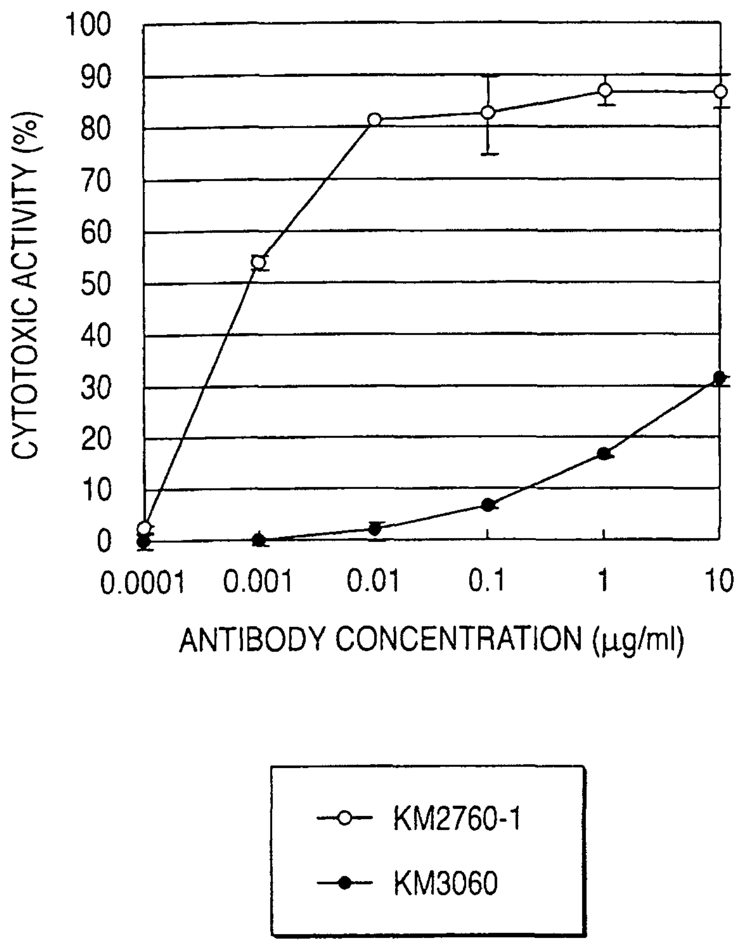
100
90
80
70
60
50
40
30
20
10
0
CYTOTOXIC ACTIVITY (%)
0.0001
0.001
0.01
0.1
1
10
ANTIBODY CONCENTRATION (μg/ml)
KM2760-1
KM3060

FIG. 5

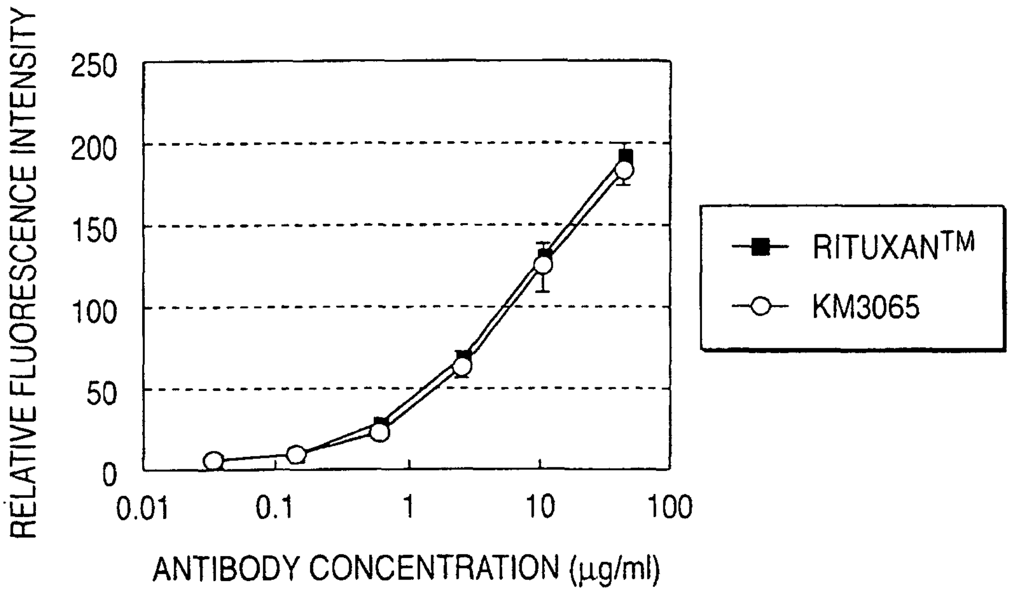
RELATIVE FLUORESCENCE INTENSITY
250
200
150
100
50
0
0.01
0.1
1
10
100
ANTIBODY CONCENTRATION (μg/ml)
RITUXAN™
KM3065

FIG. 6

A

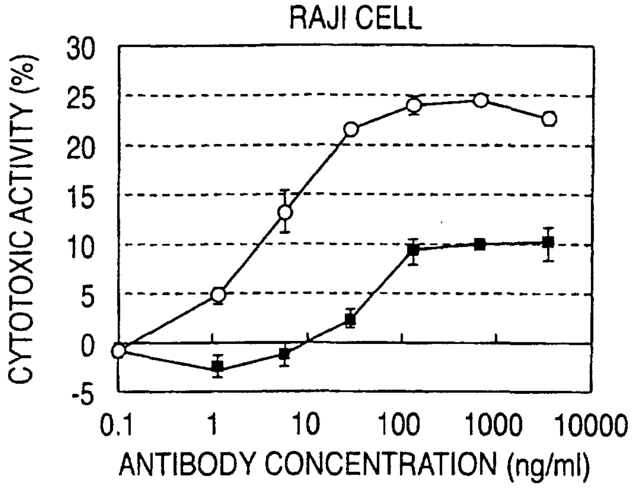
RAJI CELL
CYTOTOXIC ACTIVITY (%)
30
25
20
15
10
5
0
-5
0.1
1
10
100
1000
10000
ANTIBODY CONCENTRATION (ng/ml)

B

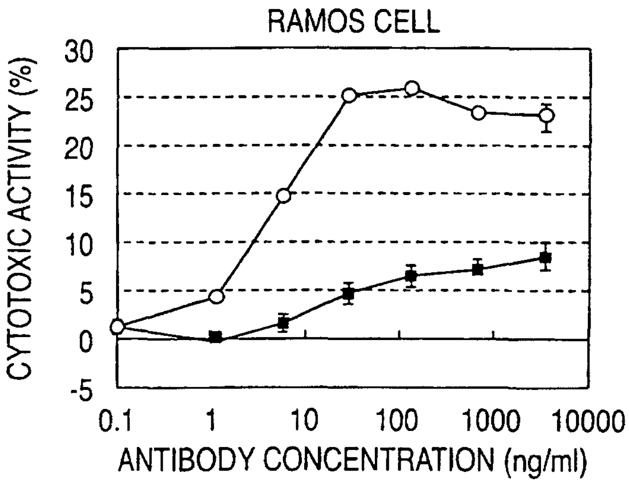
RAMOS CELL
CYTOTOXIC ACTIVITY (%)
30
25
20
15
10
5
0
-5
0.1
1
10
100
1000
10000
ANTIBODY CONCENTRATION (ng/ml)

C

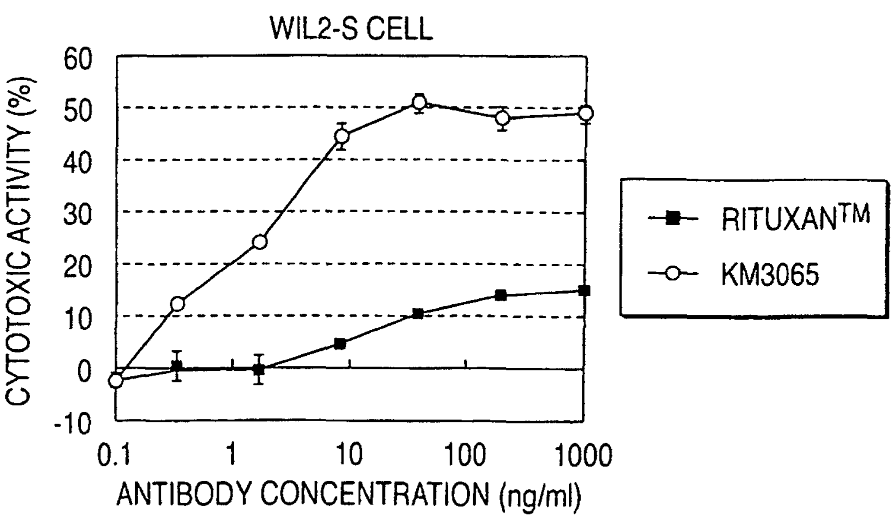
WIL2-S CELL
CYTOTOXIC ACTIVITY (%)
60
50
40
30
20
10
0
-10
0.1
1
10
100
1000
ANTIBODY CONCENTRATION (ng/ml)
RITUXAN™
KM3065

FIG. 7

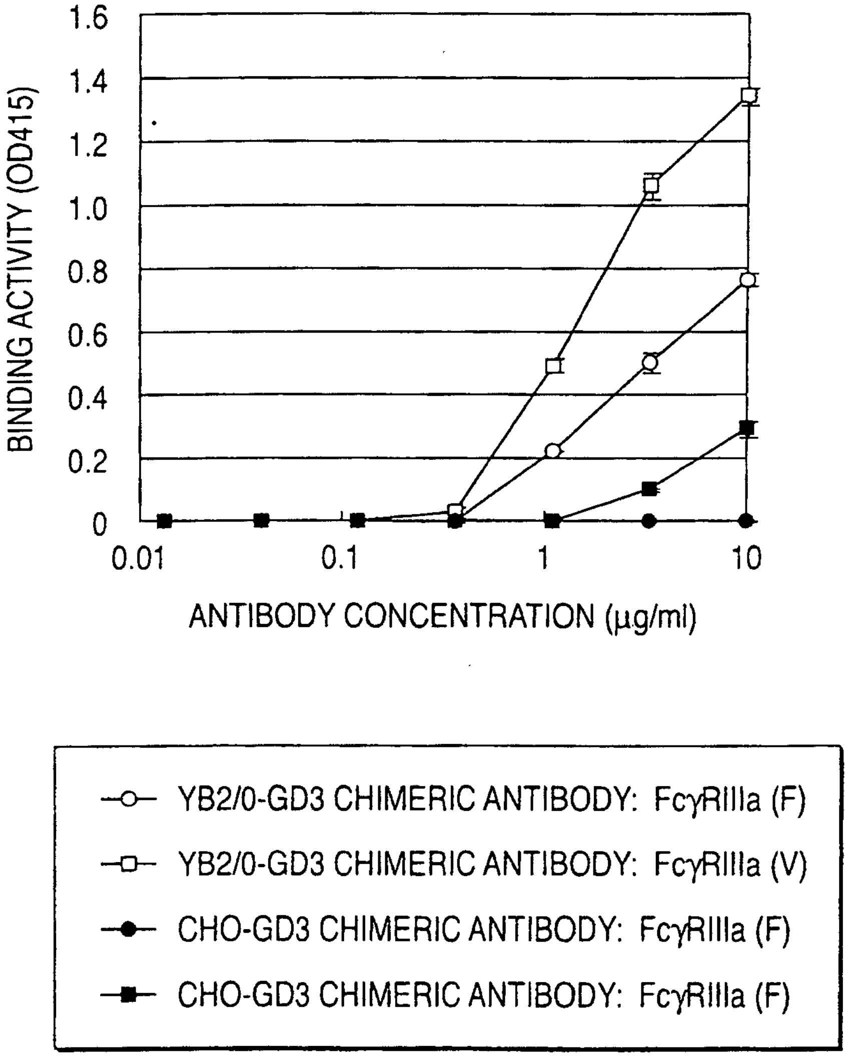
1.6
1.4
1.2
1.0
0.8
0.6
0.4
0.2
0
BINDING ACTIVITY (OD415)
0.01
0.1
1
10
ANTIBODY CONCENTRATION (μg/ml)
YB2/0-GD3 CHIMERIC ANTIBODY: FcγRIIIa (F)
YB2/0-GD3 CHIMERIC ANTIBODY: FcγRIIIa (V)
CHO-GD3 CHIMERIC ANTIBODY: FcγRIIIa (F)
CHO-GD3 CHIMERIC ANTIBODY: FcγRIIIa (F)

FIG. 8

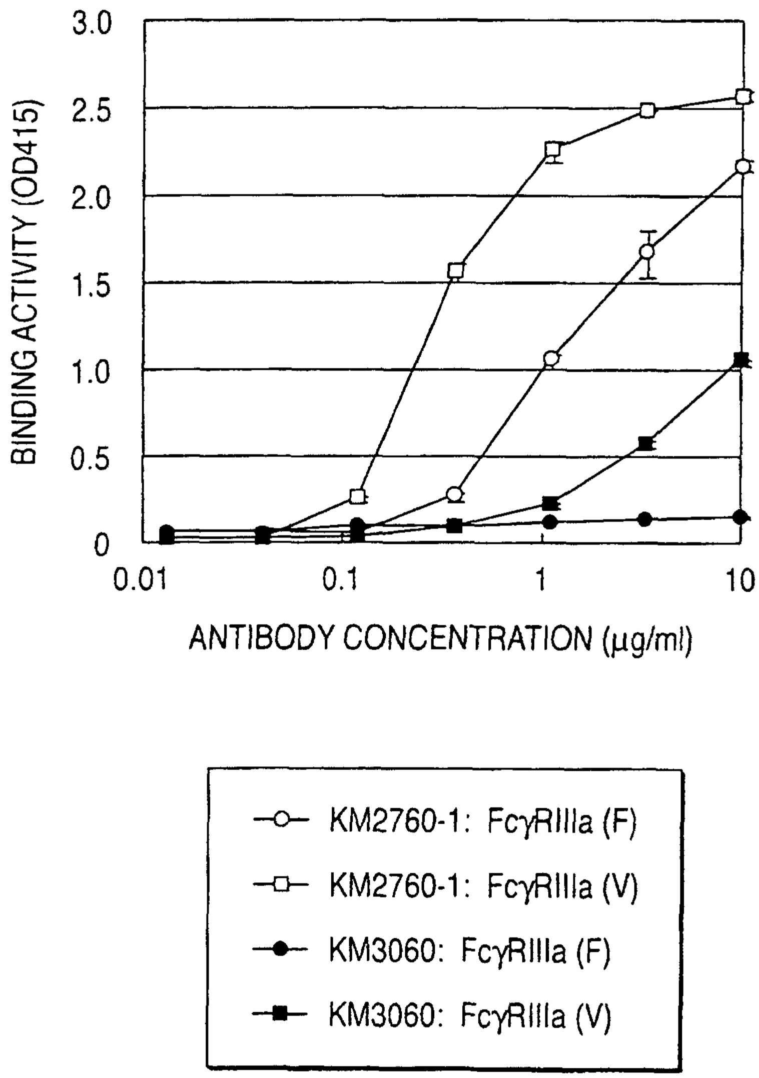
3.0
2.5
2.0
1.5
1.0
0.5
0
BINDING ACTIVITY (OD415)
0.01
0.1
1
10
ANTIBODY CONCENTRATION (μg/ml)
KM2760-1: FcγRIIIa (F)
KM2760-1: FcγRIIIa (V)
KM3060: FcγRIIIa (F)
KM3060: FcγRIIIa (V)

FIG. 9

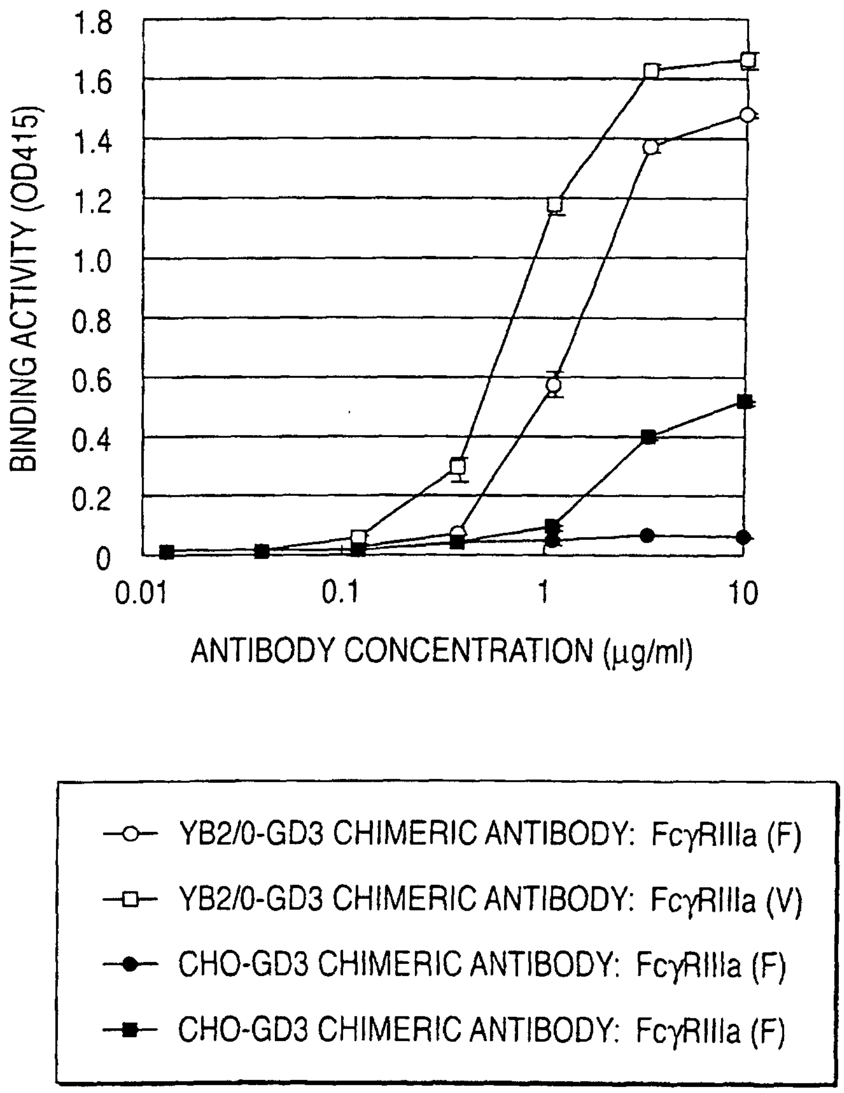
1.8
1.6
1.4
1.2
1.0
0.8
0.6
0.4
0.2
0
BINDING ACTIVITY (OD415)
0.01
0.1
1
10
ANTIBODY CONCENTRATION (μg/ml)
YB2/0-GD3 CHIMERIC ANTIBODY: FcγRIIIa (F)
YB2/0-GD3 CHIMERIC ANTIBODY: FcγRIIIa (V)
CHO-GD3 CHIMERIC ANTIBODY: FcγRIIIa (F)
CHO-GD3 CHIMERIC ANTIBODY: FcγRIIIa (F)

FIG. 10

A

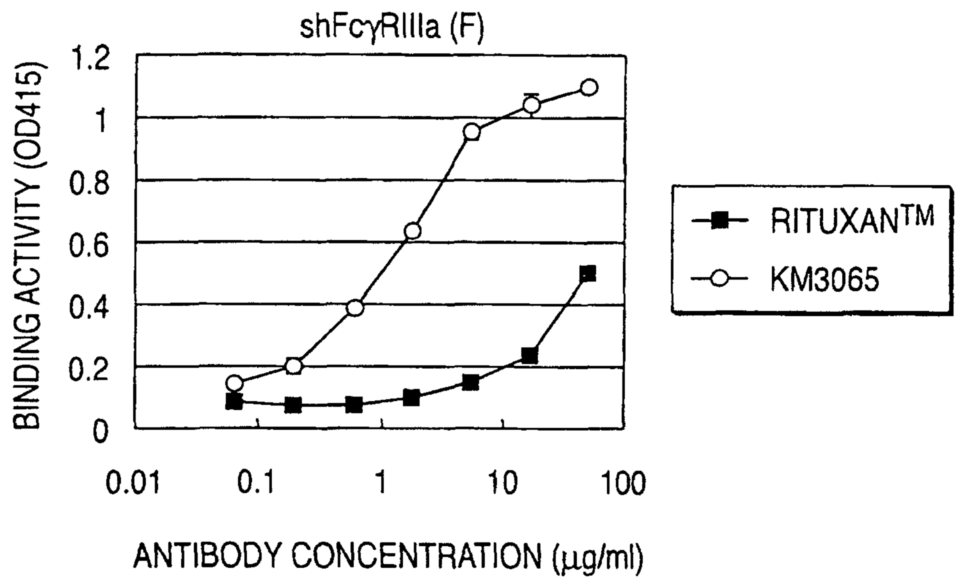
shFcγRIIIa (F)
BINDING ACTIVITY (OD415)
1.2
1
0.8
0.6
0.4
0.2
0
0.01
0.1
1
10
100
ANTIBODY CONCENTRATION (μg/ml)
RITUXAN™
KM3065

B

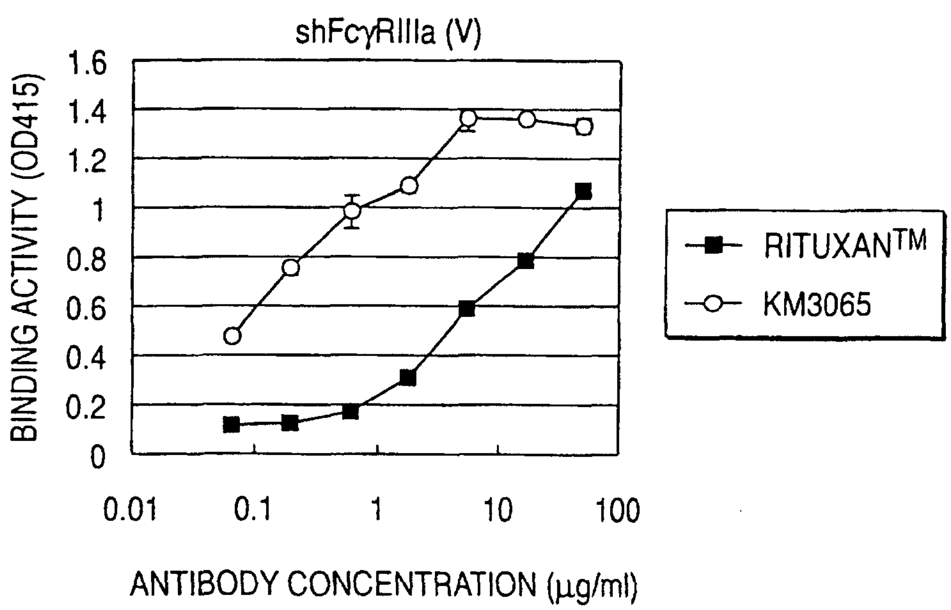
shFcγRIIIa (V)
BINDING ACTIVITY (OD415)
1.6
1.4
1.2
1
0.8
0.6
0.4
0.2
0
0.01
0.1
1
10
100
ANTIBODY CONCENTRATION (μg/ml)
RITUXAN™
KM3065

FIG. 11

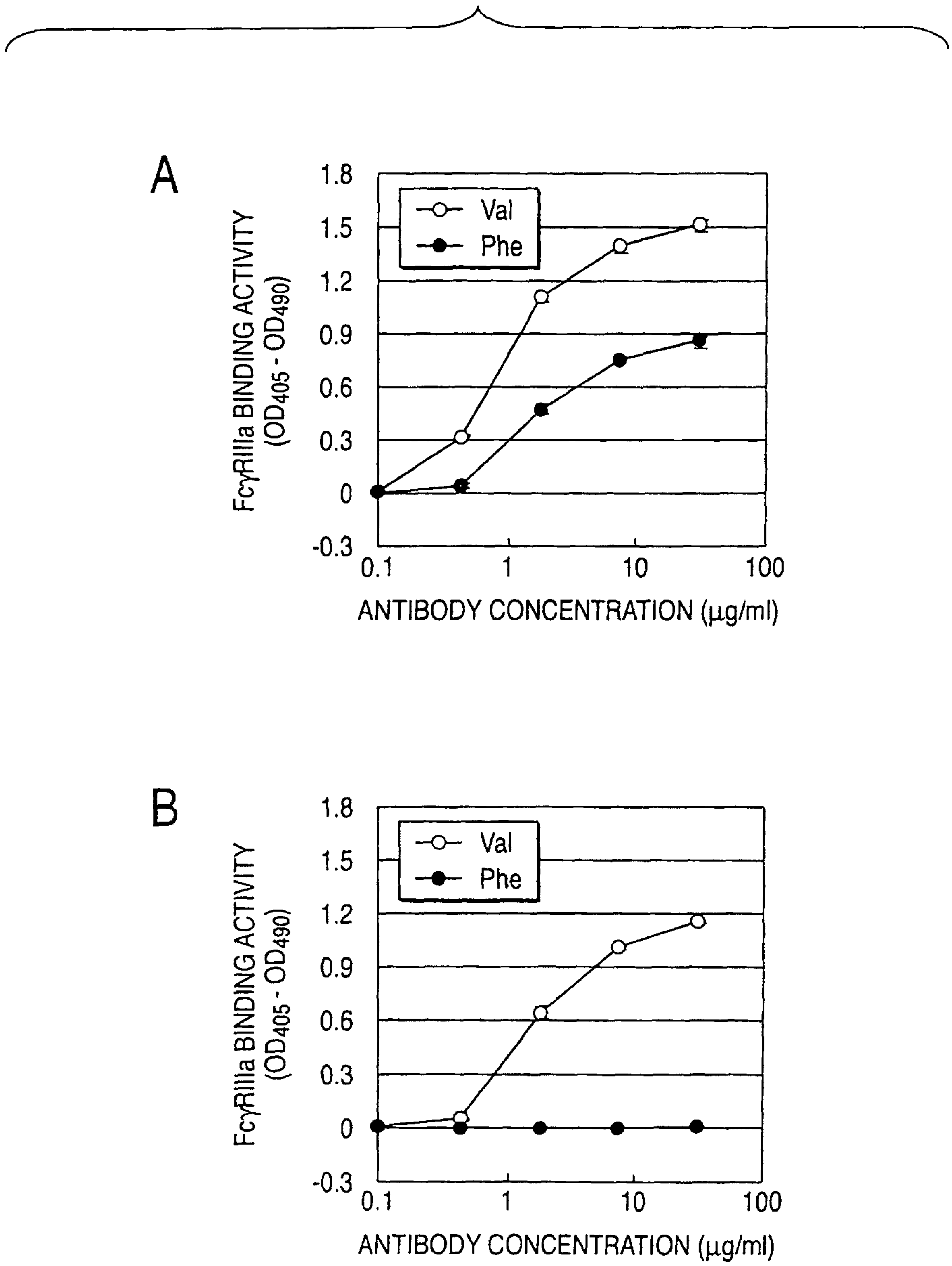
A
B
Val
Phe
FcγRIIIa BINDING ACTIVITY
(OD405 - OD490)
1.8
1.5
1.2
0.9
0.6
0.3
0
-0.3
0.1
1
10
100
ANTIBODY CONCENTRATION (μg/ml)

FIG. 12

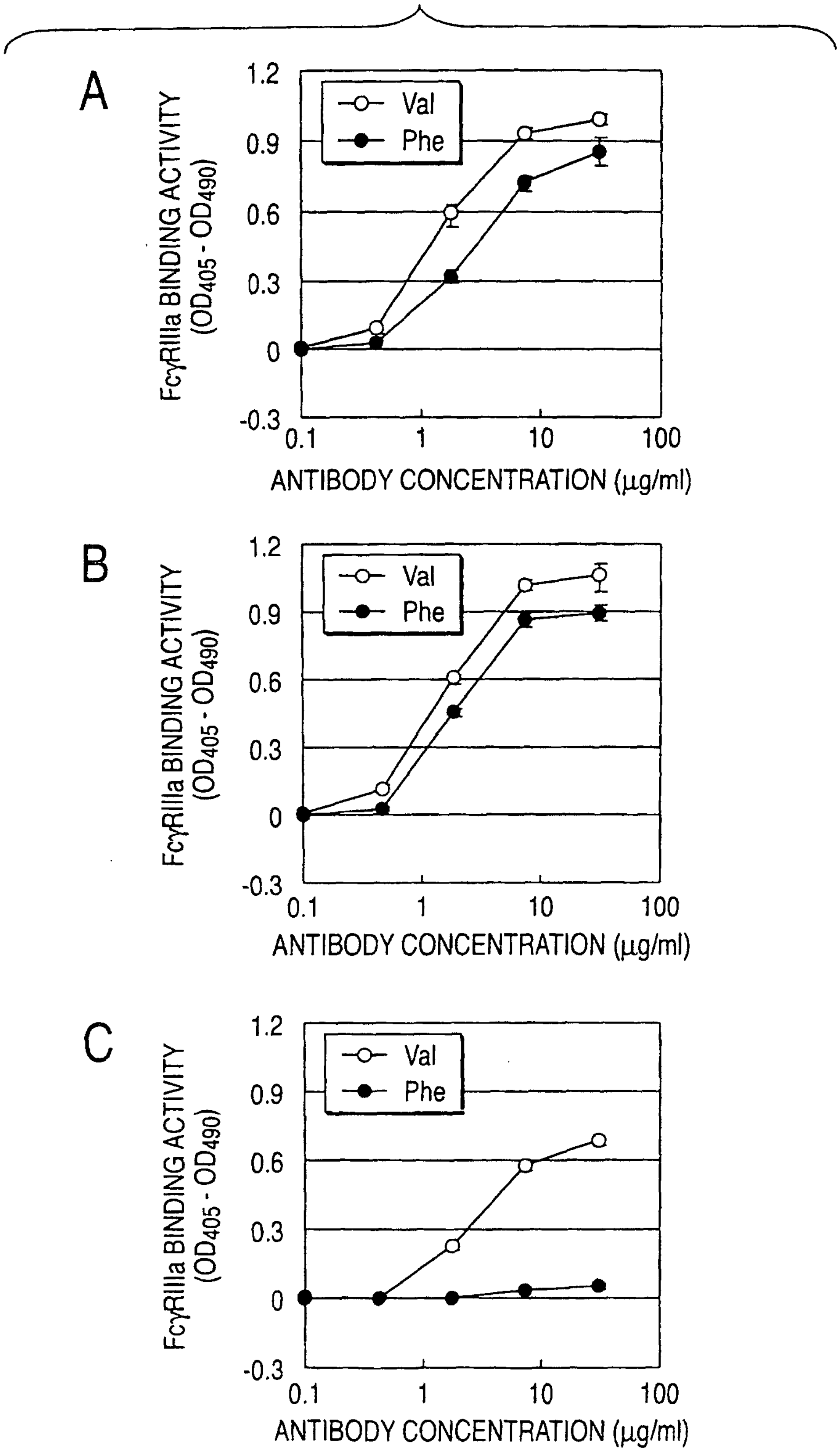
A
B
C
Val
Phe
1.2
0.9
0.6
0.3
0
-0.3
0.1
1
10
100
FcγRIIIa BINDING ACTIVITY
(OD405 - OD490)
ANTIBODY CONCENTRATION (μg/ml)

FIG. 13

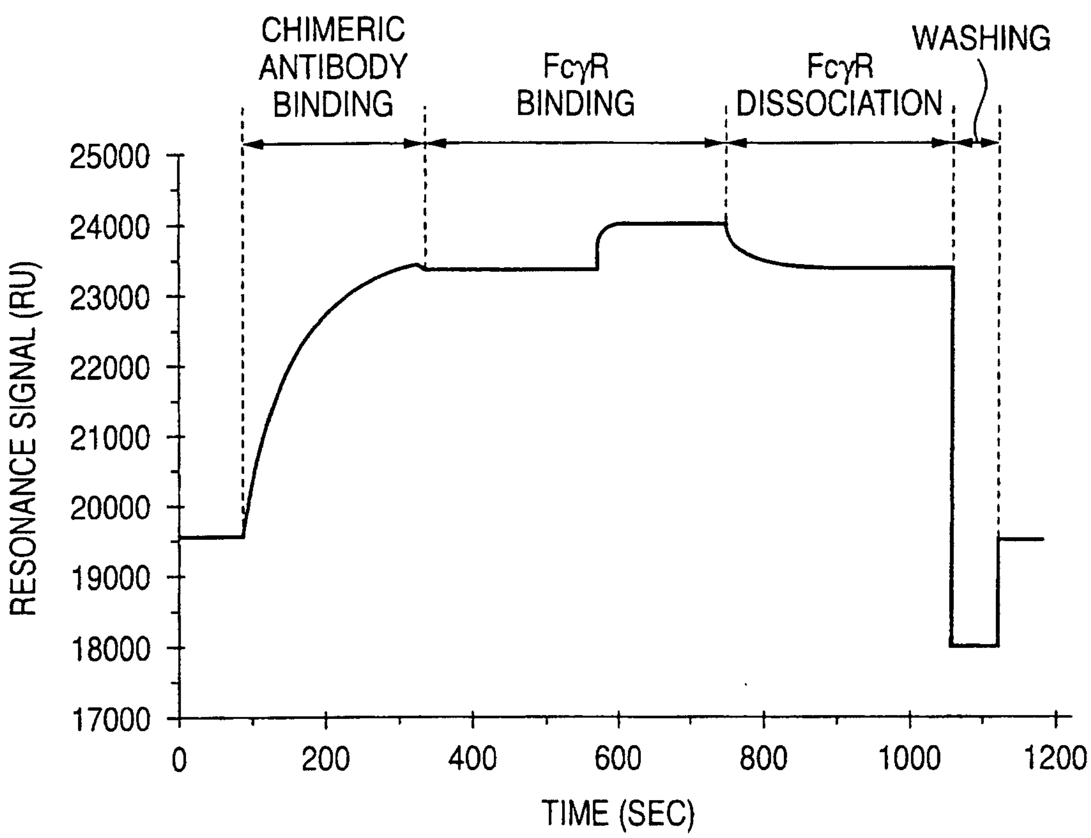
CHIMERIC ANTIBODY BINDING
FcγR BINDING
FcγR DISSOCIATION
WASHING
RESONANCE SIGNAL (RU)
25000
24000
23000
22000
21000
20000
19000
18000
17000
0
200
400
600
800
1000
1200
TIME (SEC)

FIG. 14

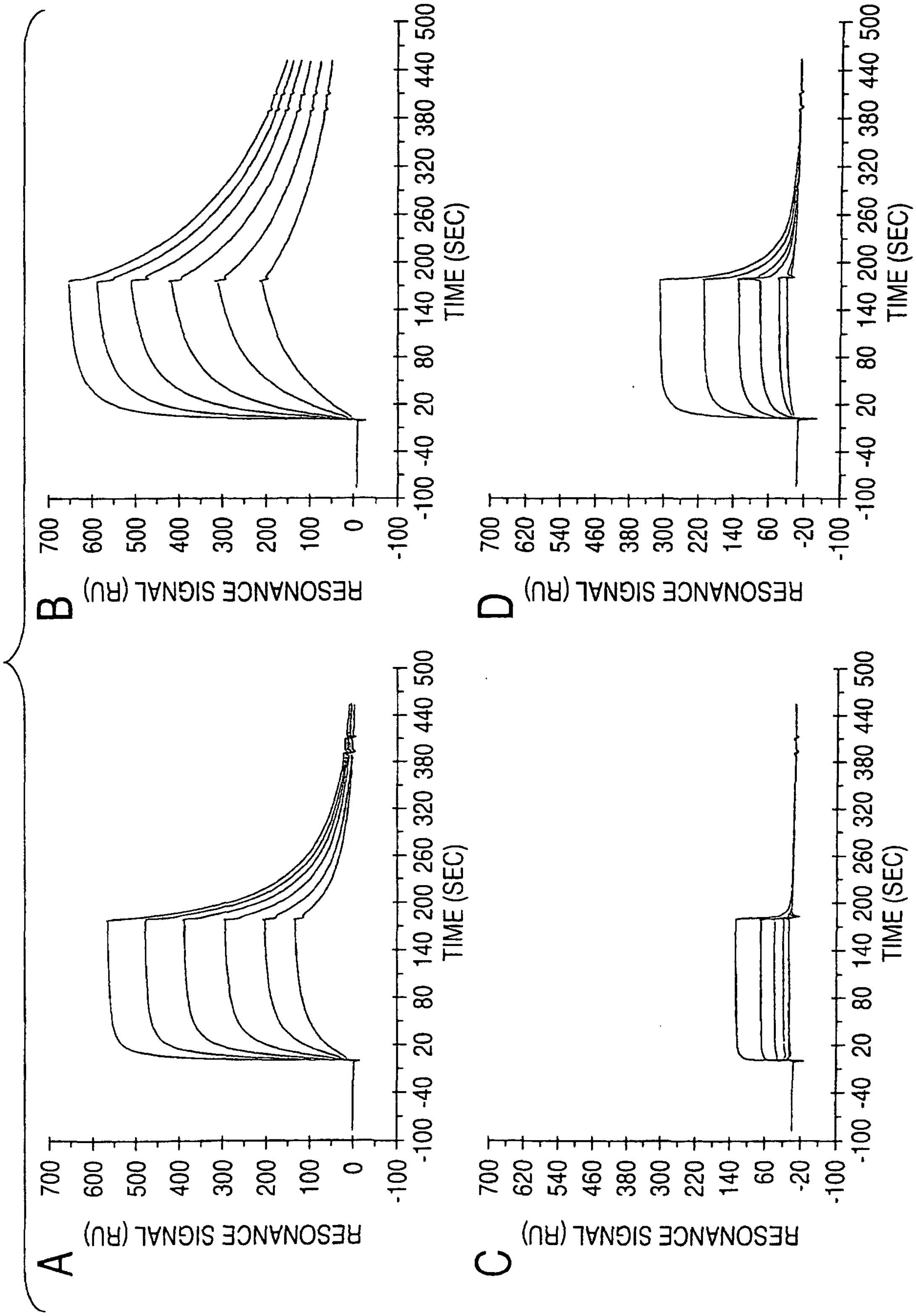
A
B
C
D
RESONANCE SIGNAL (RU)
TIME (SEC)
700
600
500
400
300
200
100
0
-100
620
540
460
380
300
220
140
60
-20
-40
20
80
140
200
260
320
380
440
500

FIG. 15

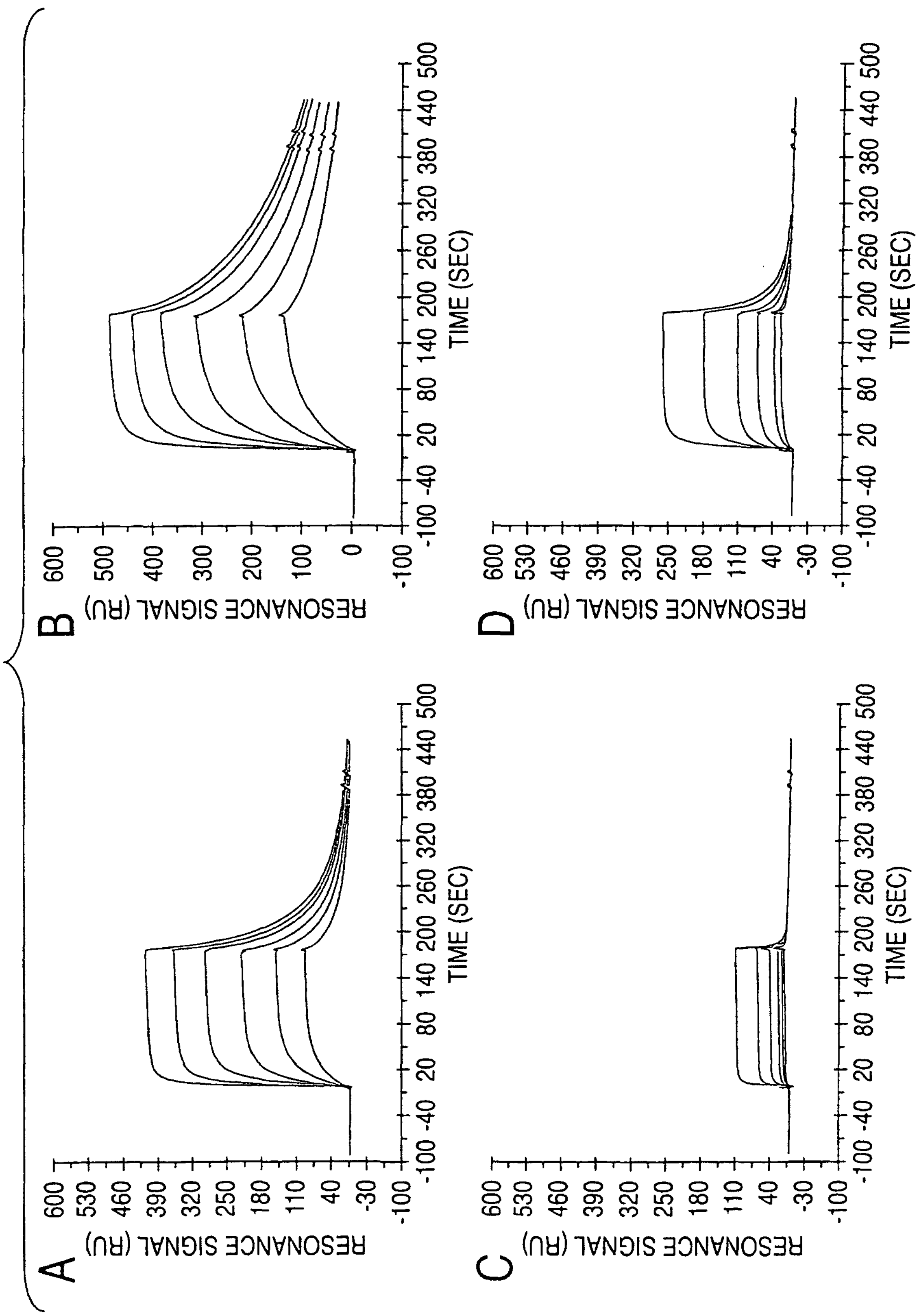
A
B
C
D
RESONANCE SIGNAL (RU)
TIME (SEC)
600 530 460 390 320 250 180 110 40 -30 -100
600 500 400 300 200 100 0 -100
-100 -40 20 80 140 200 260 320 380 440 500

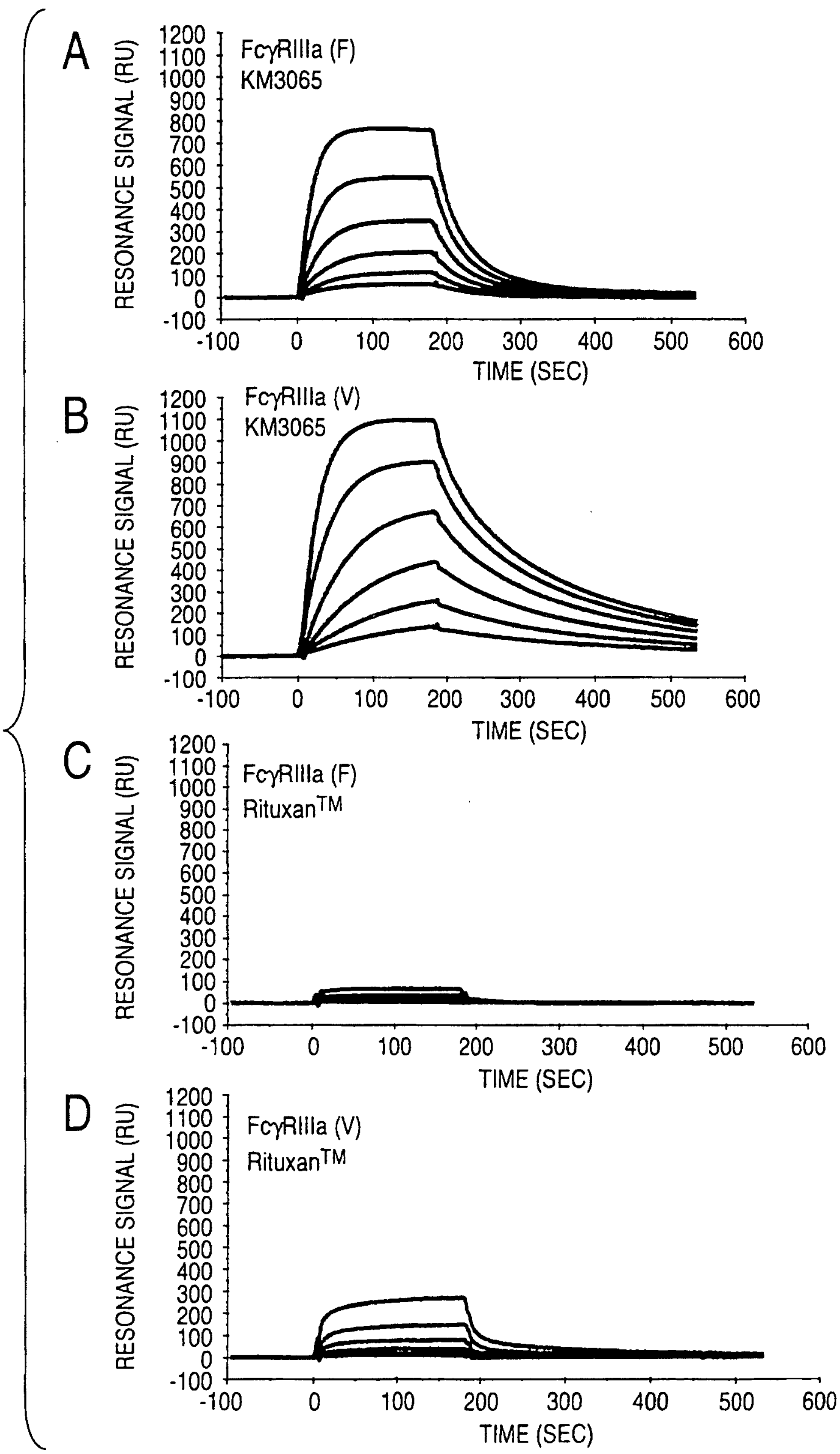
A
FcγRIIIa (F)
KM3065
B
FcγRIIIa (V)
KM3065
C
FcγRIIIa (F)
Rituxan™
D
FcγRIIIa (V)
Rituxan™
RESONANCE SIGNAL (RU)
TIME (SEC)
1200
1100
1000
900
800
700
600
500
400
300
200
100
0
-100
-100
0
100
200
300
400
500
600

FIG. 16

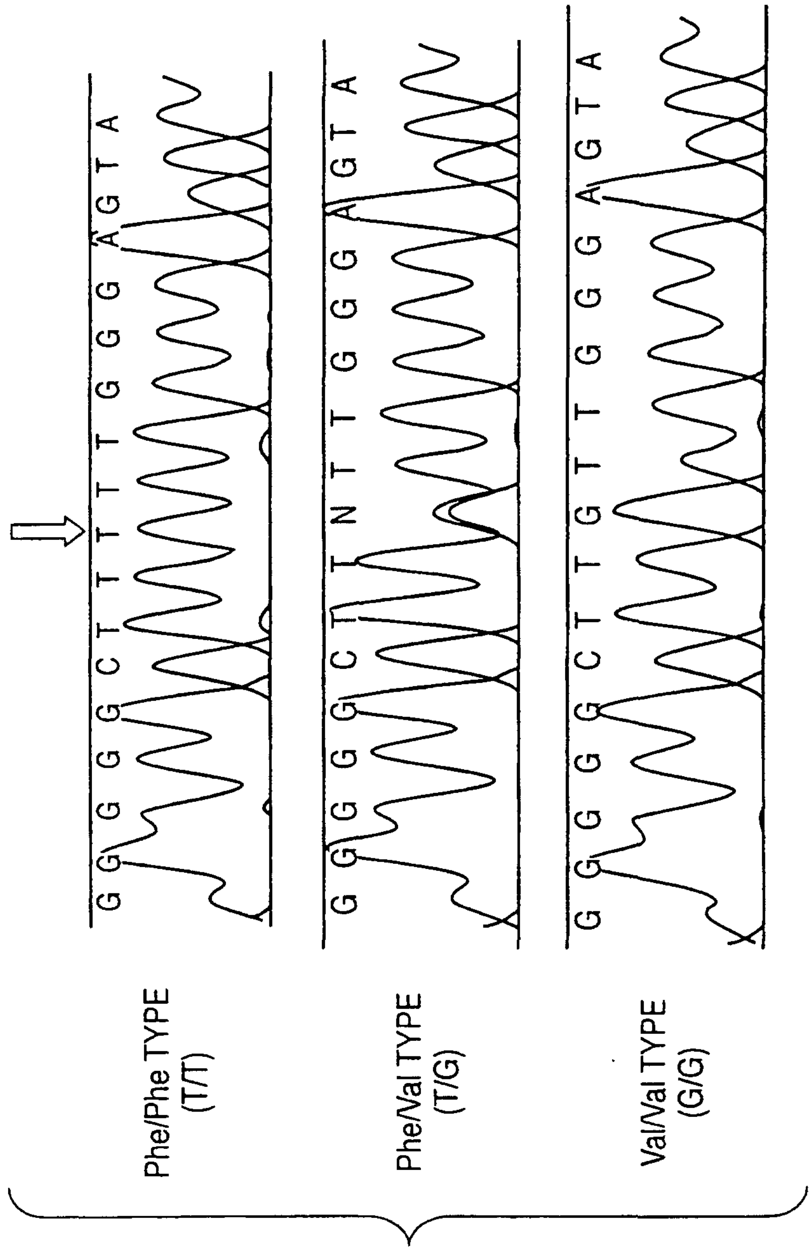
G G G G G C T T T T T G G G A G T A
G G G G G C T T N T T G G G A G T A
G G G G G C T T G T T G G G A G T A
Phe/Phe TYPE (T/T)
Phe/Val TYPE (T/G)
Val/Val TYPE (G/G)

FIG. 17

FIG. 18

ADCC ACTIVITY OF ANTI-CD20 CHIMERIC ANTIBODY TO RAJI CELL

ADCC ACTIVITY OF ANTI-CD20 CHIMERIC ANTIBODY TO WIL2-S CELL

ADCC ACTIVITY OF ANTI-CD20 CHIMERIC ANTIBODY TO G-361 CELL

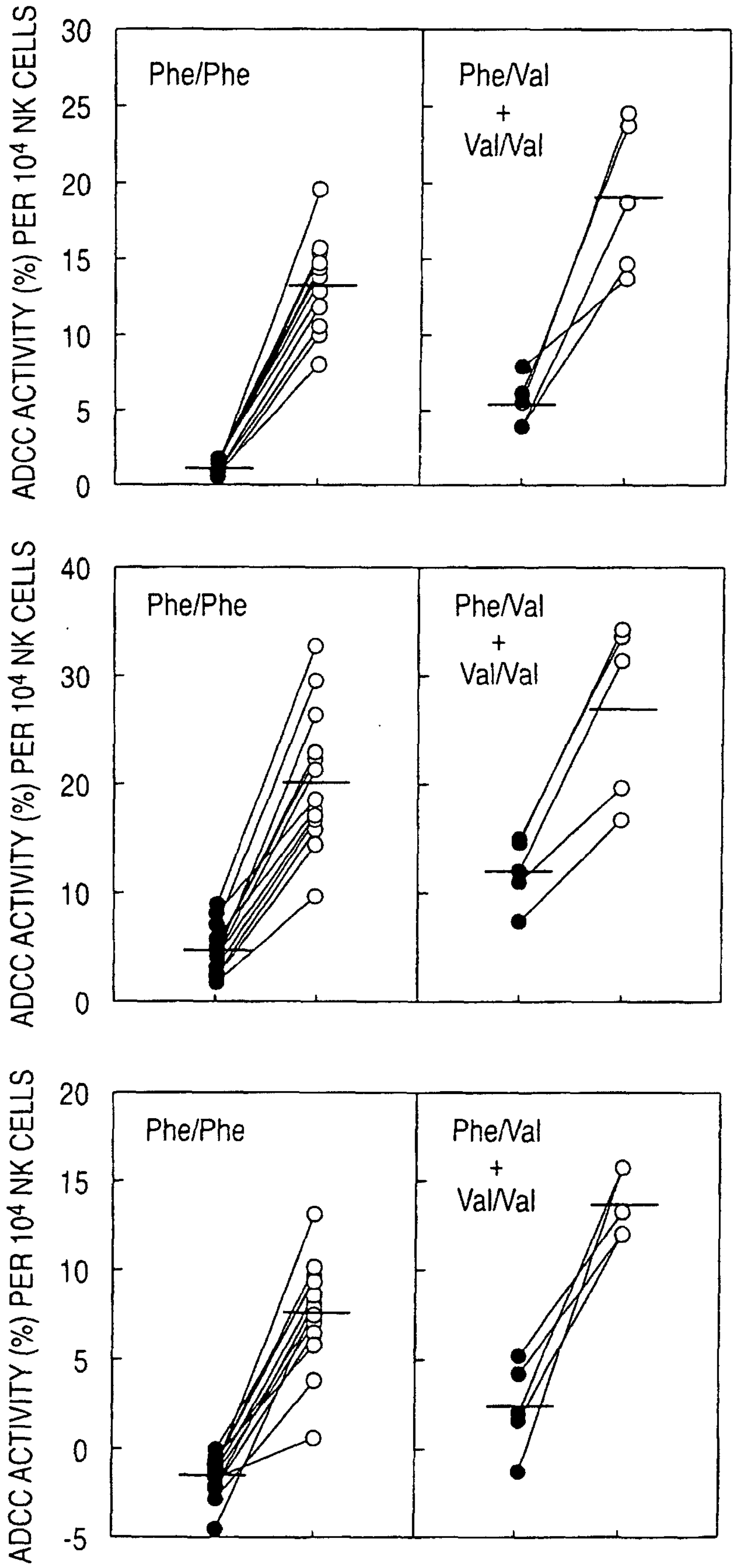

FIG. 19

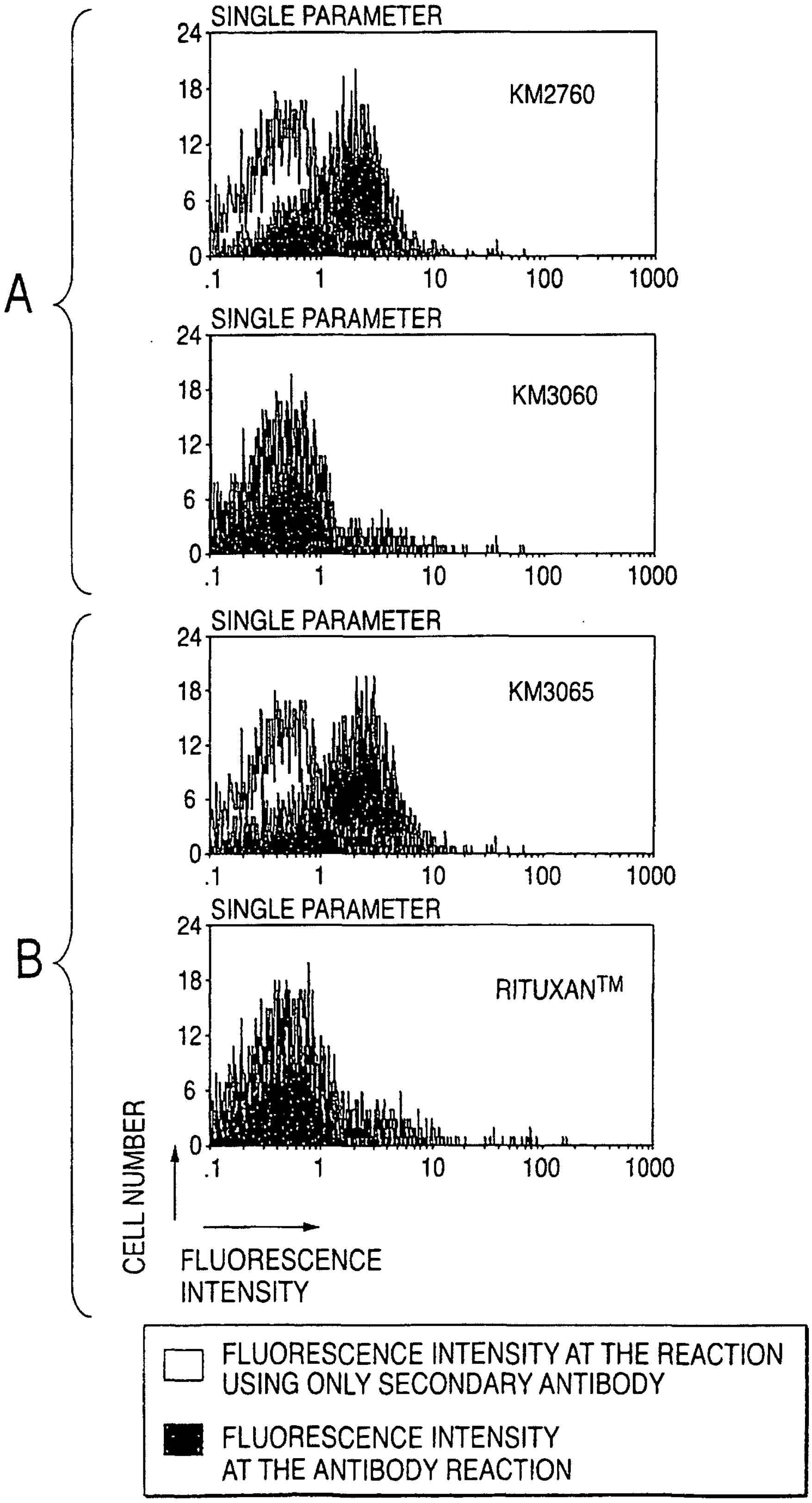
A
B
SINGLE PARAMETER
KM2760
SINGLE PARAMETER
KM3060
SINGLE PARAMETER
KM3065
SINGLE PARAMETER
RITUXAN™
24
18
12
6
0
.1
1
10
100
1000
CELL NUMBER
FLUORESCENCE INTENSITY
FLUORESCENCE INTENSITY AT THE REACTION USING ONLY SECONDARY ANTIBODY
FLUORESCENCE INTENSITY AT THE ANTIBODY REACTION

FIG. 20

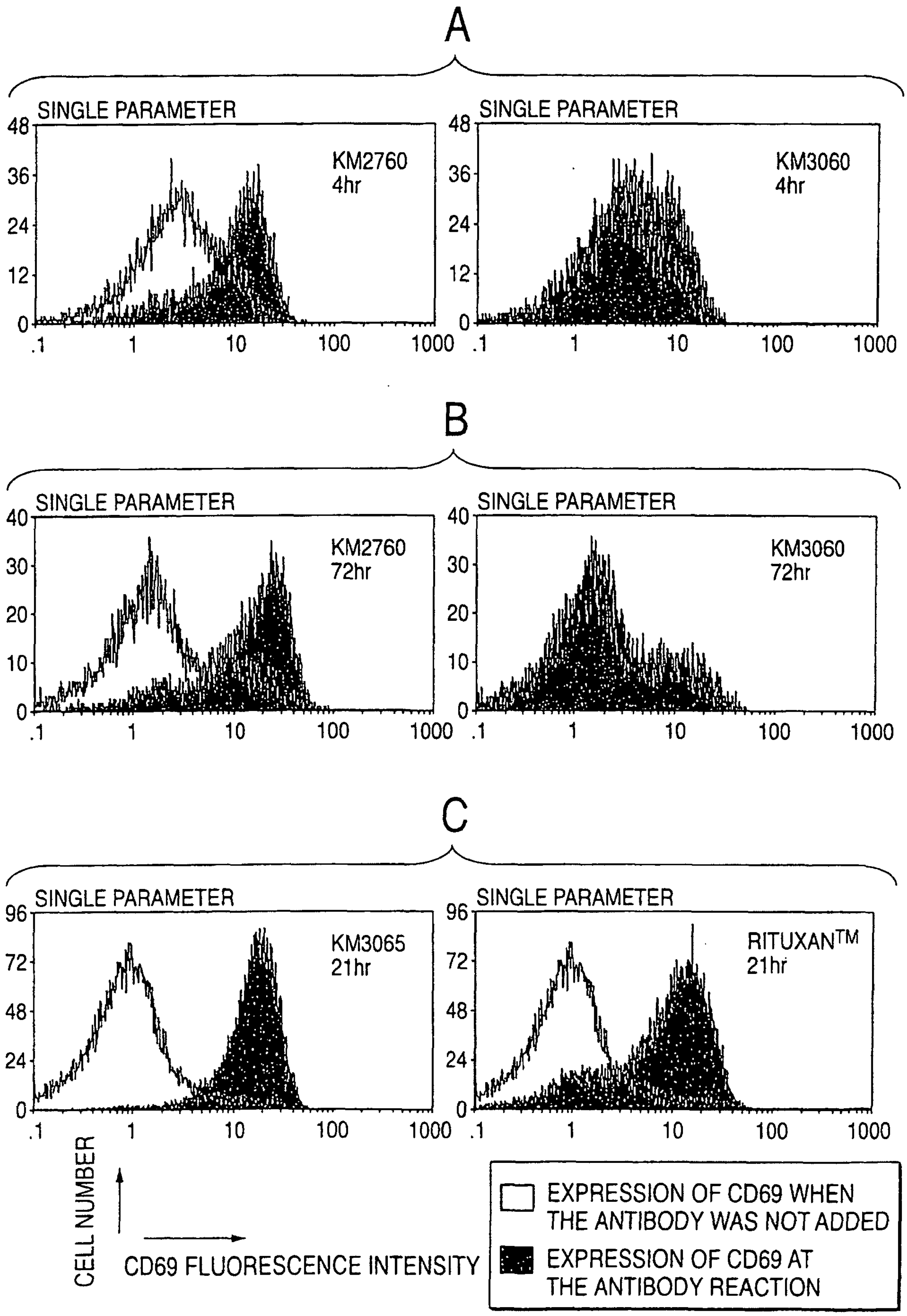
A
SINGLE PARAMETER
KM2760
4hr
SINGLE PARAMETER
KM3060
4hr
48
36
24
12
0
.1
1
10
100
1000
B
SINGLE PARAMETER
KM2760
72hr
SINGLE PARAMETER
KM3060
72hr
40
30
20
10
C
SINGLE PARAMETER
KM3065
21hr
SINGLE PARAMETER
RITUXAN™
21hr
96
72
48
24
CELL NUMBER
CD69 FLUORESCENCE INTENSITY
EXPRESSION OF CD69 WHEN THE ANTIBODY WAS NOT ADDED
EXPRESSION OF CD69 AT THE ANTIBODY REACTION

FIG. 21

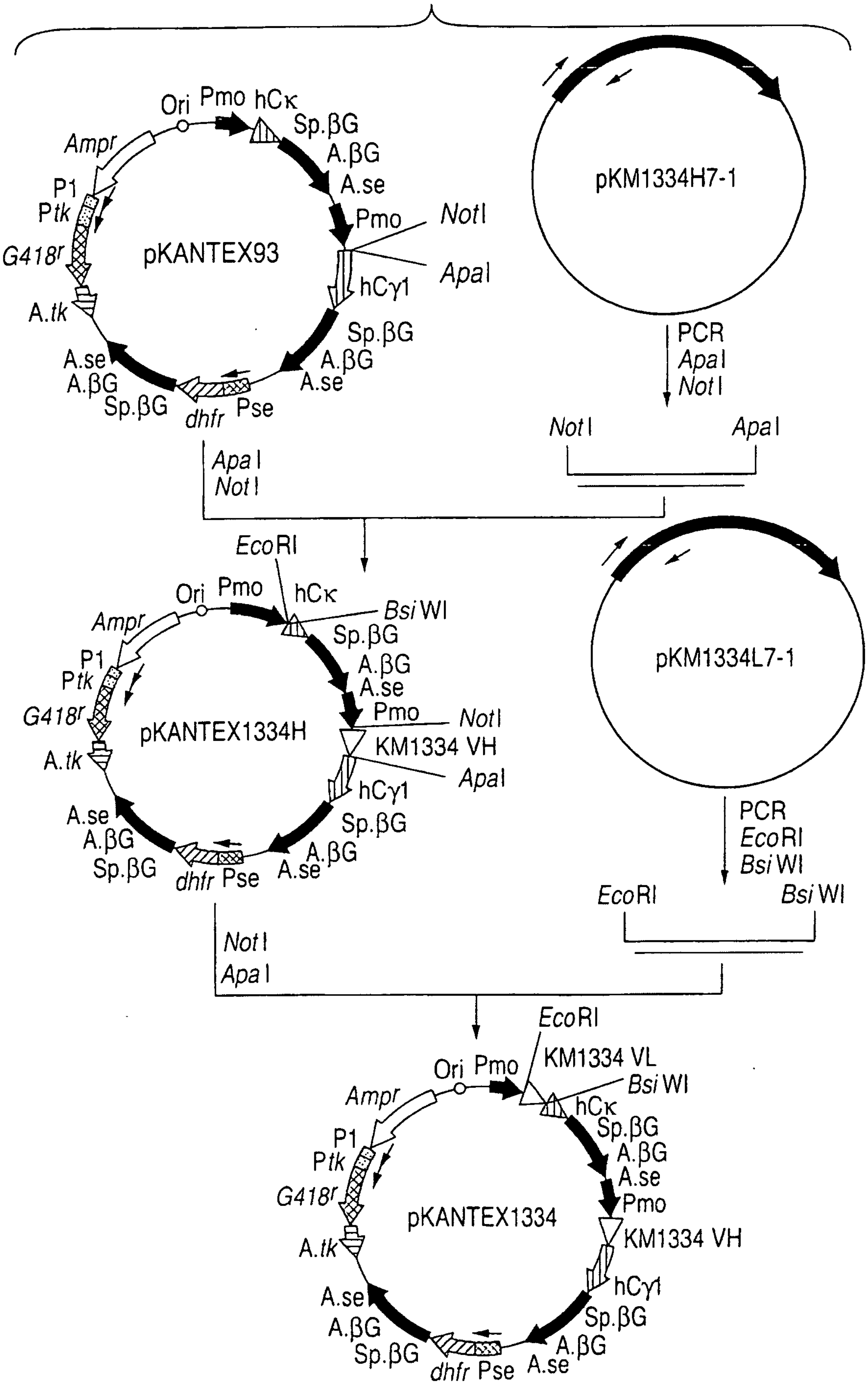
Ori
Pmo
hCκ
Sp.βG
A.βG
A.se
Pmo
NotI
ApaI
hCγ1
Ampr
P1
Ptk
G418r
A.tk
pKANTEX93
dhfr
Pse
pKM1334H7-1
PCR
ApaI
NotI
EcoRI
BsiWI
pKANTEX1334H
KM1334 VH
pKM1334L7-1
PCR
EcoRI
BsiWI
KM1334 VL
pKANTEX1334

FIG. 22

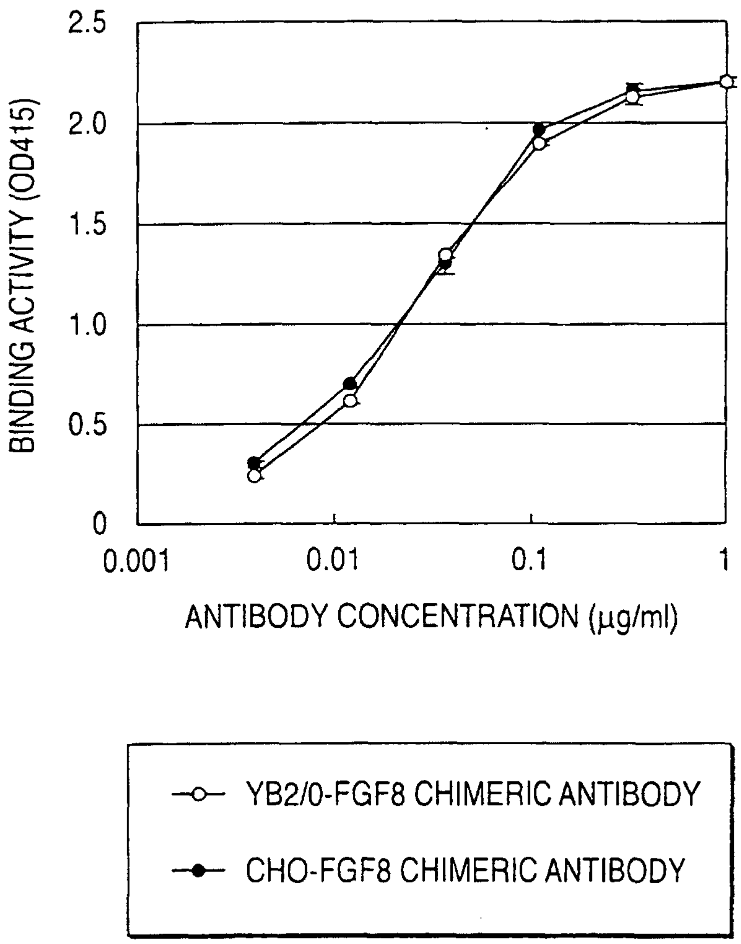

2.5
2.0
1.5
1.0
0.5
0
BINDING ACTIVITY (OD415)
0.001
0.01
0.1
1
ANTIBODY CONCENTRATION (μg/ml)
YB2/0-FGF8 CHIMERIC ANTIBODY
CHO-FGF8 CHIMERIC ANTIBODY

FIG. 23

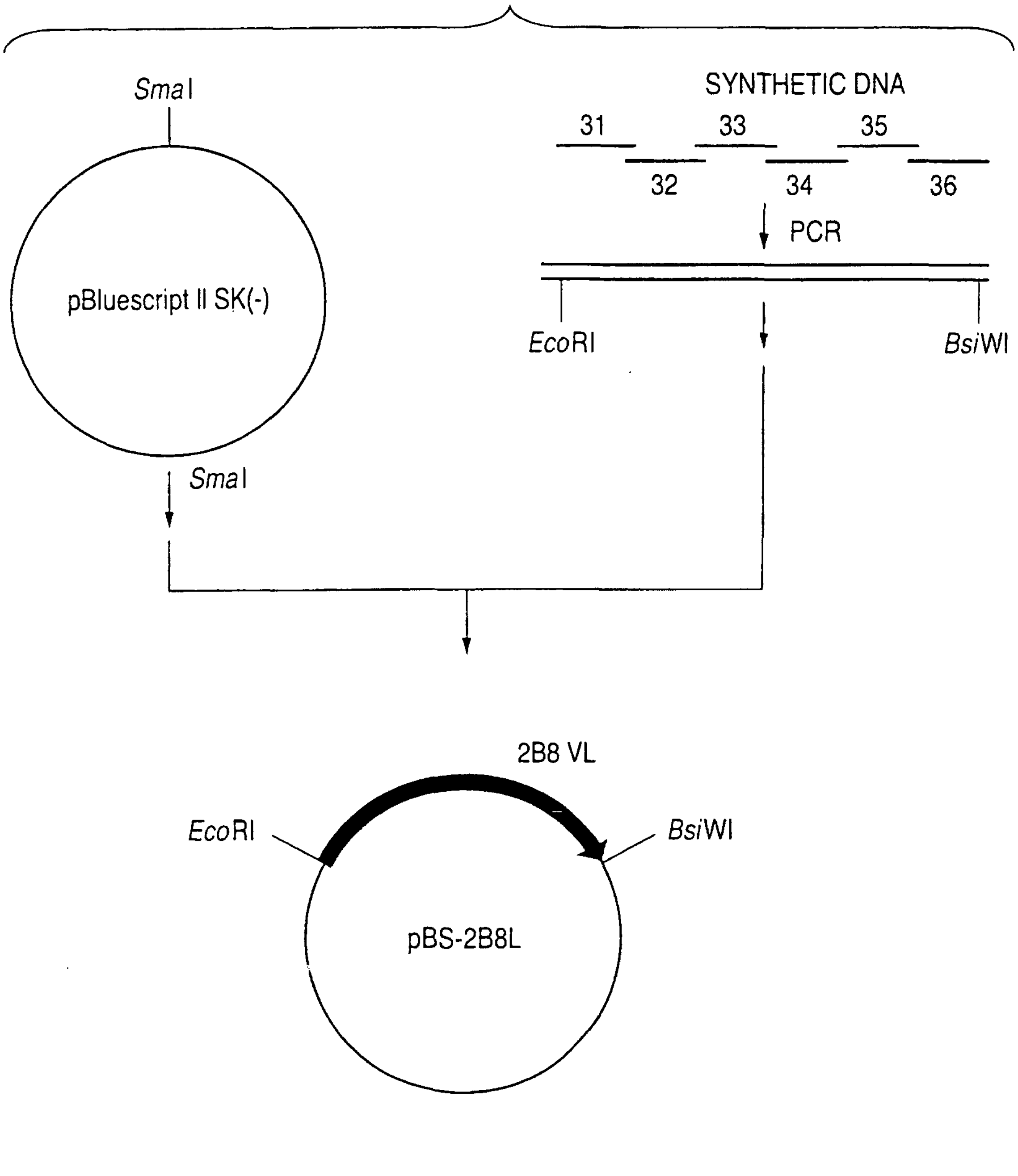
SmaI
pBluescript II SK(-)
SmaI
SYNTHETIC DNA
31
33
35
32
34
36
PCR
EcoRI
BsiWI
2B8 VL
EcoRI
BsiWI
pBS-2B8L

FIG. 24

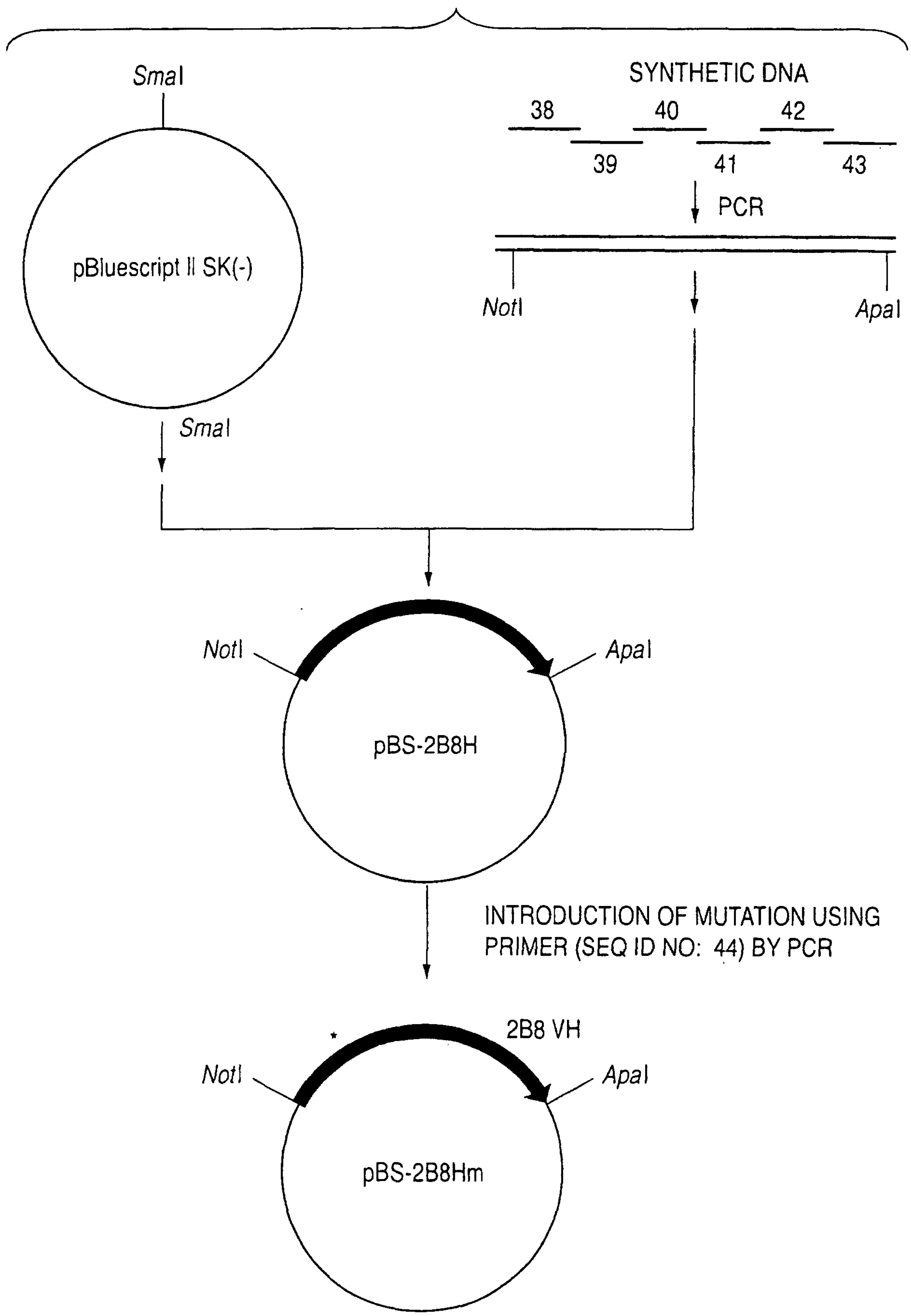
SmaI
pBluescript II SK(-)
SmaI
SYNTHETIC DNA
38
40
42
39
41
43
PCR
NotI
ApaI
NotI
ApaI
pBS-2B8H
INTRODUCTION OF MUTATION USING PRIMER (SEQ ID NO: 44) BY PCR
2B8 VH
NotI
ApaI
pBS-2B8Hm

FIG. 25

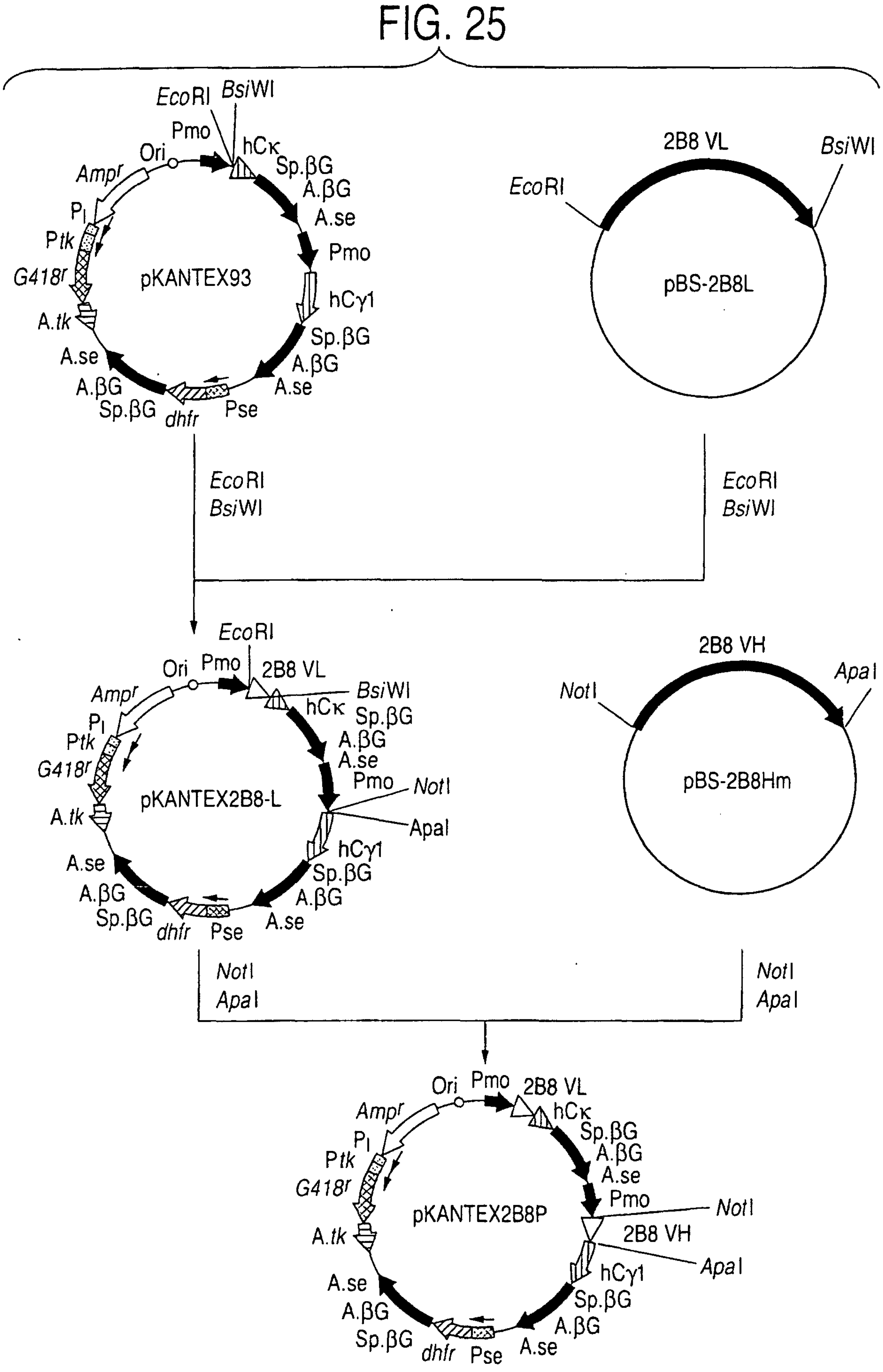
EcoRI
BsiWI
Pmo
Ori
hCκ
Ampr
Sp.βG
A.βG
Pl
A.se
Ptk
Pmo
G418r
pKANTEX93
hCγ1
A.tk
Sp.βG
A.se
A.βG
A.βG
A.se
Sp.βG
dhfr
Pse
2B8 VL
BsiWI
EcoRI
pBS-2B8L
EcoRI
BsiWI
EcoRI
BsiWI
EcoRI
Ori
Pmo
2B8 VL
BsiWI
hCκ
Sp.βG
A.βG
A.se
Pmo
NotI
pKANTEX2B8-L
ApaI
hCγ1
Sp.βG
A.βG
A.se
dhfr
Pse
2B8 VH
NotI
ApaI
pBS-2B8Hm
NotI
ApaI
NotI
ApaI
Ori
Pmo
2B8 VL
hCκ
Sp.βG
A.βG
A.se
Pmo
NotI
pKANTEX2B8P
2B8 VH
ApaI
hCγ1
Sp.βG
A.βG
A.se
Pse
dhfr
Sp.βG
A.βG
A.se
A.tk
G418r
Ptk
Pl
Ampr

FIG. 26

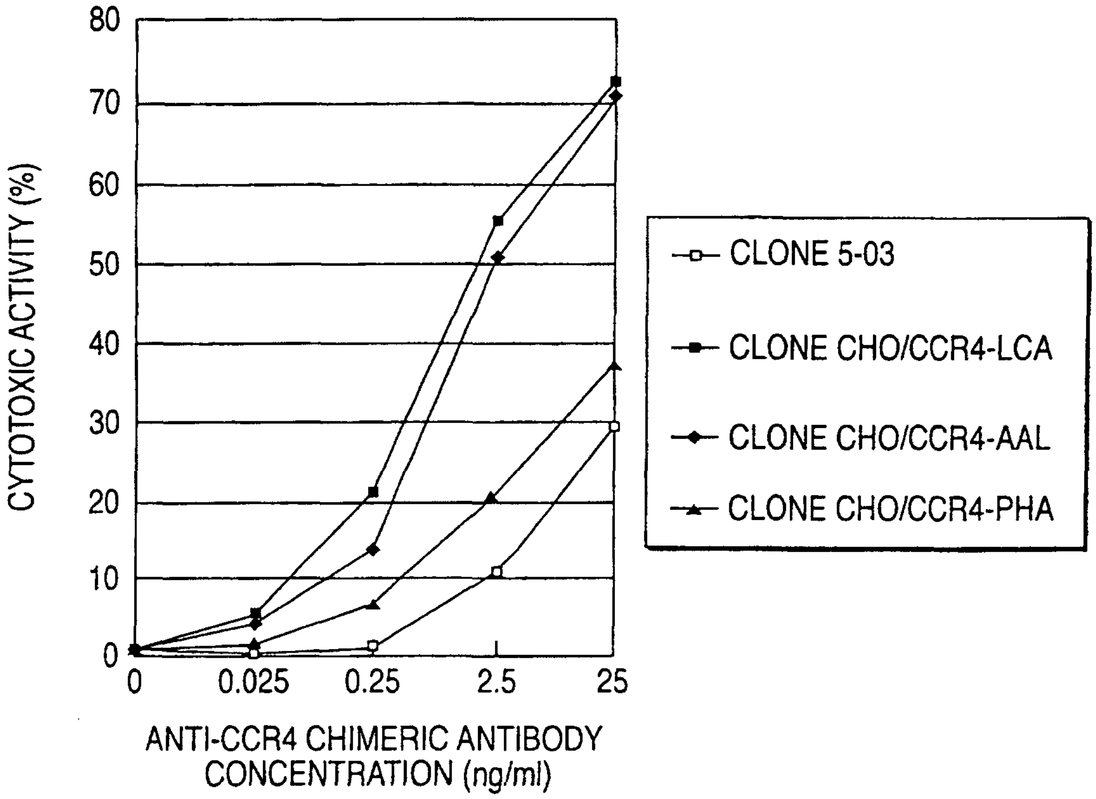
CYTOTOXIC ACTIVITY (%)
80
70
60
50
40
30
20
10
0
0
0.025
0.25
2.5
25
ANTI-CCR4 CHIMERIC ANTIBODY CONCENTRATION (ng/ml)
CLONE 5-03
CLONE CHO/CCR4-LCA
CLONE CHO/CCR4-AAL
CLONE CHO/CCR4-PHA

FIG. 27

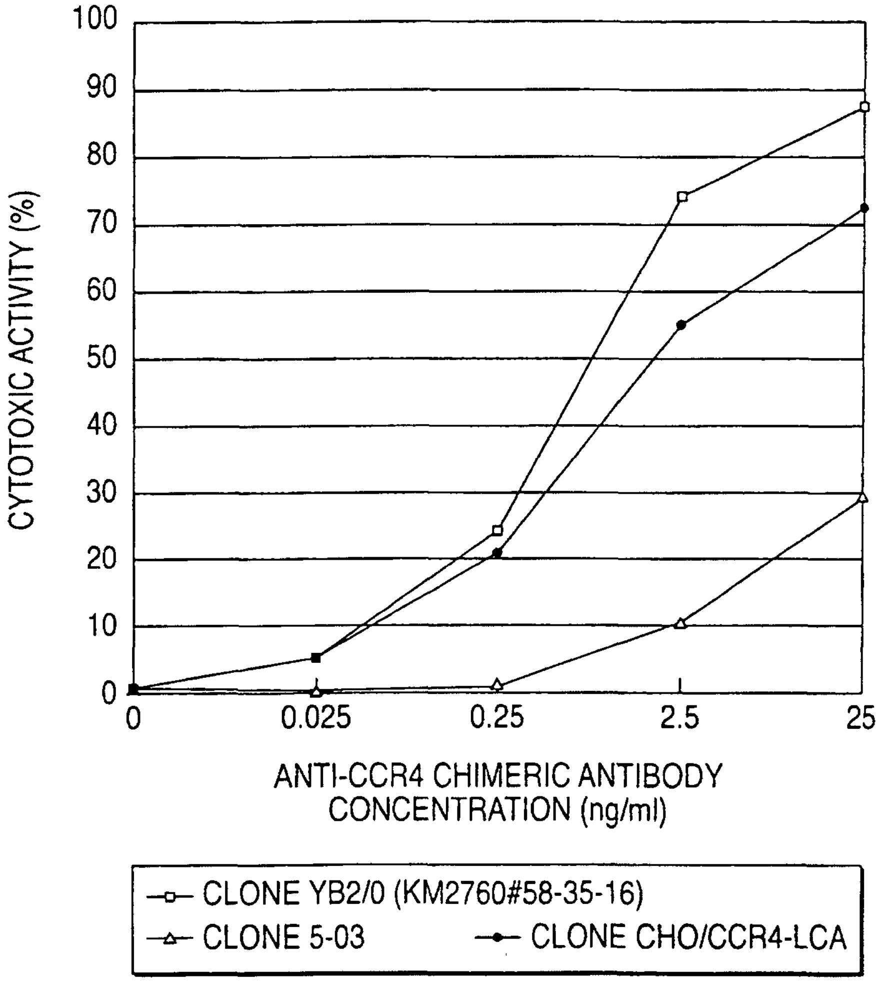
100
90
80
70
60
50
40
30
20
10
0
CYTOTOXIC ACTIVITY (%)
0
0.025
0.25
2.5
25
ANTI-CCR4 CHIMERIC ANTIBODY CONCENTRATION (ng/ml)
CLONE YB2/0 (KM2760#58-35-16)
CLONE 5-03
CLONE CHO/CCR4-LCA

FIG. 28

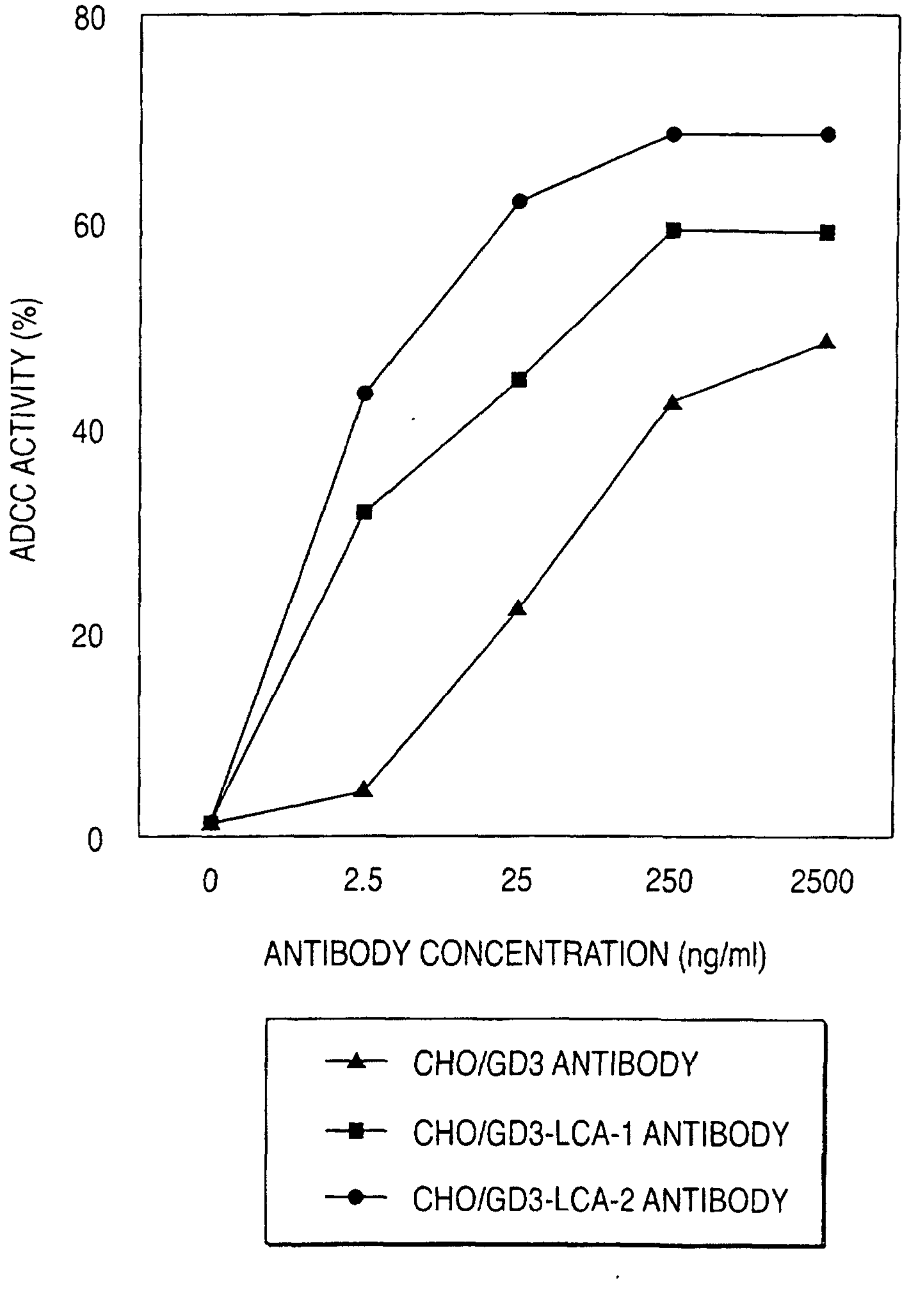
80
60
40
20
0
ADCC ACTIVITY (%)
0
2.5
25
250
2500
ANTIBODY CONCENTRATION (ng/ml)
CHO/GD3 ANTIBODY
CHO/GD3-LCA-1 ANTIBODY
CHO/GD3-LCA-2 ANTIBODY

FIG. 29

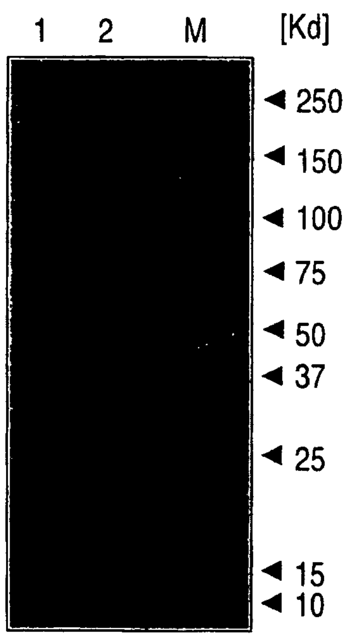

**INTERNATIONAL SEARCH REPORT**

International application No.
PCT/JP03/04505

A. CLASSIFICATION OF SUBJECT MATTER
Int.Cl$^7$ A61K39/395, A61P9/00, A61P29/00, A61P31/04, A61P31/12, A61P31/14, A61P35/00, A61P37/02, A61P37/04, A61P37/08, A61P43/00, G01N33/15, G01N33/50//C12P21/08, C12N15/09,

According to International Patent Classification (IPC) or to both national classification and IPC

B. FIELDS SEARCHED

Minimum documentation searched (classification system followed by classification symbols)
Int.Cl$^7$ A61K39/395, A61P9/00, A61P29/00, A61P31/04, A61P31/12, A61P31/14, A61P35/00, A61P37/02, A61P37/04, A61P37/08, A61P43/00, G01N33/15, G01N33/50, C12P21/08, C12N15/09, C12N5/10, C07K16/18

Documentation searched other than minimum documentation to the extent that such documents are included in the fields searched

Electronic data base consulted during the international search (name of data base and, where practicable, search terms used)
BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | WO 00/61739 A1 (Kyowa Hakko Kogyo Co., Ltd.), 19 October, 2000 (19.10.00), & EP 117619 A1 & AU 3672800 A | 1-28 |
| A | Ripka J. et al., Two Chinese hamster ovary glycosylation mutants affected in the conversion of GDP-mannose to GDP-fucose, Arch.Biochem. Biophys., 1986, 249(2), pages 533 to 545 | 1-28 |
| A | Shitara K. et al., A new vector for the high level expression of chimeric antibodies in myeloma cells, J.Immunol.Methods, 1994, 167(1-2), pages 271 to 278 | 1-28 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 May, 2003 (23.05.03) | 22 July, 2003 (22.07.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/04505

C (Continuation). DOCUMENTS CONSIDERED TO BE RELEVANT

| Category* | Citation of document, with indication, where appropriate, of the relevant passages | Relevant to claim No. |
|---|---|---|
| A | Davies J. et al., Expression of GnTIII in a recombinant anti-CD20 CHO production cell line: Expression of antibodies with altered glycoforms leads to an increase in ADCC through higher affinity for FC gamma RIII, Biotechnol.Bioeng., 2001, 74(4), pages 288 to 294 | 1-28 |
| A | Hackett J. Jr. et al., Recombinant mouse-human chimeric antibodies as calibrators in immunoassays that measure antibodies to Toxoplasma gondii, J. Clin.Microbiol., 1998, 36(5), pages 1277 to 1284 | 1-28 |
| A | Elbashir SM. et al., Duplexes of 21-nucleotide RNAs mediate RNA interference in cultured mammalian cells, Nature, 2001, 411(6836), pages 494 to 498 | 1-28 |
| A | WO 97/27303 A (Toyobo Co., Ltd.), 31 July, 1997 (31.07.97), & US 6054304 A & EP 816503 A1 & JP 9-201191 A | 1-28 |
| A | Shields RL. et al., High resolution mapping of the binding site on human IgG1 for Fc gamma RI, Fc gamma RII, Fc gamma RIII, and FcRn and design of IgG1 variants with improved binding to the Fc gamma R, J.Biol.Chem., 2001, 276(9), pages 6591 to 6604 | 1-28 |
| P,X | Shields RL. et al., Lack of fucose on human IgG1 N-linked oligosaccharide improves binding to human Fc gamma RIII and antibody-dependent cellular toxicity, J.Biol.Chem., 2002 Jul., 277(30), pages 26733 to 26740 | 1-28 |
| P,X | SHINKAWA, T. et al., The absence of fucose but not the presence of galactose or bisecting N-acetylglucosamine of human IgG1 complex-type oligosaccharides shows the critical role of enhancing antibody-dependent cellular cytotoxicity, J.Biol.Chem., 2003, 278(5), pages 3466 to 3473 | 1-28 |
| P,X | WO 02/31140 A1 (Kyowa Hakko Kogyo Co., Ltd.), 18 April, 2002 (18.04.02), & AU 9419801 A | 1-28 |

Form PCT/ISA/210 (continuation of second sheet) (July 1998)

## INTERNATIONAL SEARCH REPORT

International application No.
PCT/JP03/04505

Continuation of A. CLASSIFICATION OF SUBJECT MATTER
(International Patent Classification (IPC))

Int.Cl[7] C12N5/10, C07K16/18, (C12P21/08, C12R1:91),
(C12N5/10, C12R1:91)

(According to International Patent Classification (IPC) or to both national classification and IPC)

Continuation of B. FIELDS SEARCHED
Minimum Documentation Searched(International Patent Classification (IPC))

Int.Cl[7]

Minimum documentation searched (classification system followed by classification symbols)

Form PCT/ISA/210 (extra sheet) (July 1998)

SEQUENCE LISTING

<110> KYOWA HAKKO KOGYO CO., LTD.

<120> Therapeutic agent for patients having human FcγRIIIa

<130> P044078

<150> 2002-106951
<151> 2002-04-09

<160> 44

<170> PatentIn Ver. 2.1

<210> 1
<211> 18
<212> PRT
<213> Homo sapiens

<400> 1
Asp Glu Ser Ile Tyr Ser Asn Tyr Tyr Leu Tyr Glu Ser Ile Pro Lys
 1               5                  10                  15
Pro Cys

<210> 2
<211> 25
<212> PRT
<213> Homo sapiens

<400> 2
Gln Val Thr Val Gln Ser Ser Pro Asn Phe Thr Gln His Val Arg Glu
 1               5                  10                  15
Gln Ser Leu Val Thr Asp Gln Leu Cys
             20                  25

<210> 3
<211> 32
<212> DNA
<213> Artificial Sequence

<220>
<223> Description of Artificial Sequence: Synthetic DNA

<400> 3
taaatagaat tcggcatcat gtggcagctg ct 32

<210> 4
<211> 34
<212> DNA
<213> Artificial Sequence

<220>
<223> Description of Artificial Sequence: Synthetic DNA

<400> 4
aataaaggat cctggggtca tttgtcttga gggt 34

<210> 5
<211> 788
<212> DNA
<213> Homo sapiens

<220>
<221> CDS
<222> (13)..(774)

<400> 5
gaa ttc ggc atc atg tgg cag ctg ctc ctc cca act gct ctg cta ctt 48
Met Trp Gln Leu Leu Leu Pro Thr Ala Leu Leu Leu

```
                        1               5                   10
cta gtt tca gct ggc atg cgg act gaa gat ctc cca aag gct gtg gtg  96
Leu Val Ser Ala Gly Met Arg Thr Glu Asp Leu Pro Lys Ala Val Val
            15                  20                  25
ttc ctg gag cct caa tgg tac agg gtg ctc gag aag gac agt gtg act 144
Phe Leu Glu Pro Gln Trp Tyr Arg Val Leu Glu Lys Asp Ser Val Thr
        30                  35                  40
ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp
    45                  50                  55                  60
ttt cac aat gag agc ctc atc tca agc cag gcc tcg agc tac ttc att 240
Phe His Asn Glu Ser Leu Ile Ser Ser Gln Ala Ser Ser Tyr Phe Ile
                    65                  70                  75
gac gct gcc aca gtc gac gac agt gga gag tac agg tgc cag aca aac 288
Asp Ala Ala Thr Val Asp Asp Ser Gly Glu Tyr Arg Cys Gln Thr Asn
                80                  85                  90
ctc tcc acc ctc agt gac ccg gtg cag cta gaa gtc cat atc ggc tgg 336
Leu Ser Thr Leu Ser Asp Pro Val Gln Leu Glu Val His Ile Gly Trp
            95                  100                 105
ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile
        110                 115                 120
cac ctg agg tgt cac agc tgg aag aac act gct ctg cat aag gtc aca 432
His Leu Arg Cys His Ser Trp Lys Asn Thr Ala Leu His Lys Val Thr
125                 130                 135                 140
tat tta cag aat ggc aaa ggc agg aag tat ttt cat cat aat tct gac 480
Tyr Leu Gln Asn Gly Lys Gly Arg Lys Tyr Phe His His Asn Ser Asp
                145                 150                 155
ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
            160                 165                 170
agg ggg ctt ttt ggg agt aaa aat gtg tct tca gag act gtg aac atc 576
Arg Gly Leu Phe Gly Ser Lys Asn Val Ser Ser Glu Thr Val Asn Ile
        175                 180                 185
acc atc act caa ggt ttg gca gtg tca acc atc tca tca ttc ttt cca 624
Thr Ile Thr Gln Gly Leu Ala Val Ser Thr Ile Ser Ser Phe Phe Pro
    190                 195                 200
```

cct ggg tac caa gtc tct ttc tgc ttg gtg atg gta ctc ctt ttt gca 672
Pro Gly Tyr Gln Val Ser Phe Cys Leu Val Met Val Leu Leu Phe Ala
205 210 215 220
gtg gac aca gga cta tat ttc tct gtg aag aca aac att cga agc tca 720
Val Asp Thr Gly Leu Tyr Phe Ser Val Lys Thr Asn Ile Arg Ser Ser
225 230 235
aca aga gac tgg aag gac cat aaa ttt aaa tgg aga aag gac cct caa 768
Thr Arg Asp Trp Lys Asp His Lys Phe Lys Trp Arg Lys Asp Pro Gln
240 245 250
gac aaa tga ccc cag gat cc 788
Asp Lys

<210> 6
<211> 254
<212> PRT
<213> Homo sapiens

<400> 6
Met Trp Gln Leu Leu Leu Pro Thr Ala Leu Leu Leu Leu Val Ser Ala
1 5 10 15
Gly Met Arg Thr Glu Asp Leu Pro Lys Ala Val Val Phe Leu Glu Pro
20 25 30
Gln Trp Tyr Arg Val Leu Glu Lys Asp Ser Val Thr Leu Lys Cys Gln
35 40 45
Gly Ala Tyr Ser Pro Glu Asp Asn Ser Thr Gln Trp Phe His Asn Glu
50 55 60
Ser Leu Ile Ser Ser Gln Ala Ser Ser Tyr Phe Ile Asp Ala Ala Thr
65 70 75 80
Val Asp Asp Ser Gly Glu Tyr Arg Cys Gln Thr Asn Leu Ser Thr Leu
85 90 95
Ser Asp Pro Val Gln Leu Glu Val His Ile Gly Trp Leu Leu Leu Gln
100 105 110
Ala Pro Arg Trp Val Phe Lys Glu Glu Asp Pro Ile His Leu Arg Cys
115 120 125
His Ser Trp Lys Asn Thr Ala Leu His Lys Val Thr Tyr Leu Gln Asn
130 135 140

Gly Lys Gly Arg Lys Tyr Phe His His Asn Ser Asp Phe Tyr Ile Pro
145 150 155 160
Lys Ala Thr Leu Lys Asp Ser Gly Ser Tyr Phe Cys Arg Gly Leu Phe
165 170 175
Gly Ser Lys Asn Val Ser Ser Glu Thr Val Asn Ile Thr Ile Thr Gln
180 185 190
Gly Leu Ala Val Ser Thr Ile Ser Ser Phe Phe Pro Pro Gly Tyr Gln
195 200 205
Val Ser Phe Cys Leu Val Met Val Leu Leu Phe Ala Val Asp Thr Gly
210 215 220
Leu Tyr Phe Ser Val Lys Thr Asn Ile Arg Ser Ser Thr Arg Asp Trp
225 230 235 240
Lys Asp His Lys Phe Lys Trp Arg Lys Asp Pro Gln Asp Lys
245 250

<210> 7
<211> 788
<212> DNA
<213> Homo sapiens

<220>
<221> CDS
<222> (13)..(774)

<400> 7
gaa ttc ggc atc atg tgg cag ctg ctc ctc cca act gct ctg cta ctt 48
Met Trp Gln Leu Leu Leu Pro Thr Ala Leu Leu Leu
1 5 10
cta gtt tca gct ggc atg cgg act gaa gat ctc cca aag gct gtg gtg 96
Leu Val Ser Ala Gly Met Arg Thr Glu Asp Leu Pro Lys Ala Val Val
15 20 25
ttc ctg gag cct caa tgg tac agg gtg ctc gag aag gac agt gtg act 144
Phe Leu Glu Pro Gln Trp Tyr Arg Val Leu Glu Lys Asp Ser Val Thr
30 35 40
ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp

```
 45                  50                  55                  60
ttt cac aat gag agc ctc atc tca agc cag gcc tcg agc tac ttc att 240
Phe His Asn Glu Ser Leu Ile Ser Ser Gln Ala Ser Ser Tyr Phe Ile
                65                  70                  75
gac gct gcc aca gtc gac gac agt gga gag tac agg tgc cag aca aac 288
Asp Ala Ala Thr Val Asp Asp Ser Gly Glu Tyr Arg Cys Gln Thr Asn
            80                  85                  90
ctc tcc acc ctc agt gac ccg gtg cag cta gaa gtc cat atc ggc tgg 336
Leu Ser Thr Leu Ser Asp Pro Val Gln Leu Glu Val His Ile Gly Trp
        95                  100                 105
ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile
    110                 115                 120
cac ctg agg tgt cac agc tgg aag aac act gct ctg cat aag gtc aca 432
His Leu Arg Cys His Ser Trp Lys Asn Thr Ala Leu His Lys Val Thr
125                 130                 135                 140
tat tta cag aat ggc aaa ggc agg aag tat ttt cat cat aat tct gac 480
Tyr Leu Gln Asn Gly Lys Gly Arg Lys Tyr Phe His His Asn Ser Asp
                145                 150                 155
ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
            160                 165                 170
agg ggg ctt gtt ggg agt aaa aat gtg tct tca gag act gtg aac atc 576
Arg Gly Leu Val Gly Ser Lys Asn Val Ser Ser Glu Thr Val Asn Ile
        175                 180                 185
acc atc act caa ggt ttg gca gtg tca acc atc tca tca ttc ttt cca 624
Thr Ile Thr Gln Gly Leu Ala Val Ser Thr Ile Ser Ser Phe Phe Pro
    190                 195                 200
cct ggg tac caa gtc tct ttc tgc ttg gtg atg gta ctc ctt ttt gca 672
Pro Gly Tyr Gln Val Ser Phe Cys Leu Val Met Val Leu Leu Phe Ala
205                 210                 215                 220
gtg gac aca gga cta tat ttc tct gtg aag aca aac att cga agc tca 720
Val Asp Thr Gly Leu Tyr Phe Ser Val Lys Thr Asn Ile Arg Ser Ser
                225                 230                 235
aca aga gac tgg aag gac cat aaa ttt aaa tgg aga aag gac cct caa 768
Thr Arg Asp Trp Lys Asp His Lys Phe Lys Trp Arg Lys Asp Pro Gln
            240                 245                 250
```

gac aaa tga ccc cag gat cc 788
Asp Lys

<210> 8
<211> 254
<212> PRT
<213> Homo sapiens

<400> 8
Met Trp Gln Leu Leu Leu Pro Thr Ala Leu Leu Leu Leu Val Ser Ala
1 5 10 15
Gly Met Arg Thr Glu Asp Leu Pro Lys Ala Val Val Phe Leu Glu Pro
20 25 30
Gln Trp Tyr Arg Val Leu Glu Lys Asp Ser Val Thr Leu Lys Cys Gln
35 40 45
Gly Ala Tyr Ser Pro Glu Asp Asn Ser Thr Gln Trp Phe His Asn Glu
50 55 60
Ser Leu Ile Ser Ser Gln Ala Ser Ser Tyr Phe Ile Asp Ala Ala Thr
65 70 75 80
Val Asp Asp Ser Gly Glu Tyr Arg Cys Gln Thr Asn Leu Ser Thr Leu
85 90 95
Ser Asp Pro Val Gln Leu Glu Val His Ile Gly Trp Leu Leu Leu Gln
100 105 110
Ala Pro Arg Trp Val Phe Lys Glu Glu Asp Pro Ile His Leu Arg Cys
115 120 125
His Ser Trp Lys Asn Thr Ala Leu His Lys Val Thr Tyr Leu Gln Asn
130 135 140
Gly Lys Gly Arg Lys Tyr Phe His His Asn Ser Asp Phe Tyr Ile Pro
145 150 155 160
Lys Ala Thr Leu Lys Asp Ser Gly Ser Tyr Phe Cys Arg Gly Leu Val
165 170 175
Gly Ser Lys Asn Val Ser Ser Glu Thr Val Asn Ile Thr Ile Thr Gln
180 185 190
Gly Leu Ala Val Ser Thr Ile Ser Ser Phe Phe Pro Pro Gly Tyr Gln
195 200 205
Val Ser Phe Cys Leu Val Met Val Leu Leu Phe Ala Val Asp Thr Gly

210 215 220
Leu Tyr Phe Ser Val Lys Thr Asn Ile Arg Ser Ser Thr Arg Asp Trp
225 230 235 240
Lys Asp His Lys Phe Lys Trp Arg Lys Asp Pro Gln Asp Lys
245 250

<210> 9
<211> 51
<212> DNA
<213> Artificial Sequence

<220>
<223> Description of Artificial Sequence: Synthetic DNA

<400> 9
tgttggatcc tgtcaatgat gatgatgatg atgaccttga gtgatggtga t 51

<210> 10
<211> 620
<212> DNA
<213> Homo sapiens

<220>
<221> CDS
<222> (13)..(609)

<400> 10
gaa ttc ggc atc atg tgg cag ctg ctc ctc cca act gct ctg cta ctt 48
Met Trp Gln Leu Leu Leu Pro Thr Ala Leu Leu Leu
1 5 10
cta gtt tca gct ggc atg cgg act gaa gat ctc cca aag gct gtg gtg 96
Leu Val Ser Ala Gly Met Arg Thr Glu Asp Leu Pro Lys Ala Val Val
15 20 25
ttc ctg gag cct caa tgg tac agg gtg ctc gag aag gac agt gtg act 144
Phe Leu Glu Pro Gln Trp Tyr Arg Val Leu Glu Lys Asp Ser Val Thr

30 35 40

ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp
45 50 55 60

ttt cac aat gag agc ctc atc tca agc cag gcc tcg agc tac ttc att 240
Phe His Asn Glu Ser Leu Ile Ser Ser Gln Ala Ser Ser Tyr Phe Ile
65 70 75

gac gct gcc aca gtc gac gac agt gga gag tac agg tgc cag aca aac 288
Asp Ala Ala Thr Val Asp Asp Ser Gly Glu Tyr Arg Cys Gln Thr Asn
80 85 90

ctc tcc acc ctc agt gac ccg gtg cag cta gaa gtc cat atc ggc tgg 336
Leu Ser Thr Leu Ser Asp Pro Val Gln Leu Glu Val His Ile Gly Trp
95 100 105

ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile
110 115 120

cac ctg agg tgt cac agc tgg aag aac act gct ctg cat aag gtc aca 432
His Leu Arg Cys His Ser Trp Lys Asn Thr Ala Leu His Lys Val Thr
125 130 135 140

tat tta cag aat ggc aaa ggc agg aag tat ttt cat cat aat tct gac 480
Tyr Leu Gln Asn Gly Lys Gly Arg Lys Tyr Phe His His Asn Ser Asp
145 150 155

ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
160 165 170

agg ggg ctt ttt ggg agt aaa aat gtg tct tca gag act gtg aac atc 576
Arg Gly Leu Phe Gly Ser Lys Asn Val Ser Ser Glu Thr Val Asn Ile
175 180 185

acc atc act caa ggt cat cat cat cat cat cat tga cag gat cc 620
Thr Ile Thr Gln Gly His His His His His His
190 195

<210> 11
<211> 199
<212> PRT
<213> Homo sapiens

<400> 11

Met Trp Gln Leu Leu Leu Pro Thr Ala Leu Leu Leu Leu Val Ser Ala
1 5 10 15

Gly Met Arg Thr Glu Asp Leu Pro Lys Ala Val Val Phe Leu Glu Pro
20 25 30

Gln Trp Tyr Arg Val Leu Glu Lys Asp Ser Val Thr Leu Lys Cys Gln
35 40 45

Gly Ala Tyr Ser Pro Glu Asp Asn Ser Thr Gln Trp Phe His Asn Glu
50 55 60

Ser Leu Ile Ser Ser Gln Ala Ser Ser Tyr Phe Ile Asp Ala Ala Thr
65 70 75 80

Val Asp Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Gly Arg Lys Tyr Phe His His Asn Ser Asp Phe Tyr Ile Pro
145 150 155 160

Lys Ala Thr Leu Lys Asp Ser Gly Ser Tyr Phe Cys Arg Gly Leu Phe
165 170 175

Gly Ser Lys Asn Val Ser Ser Glu Thr Val Asn Ile Thr Ile Thr Gln
180 185 190

Gly His His His His His His
195

<210> 12
<211> 620
<212> DNA
<213> Homo sapiens

<220>
<221> CDS

<222> (13)..(609)

<400> 12

```
gaa ttc ggc atc atg tgg cag ctg ctc ctc cca act gct ctg cta ctt  48
                Met Trp Gln Leu Leu Leu Pro Thr Ala Leu Leu Leu
                1               5                   10
cta gtt tca gct ggc atg cgg act gaa gat ctc cca aag gct gtg gtg  96
Leu Val Ser Ala Gly Met Arg Thr Glu Asp Leu Pro Lys Ala Val Val
        15                  20                  25
ttc ctg gag cct caa tgg tac agg gtg ctc gag aag gac agt gtg act 144
Phe Leu Glu Pro Gln Trp Tyr Arg Val Leu Glu Lys Asp Ser Val Thr
    30                  35                  40
ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp
45                  50                  55                  60
ttt cac aat gag agc ctc atc tca agc cag gcc tcg agc tac ttc att 240
Phe His Asn Glu Ser Leu Ile Ser Ser Gln Ala Ser Ser Tyr Phe Ile
                65                  70                  75
gac gct gcc aca gtc gac gac agt gga gag tac agg tgc cag aca aac 288
Asp Ala Ala Thr Val Asp Asp Ser Gly Glu Tyr Arg Cys Gln Thr Asn
            80                  85                  90
ctc tcc acc ctc agt gac ccg gtg cag cta gaa gtc cat atc ggc tgg 336
Leu Ser Thr Leu Ser Asp Pro Val Gln Leu Glu Val His Ile Gly Trp
        95                  100                 105
ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile
    110                 115                 120
cac ctg agg tgt cac agc tgg aag aac act gct ctg cat aag gtc aca 432
His Leu Arg Cys His Ser Trp Lys Asn Thr Ala Leu His Lys Val Thr
125                 130                 135                 140
tat tta cag aat ggc aaa ggc agg aag tat ttt cat cat aat tct gac 480
Tyr Leu Gln Asn Gly Lys Gly Arg Lys Tyr Phe His His Asn Ser Asp
                145                 150                 155
ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
            160                 165                 170
agg ggg ctt gtt ggg agt aaa aat gtg tct tca gag act gtg aac atc 576
```

Arg Gly Leu Val Gly Ser Lys Asn Val Ser Ser Glu Thr Val Asn Ile
175 180 185

acc atc act caa ggt cat cat cat cat cat cat tga cag gat cc 620
Thr Ile Thr Gln Gly His His His His His His
190 195

<210> 13
<211> 199
<212> PRT
<213> Homo sapiens

<400> 13
Met Trp Gln Leu Leu Leu Pro Thr Ala Leu Leu Leu Leu Val Ser Ala
1 5 10 15
Gly Met Arg Thr Glu Asp Leu Pro Lys Ala Val Val Phe Leu Glu Pro
20 25 30
Gln Trp Tyr Arg Val Leu Glu Lys Asp Ser Val Thr Leu Lys Cys Gln
35 40 45
Gly Ala Tyr Ser Pro Glu Asp Asn Ser Thr Gln Trp Phe His Asn Glu
50 55 60
Ser Leu Ile Ser Ser Gln Ala Ser Ser Tyr Phe Ile Asp Ala Ala Thr
65 70 75 80
Val Asp Asp Ser Gly Glu Tyr Arg Cys Gln Thr Asn Leu Ser Thr Leu
85 90 95
Ser Asp Pro Val Gln Leu Glu Val His Ile Gly Trp Leu Leu Leu Gln
100 105 110
Ala Pro Arg Trp Val Phe Lys Glu Glu Asp Pro Ile His Leu Arg Cys
115 120 125
His Ser Trp Lys Asn Thr Ala Leu His Lys Val Thr Tyr Leu Gln Asn
130 135 140
Gly Lys Gly Arg Lys Tyr Phe His His Asn Ser Asp Phe Tyr Ile Pro
145 150 155 160
Lys Ala Thr Leu Lys Asp Ser Gly Ser Tyr Phe Cys Arg Gly Leu Val
165 170 175
Gly Ser Lys Asn Val Ser Ser Glu Thr Val Asn Ile Thr Ile Thr Gln
180 185 190

Gly His His His His His His
195

<210> 14
<211> 420
<212> DNA
<213> Mus musculus
<220>
<221> CDS
<222> (1)..(420)
<400> 14

atg gaa tgg atc tgg atc ttt ctc ttc ttc ctc tca gga act aca ggt 48
Met Glu Trp Ile Trp Ile Phe Leu Phe Phe Leu Ser Gly Thr Thr Gly
1 5 10 15
gtc tac tcc cag gtt cag ctg cag cag tct gga gct gag gtg gcg agg 96
Val Tyr Ser Gln Val Gln Leu Gln Gln Ser Gly Ala Glu Val Ala Arg
20 25 30
ccc ggg gct tca gtg aaa ctg tcc tgc aag gct tct ggc tac acc ttc 144
Pro Gly Ala Ser Val Lys Leu Ser Cys Lys Ala Ser Gly Tyr Thr Phe
35 40 45
act gac tac tat cta aac tgg gtg aag cag agg tct gga cag ggc ctt 192
Thr Asp Tyr Tyr Leu Asn Trp Val Lys Gln Arg Ser Gly Gln Gly Leu
50 55 60
gag tgg att gga gag att gat cct gga agt gat agt ata tat tat aat 240
Glu Trp Ile Gly Glu Ile Asp Pro Gly Ser Asp Ser Ile Tyr Tyr Asn
65 70 75 80
gaa aac ttg gag ggc agg gcc aca ctg act gca gac aaa tcc tcc agc 288
Glu Asn Leu Glu Gly Arg Ala Thr Leu Thr Ala Asp Lys Ser Ser Ser
85 90 95
aca gcc tac atg cag ctc aac agc ctg aca tct gag gac tct gca gtc 336
Thr Ala Tyr Met Gln Leu Asn Ser Leu Thr Ser Glu Asp Ser Ala Val
100 105 110
tat ttc tgt gca aga tat ggg tat tct aga tac gac gta agg ttt gtc 384
Tyr Phe Cys Ala Arg Tyr Gly Tyr Ser Arg Tyr Asp Val Arg Phe Val
115 120 125
tac tgg ggc caa ggg act ctg gtc act gtc tct aca 420

Tyr Trp Gly Gln Gly Thr Leu Val Thr Val Ser Thr
130 135 140

<210> 15
<211> 140
<212> PRT
<213> Mus musculus

<400> 15
Met Glu Trp Ile Trp Ile Phe Leu Phe Phe Leu Ser Gly Thr Thr Gly
1 5 10 15
Val Tyr Ser Gln Val Gln Leu Gln Gln Ser Gly Ala Glu Val Ala Arg
20 25 30
Pro Gly Ala Ser Val Lys Leu Ser Cys Lys Ala Ser Gly Tyr Thr Phe
35 40 45
Thr Asp Tyr Tyr Leu Asn Trp Val Lys Gln Arg Ser Gly Gln Gly Leu
50 55 60
Glu Trp Ile Gly Glu Ile Asp Pro Gly Ser Asp Ser Ile Tyr Tyr Asn
65 70 75 80
Glu Asn Leu Glu Gly Arg Ala Thr Leu Thr Ala Asp Lys Ser Ser Ser
85 90 95
Thr Ala Tyr Met Gln Leu Asn Ser Leu Thr Ser Glu Asp Ser Ala Val
100 105 110
Tyr Phe Cys Ala Arg Tyr Gly Tyr Ser Arg Tyr Asp Val Arg Phe Val
115 120 125
Tyr Trp Gly Gln Gly Thr Leu Val Thr Val Ser Thr
130 135 140

<210> 16
<211> 393
<212> DNA
<213> Mus musculus

<220>
<221> CDS

<222> (1)..(393)

<400> 16

```
atg aag ttg cct gtt agg ctg ttg gtg ctg atg ttc tgg att cct gct      48
Met Lys Leu Pro Val Arg Leu Leu Val Leu Met Phe Trp Ile Pro Ala
 1               5                  10                  15
tcc agg agt gat gtt ttg atg acc caa act cca ctc tcc ctg cct gtc      96
Ser Arg Ser Asp Val Leu Met Thr Gln Thr Pro Leu Ser Leu Pro Val
            20                  25                  30
agt ctt gga gat caa gcc tcc atc tct tgc aga tct agt cag agt ctt     144
Ser Leu Gly Asp Gln Ala Ser Ile Ser Cys Arg Ser Ser Gln Ser Leu
        35                  40                  45
gta cat agt aat gga aga acc tat tta gaa tgg tac ctg cag aaa cct     192
Val His Ser Asn Gly Arg Thr Tyr Leu Glu Trp Tyr Leu Gln Lys Pro
    50                  55                  60
ggc cag tca cca aag gtc ctg atc tac aaa gtt tcc aac cga att tct     240
Gly Gln Ser Pro Lys Val Leu Ile Tyr Lys Val Ser Asn Arg Ile Ser
65                  70                  75                  80
ggg gtc cca gac agg ttc agt ggc agt gga tca ggg aca gat ttc aca     288
Gly Val Pro Asp Arg Phe Ser Gly Ser Gly Ser Gly Thr Asp Phe Thr
                85                  90                  95
ctc aaa atc agc aga gtg gag gct gag gat ctg gga gtt tat ttc tgc     336
Leu Lys Ile Ser Arg Val Glu Ala Glu Asp Leu Gly Val Tyr Phe Cys
            100                 105                 110
ttt cag ggt tca cat gtt ccg tac acg ttc gga ggg ggg acc aag ctg     384
Phe Gln Gly Ser His Val Pro Tyr Thr Phe Gly Gly Gly Thr Lys Leu
        115                 120                 125
gaa ata aaa                                                         393
Glu Ile Lys
    130
```

<210> 17

<211> 131

<212> PRT

<213> Mus musculus

<400> 17

Met Lys Leu Pro Val Arg Leu Leu Val Leu Met Phe Trp Ile Pro Ala
1 5 10 15

Ser Arg Ser Asp Val Leu Met Thr Gln Thr Pro Leu Ser Leu Pro Val
20 25 30

Ser Leu Gly Asp Gln Ala Ser Ile Ser Cys Arg Ser Ser Gln Ser Leu
35 40 45

Val His Ser Asn Gly Arg Thr Tyr Leu Glu Trp Tyr Leu Gln Lys Pro
50 55 60

Gly Gln Ser Pro Lys Val Leu Ile Tyr Lys Val Ser Asn Arg Ile Ser
65 70 75 80

Gly Val Pro Asp Arg Phe Ser Gly Ser Gly Ser Gly Thr Asp Phe Thr
85 90 95

Leu Lys Ile Ser Arg Val Glu Ala Glu Asp Leu Gly Val Tyr Phe Cys
100 105 110

Phe Gln Gly Ser His Val Pro Tyr Thr Phe Gly Gly Gly Thr Lys Leu
115 120 125

Glu Ile Lys
130

<210> 18
<211> 5
<212> PRT
<213> Mus musculus
<400> 18

Asp Tyr Tyr Leu Asn
1 5

<210> 19
<211> 17
<212> PRT
<213> Mus musculus

<400> 19

Glu Ile Asp Pro Gly Ser Asp Ser Ile Tyr Tyr Asn Glu Asn Leu Glu

1 5 10 15
Gly

<210> 20
<211> 12
<212> PRT
<213> Mus musculus

<400> 20
Tyr Gly Tyr Ser Arg Tyr Asp Val Arg Phe Val Tyr
1 5 10

<210> 21
<211> 16
<212> PRT
<213> Mus musculus

<400> 21
Arg Ser Ser Gln Ser Leu Val His Ser Asn Gly Arg Thr Tyr Leu Glu
1 5 10 15

<210> 22
<211> 7
<212> PRT
<213> Mus musculus
<400> 22
Lys Val Ser Asn Arg Ile Ser
1 5

<210> 23
<211> 9
<212> PRT
<213> Mus musculus

<400> 23
Phe Gln Gly Ser His Val Pro Tyr Thr
1 5

<210> 24
<211> 22
<212> DNA
<213> Artificial Sequence

<220>
<223> Description of Artificial Sequence:synthetic DNA

<400> 24
ctgaattcgc ggccgctagt cc 22

<210> 25
<211> 39
<212> DNA
<213> Artificial Sequence

<220>
<223> Description of Artificial Sequence:synthetic DNA

<400> 25
atgggccctt ggtggaggct gtagagacag tgaccagag 39

<210> 26
<211> 22
<212> DNA
<213> Artificial Sequence

<220>
<223> Description of Artificial Sequence:synthetic DNA

<400> 26
ctgaattcgc ggccgctgct gt 22

<210> 27
<211> 28
<212> DNA
<213> Artificial Sequence

<220>
<223> Description of Artificial Sequence: synthetic DNA

<400> 27
atcgtacgtt ttatttccag cttggtcc 28

<210> 28
<211> 21
<212> DNA
<213> Artificial Sequence

<220>
<223> Description of Artificial Sequence: Synthetic DNA

<400> 28
atatttacag aatggcacag g 21

<210> 29
<211> 20
<212> DNA
<213> Artificial Sequence

<220>
<223> Description of Artificial Sequence: Synthetic DNA

<400> 29
gacttggtac ccaggttgaa 20

<210> 30
<211> 384
<212> DNA
<213> Mus musculus

<400> 30
atg gat ttt cag gtg cag att atc agc ttc ctg cta atc agt gct tca 48
Met Asp Phe Gln Val Gln Ile Ile Ser Phe Leu Leu Ile Ser Ala Ser
1 5 10 15
gtc ata atg tcc aga gga caa att gtt ctc tcc cag tct cca gca atc 96
Val Ile Met Ser Arg Gly Gln Ile Val Leu Ser Gln Ser Pro Ala Ile
20 25 30
ctg tct gca tct cca ggg gag aag gtc aca atg act tgc agg gcc agc 144
Leu Ser Ala Ser Pro Gly Glu Lys Val Thr Met Thr Cys Arg Ala Ser
35 40 45
tca agt gta agt tac atc cac tgg ttc cag cag aag cca gga tcc tcc 192
Ser Ser Val Ser Tyr Ile His Trp Phe Gln Gln Lys Pro Gly Ser Ser
50 55 60
ccc aaa ccc tgg att tat gcc aca tcc aac ctg gct tct gga gtc cct 240
Pro Lys Pro Trp Ile Tyr Ala Thr Ser Asn Leu Ala Ser Gly Val Pro
65 70 75 80
gtt cgc ttc agt ggc agt ggg tct ggg act tct tac tct ctc acc atc 288
Val Arg Phe Ser Gly Ser Gly Ser Gly Thr Ser Tyr Ser Leu Thr Ile
85 90 95
agc aga gtg gag gct gaa gat gct gcc act tat tac tgc cag cag tgg 336
Ser Arg Val Glu Ala Glu Asp Ala Ala Thr Tyr Tyr Cys Gln Gln Trp
100 105 110
act agt aac cca ccc acg ttc gga ggg ggg acc aag ctg gaa atc aaa 384
Thr Ser Asn Pro Pro Thr Phe Gly Gly Gly Thr Lys Leu Glu Ile Lys
115 120 125

<210> 31

<211> 91
<212> DNA
<213> Artificial Sequence

<220>
<223> Description of Artificial Sequence: Synthetic DNA

<400> 31
caggaaacag ctatgacgaa ttcgcctcct caaaatggat tttcaggtgc agattatcag 60
cttcctgcta atcagtgctt cagtcataat g 91

<210> 32
<211> 91
<212> DNA
<213> Artificial Sequence

<220>
<223> Description of Artificial Sequence: Synthetic DNA

<400> 32
gtgaccttct cccctggaga tgcagacagg attgctggag actgggagag aacaatttgt 60
cctctggaca ttatgactga agcactgatt a 91

<210> 33
<211> 90
<212> DNA
<213> Artificial Sequence

<220>
<223> Description of Artificial Sequence: Synthetic DNA

<400> 33
ctccagggga gaaggtcaca atgacttgca gggccagctc aagtgtaagt tacatccact 60
ggttccagca gaagccagga tcctccccca 90

<210> 34
<211> 89
<212> DNA
<213> Artificial Sequence

<220>
<223> Description of Artificial Sequence: Synthetic DNA

<400> 34
ccagacccac tgccactgaa gcgaacaggg actccagaag ccaggttgga tgtggcataa 60
atccagggtt tgggggagga tcctggctt 89

<210> 35
<211> 91
<212> DNA
<213> Artificial Sequence

<220>
<223> Description of Artificial Sequence: Synthetic DNA

<400> 35
tcagtggcag tgggtctggg acttcttact ctctcaccat cagcagagtg gaggctgaag 60
atgctgccac ttattactgc cagcagtgga c 91

<210> 36
<211> 90
<212> DNA
<213> Artificial Sequence

<220>
<223> Description of Artificial Sequence: Synthetic DNA

<400> 36
gttttcccag tcacgaccgt acgtttgatt tccagcttgg tcccccctcc gaacgtgggt 60

gggttactag tccactgctg gcagtaataa 90

<210> 37
<211> 420
<212> DNA
<213> Mus musculus

<400> 37
atg ggt tgg agc ctc atc ttg ctc ttc ctt gtc gct gtt gct acg cgt 48
Met Gly Trp Ser Leu Ile Leu Leu Phe Leu Val Ala Val Ala Thr Arg
1 5 10 15
gtc ctg tcc cag gta caa ctg cag cag cct ggg gct gag ctg gtg aag 96
Val Leu Ser Gln Val Gln Leu Gln Gln Pro Gly Ala Glu Leu Val Lys
20 25 30
cct ggg gcc tca gtg aag atg tcc tgc aag gct tct ggc tac aca ttt 144
Pro Gly Ala Ser Val Lys Met Ser Cys Lys Ala Ser Gly Tyr Thr Phe
35 40 45
acc agt tac aat atg cac tgg gta aaa cag aca cct ggt cgg ggc ctg 192
Thr Ser Tyr Asn Met His Trp Val Lys Gln Thr Pro Gly Arg Gly Leu
50 55 60
gaa tgg att gga gct att tat ccc gga aat ggt gat act tcc tac aat 240
Glu Trp Ile Gly Ala Ile Tyr Pro Gly Asn Gly Asp Thr Ser Tyr Asn
65 70 75 80
cag aag ttc aaa ggc aag gcc aca ttg act gca gac aaa tcc tcc agc 288
Gln Lys Phe Lys Gly Lys Ala Thr Leu Thr Ala Asp Lys Ser Ser Ser
85 90 95
aca gcc tac atg cag ctc agc agc ctg aca tct gag gac tct gcg gtc 336
Thr Ala Tyr Met Gln Leu Ser Ser Leu Thr Ser Glu Asp Ser Ala Val
100 105 110
tat tac tgt gca aga tcg act tac tac ggc ggt gac tgg tac ttc aat 384
Tyr Tyr Cys Ala Arg Ser Thr Tyr Tyr Gly Gly Asp Trp Tyr Phe Asn
115 120 125
gtc tgg ggc gca ggg acc acg gtc acc gtc tct gca 420
Val Trp Gly Ala Gly Thr Thr Val Thr Val Ser Ala
130 135 140

<210> 38
<211> 99
<212> DNA
<213> Artificial Sequence

<220>
<223> Description of Artificial Sequence: Synthetic DNA

<400> 38
caggaaacag ctatgacgcg gccgcgaccc ctcaccatgg gttggagcct catcttgctc 60
ttccttgtcg ctgttgctac gcgtgtcctg tcccaggta 99

<210> 39
<211> 98
<212> DNA
<213> Artificial Sequence

<220>
<223> Description of Artificial Sequence: Synthetic DNA

<400> 39
atgtgtagcc agaagccttg caggacatct tcactgaggc cccagccttc accagctcag 60
ccccaggctg ctgcagttgt acctgggaca ggacacgc 98

<210> 40
<211> 97
<212> DNA
<213> Artificial Sequence

<220>
<223> Description of Artificial Sequence: Synthetic DNA

<400> 40
caaggcttct ggctacacat ttaccagtta caatatgcac tgggtaaaac agacacctgg 60

tcggggcctg gaatggattg gagctattta tcccgga 97

<210> 41
<211> 99
<212> DNA
<213> Artificial Sequence

<220>
<223> Description of Artificial Sequence: Synthetic DNA

<400> 41
gtaggctgtg ctggaggatt tgtctgcagt caatgtggcc ttgcctttga acttctgatt 60
gtaggaagta tcaccatttc cgggataaat agctccaat 99

<210> 42
<211> 99
<212> DNA
<213> Artificial Sequence

<220>
<223> Description of Artificial Sequence: Synthetic DNA

<400> 42
aatcctccag cacagcctac atgcagctca gcagcctgac atctgaggac tctgcggtct 60
attactgtgc aagatcgact tactacggcg gtgactggt 99

<210> 43
<211> 98
<212> DNA
<213> Artificial Sequence

<220>
<223> Description of Artificial Sequence: Synthetic DNA

<400> 43
gttttcccag tcacgacggg cccttggtgg aggctgcaga gacggtgacc gtggtccctg 60
cgccccagac attgaagtac cagtcaccgc cgtagtaa 98

<210> 44
<211> 25
<212> DNA
<213> Artificial Sequence

<220>
<223> Description of Artificial Sequence: Synthetic DNA

<400> 44
gagctggtga agcctggggc ctcag 25